# EUROPEAN PATENT APPLICATION

(11) **EP 2 177 623 A1**
(43) Date of publication of application: **21.04.2010**
(21) Application number: 08791346.3
(22) Date of filing: 18.07.2008
(51) Int. Cl.: C12Q 1/37, G01N 33/50

(54) **METHOD OF SCREENING USING c-MET, A NOVEL SUBSTRATE FOR GAMMA-SECRETASE**

(30) Priority: 19.07.2007 US 950621 P; 24.07.2007 US 951493 P
(71) Applicant: Eisai R&D Management Co., Ltd., Tokyo 112-8088 (JP)
(72) Inventor: INOUE, Eiji, c/o KAN Research Institute, Inc., Kobe-shi Hyogo 650-0047 (JP)
(74) Representative: Woods, Geoffrey Corlett
(86) International application number: PCT/JP2008/063037
(87) International publication number: WO 2009/011426

(57) **Abstract**

The present invention relates to a method of screening for compounds which affect the processing of c-Met by γ-secretase, comprising the following steps:
(i) contacting a first biological composition containing γ-secretase or a biologically active fragment thereof with a second biological composition containing c-Met in the presence and absence of a candidate compound;
(ii) measuring the cleavage of the c-Met in the presence and absence of the candidate compound;
(iii) selecting those candidate compounds which affect the cleavage of the c-Met by γ-secretase; and
(iv) identifying the candidate compounds selected in step (iii) as compounds which affect the processing of c-Met by γ-secretase.

## Description

### FIELD OF THE INVENTION

The present invention relates to a screening method using c-Met, which is a novel substrate for γ-secretase, and a kit for use in the method.

### BACKGROUND ART

γ-Secretase is a complex protein (aspartate protease) comprising presenilin, nicastrin, Aph-1 and Pen-2 as basic components. Presenilin is the catalytic domain; presenilin gene has been identified as a causative gene for familial Alzheimer's disease (AD). γ-Secretase acts on single-pass transmembrane proteins as its substrates. As most representative substrates thereof, amyloid precursor protein (APP) and Notch are known. When cleaved by β-secretase at β-site and by γ-secretase at γ-site, APP produces amyloid β protein (Aβ). The thus-produced Aβ is classified into peptides with different lengths depending on the cleavage site in the amino acid sequence (C-terminal side). Of these peptides, Aβ42 which is strongly hydrophobic and ready to aggregate (ready to take the β-sheet structure) exhibits neurotoxicity It has been considered that this phenomenon may be the major cause of Alzheimer's disease. Recently, however, a report has been made that presenilin 1 (PS 1) and presenilin 2 (PS2) double-knockout mice capable of producing no Aβ show AD-like phenotypes such as decrease of synapses and neuronal death; this suggests existence of a pathogenic mechanism of AD independent from APP (1).

On the other hand, c-Met is a member of the receptor tyrosine kinase family and is a receptor for Hepatocyte Growth Factor (HGF). It is known that HGF is also expressed in the nervous system and is a molecule regulating the morphogenesis of post-synapses (Non-Patent Document 2). It is known that addition of HGF to hippocampal neurons increases the number of NMDA receptors and enhances neuronal function (2 and 3). Further, it is also known that HGF has a neuron protective effect (Japanese Patent No. 3680114).

However, it has never been reported to date that c-Met is a substrate for γ-secretase.
1. Saura CA, Choi SY, Beglopoulos V, Malkani S, Zhang D, Shankaranarayana Rao BS, Chattarji S, Kelleher RJ 3rd, Kandel ER, Duff K, Kirkwood A, and Shen J., Loss of presenilin function causes impairments of memory and synaptic plasticity followed by age-dependent neurodegeneration, Neuron. 2004 Apr 8, 42(1):23-36.
2. Tyndall SJ, Walikonis RS. The receptor tyrosine kinase Met and its ligand hepatocyte growth factor are clustered at excitatory synapses and can enhance clustering of synaptic proteins. Cell Cycle. 2006 Jul; 5(14):1560-8. Epub 2006 Jul 17.
3. Akimoto M, Baba A, Ikeda-Matsuo Y, Yamada MK, Itamura R, Nishiyama N, Ikegaya Y, Matsuki N. Hepatocyte growth factor as an enhancer of nmda currents and synaptic plasticity in the hippocampus. Neuroscience. 2004; 128(1):155-62.

### DISCLOSURE OF THE INVENTION

### PROBLEMS TO BE SOLVED BY THE INVENTION

It is an object of the present invention to provide a screening method using c-Met, a novel substrate for γ-secretase, in particular a method of screening for compounds which affect the processing of c-Met by γ-secretase.

### MEANS TO SOLVE THE PROBLEM

The present inventors have proved for the first time that c-Met is cleaved by γ-secretase in HEK293 cells by using a γ-secretase inhibitor (2S)-2-{[(3,5-difluorophenyl)acetyl]amino}-N-[(3S)-1-methyl-2-oxo-5-phenyl-2,3-dihydro-1H-1,4-benzodiazepin-3-yl]propanamide. The present inventors have also found for the first time that it is possible to detect the cleavage of c-Met by using an antibody specific to a hemagglutinin tag at the C-terminus of c-Met.

Briefly, the present inventors have demonstrated that a screening method with c-Met utilizing the c-Met degradation activity of γ-secretase (in particular, cleavage accelerating activity or cleavage inhibiting activity) is effective by showing that the cleavage of c-Met is inhibited by γ-secretase inhibitor.

An accelerator for the c-Met degradation activity of γ-secretase obtainable by the screening method of the present invention is a compound which accelerates the processing of c-Met through γ-secretase. An inhibitor for the c-Met degradation activity of γ-secretase obtainable by the screening method of the present invention is a compound which reduces the processing of c-Met through γ-secretase. According to the present invention, it has become possible to develop therapeutics for memory disorders of interest (preferably AD) by selecting those compounds which act on γ-secretase selectively.

The present invention provides the following embodiments.

In one embodiment, the present invention relates to a method of screening for compounds which affect the processing of c-Met by γ-secretase. More specifically, the method comprises the following steps: (a) a step of detecting the cleavage of c-Met by γ-secretase, wherein a first biological composition containing γ-secretase or a biologically active fragment thereof is contacted with a second biological composition containing c-Met to thereby measure the cleavage of the c-Met; and (b) a step of evaluating whether or not candidate compounds affect the cleavage of c-Met by γ-secretase, wherein those candidate compounds which affect the cleavage of the c-Met by γ-secretase are selected and the thus selected compounds are identified as compounds which affect the processing of c-Met by γ-secretase.

In another embodiment, the present invention relates to a screening method further comprising, in addition to the above-described steps, a step of evaluating a candidate compound as a compound which inhibits the processing of c-Met through γ-secretase or an inhibitor for the c-Met degradation activity of γ-secretase, when c-Met undegraded product in the presence of the candidate compound was increased relative to c-Met undegraded product in the absence of the candidate compound.

In still another embodiment, the present invention relates to a screening method further comprising, in addition to the above-described steps, a step of evaluating a candidate compound as a compound which accelerates the processing of c-Met through γ-secretase or an accelerator for the c-Met degradation activity of γ-secretase, when c-Met undegraded product in the presence of the candidate compound was decreased relative to c-Met undegraded product in the absence of the candidate compound.

In still another embodiment, the present invention relates to a screening method further comprising, in addition to the above-described steps, a step of evaluating a candidate compound as a compound which accelerates the processing of c-Met through γ-secretase or an accelerator for the c-Met degradation activity of γ-secretase, when c-Met cleavage product in the presence of the candidate compound was increased relative to c-Met cleavage product in the absence of the candidate compound.

In still another embodiment, the present invention relates to a screening method further comprising, in addition to the above-described steps, a step of evaluating a candidate compound as a compound which inhibits the processing of c-Met through γ-secretase or an inhibitor for the c-Met degradation activity of γ-secretase, when c-Met cleavage product in the presence of the candidate compound was decreased relative to c-Met cleavage product in the absence of the candidate compound.

In still another embodiment, the present invention relates to a screening method further comprising, in addition to the above-described method, a step of measuring the cleavage of APP or a polypeptide comprising a γ-secretase cleavage site of APP (hereinafter, expressed as "polypeptide comprising an APP γ-secretase cleavage site").

In still another embodiment, the present invention relates to a screening method further comprising, in addition to the above-described method, a step of measuring the cleavage of Notch or a polypeptide comprising a γ-secretase cleavage site of Notch (hereinafter, expressed as "polypeptide comprising a Notch γ-secretase cleavage site").

In still another embodiment, the present invention provides a pharmaceutical composition comprising at least one compound identified by the screening method of the present invention and a pharmacologically acceptable carrier. Preferably, the above compound is (2S)-2-{[(3,5-difluorophenyl)acetyl]amino}-N-[(3S)-1-methyl-2-oxo-5-phenyl-2,3-dihydro-1H-1,4-benzodiazepin-3-yl]propanamide (hereinafter, sometimes referred to as "Compound E").

In still another embodiment, the present invention provides a method of treating a disease or condition characterized by abnormality in the processing of c-Met by γ-secretase, comprising a step of administering to a patient in need of treatment (e.g., a patient with a memory disorder, especially a condition of dementia (preferably AD)) an effective dose of the compound of the present invention or a pharmaceutical composition comprising the same (preferably a therapeutic for dementia), wherein preferably the dose is effective for altering the c-Met processing activity of γ-secretase in the cells of the patient.

In still another embodiment, the present invention provides a test kit for measuring the processing of c-Met by γ-secretase, which is applicable to the method of the present invention, comprising c-Met and preferably further comprising a substrate for γ-secretase other than c-Met (preferably APP and/or Notch).

In still another embodiment, the present invention provides a test kit for measuring the processing of c-Met by γ-secretase, comprising a first biological composition containing γ-secretase or a biologically active fragment thereof and a second biological composition containing c-Met.

### EFFECT OF THE INVENTION

According to the present invention, there is provided a method for screening for compounds which affect the processing of c-Met by γ-secretase. The compound screened by the present invention is useful as a therapeutic for a memory disorder of interest, especially dementia (preferably AD).

### BRIEF DESCRIPTION OF THE DRAWINGS

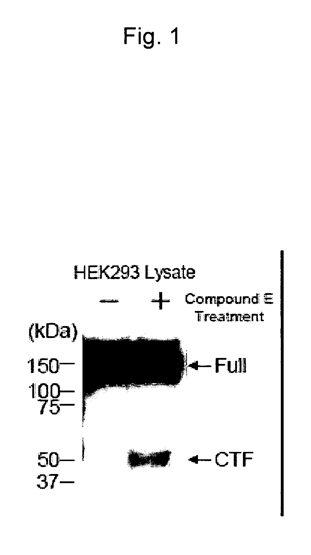

Fig. 1 is a diagram showing the results of analysis of c-Met processing using *c*-*Met*-transfected 293/EBNA-1 cell strain.

### DETAILED DESCRIPTION OF THE INVENTION

Hereinbelow, the present invention will be described in more detail. The present specification encompasses the contents disclosed in the specifications and the drawings of US Provisional Patent Applications No. 60/950,621 (filed on July 19, 2007) and No. 60/951,493 (filed on July 24, 2007) based on which the present patent application claims priority.

The term "patient" used herein refers to an animal, preferably a mammal.

The term "mammal" used herein means any animal classified as mammal, including human and non-human mammals (such as mouse, rat, hamster, guinea pig, rabbit, pig, dog, horse, cattle, monkey, etc.). Preferably, the mammal in the present specification is human. When the mammal is human, the term "patient" include adults and children, and also male and female. Children include newborn infants, infants and adolescents.

The term "γ-secretase" used herein means an enzyme or a complex composed of a plurality of molecules, each of which is in charge of the production of Aβ by cleaving (degrading) APP within its transmembrane domain. The plurality of molecules comprise at least one molecule selected from presenilin, nicastrin, Aph-1 and Pen-2. Examples of the γ-secretase of the present invention include mouse Presenilin 1(NM_008943), rat Presenilin1 (D82363), human Presenilin1 (NM_000021), mouse Presenilin2 (NM_011183), rat Presenilin2 (NM_031087), human Presenilin2 (NM_000447), mouse Nicastrin (NM_021607), rat Nicastrin (NM_174864), human Nicastrin (NM_015331), mouse Aph-1 (NM_146104), rat Aph-1 (NM_001014255), human Aph-1 (NM_016022), mouse Pen-2 (NM_025498), rat Pen-2 (NM_001008764) and human Pen-2 (NM_172341). Each component molecule of the γ-secretase of the present invention may be a full-length molecule or a part thereof, as long as that γ-secretase has an enzyme activity equivalent to that of γ-secretase functioning *in vivo.* Further, the γ-secretase of the present invention may be a mutant γ-secretase. The mutant γ-secretase is a polypeptide composed of full-length component molecules which may have deletion, substitution, insertion and/or addition of one or more (preferable one or several) amino acids, or a polypeptide having an amino acid sequence comprising a combination of such mutations, each of the above polypeptide having an enzyme activity equivalent to that of γ-secretase functioning *in vivo.*

The term "biologically active fragment of γ-secretase" means a fragment having an enzyme activity equivalent to that of γ-secretase functioning *in vivo.* Examples of such fragments include fragments capable of cleaving APP or c-Met.

It should be noted that sometimes, the term "γ-secretase" is intended to include the "biologically active fragment of γ-secretase" in the present specification.

The term "c-Met" used herein refers to a known polypeptide which is a member of the receptor tyrosine kinase family and is a receptor for Hepatocyte Growth Factor (HGF), a regulator for the morphogenesis of postsynapses (Tyndall SJ, Walikonis RS. The receptor tyrosine kinase Met and its ligand hepatocyte growth factor are clustered at excitatory synapses and can enhance clustering of synaptic proteins. Cell Cycle. 2006 Jul; 5(14):1560-8. Epub 2006 Jul 17). Structurally, c-Met comprises an HGF binding domain, a transmembrane domain and a kinase activity site (Maulik G et al., Cytokine Growth Factor Rev. 2002 Feb; 13(1):41-59).

The c-Met used in the present invention is preferably a c-Met derived from a mammal. For example, human c-Met (BC130420 or X54559), rheus monkey (*Macaca mulatta*) c-Met (XM_001101874), chimpanzee (*Pan troglodytes*) c-Met (XM_519326), rat c-Met (X96786, NM_031517, XM_346692 or XM_347367), mouse c-Met (Y00671, BC117969 or NM_008591), horse (*Equus caballus*) c-Met (XM_001501820), pig (*Sus scrofa*) c-Met (NM_001038008), dog (*Canis lupus familiaris*) c-Met (NM_001002963), cattle (*Bos Taurus*) c-Met (NM_001012999, XM_608832 or XM_612507) and the like are included in the c-Met of the present invention.

Genes encoding the above-listed c-Mets and the corresponding c-Met polypeptides are included in the c-Met of the present invention. Table 1 below shows correspondence between various animal-derived c-Met polypeptides and their nucleotide and amino acid sequences.

**Table 1**

| Source or Type | Accession No. | Nucleotide Sequence | Amino Acid Sequence |
|---|---|---|---|
| Human (Homo sapiens) | BC130420 | SEQ ID NO: 1 | SEQ ID NO: 2 |
| | X54559 | SEQ ID NO: 3 | SEQ ID NO: 4 |
| Rheus monkey (Macaca mulatta) | XM_001101874 | SEQ ID NO: 5 | SEQ ID NO: 6 |
| Chimpanzee (Pan troglodytes) | XM_519326 | SEQ ID NO: 7 | SEQ ID NO: 8 |
| Rat (Rattus norvegicus) | X96786 | SEQ ID NO: 9 | SEQ ID NO: 10 |
| | NM_031517, XM_346692 or XM_347367 | SEQ ID NO: 11 | SEQ ID NO: 12 |
| Rat-HA (Rattus norvegicus) | | SEQ ID NO: 13 | SEQ ID NO: 14 |
| Mouse (Mus musculus) | Y00671 | SEQ ID NO: 15 | SEQ ID NO: 16 |
| | BC117969 | SEQ ID NO: 17 | SEQ ID NO: 18 |
| | NM_008591 | SEQ ID NO: 19 | SEQ ID NO: 20 |
| Horse (Equus caballus) | XM_001501820 | SEQ ID NO: 21 | SEQ ID NO: 22 |
| Pig (Sus scrofa) | NM_001038008 | SEQ ID NO: 23 | SEQ ID NO: 24 |
| Dog (Canis lupus familiaris) | NM_001002963 | SEQ ID NO: 25 | SEQ ID NO: 26 |
| Cattle (Bos Taurus) | NM_001012999, XM_608832 or XM_612507 | SEQ ID NO: 27 | SEQ ID NO: 28 |

In the present invention, c-Met may be a polypeptide derived from any of the above-listed animals, a recombinant polypeptide or a synthetic polypeptide.

The c-Met of the present invention may be either a full-length polypeptide or a partial sequence thereof, as long as it comprises the γ-secretase cleavage site of c-Met.

Further, in the present invention, c-Met may be a mutant c-Met. The mutant c-Met is a full-length c-Met polypeptide which may have deletion, substitution, insertion and/or addition of one or more (preferable one or several) amino acids, or a c-Met polypeptide having an amino acid sequence comprising a combination of such mutations, each of the above polypeptides being functionally substantially identical with c-Met.

The "polypeptide which is functionally substantially identical with c-Met" means a polypeptide having an activity of c-Met, for example, a polypeptide which has a cleavage activity in a γ-secretase-dependent manner, and includes any and all c-Met derivatives comprising at least the γ-secretase cleavage site of c-Met. These polypeptides are especially useful in detecting and purifying c-Met.

The term "substitution" used herein means preferably conservative substitution in which one or more (preferably one or several) amino acid residues are substituted with other chemically similar amino acid residues so that the activity of the peptide is not substantially modified. Examples of conservative substitution include substitution of a hydrophobic residue with other hydrophobic residue and substitution of a polar residue with other polar residue with the same electric charge. Functionally similar amino acids which allow such substitution are known to those skilled in the art for each amino acid. Specifically, examples of non-polar (hydrophobic) amino acids include alanine, valine, isoleucine, leucine, proline, tryptophan, phenylalanine and methionine; examples of polar (neutral) amino acids include glycine, serine, threonine, tyrosine, glutamine, asparagine and cystein. Examples of positively charged (basic) amino acids include arginine, histidine and lysine. Examples of negatively charged (acidic) amino acids include aspartic acid and glutamic acid.

The number of amino acids which may be deleted, substituted, inserted and/or added as described above is, for example, 1 to 30, preferably 1 to 20, more preferably 1 to 10, still more preferably 1 to 5, particularly preferably 1 to 2.

The above-described identify means that the amino acid sequence of a polypeptide may not be identical with the amino acid sequence of c-Met as long as the polypeptide has substantially the same function as that of c-Met. Therefore, when a polypeptide has an activity functionally substantially the same as that of c-Met (e.g., γ-secretase-dependent degradation activity), the degree of homology of the amino acid sequence of the relevant polypeptide is disregarded. The animal species from which the polypeptide is derived is also disregarded.

In a preferred embodiment of the present invention, a polypeptide functionally substantially identical to c-Met is an amino acid sequence which has preferably 80% or more, more preferably 85% or more, still more preferably 90% or more, still more preferably 95% or more, particularly preferably 98% or more, most preferably 99% or more homology to the amino acid sequence of c-Met (e.g., the amino acid sequence of a polypeptide consisting of SEQ ID NO: 10 (rat c-Met)) and has substantially the same activity as that of c-Met. When a polypeptide has these features, the polypeptide may be any of a polypeptide derived from the above-listed animals, a recombinant polypeptide or a synthetic polypeptide.

The identity described above may be values calculated by homology search programs known to those skilled in the art. For example, identity can be calculated by using default parameters in the homology algorithm BLAST (Basic local alignment search tool; http://www.ncbi.nlm.nih.gov/BLAST/) of The National Center for Biotechnology Information (NCBI).

The c-Met may take any of the following forms: a fusion polypeptide fused to other polypeptide, a tagged or labeled polypeptide or a polypeptide otherwise modified. These polypeptides may be obtained by recombinant DNA techniques, site-directed mutagenesis, treatment with mutagenic agents (such as hydroxylamine), or automated peptide synthesis.

Examples of particularly useful systems as tagged c-Met polypeptides include hemagglutinin (HA) system, glutathione-S-transferase (GST) system, maltose-binding protein system, 6x histidine system, 8x histidine system and the like.

Examples of modification incorporating the above-mentioned label or other detectable moieties include biotin label, radioactive label, fluorescence label, chemiluminescence label and the like. The c-Met of the present invention may be labeled with one, two or more of these labels.

In a preferred embodiment of the present invention, c-Met is a rat c-Met, for example, a polypeptide comprising the amino acid sequence as shown in SEQ ID NO: 10 or SEQ ID NO: 12. In a still preferred embodiment of the present invention, c-Met is a rat c-Met to which an HA tag is added. For example, a rat c-Met polypeptide with an HA tag added at its C-terminus (SEQ ID NO: 14) may be used (Example 1). It is for granted that polypeptides comprising the entire human c-Met amino acid sequence or a part thereof (e.g., polypeptides comprising the amino acid sequence as shown in SEQ ID NO: 2 or 4) or c-Met polypeptide sequences derived from other animals listed above may also be used in the same manner as rat c-Met polypeptides.

The present invention further provides a polynucleotide comprising a nucleotide sequence encoding the above-described c-Met. The polynucleotide encoding the c-Met of the present invention is a polynucleotide encoding a polypeptide having substantially the same activity as that of c-Met (e.g., γ-secretase-dependent degradation activity). In a preferred embodiment of the present invention, the polynucleotide encoding the c-Met of the present invention is a polynucleotide encoding a rat c-Met, e.g., the polynucleotide as shown in SEQ ID NO: 9 or SEQ ID NO: 11. Further, in a preferred embodiment of the present invention, the polynucleotide encoding the c-Met of the present invention is a polynucleotide encoding a rat c-Met polypeptide with an HA tag added. For example, a polynucleotide encoding a rat c-Met polypeptide to which an HA tag is added at its C-terminus (SEQ ID NO: 13) may be given (Example 1). It is for granted that polynucleotides comprising the entire human c-Met nucleotide sequence or a part thereof (e.g., the nucleotide sequence as shown in SEQ ID NO: 1 or 3) or nucleotide sequences derived from other animals listed above may also be used in the same manner as rat c-Met polynucleotides.

The polynucleotide encoding the c-Met of the present invention may be a polynucleotide encoding the above-described c-Met mutant or c-Met derivative. For example, a polynucleotide which comprises a nucleotide sequence having 80% or more, preferably 85% or more, still more preferably 90% or more, yet still more preferably 95% or more, particularly preferably 98% or more, most preferably 99% or more homology (identity) with the nucleotide sequence as shown in SEQ ID NO: 9 or SEQ ID NO: 11 and encodes a polypeptide having substantially the same activity as that of c-Met may be included.

Further, the polynucleotide encoding the c-Met of the present invention includes a polynucleotide which hybridizes to a polynucleotide consisting of a nucleotide sequence complementary to the nucleotide sequence as shown in SEQ ID NO: SEQ ID NO: 9 or SEQ ID NO: 11 under stringent conditions and yet encodes a polypeptide having substantially the same activity as that of c-Met. The stringent conditions refer to, for example, "2 x SSC, 0.1% SDS, 42°C" or "1 x SSC, 0.1% SDS, 37°C" as washing conditions after hybridization. As more stringent conditions, "1 x SSC, 0.1% SDS, 65°C" or "0.5 x SSC, 0.1% SDS, 50°C" may be given, for example. More specifically, a method using Rapid-hyb buffer (Amersham Life Science) may be employed in which pre-hybridization is performed at 68 °C for more than 30 minutes; then, with addition of a probe, a hybrid is formed while keeping the reaction solution at 68 °C for more than 1 hour, followed by washing in 2 x SSC, 0.1% SDS at room temperature for 20 minutes 3 times, washing in 1 x SSC, 0.1% SDS at 37°C for 20 minutes 3 times and finally washing in 1 x SSC, 0.1% SDS at 50°C for 20 minutes twice. Alternatively, other method may be employed in which pre-hybridization is performed in Expresshyb Hybridization Solution (CLONTECH) at 55 °C for more than 30 minutes; then, with addition of a labeled probe, the reaction solution is incubated at 37-55°C for more than 1 hour, followed by washing in 2 x SSC, 0.1% SDS at room temperature for 20 minutes 3 times, washing in 1 x SSC, 0.1% SDS at 37°C for 20 minutes once. In these methods, more stringent conditions may be achieved, for example, by raising the temperature of prehybridization, hybridization or the second washing. For example, the temperatures of prehybridization and hybridization may be raised to 60°C, respectively; for more stringent conditions, the temperatures may be raised to 68°C, respectively. Those skilled in the art could appropriately select conditions for obtaining c-Met isoforms, allelic mutants, and corresponding genes derived from other organisms, by taking into account various conditions such as probe concentration, probe length, reaction time, etc. in addition to the above-described salt concentrations and reaction temperatures.

Such polynucleotides may be obtained by gene amplification techniques, hybridization techniques, recombinant DNA techniques, and the like.

The term "biological composition" used herein means a composition comprising γ-secretase or a biologically active fragment thereof, or c-Met, and is not particularly limited. For example, the biological composition may be a cell-free reconstruction system, a mammal or a part thereof, or a transgenic non-human mammal so engineered to overexpress APP or a part of this transgenic mammal.

In the expressions "first biological composition containing γ-secretase or a biologically active fragment thereof" and "second biological composition containing c-Met" used herein, the γ-secretase and/or c-Met may be either endogenous or exogenous.

When the γ-secretase or c-Met is endogenous, the composition may be any composition as long as it contains γ-secretase or c-Met derived from the above-mentioned animal or a part thereof

The term "a part of the above-mentioned animal" include tissues, cells, cell lysates, cell membrane fractions or purified membranes of the above-mentioned animal. As examples of the cells, cells in the central nervous system; neurons such as brain-derived neurons, cerebral cortex-derived neurons, cerebral cortex-derived primarily cultured neurons, or hippocampus-derived primarily cultured neurons; or glial cells may be enumerated. The γ-secretase or c-Met may be in the state of being contained in a mammal or a part thereof Alternatively, the γ-secretase or c-Met may be a γ-secretase fraction or a c-Met fraction of cell lysate prepared from a mammal. The cell lysate may be obtained by subjecting γ-secretase- or c-Met-containing cells to lysis with a hypotonic solution or surfactant, or to sonication or other physical disruption. Optionally, the cell lysate may be purified with some purification means such as columns.

When the γ-secretase or c-Met is exogenous, the biological composition may be γ-secretase expressing cells or c-Met expressing cells prepared by allowing host cells to express the whole or a part of the sequences in expression vectors comprising a polynucleotide encoding the individual molecules constituting γ-secretase or a polynucleotide encoding c-Met. Alternatively, the biological composition may be the γ-secretase fraction of a cell lysate derived from γ-secretase expressing cells, or the c-Met fraction or cell membrane fraction of a cell lysate derived from c-Met expressing cells. The cell lysate may be obtained by subjecting γ-secretase- or c-Met-containing cells to lysis with a hypotonic solution or surfactant, or to sonication or physical disruption. Optionally, the cell lysate may be purified with some purification means such as columns. The expression vector may be a vector which is transformed or transfected into a host cell and temporarily expresses the gene of interest. Alternatively, the expression vector may be a vector which is integrated into the genome of a host cell and expresses the gene of interest stably.

The term "transformation" or "transfection" used herein means any and all methods which change DNA contents in eukaryotic cells or microorganisms. These methods include calcium phosphate transfection, protoplast fusion transfection, electroporation transfection, DEAE-dextran transfection, liposome transfection, polybrene transfection and direct microinjection transfection (Sambrook, et al., Molecular Cloning 3: 16.30-16.31 (1989)).

The host cell into which the above-described expression vector is to be transformed or transfected may be any cell (or cell line) or microorganism capable of expressing the gene of interest. Known cultured cells may be used as host cells. Examples of mammal cells or cell lines which may be used as host cells include, but are not limited to, HEK 293 cells, Chinese hamster ovary (CHO) cells, fibroblast cells, primary endothelial cells (HUVEC cells), human glioma cells, HeLa cells, COS cells, PC 12 cells, lymphoblast cells, melanoma cells, hybridoma cells, oocytes and embryonic stem cells. Examples of known microorganisms which may be used as host cells include *Escherichia coli* and yeast. Insect cells such as BmN4 cells may also be used.

The expression vector used in the above-described transformation or transfection is not particularly limited as long as the vector comprises a polynucleotide encoding the individual molecules constituting γ-secretase or a polynucleotide encoding c-Met. Such an expression vector may be a plasmid obtainable by introducing the polynucleotide into a known expression vector selected appropriately depending on the host cell to be used.

Examples of the above-mentioned known expression vector include pUC, pTV, pGEX, pKK or pTrcHis for *E. coli*; pEMBLY or pYES2 for yeast; pcDNA3, pMAMneo and pBabe Puro for CHO cells, HEK293 cells or COS cells; and a vector comprising the polyhedrin promoter of *Bombyx mori* nuclear polyhedrosis virus (BmNPV) (such as pBK283) for BmN4 cells.

In mammal cells, examples of promoters which give a strong transcription activity include CMV immediate early promoter, retrovirus promoters (e.g., LTR from MLV or MMTV), promoters of SV40, RSV LTR, HIV- 1 LTR and HIV-2 LTR, adenovirus promoters (e.g., those from E1A region, E2A region or MLP region), and promoters of AAV LTR, cauliflower mosaic virus, HSV-TK and avian sarcoma virus.

The above-described transformed or transfected host cell is not particularly limited as long as the host cell comprises a polynucleotide encoding the individual molecules constituting γ-secretase or a polynucleotide encoding c-Met. For example, the transformed cell may be a transformant in which the polynucleotide has been integrated into the chromosome thereof Alternatively, the transformed cell may be a transformant comprising the polynucleotide in the form of a plasmid. It is also possible that the transformed cell is a transformant which is not expressing γ-secretase or c-Met. These transformants may be obtained by transforming a desired host cell with the above-mentioned plasmid or the above-described polynucleotide *per se.*

Cells containing the above-described γ-secretase and/or c-Met are not particularly limited as long as the cell is capable of expressing γ-secretase and/or c-Met on the surface of its cell membrane. As examples of such cells, a cell expressing endogenous γ-secretase and endogenous c-Met, a cell expressing γ-secretase and c-Met one of which is endogenous and the other is exogenous or a cell expressing exogenous γ-secretase and exogenous c-Met may be given. Such cells may also be obtained by culturing under conditions which allow expression of γ-secretase and/or c-Met. Alternatively, such cells may be obtained by injecting into an appropriate cell an RNA encoding the individual molecules constituting γ-secretase and/or an RNA encoding c-Met and culturing the resultant cell under conditions which allow expression of γ-secretase and/or c-Met.

The above-described cell membrane fraction may be obtained, for example, by disrupting cells expressing the γ-secretase or c-Met of the present invention and isolating cell membrane-rich fractions. As methods for disrupting cells, homogenizing in a homogenizer; disrupting in a Waring blender or Polytron; disrupting by sonication; ejecting cells from a thin nozzle while applying pressure with a French press; and so on may be used. As methods for fractionating cell membrane, fractionation methods with centrifugal force such as differential centrifugation or density gradient centrifugation may be used.

For purification, a known method for protein purification may be used. The method comprises a step of crudely fractionating cells into polypeptide fractions and non-polypeptide fractions. After the γ-secretase or c-Met of the present invention has been isolated from other polypeptides with a column or the like, the desired γ-secretase or c-Met is further purified by chromatography or electrophoresis to thereby achieve partial purification or complete purification (or homogeneity by purification). Examples of analysis methods particularly suitable for preparation/purification of pure peptides include precipitation using ammonium sulfate, PEG, antibodies, etc.; centrifugation after thermal denaturation; chromatography step; isoelectric focusing; gel electrophoresis; SDS (sodium dodecyl sulfate)-polyacrylamide electrophoresis (SDS-PAGE); and a combination of these methods and other method. Examples of chromatography step includes ion exchange chromatography, gel filtration chromatography, reversed-phase chromatography, hydroxyapatite chromatography, affinity chromatography, fast protein liquid chromatography (FPLC), high performance liquid chromatography (HPLC) and immobilized metal ion affinity chromatography (IMAC).

Alternatively, γ-secretase or c-Met may be tagged in advance; then, a crude polypeptide may be applied to a purification column to which a protein that recognizes the tag has been bound; the desired γ-secretase or c-Met adsorbed onto the column may be desorbed from the column by feeding an appropriate solvent thereinto.

Various purification steps may be performed in a different order, or some of the steps may be omitted. A preferable method for evaluating the purity of a fraction is a method in which the specific activity of the fraction is calculated and compared with the specific activity of the first extract, followed by calculation of the magnitude of purity for evaluation.

The term "cleavage of c-Met" used herein refers to an event in which c-Met, cut by γ-secretase, produces a fragment shorter than the initial c-Met before cutting. The term "c-Met undegraded product" used herein refers to a polypeptide which is produced as a result of non-cleavage of c-Met; this polypeptide means the c-Met polypeptide before degradation by γ-secretase.

For monitoring the cleavage of c-Met by γ-secretase, any of the following antibodies may be used: an anti-c-Met antibody; an antibody which recognizes c-Met undegraded product produced as a result of non-cleavage of c-Met; or an antibody which recognizes c-Met cleaved product produced as a result of cleavage of c-Met, preferably an antibody which recognizes the intracellular domain of c-Met, more preferably an antibody which recognizes the C-terminal domain of c-Met. When the undegraded product of a tagged c-Met polypeptide is to be detected, an antibody which recognizes the selected tag may be used. For example, when an HA tag has been added to the C-terminus of c-Met, it is possible to detect the undegradation or cleavage of c-Met using an anti-HA tag antibody. In this case, the antibody is capable of clarifying the presence and concentration of a C-terminal fragment of c-Met that is produced as a result of non-cleavage of c-Met or the presence and concentration of a C-terminal fragment of c-Met that is produced as a result of cleavage of c-Met.

The term "APP" used herein β-amyloid precursor protein (βAPP) or a mutant thereof APP is a single-pass transmembrane protein comprising an Aβ domain in its C-terminal region, expressed in a large variety of cells in many mammals. In human, APP is encoded by the gene *APP* located in the long arm of chromosome No. 21 and has three major isotypes (APP695, APP751 and APP770). APP695, APP751 and APP770 consist of 695, 751 and 770 amino acid residues, respectively. Examples of APP proteins include human APP695 (NM_201414, NP_958817 and P05067-4), human APP751 (NM_201413, NP_958816 and P05067-8), human APP770 (NM_000484, NP_000475, P05067-1 and P05067), mouse APP695 (NM_007471, NP_031497 and P12023-2), mouse APP751 (P12023-3), mouse APP770 (AY267348, AAP23169, P12023-1 and P12023), rat APP695 (P08592-2), rat APP751 (P08592-7) and rat APP770 (NM_019288, NP_062161, P08592-1 and P08592). Examples of APP mutants include Swedish FAD double mutant, London mutant, valine717 to phenylalanine mutant, valine717 to isoleucine mutant and valine717 to glycine mutant.

The term "Aβ" used herein means the term β-amyloid protein, amyloid β protein, β-amyloid peptide, amyloid β peptide or amyloid beta. For example, Aβ is a peptide consisting of about 33-44 amino acids residues in human APP695 amino acid isotype. Preferably, Aβ includes any peptide comprising a part or all of the amino acid residues from positions 597 to 640 in APP, and means every peptide produced from APP by its N-terminal protein degradation and subsequent C-terminal protein degradation. Aβ40 and Aβ42 are peptides comprising 40 amino acid residues and 42 amino acid residues, respectively.

The term "Notch" used herein refers to one of the competing substrates for γ-secretase belonging to the cell surface receptor Notch family. For example, human Notch 1 (AF308602.1), mouse Notch 1 (NM_008714.2) and rat Notch 1 (NM_001105721.1) may be enumerated. Since Notch 1 has an important function in hematopoiesis, inhibition of the processing of Notch 1 may potentially cause immunodeficiency and anemia.

The term "candidate compound" used herein means a compound which is tested in the compound screening method. Although every molecule may be used as a candidate compound, preferably a compound capable of changing the activity of γ-secretase (preferably the activity of γ-secretase of mammals) is used. The candidate compound is one or more compounds contained in expression products of gene libraries; natural or synthetic low molecular weight compound libraries; nucleic acids (oligo DNA or oligo RNA); natural or synthetic peptide libraries; antibodies; substances released from bacteria (including those released from bacteria as a result of metabolism); cell (from microorganisms, plant cells or animal cells) extracts; cell (microorganism, plant cell or animal cell) culture supernatants; purified or partially purified peptides; extracts from marine organisms, plants or animals; soils; or random phage peptide display libraries. The above-described compound may be either a novel compound or a known compound. Further, the above-described compound may be a compound modified by conventional chemical means, physical means and/or biochemical means. For example, the above-desribed compound may be a structural analogue which is obtained by subjecting the initial compound to direct chemical modification (such as acylation, alkylation, esterification or amidation) or random chemical modification. The candidate compound may also be a compound identified by pharmacophore search of the above-described compound or structure comparison programs with computer. The candidate compound may be in the form of a salt. Further, the candidate compound or a salt thereof may be in the form of a solvate (including hydrate).
Further, the candidate compound may be a known γ-secretase accelerator or γ-secretase inhibitor involved in the processing of APP and/or the processing of Notch, or a structural analogue of the above accelerator or inhibitor. A known compound which accelerates or inhibits the activity of γ-secretase and/or the processing of APP and/or the processing of Notch may be a compound that can be designed through rational drug design. For example, DAPT (N-[N-(3,5-difluorophenacetyl)-L-alanyl]-S-phenylglycine t-butyl ester), CM256 (N-[(1,1 dimethylethoxy) carbonyl]-L-valyl- (4S, 5S) -4-amino-2, 2-difluoro-5-methyl-3-oxoheptanoyl-L-valyl-L-Isoleucine methyl ester) and (2S)-2-{[(3,5-difluorophenyl)acetyl]amino}-N-[(3S)-1-methyl-2-oxo-5-phenyl-2,3-dihydro-1H-1,4-benzodiazepin-3-yl]propanamide (Alexis Biochemicals) may be enumerated.

The expression "compound which affects the processing of c-Met by γ-secretase" used herein means either a compound which inhibits the c-Met cleavage activity by γ-secretase (γ-secretase inhibitor) or a compound which accelerates the c-Met cleavage activity by γ-secretase (γ-secretase accelerator). It should be noted that γ-secretase inhibitor includes antagonist to γ-secretase and that γ-secretase accelerator includes agonist to γ-secretase. γ-Secretase inhibitor and γ-secretase accelerator also include those compounds which alter the cleavage site of c-Met cleavage products by γ-secretase to thereby produce c-Met cleavage products with different peptide lengths.

The term "salt" used herein refers to a pharmacologically acceptable salt, and is not particularly limited as long as it forms a pharmacologically acceptable salt with the above-described compound. Preferred examples thereof are hydrohalogenic acid salts (such as hydrofluoride, hydrochloride, hydrobromide or hydroiodide), inorganic acid salts (such as sulfate, nitrate, perchlorate, phosphate, carbonate or hydrogencarbonate), organic carboxylic acid salts (such as acetate, oxalate, maleate, tartrate, fumarate or citrate), organic sulfonic acid salts (such as methanesulfonate, trifluoromethanesulfonate, ethanesulfonate, benzenesulfonate, toluenesulfonate or camphorsulfonate), amino acid salts (such as aspartate or glutamate), quaternary amine salts, alkali metal salts (such as lithium salt, sodium salt or potassium salt) and alkaline earth metal salts (such as magnesium salt or calcium salt).

According to the present invention, there are provided (a) an assay method for examining the cleavage of c-Met and (b) a method of evaluating whether or not a candidate compound is a compound which affects γ-secretase (screening method) utilizing the assay method. The method of the present invention is as described below.

The method of screening for compounds which affect the processing of c-Met by γ-secretase (which is an embodiment of the present invention) comprises the following steps:
(i) contacting a first biological composition containing γ-secretase or a biologically active fragment thereof with a second biological composition containing c-Met in the presence and absence of a candidate compound;
(ii) measuring the cleavage of the c-Met in the presence and absence of the candidate compound;
(iii) selecting those candidate compounds which affect the cleavage of the c-Met by γ-secretase; and
(iv) identifying the candidate compounds selected in step (iii) as compounds which affect the processing of c-Met by γ-secretase.

The method of the present invention may be performed in an appropriate cell system containing γ-secretase and c-Met or a cell-free system containing γ-secretase and c-Met.

The cell system containing γ-secretase and c-Met may be either a cell system expressing endogenous genes or a cell system containing an exogenous gene(s). It is possible to contact a first biological composition containing γ-secretase with a second biological composition containing c-Met by culturing a cell containing γ-secretase and c-Met in an appropriate medium in the presence and absence of a candidate compound and incubating the cell under reaction conditions which allow the cleavage of c-Met by γ-secretase activity. If a cell system containing an exogenous gene(s) is used, the above-described contact may be performed under culture conditions which allow the expression of the exogenous gene. It is also possible to apply conditions that allow the cleavage of other γ-secretase substrate, e.g., reaction conditions known to those skilled in the art when the substrate is APP.

Examples of reaction conditions are enumerated below. For cell systems expressing endogenous genes, in the case of primary culture of neurons, culture conditions are in MEM (Invitrogen) medium supplemented with 5% FBS (Hyclone), 1X B27 Supplement (Invitrogen), 0.5 mM L-glutamine (Invitrogen), 25 µg/ml insulin (SIGMA) and 8 µM AraC (SIGMA); under 5% CO₂; and at 37°C. For cell systems containing an exogenous gene(s), in the case of HEK293 cell line, culture conditions are in 10% FBS (Hyclone)/DMEM (Invitrogen), under 5% CO₂ and at 37°C.

In cell-free systems, a first biological composition containing γ-secretase or a biologically active fragment thereof (e.g., cell membrane fraction containing γ-secretase) and a second biological composition containing c-Met (e.g., cell membrane fraction containing c-Met) may be contacted with each other by incubating these compositions by mixing them in the presence and absence of a candidate compound. These compositions may be mixed under reaction conditions which allow the cleavage of c-Met by γ-secretase activity, e.g., 10 mM HEPES, pH 7.4, 150 mM NaCl, 10% glycerol, 5 mM EDTA, 5 mM 1,10-phenanthroline, 10 µg/ml phosphoramidon, Complete protease inhibitor cocktail (Roche Biochemicals) (Tomita et al., Molecular Neurodegeneration 2006 1:2). Alternatively, these compositions may be mixed under conditions which allow the cleavage of other γ-secretase substrate, e.g., reaction conditions known to those skilled in the art when the substrate is APP. γ-Secretase or c-Met may be a purified γ-secretase or c-Met, a biologically active fragment of γ-secretase or c-Met, an analogue of γ-secretase or c-Met, or a mutant of γ-secretase or c-Met.

The candidate compound may be added generally within a range from approx. 1 nM to 1 mM, usually within a range from approx. 10 µM to 1 mM.

By contacting a first biological composition containing γ-secretase with a second biological composition containing c-Met as described above, c-Met cleavage reaction by γ-secretase occurs.

In order to identify a compound which changes the cleavage of c-Met by γ-secretase, the steps described above are performed in the presence and absence of a candidate compound. Then, the c-Met cleavage activity of γ-secretase in the presence of the candidate compound is compared with that activity in the absence of the candidate compound (control) to evaluate the effect of the candidate compound upon cleavage activity. By these procedures, it is possible to identify compounds which change the cleavage of c-Met by γ-secretase.

Even a slight change in the quantity or degree of c-Met in the presence of a candidate compound indicates that the c-Met cleavage activity of γ-secretase has been changed in the presence of the candidate compound. Therefore, the candidate compound can be identified as a compound which affects the processing of c-Met by γ-secretase. For example, a compound which increases c-Met cleavage product or decreases c-Met undegraded product compared with control is evaluated as an accelerator for the c-Met degradation activity of γ-secretase. On the other hand, a compound which decreases c-Met cleavage product or increases c-Met undegraded product compared with control is evaluated as an inhibitor for the c-Met degradation activity of γ-secretase. The accelerator for the degradation activity of γ-secretase obtained by the method of the present invention is potentially useful for treatment ofAD.

Analysis of c-Met cleavage is performed by measuring an indicator of cleavage for one or both of the N-terminal fragment and C-terminal fragment of c-Met.

As indicators of cleavage, the following antibodies may be used: anti-c-Met antibodies; antibodies recognizing a c-Met derivative (c-Met undegraded product) before degradation by γ-secretase, produced as a result of non-cleavage of c-Met; antibodies recognizing a c-Met cleavage product produced as a result of cleavage of c-Met; or antibodies recognizing the intracellular domain of c-Met.

When c-Met is tagged, a substance which binds to the tag (e.g., antibody) may be used to detect the indicator.

For example, when a tagged undegraded product of c-Met polypeptide is to be detected, an hemagglutinin (HA) tag may be added to the C-terminus of c-Met, followed by detection with an anti-HA tag antibody In this case, it is possible to clarify the presence and concentration of a C-terminal fragment of c-Met produced as c-Met undegraded product, by detecting or quantitatively determining the HA tag. When c-Met or tagged c-Met is labeled, it is possible to detect its undegraded product by detecting or quantitatively determining the label.

On the other hand, when a cleavage product of c-Met polypeptide is to be detected, the membrane fraction is purified from c-Met-expressing cells; then, the purified membrane fraction is subjected to cleavage reaction by γ-secretase to thereby allow the c-Met cleavage product to be released from the membrane fraction. When this reaction product is centrifuged, the c-Met undegraded product is precipitated into the membrane fraction. Thus, the membrane fraction and other fractions can be separated. By detecting the released fragment, the c-Met cleavage product can be detected. For this detection, an antibody recognizing the intracellular domain of c-Met is used when endogenous c-Met is used. When exogenous recombinant c-Met is used, a cDNA encoding c-Met with a tag sequence added to its C-terminus is used to express the recombinant c-Met. Then, the c-Met cleavage product is detected using an antibody that recognizes the tag. The tag is not particularly limited. For example, HA, Myc, FLAG or the like may be used.

The antibody to c-Met is not particularly limited as long as the antibody recognizes c-Met. Preferably, an antibody which recognizes the intracellular domain of c-Met is used.

Those skilled in the art could prepare an antibody which recognizes c-Met by immunizing an animal with an immunogen (antigen) and following the conventional, general procedures for preparing monoclonal antibodies. As the immunogen for example, c-Met or a fragment thereof, or a fusion protein prepared by adding a tag or label to c-Met or a fragment thereof may be used.

For example, a non-human mammal is immunized with the immunogen alone or, if necessary, together with Freund's adjuvant. Polyclonal antibodies may be obtained from the serum collected from the immunized animal. Monoclonal antibodies may be obtained by fusing antibody producing cells from the immunized animal with myeloma cells without autoantibody producing ability to prepare fusion cells (hybridomas), cloning the hybridomas, and selecting those clones which produce a monoclonal antibody showing specific affinity to the antigen used for immunizing the animal. The preparation of monoclonal antibodies from hybridomas may be performed *in vitro.* Alternatively, the preparation may be performed *in vivo* in a non-human mammal, preferably mouse or rat, more preferably in the abdominal dropsy in mouse. Monoclonal antibodies may be isolated from the resultant culture supernatant or the abdominal dropsy of the mammal. The isolation and purification of monoclonal antibodies may be performed by subjecting the above-mentioned culture supernatant or abdominal dropsy to methods such as saturated ammonium sulfate precipitation, euglobulin precipitation, caproic acid method, caprilic acid method, ion exchange chromatography (DEAE, DE52, etc.), or affinity column chromatography using anti-immunoglobulin column or protein A column. The monoclonal antibody include those monoclonal antibodies consisting of heavy chains and/or light chains having the amino acid sequences which have deletion, substitution or addition of one or several amino acids in the heavy chains and/or light chains constituting the initial antibody

By the procedures described above, it is possible to incubate γ-secretase and c-Met in the presence or absence of a candidate compound and to evaluate the cleavage of c-Met by γ-secretase.

In another embodiment of the present invention, it is possible to evaluate whether or not a candidate compound affects the processing of APP and/or Notch, in parallel with, simultaneously with, or before or after the above-described method of the present invention. For example, a step of measuring the cleavage of APP or a polypeptide containing an APP cleavage site by γ-secretase (i.e., polypeptide containing APP γ-secretase cleavage site) may be included in parallel with, simultaneously with, or before or after the above-described method of the present invention. Further, a step of measuring the cleavage of Notch or a polypeptide containing a Notch cleavage site by γ-secretase (i.e., polypeptide containing Notch γ-secretase cleavage site) may be included.

By including such steps, it is possible to evaluate whether or not a candidate compound selectively acts on the processing of c-Met compared to the processing of APP and/or Notch. The expression "selectively act on" used herein means to have more inhibitory effect or accelerating effect upon the cleavage of substrate c-Met by γ-secretase than upon the cleavage of other substrate(s). Specifically, according to this embodiment of the present invention, it is possible to identify a compound which selectively acts only on the processing of c-Met; a compound which selectively acts only on the processing of APP; a compound which selectively acts only on the processing of Notch; or a compound which selectively acts on the processing of APP and c-Met. This means that, with the method of the present invention, it is possible to obtain compounds which selectively inhibit or activate the γ-secretase's cleavage activity on c-Met in addition to γ-secretase's known substrates such as APP or Notch. Therefore, it is possible to obtain compounds which selectively inhibit or activate the cleavage function of γ-secretase on a specific substrate; compounds with increased selectivity on the substrate of γ-secretase can be obtained.

As a candidate compound in drug development, a compound which acts in the same manner as metabolic activity in healthy animals *in vivo* or a compound which regulates the metabolic activity is preferable. A preferable example of such a candidate compound is a compound which inhibits the production of Aβ42 by inhibiting the APP cleavage activity of γ-secretase, does not inhibit the c-Met cleavage activity of γ-secretase, and does not inhibit the Notch cleavage activity of γ-secretase. Another preferable example is a compound which inhibits the production of Aβ42 by accelerating the production of Aβ40 through acceleration of the APP cleavage activity of γ-secretase; accelerates the degradation of c-Met by accelerating the c-Met cleavage activity of γ-secretase; and does not inhibit the Notch cleavage activity of γ-secretase.

As methods for measuring the cleavage by γ-secretase of APP, Notch or a polypeptide containing an APP or Notch γ-secretase cleavage site, assay methods known to those skilled in the art may be applicable (Song et al. PNAS 1999 96 6959-6963; Moehlmann et al. PNAS 2002 99 8025-8030). For example, a first biological composition containing γ-secretase or a biologically active fragment thereof may be contacted with a biological composition containing APP or a polypeptide containing an APP γ-secretase cleavage site or a biological composition containing Notch or a polypeptide containing a Notch γ-secretase cleavage site in the presence and absence of a candidate compound, and then the cleavage of the APP or the polypeptide containing an APP γ-secretase cleavage site or the cleavage of the Notch or the polypeptide containing a Notch γ-secretase cleavage site. This measurement may be performed by measuring the cleavage product from the APP or the polypeptide containing an APP γ-secretase cleavage site or the cleavage product from the Notch or the polypeptide containing a Notch γ-secretase cleavage site. As one example of the cleavage product from APP or a polypeptide containing an APP γ-secretase cleavage site, Aβ may be given. The quantity of Aβ may be measured, and changes in the quantity between the presence and absence of the candidate compound may be compared. Alternatively, the degree of cleavage and the quantity of cleavage product may be measured by using a known antibody which recognizes the cleavage product from APP or a polypeptide containing an APP γ-secretase cleavage site or the cleavage product from Notch or a polypeptide containing a Notch γ-secretase cleavage site. As the antibody which recognizes the cleavage product from APP or a polypeptide containing an APP γ-secretase cleavage site, commercial antibodies (Sigma or Chemicon) may be used. The measurement may be performed, for example, by Western blotting. As the antibody which recognizes the cleavage product from Notch or a polypeptide containing a Notch γ-secretase cleavage site, commercial antibodies (Santacruze) may be used. The measurement may be performed, for example, by Western blotting.

The method of the present invention also includes a high through put screening (HTS) known to those skilled in the art, which tests a large number of compounds simultaneously (US5876946; US5902732; Jayawickreme and Kost, Curr. Opin. Biotechnol., 8, pp. 629-634, 1997; Houston and Banks, Curr. Opin. Biotechnol., 8, pp. 734-740, 1997).

The method of the present invention also includes the use of known model animals. It is possible to analyze the *in vivo* effect of a compound selected by the *in vitro* method of the present invention by using, for example, non-human models for APP processing and/or AD. As non-human models for AD, APP transgenic non-human animal models are well-known in the art. For example, Tg2576 mouse (J. Neurosci. 21(2), 372-381, 2001; J. Clin. Invest., 112, 440-449, 2003) may be used. Further, the following evaluations may be made after administering to Tg2576 mouse a known γ-secretase inhibitor DAPT, (2S)-2-{[(3,5-difluorophenyl)acetyl]amino}-N-[(3S)-1-methyl-2-oxo-5-phenyl-2,3-dihydro-1H-1,4-benzodiazepin-3-yl]propanamide (Alexis Biochemicals) or a compound of the present invention: evaluation by a method of measuring the Aβ quantities in the brain, cerebrospinal fluid and serum of the mouse (J. Pharmacol. Exp. Ther. 305, 864-871, 2003); pathological examination of changes in the brain (e.g., changes in Aβ yield, the degree of cerebral atrophy, etc.) resulted from changes in γ-secretase activity; and evaluation of the survival ratio, momentum or food consumption of the mouse.

The pharmaceutical composition comprising the compound identified by the method of the present invention, preferably the AD therapeutic of the present invention, may be administered to patients in various forms through an oral or parenteral (e.g., intravenous injection, muscle injection, subcutaneous administration, rectal administration or transdermal administration) route. Therefore, the pharmaceutical composition comprising the compound of the present invention may be formulated into various preparations using a pharmacologically acceptable carrier by a conventional method depending on the administration route, though the pharmaceutical composition may be used alone.

Preferred dosage forms include oral preparations such as tablets, powders, subtle granules, granules, coated tablets, capsules, syrups and troches; and parenteral preparations such as inhalants, suppositories, injections (including drops), ointments, eye drops, ophthalmic ointments, nasal drops, ear drops, cataplasms, and lotions and liposomes.

Examples of carriers used in the formulation include conventionally used fillers, binders, disintegrants, lubricants, coloring agents and flavoring agents, as well as stabilizers, emulsifiers, absorbefacients, surfactants, pH adjusting agents, antiseptics, antioxidants, expanders, wetting agents, surface activators, dispersing agents, buffers, preservatives, dissolution aids and analgesic agents according to necessity. They can be formulated according to a conventional procedure using components commonly used as raw materials for pharmaceutical preparations. Examples of nontoxic these components which may be used in the present invention include animal and vegetable oils such as soybean oil, beef tallow and synthetic glycerides; hydrocarbons such as liquid paraffins, squalane and solid paraffins; ester oils such as octyldodecyl myristate and isopropyl myristate; higher alcohols such as cetostearyl alcohol and behenyl alcohol; silicone resins; silicone oils; surfactants such as polyoxyethylene fatty acid esters, sorbitan fatty acid esters, glycerin fatty acid esters, polyoxyethylene sorbitan fatty acid esters, polyoxyethylene hydrogenated castor oils and polyoxyethylene-polyoxypropylene block copolymers; water-soluble polymers such as hydroxyethyl cellulose, polyacrylic acids, carboxyvinyl polymers, polyethylene glycol, polyvinylpyrrolidone and methylcellulose; lower alcohols such as ethanol and isopropanol; polyhydric alcohols (polyols) such as glycerol, propylene glycol, dipropylene glycol, sorbitol and polyethylene glycol; sugars such as glucose and sucrose; inorganic powders such as silicic anhydride, magnesium aluminium silicate and aluminium silicate; inorganic salts such as sodium chloride and sodium phosphate; and purified water.

The fillers include, for example, lactose, fructose, com starch, white sugar, glucose, mannitol, sorbitol, crystalline cellulose and silicon dioxide. The binders include, for example, polyvinyl alcohol, polyvinyl ether, methylcellulose, ethylcellulose, gum arabic, gum tragacanth, gelatin, shellac, hydroxypropyl methylcellulose, hydroxypropyl cellulose, polyvinylpyrrolidone, polypropylene glycol-polyoxyethylene block polymers and meglumine. The disintegrants include, for example, starch, agar, gelatin powder, crystalline cellulose, calcium carbonate, sodium hydrogencarbonate, calcium citrate, dextrin, pectin and carboxymethylcellulose calcium. The lubricants include, for example, magnesium stearate, talc, polyethylene glycol, silica and hardened vegetable oils. The coloring agents may be any coloring agents which are approved to be added to pharmaceutical preparations. The flavoring agents include, for example, cocoa powder, menthol, aromatic powder, peppermint oil, camphol and cinnamon powder. The above-listed components may be in the form of a salt or solvate thereof.

The oral preparation is produced by mixing the compound of the present invention with a filler, and if necessary, a binder, disintegrant, lubricant, coloring agent, flavoring agent, etc. and formulating the mixture according to conventional procedures into, for example, a powder, subtle granules, granules, tablet, coated tablet, capsules or the like. Resultant tablets and granules can be appropriately coated with, for example, sugar according to necessity. The syrups and injection preparations can be prepared according to conventional procedures by adding a pH adjusting agent, solubilizer, and isotonizing agent, and if necessary, a dissolution aid, stabilizer, etc. The external preparations can be produced according to conventional procedures not specifically limited. Base materials which may be used in the present invention include various raw materials conventionally used in pharmaceutical preparations, quasi drugs and cosmetics. Such raw materials include, for example, animal and vegetable oils, mineral oils, ester oils, waxes, higher alcohols, fatty acids, silicone oils, surfactants, phospholipids, alcohols, polyhydric alcohols, water-soluble polymers, clay minerals and purified water. If necessary, pH adjusting agents, antioxidants, chelating agents, antiseptics and antimolds, coloring agents, flavors, or the like can be added. In addition, components such as blood-flow accelerators, bactericides, anti-inflammatory agents, cell activators, vitamins, amino acids, humectants, keratolytic agents or the like be added according to necessity. The ratio of the active ingredient to carriers may vary from 1 to 90% by weight. When the compounds used in the present invention, the peptides used in the present invention or the polynucleotides used in the present invention are used in the above-described treatment, it is preferable to use those compounds, peptides or polynucleotides purified to 90% or more, preferably 95% or more, more preferably 98% or more, still more preferably 99% or more.

The effective dose of the pharmaceutical composition comprising the compound of the present invention varies depending on the severity of symptom, the age, sex and body weight of the patient, administration mode, type of the salt, specific type of the disease and other factors. Generally, the pharmaceutical composition may be administered to an adult (body weight: 60 kg) in one to several divided doses at a daily dose of about 30 µg to about 10 g, preferably 100 µg to 5 g, and more preferably 100 µg to 100 mg for oral administration, or at a daily dose of about 30 µg to about 1 g, preferably 100 µg to 500 mg, and more preferably 100 µg to 30 mg for injection administration. Considering that efficacy varies depending on the administration route, the required dose is expected to vary widely. For example, it is expected that oral administration requires a higher dose than intravenous injection. When administered to children, the dose may be smaller than the dose for adults. These variations in the dose level can be adjusted by standard empirical optimization procedures which are well understood in the industry.

The term "treatment" is herein to generally mean obtaining a desired pharmacological and/or physiological effect. The effect may be prophylactic in terms of completely or partially preventing a disease and/or a symptom and may be therapeutic in terms of partially or completely curing a disease and/or an adverse effect attributed to the disease. The term "treatment" as used herein covers any treatment of a disease in a patient, preferably a human, and includes at least one treatment selected from the following (a) to (c):
(a) preventing a disease or a symptom from occurring in a patient who may be predisposed to the disease but has not yet been diagnosed as having it;
(b) inhibiting a disease symptom, i.e. preventing or delaying its progress; or
(c) relieving a disease symptom, i.e. causing regression or elimination of the disease or symptom, or causing reversal of the progress of the disease.

For example, as clinical symptoms of AD, progressive disorientation, memory loss and aphasia are enumerated. Finally, disablement, speech loss and akinesia occur. Pathological signs of AD include neurofibrillary tangle, senile plaques and amyloid angiopathy. To prevent the progress of AD is interpreted to mean to prevent the onset or further progress of the clinical symptoms and/or pathological signs of AD. For example, in patients who do not have the clinical symptoms or pathological signs of AD, it is possible to prevent the progress of clinical symptoms or pathological signs. In patients suffering from mild AD, it is possible to prevent the development of more severe AD forms. To delay the progress of AD is interpreted to mean to delay the point of onset of AD-related symptoms and/or pathological signs, or to reduce the speed of progress of AD that is determined by the speed of progress of clinical symptoms and pathological signs. To reverse the progress of AD is interpreted to mean to relieve the severity of AD symptoms, i.e., to change the severity of AD conditions of patients from severe to mild. At that time, the change to mild is indicated by decrease of clinical symptoms or pathological signs.

Diagnosis of AD in patients may be performed by various known methods. Typically, AD is diagnosed by combining clinical and pathological assessments. For example, the progress or severity of AD may be judged using Mini Mental State Examination (MMSE) (Mohs et al. (1996) Int Psychogeriatr 8: 195-203), Alzheimer's Disease Assessment Scale-Cognitive Subscale (ADAS-cog) (Galasko et al., (1997) Alzheimer Dis Assoc Disord, 11 suppl 2: S33-9), Alzheimer's Disease Cooperative Study-Activities of Daily Living (ADCS-ADL) (McKhann et al., (1984) Neurology 34: 939-944) and Criteria of National Institute of Neurologic Communicative Disorders and Stroke-Alzheimer's Disease and Related Disorders Association (NINCDS-ADRDA) (Folstein et al., (1975) J Psychiatr Res 12: 189-198; McKhann et al., (1984) Neurology 34: 939-944). Further, methods which evaluate various regions of the brain and enable the estimation of frequency of senile plaques or neurofibrillary tangle may be used (Braak et al., (1991) Acta Neuropathol 82: 239-259; Khachaturian (1985) Arch Neuro 42: 1097-1105; Mirra et al., (1991) Neurology 41: 479-486; and Mirra et al., (1993) Arch Pathol Lab Med 117: 132-144).

The present invention provides a test kit for measuring the processing of c-Met by γ-secretase as a test kit for use in the method of the present invention.

The kit of the present invention comprises at least γ-secretase or a biological composition containing γ-secretase, and a biological composition containing c-Met. The kit may further comprise a substrate for γ-secretase other than c-Met (e.g., APP and/or Notch) or a biological composition containing such a substrate. Preferably, the kit comprises a plurality of substrates for γ-secretase. It is preferred that the kit comprises APP and/or Notch in addition to c-Met.

Further, the kit may comprise tools, reagents, instructions, and so forth which are used in technologies for detecting the processing of c-Met by γ-secretase. Examples of technologies for detecting the processing of c-Met by γ-secretase include immunoblotting and Western blotting. Examples of tools used in these technologies include reaction vessels and blotting membranes; and examples of reagents used in these technologies include buffers, culture broths and anti-c-Met antibodies.

With the above-described kit, it is possible to measure the processing of c-Met by γ-secretase. As a result, it becomes possible to evaluate whether or not a candidate compound affects the processing of c-Met by γ-secretase. Therefore, this kit can serve as a test kit for screening for compounds which affect the processing of c-Met by γ-secretase. The test kit of the present invention includes a test kit for screening for compounds which affect the processing of c-Met by γ-secretase, the kit having the same constitution as that of the test kit for measuring the processing of c-Met by γ-secretase.

Further, the present invention includes the use of the above-described kit in measuring the processing of c-Met, or in methods of screening or testing γ-secretase inhibitors.

### EXAMPLES

Hereinbelow, the present invention will be described in more detail with reference to the following Examples and Preparation Examples. However, the present invention is not limited to these Examples, which are provided only for the purpose of full disclosure of the present invention to those skilled in the art. It is not meant or even implied that the experiments described herein are all or only one experiment actually carried out. Although efforts have been made to guarantee the accuracy of the numerical values used herein (e.g., volume, temperature, concentration, etc.), experimental errors and deviations are considered to some extent. Thus, such values may be changed within a range which does not depart from the scope of the present invention.

### Example 1: Analysis of c-Met Processing in c-Met-Transfected 293/EBNA-1 Cell Strain

Whether or not c-Met is a substrate for γ-secretase was evaluated using HEK293 cells expressing a gene encoding c-Met with an HA tag added to its C-terminus, in the presence of a γ-secretase inhibitor. As the γ-secretase inhibitor, (2S)-2-{[(3,5-difluorophenyl)acetyl]amino}-N-[(3S)-1-methyl-2-oxo-5-phenyl-2,3-dihydro-1H-1,4-benzodiazepin-3-yl]propanamide (hereinafter, sometimes referred to as "Compound E") (Alexis Biochemicals) was used.

### 1. Experimental Conditions and Methods

### (1) Cloning of Rat c-Met

RNA was purified from rat brain with Trisol (Invitrogen), followed by synthesis of 1st strand cDNA with RNA PCR Kit (TaKaRa). *c-Met* was amplified in two separate fragments of 1-2100 bp (primers 1 and 2) and 2041 bp-stop (primers 3 and 4). Specifically, *c-Met* was amplified using 1 µl of the finally synthesized 1st strand cDNA product, the following primers and Pfu (Stratagene).

Primer 1 - SalI site added: GAGGTCGACGCCACCATGAAGGCTCCCACCGCGCTGGC (SEQ ID NO: 29)
Primer 2: TAAAGTACATGTTTTCCCTCCGAT (SEQ ID NO: 30)
Primer 3: AAATACCTCAACAGCGGCAATTC (SEQ ID NO: 31)
Primer 4 - NotI site added: GAGGCGGCCGCTGTGTTCGCTTCGCCGTCAATGTT (SEQ ID NO: 32)
   PCR conditions were as follows: first reaction at 95°C for 2 minutes; then 35 cycles of (at 95°C for 45 seconds → at 60°C for 45 seconds→at 72°C for 6 minutes); then final reaction at 72°C for 10 minutes. The PCR products were purified with Quiaquick PCR purification kit (QIAGEN). Then, 1-2100 bp fragment was treated with restriction enzymes SalI (TaKaRa) and XbaI (TaKaRa), and 2041 bp-stop fragment was treated with XbaI (TaKaRa) and NotI (TaKaRa). Subsequently, they were cloned into pBluescript (Stratagene), separately. The resultant pBluescripts were subjected to sequence analysis with a DNA sequencer (Applied Biosystems; 3130x1). The pBluescript into which 1-2100 bp fragment had been inserted was treated with restriction enzymes XbaI and NotI, and then 2041 bp-stop fragment was inserted thereto.

### (2) Construction of a Gene Encoding Rat c-Met with HA Tag Added to Its C-Terminus and an Expression Vector

pcDNA (Invitrogen) was treated with restriction enzymes SalI (TaKaRa) and NotI (TaKaRa). c-Met obtained by treating the pBluescript obtained in (1) above with restriction enzymes SalI (TaKaRa) and NotI (TaKaRa) was inserted into the resultant pcDNA to thereby prepare an expression vector. This expression vector was constructed so that an HA tag is added to the C-terminus of the incorporated c-Met. The DNA sequence of the resultant c-Met-HA was analyzed with a DNA sequencer (Applied Biosystems; 3130x1). The resultant DNA sequence is shown in SEQ ID NO: 13.

In the resultant DNA sequence, the nucleotide at position 156 was changed from c to t, compared with rat c-Met (X96786). (Hereinafter, this mutation is expressed as "c→t". Other mutations will also be expressed in the same manner.) Besides, the resultant DNA sequence had c→a mutation at position 173, a→c mutation at position 744, g→a mutation at position 1095, g→a mutation at position 1269, c→g mutation at position 1597, t→c mutation at position 1854, c→t mutation at position 2189, g→a mutation at position 2423, t→g mutation at position 2843, t→c mutation at position 3083, c→a mutation at position 3203, t→c mutation at position 3590, g→c mutation at position 3991 and t→g mutation at position 3992, compared with rat c-Met (X96786). At the amino acid level, the sequence had P→H mutation (mutation from P to H) at position 58, P→A mutation at position 533, A→V mutation at position 730, R→Q mutation at position 808, L→W mutation at position 948, L→P mutation at position 1028, P→Q mutation at position 1068, V→A mutation at position 1197 and V→R mutation at position 1331. The expression vector was prepared in large quantity with Endofree Plasmid Maxi Kit (QIAGEN).

The 3' terminal sequence (from g at 4156 to t at 4185) of the nucleotide sequence shown in SEQ ID NO: 13 is a nucleotide sequence encoding the HA tag.

### (3) Cells Expressing the Gene Encoding Rat c-Met with HA Tag Added to Its C-Terminus

293/EBNA-1 cell strain (Invitrogen) was cultured in 10% FBS (Hyclone)/DMEM (Invitrogen) under 5% CO₂ at 37°C, followed by transfection thereinto of the gene encoding rat c-Met with an HA tag added to its C-terminus using Lipofectamine 2000 (Invitrogen). After one day culture under the same conditions, 50 mM Compound E (Alexis Biochemicals) was added to the culture broth. Cells were cultured for another day under the same conditions. Then, the transfected HEK293 cells were collected with PBS (Sigma) and sonicated with a sonicator (Taitec VP-5S) to disrupt cells. Then, the quantity of protein was determined with Protein Assay Kit (BioRad). Samples (2 µg each) were taken from proteins obtained from Compound E-added cells and Compound E-not added cells, respectively, and subjected to SDS-PAGE, followed by Western blotting with an anti-HA antibody (Roche) (final concentration: 0.2 µg/ml).

### 2. Experimental Results

Fig. 1 shows the results.

In Fig. 1, the left lane represents the sample untreated with Compound E and the right lane represents the sample treated with Compound E. "Full" represents the full-length of c-Met (about 150 kDa). When Compound E (γ-secretase inhibitor) was added, a band around 50 kDa (CTF: C terminal fragment) was accumulated specifically as a c-Met undegraded product. Since this band is almost equal in size to the region spanning from the transmembrane domain of c-Met to its C-terminal site, it has become clear that c-Met is cleaved by γ-secretase in HEK293 cells.

The technical terms used herein are used only for the purpose of illustrating a specific embodiment and not intended to limit the embodiment.

Unless otherwise specifically defined, all technical terms and scientific terms used herein have the same meaning as generally understood by those skilled in the art. Although any methods and materials similar to or equivalent to those described herein may be used in the practice or test of the present invention, those skilled in the art can consult the description provided herein, for preferable methods and materials.

All publications cited herein are incorporated herein by reference in their entirety for the purpose of describing and disclosing, for example, the cell systems, constructs and methods described in the publications that are used in connection with the present invention, or incorporated herein as references with respect to the disclosure of the compound identification method, screening method and methodologies therefor, and composition of the present invention; such publications may be used for the practice of the present invention.

## Claims

1. A method of screening for compounds which affect the processing of c-Met by γ-secretase, comprising the following steps:
(i) contacting a first biological composition containing γ-secretase or a biologically active fragment thereof with a second biological composition containing c-Met in the presence and absence of a candidate compound;
(ii) measuring the cleavage of the c-Met in the presence and absence of the candidate compound;
(iii) selecting those candidate compounds which affect the cleavage of the c-Met by γ-secretase; and
(iv) identifying the candidate compounds selected in step (iii) as compounds which affect the processing of c-Met by γ-secretase.

2. The method of claim 1, further comprising evaluating a candidate compound as an inhibitor for the c-Met degradation activity of γ-secretase when c-Met undegraded product in the presence of said candidate compound was increased relative to c-Met undegraded product in the absence of said candidate compound in the cleavage of c-Met measured in step (ii).

3. The method of claim 1, further comprising evaluating a candidate compound as an accelerator for the c-Met degradation activity of γ-secretase when c-Met undegraded product in the presence of said candidate compound was decreased relative to c-Met undegraded product in the absence of said candidate compound in the cleavage of c-Met measured in step (ii).

4. The method of claim 1, further comprising evaluating a candidate compound as an accelerator for the c-Met degradation activity of γ-secretase when c-Met cleavage product in the presence of said candidate compound was increased relative to c-Met cleavage product in the absence of said candidate compound in the cleavage of c-Met measured in step (ii).

5. The method of claim 1, further comprising evaluating a candidate compound as an inhibitor for the c-Met degradation activity of γ-secretase when c-Met cleavage product in the presence of said candidate compound was decreased relative to c-Met cleavage product in the absence of said candidate compound in the cleavage of c-Met measured in step (ii).

6. The method of any one of claims 1 to 5, further comprising measuring the cleavage ofAPP or a polypeptide comprising an APP γ-secretase cleavage site.

7. The method of any one of claims 1 to 5, further comprising measuring the cleavage of Notch or a polypeptide comprising a Notch γ-secretase cleavage site.

8. A test kit for measuring the processing of c-Met by γ-secretase, comprising a first biological composition containing γ-secretase or a biologically active fragment thereof and a second biological composition containing c-Met.


SEQUENCE LISTING

<110> EISAI R&D MANAGEMENT CO., LTD.

<120> A method of screening using a novel substrate c-Met of
γ-secretase

<130> G09-0094EP

<150> US 60/950,621
<151> 2007-07-19

<150> US 60/951,493
<151> 2007-07-24

<160> 32

<170> PatentIn version 3.4

<210> 1
<211> 4173
<212> DNA
<213> Homo sapiens

<220>
<221> CDS
<222> (1)..(4170)

<400> 1
atg aag gcc ccc gct gtg ctt gca cct ggc atc ctc gtg ctc ctg ttt 48
Met Lys Ala Pro Ala Val Leu Ala Pro Gly Ile Leu Val Leu Leu Phe
1               5                   10                  15

acc ttg gtg cag agg agc aat ggg gag tgt aaa gag gca cta gca aag 96
Thr Leu Val Gln Arg Ser Asn Gly Glu Cys Lys Glu Ala Leu Ala Lys
            20                  25                  30

tcc gag atg aat gtg aat atg aag tat cag ctt ccc aac ttc acc gcg 144
Ser Glu Met Asn Val Asn Met Lys Tyr Gln Leu Pro Asn Phe Thr Ala
        35                  40                  45

gaa aca ccc atc cag aat gtc att cta cat gag cat cac att ttc ctt 192
Glu Thr Pro Ile Gln Asn Val Ile Leu His Glu His His Ile Phe Leu
    50                  55                  60

ggt gcc act aac tac att tat gtt tta aat gag gaa gac ctt cag aag 240
Gly Ala Thr Asn Tyr Ile Tyr Val Leu Asn Glu Glu Asp Leu Gln Lys
65                  70                  75                  80

gtt gct gag tac aag act ggg cct gtg ctg gaa cac cca gat tgt ttc 288
Val Ala Glu Tyr Lys Thr Gly Pro Val Leu Glu His Pro Asp Cys Phe
                85                  90                  95

cca tgt cag gac tgc agc agc aaa gcc aat tta tca gga ggt gtt tgg 336
Pro Cys Gln Asp Cys Ser Ser Lys Ala Asn Leu Ser Gly Gly Val Trp
            100                 105                 110

aaa gat aac atc aac atg gct cta gtt gtc gac acc tac tat gat gat 384
Lys Asp Asn Ile Asn Met Ala Leu Val Val Asp Thr Tyr Tyr Asp Asp
        115                 120                 125

caa ctc att agc tgt ggc agc gtc aac aga ggg acc tgc cag cga cat 432
Gln Leu Ile Ser Cys Gly Ser Val Asn Arg Gly Thr Cys Gln Arg His
    130                 135                 140

gtc ttt ccc cac aat cat act gct gac ata cag tcg gag gtt cac tgc 480
Val Phe Pro His Asn His Thr Ala Asp Ile Gln Ser Glu Val His Cys
145                 150                 155                 160

ata ttc tcc cca cag ata gaa gag ccc agc cag tgt cct gac tgt gtg 528
Ile Phe Ser Pro Gln Ile Glu Glu Pro Ser Gln Cys Pro Asp Cys Val
                165                 170                 175

gtg agc gcc ctg gga gcc aaa gtc ctt tca tct gta aag gac cgg ttc 576
Val Ser Ala Leu Gly Ala Lys Val Leu Ser Ser Val Lys Asp Arg Phe
            180                 185                 190

atc aac ttc ttt gta ggc aat acc ata aat tct tct tat ttc cca gat 624
Ile Asn Phe Phe Val Gly Asn Thr Ile Asn Ser Ser Tyr Phe Pro Asp
        195                 200                 205

cat cca ttg cat tcg ata tca gtg aga agg cta aag gaa acg aaa gat 672
His Pro Leu His Ser Ile Ser Val Arg Arg Leu Lys Glu Thr Lys Asp
    210                 215                 220

ggt ttt atg ttt ttg acg gac cag tcc tac att gat gtt tta cct gag 720
Gly Phe Met Phe Leu Thr Asp Gln Ser Tyr Ile Asp Val Leu Pro Glu
225                 230                 235                 240

ttc aga gat tct tac ccc att aag tat gtc cat gcc ttt gaa agc aac 768
Phe Arg Asp Ser Tyr Pro Ile Lys Tyr Val His Ala Phe Glu Ser Asn
                245                 250                 255

aat ttt att tac ttc ttg acg gtc caa agg gaa act cta gat gct cag 816
Asn Phe Ile Tyr Phe Leu Thr Val Gln Arg Glu Thr Leu Asp Ala Gln
            260                 265                 270

act ttt cac aca aga ata atc agg ttc tgt tcc ata aac tct gga ttg 864
Thr Phe His Thr Arg Ile Ile Arg Phe Cys Ser Ile Asn Ser Gly Leu
        275                 280                 285

cat tcc tac atg gaa atg cct ctg gag tgt att ctc aca gaa aag aga 912
His Ser Tyr Met Glu Met Pro Leu Glu Cys Ile Leu Thr Glu Lys Arg
    290                 295                 300

aaa aag aga tcc aca aag aag gaa gtg ttt aat ata ctt cag gct gcg 960
Lys Lys Arg Ser Thr Lys Lys Glu Val Phe Asn Ile Leu Gln Ala Ala
305                 310                 315                 320

tat gtc agc aag cct ggg gcc cag ctt gct aga caa ata gga gcc agc 1008
Tyr Val Ser Lys Pro Gly Ala Gln Leu Ala Arg Gln Ile Gly Ala Ser
                325                 330                 335

ctg aat gat gac att ctt ttc ggg gtg ttc gca caa agc aag cca gat 1056
Leu Asn Asp Asp Ile Leu Phe Gly Val Phe Ala Gln Ser Lys Pro Asp
            340                 345                 350

tct gcc gaa cca atg gat cga tct gcc atg tgt gca ttc cct atc aaa 1104
Ser Ala Glu Pro Met Asp Arg Ser Ala Met Cys Ala Phe Pro Ile Lys
        355                 360                 365

tat gtc aac gac ttc ttc aac aag atc gtc aac aaa aac aat gtg aga 1152
Tyr Val Asn Asp Phe Phe Asn Lys Ile Val Asn Lys Asn Asn Val Arg
    370                 375                 380

tgt ctc cag cat ttt tac gga ccc aat cat gag cac tgc ttt aat agg 1200
Cys Leu Gln His Phe Tyr Gly Pro Asn His Glu His Cys Phe Asn Arg
385                 390                 395                 400

aca ctt ctg aga aat tca tca ggc tgt gaa gcg cgc cgt gat gaa tat 1248
Thr Leu Leu Arg Asn Ser Ser Gly Cys Glu Ala Arg Arg Asp Glu Tyr
                405                 410                 415

cga aca gag ttt acc aca gct ttg cag cgc gtt gac tta ttc atg ggt 1296
Arg Thr Glu Phe Thr Thr Ala Leu Gln Arg Val Asp Leu Phe Met Gly
            420                 425                 430

caa ttc agc gaa gtc ctc tta aca tct ata tcc acc ttc att aaa gga 1344
Gln Phe Ser Glu Val Leu Leu Thr Ser Ile Ser Thr Phe Ile Lys Gly
        435                 440                 445

gac ctc acc ata gct aat ctt ggg aca tca gag ggt cgc ttc atg cag 1392
Asp Leu Thr Ile Ala Asn Leu Gly Thr Ser Glu Gly Arg Phe Met Gln
    450                 455                 460

gtt gtg gtt tct cga tca gga cca tca acc cct cat gtg aat ttt ctc 1440

Val Val Val Ser Arg Ser Gly Pro Ser Thr Pro His Val Asn Phe Leu
465                 470                 475                 480

ctg gac tcc cat cca gtg tct cca gaa gtg att gtg gag cat aca tta      1488
Leu Asp Ser His Pro Val Ser Pro Glu Val Ile Val Glu His Thr Leu
                485                 490                 495

aac caa aat ggc tac aca ctg gtt atc act ggg aag aag atc acg aag      1536
Asn Gln Asn Gly Tyr Thr Leu Val Ile Thr Gly Lys Lys Ile Thr Lys
            500                 505                 510

atc cca ttg aat ggc ttg ggc tgc aga cat ttc cag tcc tgc agt caa      1584
Ile Pro Leu Asn Gly Leu Gly Cys Arg His Phe Gln Ser Cys Ser Gln
        515                 520                 525

tgc ctc tct gcc cca ccc ttt gtt cag tgt ggc tgg tgc cac gac aaa      1632
Cys Leu Ser Ala Pro Pro Phe Val Gln Cys Gly Trp Cys His Asp Lys
    530                 535                 540

tgt gtg cga tcg gag gaa tgc ctg agc ggg aca tgg act caa cag atc      1680
Cys Val Arg Ser Glu Glu Cys Leu Ser Gly Thr Trp Thr Gln Gln Ile
545                 550                 555                 560

tgt ctg cct gca atc tac aag gtt ttc cca aat agt gca ccc ctt gaa      1728
Cys Leu Pro Ala Ile Tyr Lys Val Phe Pro Asn Ser Ala Pro Leu Glu
                565                 570                 575

gga ggg aca agg ctg acc ata tgt ggc tgg gac ttt gga ttt cgg agg      1776
Gly Gly Thr Arg Leu Thr Ile Cys Gly Trp Asp Phe Gly Phe Arg Arg
            580                 585                 590

aat aat aaa ttt gat tta aag aaa act aga gtt ctc ctt gga aat gag      1824
Asn Asn Lys Phe Asp Leu Lys Lys Thr Arg Val Leu Leu Gly Asn Glu
        595                 600                 605

agc tgc acc ttg act tta agt gag agc acg atg aat aca ttg aaa tgc      1872
Ser Cys Thr Leu Thr Leu Ser Glu Ser Thr Met Asn Thr Leu Lys Cys
    610                 615                 620

aca gtt ggt cct gcc atg aat aag cat ttc aat atg tcc ata att att      1920
Thr Val Gly Pro Ala Met Asn Lys His Phe Asn Met Ser Ile Ile Ile
625                 630                 635                 640

tca aat ggc cac ggg aca aca caa tac agt aca ttc tcc tat gtg gat      1968
Ser Asn Gly His Gly Thr Thr Gln Tyr Ser Thr Phe Ser Tyr Val Asp
                645                 650                 655

cct gta ata aca agt att tcg ccg aaa tac ggt cct atg gct ggt ggc      2016
Pro Val Ile Thr Ser Ile Ser Pro Lys Tyr Gly Pro Met Ala Gly Gly

            660                 665                 670

act tta ctt act tta act gga aat tac cta aac agt ggg aat tct aga 2064
Thr Leu Leu Thr Leu Thr Gly Asn Tyr Leu Asn Ser Gly Asn Ser Arg
        675                 680                 685

cac att tca att ggt gga aaa aca tgt act tta aaa agt gtg tca aac 2112
His Ile Ser Ile Gly Gly Lys Thr Cys Thr Leu Lys Ser Val Ser Asn
    690                 695                 700

agt att ctt gaa tgt tat acc cca gcc caa acc att tca act gag ttt 2160
Ser Ile Leu Glu Cys Tyr Thr Pro Ala Gln Thr Ile Ser Thr Glu Phe
705                 710                 715                 720

gct gtt aaa ttg aaa att gac tta gcc aac cga gag aca agc atc ttc 2208
Ala Val Lys Leu Lys Ile Asp Leu Ala Asn Arg Glu Thr Ser Ile Phe
                725                 730                 735

agt tac cgt gaa gat ccc att gtc tat gaa att cat cca acc aaa tct 2256
Ser Tyr Arg Glu Asp Pro Ile Val Tyr Glu Ile His Pro Thr Lys Ser
            740                 745                 750

ttt att agt ggt ggg agc aca ata aca ggt gtt ggg aaa aac ctg aat 2304
Phe Ile Ser Gly Gly Ser Thr Ile Thr Gly Val Gly Lys Asn Leu Asn
        755                 760                 765

tca gtt agt gtc ccg aga atg gtc ata aat gtg cat gaa gca gga agg 2352
Ser Val Ser Val Pro Arg Met Val Ile Asn Val His Glu Ala Gly Arg
    770                 775                 780

aac ttt aca gtg gca tgt caa cat cgc tct aat tca gag ata atc tgt 2400
Asn Phe Thr Val Ala Cys Gln His Arg Ser Asn Ser Glu Ile Ile Cys
785                 790                 795                 800

tgt acc act cct tcc ctg caa cag ctg aat ctg caa ctc ccc ctg aaa 2448
Cys Thr Thr Pro Ser Leu Gln Gln Leu Asn Leu Gln Leu Pro Leu Lys
                805                 810                 815

acc aaa gcc ttt ttc atg tta gat ggg atc ctt tcc aaa tac ttt gat 2496
Thr Lys Ala Phe Phe Met Leu Asp Gly Ile Leu Ser Lys Tyr Phe Asp
            820                 825                 830

ctc att tat gta cat aat cct gtg ttt aag cct ttt gaa aag cca gtg 2544
Leu Ile Tyr Val His Asn Pro Val Phe Lys Pro Phe Glu Lys Pro Val
        835                 840                 845

atg atc tca atg ggc aat gaa aat gta ctg gaa att aag gga aat gat 2592
Met Ile Ser Met Gly Asn Glu Asn Val Leu Glu Ile Lys Gly Asn Asp
    850                 855                 860

att gac cct gaa gca gtt aaa ggt gaa gtg tta aaa gtt gga aat aag 2640
Ile Asp Pro Glu Ala Val Lys Gly Glu Val Leu Lys Val Gly Asn Lys
865                 870                 875                 880

agc tgt gag aat ata cac tta cat tct gaa gcc gtt tta tgc acg gtc 2688
Ser Cys Glu Asn Ile His Leu His Ser Glu Ala Val Leu Cys Thr Val
                885                 890                 895

ccc aat gac ctg ctg aaa ttg aac agc gag cta aat ata gag tgg aag 2736
Pro Asn Asp Leu Leu Lys Leu Asn Ser Glu Leu Asn Ile Glu Trp Lys
            900                 905                 910

caa gca att tct tca acc gtc ctt gga aaa gta ata gtt caa cca gat 2784
Gln Ala Ile Ser Ser Thr Val Leu Gly Lys Val Ile Val Gln Pro Asp
        915                 920                 925

cag aat ttc aca gga ttg att gct ggt gtt gtc tca ata tca aca gca 2832
Gln Asn Phe Thr Gly Leu Ile Ala Gly Val Val Ser Ile Ser Thr Ala
    930                 935                 940

ctg tta tta cta ctt ggg ttt ttc ctg tgg ctg aaa aag aga aag caa 2880
Leu Leu Leu Leu Leu Gly Phe Phe Leu Trp Leu Lys Lys Arg Lys Gln
945                 950                 955                 960

att aaa gat ctg ggc agt gaa tta gtt cgc tac gat gca aga gta cac 2928
Ile Lys Asp Leu Gly Ser Glu Leu Val Arg Tyr Asp Ala Arg Val His
                965                 970                 975

act cct cat ttg gat agg ctt gta agt gcc cga agt gta agc cca act 2976
Thr Pro His Leu Asp Arg Leu Val Ser Ala Arg Ser Val Ser Pro Thr
            980                 985                 990

aca gaa atg gtt tca aat gaa tct gta gac tac cga gct act ttt cca 3024
Thr Glu Met Val Ser Asn Glu Ser Val Asp Tyr Arg Ala Thr Phe Pro
        995                 1000                1005

gaa gat cag ttt cct aat tca tct cag aac ggt tca tgc cga caa 3069
Glu Asp Gln Phe Pro Asn Ser Ser Gln Asn Gly Ser Cys Arg Gln
    1010                1015                1020

gtg cag tat cct ctg aca gac atg tcc ccc atc cta act agt ggg 3114
Val Gln Tyr Pro Leu Thr Asp Met Ser Pro Ile Leu Thr Ser Gly
    1025                1030                1035

gac tct gat ata tcc agt cca tta ctg caa aat act gtc cac att 3159
Asp Ser Asp Ile Ser Ser Pro Leu Leu Gln Asn Thr Val His Ile
    1040                1045                1050

gac ctc agt gct cta aat cca gag ctg gtc cag gca gtg cag cat 3204
Asp Leu Ser Ala Leu Asn Pro Glu Leu Val Gln Ala Val Gln His
    1055                1060                1065

gta gtg att ggg ccc agt agc ctg att gtg cat ttc aat gaa gtc 3249
Val Val Ile Gly Pro Ser Ser Leu Ile Val His Phe Asn Glu Val
    1070                1075                1080

ata gga aga ggg cat ttt ggt tgt gta tat cat ggg act ttg ttg 3294
Ile Gly Arg Gly His Phe Gly Cys Val Tyr His Gly Thr Leu Leu
    1085                1090                1095

gac aat gat ggc aag aaa att cac tgt gct gtg aaa tcc ttg aac 3339
Asp Asn Asp Gly Lys Lys Ile His Cys Ala Val Lys Ser Leu Asn
    1100                1105                1110

aga atc act gac ata gga gaa gtt tcc caa ttt ctg acc gag gga 3384
Arg Ile Thr Asp Ile Gly Glu Val Ser Gln Phe Leu Thr Glu Gly
    1115                1120                1125

atc atc atg aaa gat ttt agt cat ccc aat gtc ctc tcg ctc ctg 3429
Ile Ile Met Lys Asp Phe Ser His Pro Asn Val Leu Ser Leu Leu
    1130                1135                1140

gga atc tgc ctg cga agt gaa ggg tct ccg ctg gtg gtc cta cca 3474
Gly Ile Cys Leu Arg Ser Glu Gly Ser Pro Leu Val Val Leu Pro
    1145                1150                1155

tac atg aaa cat gga gat ctt cga aat ttc att cga aat gag act 3519
Tyr Met Lys His Gly Asp Leu Arg Asn Phe Ile Arg Asn Glu Thr
    1160                1165                1170

cat aat cca act gta aaa gat ctt att ggc ttt ggt ctt caa gta 3564
His Asn Pro Thr Val Lys Asp Leu Ile Gly Phe Gly Leu Gln Val
    1175                1180                1185

gcc aaa ggc atg aaa tat ctt gca agc aaa aag ttt gtc cac aga 3609
Ala Lys Gly Met Lys Tyr Leu Ala Ser Lys Lys Phe Val His Arg
    1190                1195                1200

gac ttg gct gca aga aac tgt atg ctg gat gaa aaa ttc aca gtc 3654
Asp Leu Ala Ala Arg Asn Cys Met Leu Asp Glu Lys Phe Thr Val
    1205                1210                1215

aag gtt gct gat ttt ggt ctt gcc aga gac atg tat gat aaa gaa 3699
Lys Val Ala Asp Phe Gly Leu Ala Arg Asp Met Tyr Asp Lys Glu
    1220                1225                1230

tac tat agt gta cac aac aaa aca ggt gca aag ctg cca gtg aag 3744

Tyr Tyr Ser Val His Asn Lys Thr Gly Ala Lys Leu Pro Val Lys
    1235                1240                1245

tgg atg gct ttg gaa agt ctg caa act caa aag ttt acc acc aag 3789
Trp Met Ala Leu Glu Ser Leu Gln Thr Gln Lys Phe Thr Thr Lys
    1250                1255                1260

tca gat gtg tgg tcc ttt ggc gtg ctc ctc tgg gag ctg atg aca 3834
Ser Asp Val Trp Ser Phe Gly Val Leu Leu Trp Glu Leu Met Thr
    1265                1270                1275

aga gga gcc cca cct tat cct gac gta aac acc ttt gat ata act 3879
Arg Gly Ala Pro Pro Tyr Pro Asp Val Asn Thr Phe Asp Ile Thr
    1280                1285                1290

gtt tac ttg ttg caa ggg aga aga ctc cta caa ccc gaa tac tgc 3924
Val Tyr Leu Leu Gln Gly Arg Arg Leu Leu Gln Pro Glu Tyr Cys
    1295                1300                1305

cca gac ccc tta tat gaa gta atg cta aaa tgc tgg cac cct aaa 3969
Pro Asp Pro Leu Tyr Glu Val Met Leu Lys Cys Trp His Pro Lys
    1310                1315                1320

gcc gaa atg cgc cca tcc ttt tct gaa ctg gtg tcc cgg ata tca 4014
Ala Glu Met Arg Pro Ser Phe Ser Glu Leu Val Ser Arg Ile Ser
    1325                1330                1335

gcg atc ttc tct act ttc att ggg gag cac tat gtc cat gtg aac 4059
Ala Ile Phe Ser Thr Phe Ile Gly Glu His Tyr Val His Val Asn
    1340                1345                1350

gct act tat gtg aac gta aaa tgt gtc gct ccg tat cct tct ctg 4104
Ala Thr Tyr Val Asn Val Lys Cys Val Ala Pro Tyr Pro Ser Leu
    1355                1360                1365

ttg tca tca gaa gat aac gct gat gat gag gtg gac aca cga cca 4149
Leu Ser Ser Glu Asp Asn Ala Asp Asp Glu Val Asp Thr Arg Pro
    1370                1375                1380

gcc tcc ttc tgg gag aca tca tag 4173
Ala Ser Phe Trp Glu Thr Ser
    1385                1390

<210> 2
<211> 1390
<212> PRT
<213> Homo sapiens

<400> 2

Met Lys Ala Pro Ala Val Leu Ala Pro Gly Ile Leu Val Leu Leu Phe
1               5                   10                  15

Thr Leu Val Gln Arg Ser Asn Gly Glu Cys Lys Glu Ala Leu Ala Lys
            20                  25                  30

Ser Glu Met Asn Val Asn Met Lys Tyr Gln Leu Pro Asn Phe Thr Ala
        35                  40                  45

Glu Thr Pro Ile Gln Asn Val Ile Leu His Glu His His Ile Phe Leu
    50                  55                  60

Gly Ala Thr Asn Tyr Ile Tyr Val Leu Asn Glu Glu Asp Leu Gln Lys
65                  70                  75                  80

Val Ala Glu Tyr Lys Thr Gly Pro Val Leu Glu His Pro Asp Cys Phe
                85                  90                  95

Pro Cys Gln Asp Cys Ser Ser Lys Ala Asn Leu Ser Gly Gly Val Trp
            100                 105                 110

Lys Asp Asn Ile Asn Met Ala Leu Val Val Asp Thr Tyr Tyr Asp Asp
        115                 120                 125

Gln Leu Ile Ser Cys Gly Ser Val Asn Arg Gly Thr Cys Gln Arg His
    130                 135                 140

Val Phe Pro His Asn His Thr Ala Asp Ile Gln Ser Glu Val His Cys
145                 150                 155                 160

Ile Phe Ser Pro Gln Ile Glu Glu Pro Ser Gln Cys Pro Asp Cys Val
                165                 170                 175

Val Ser Ala Leu Gly Ala Lys Val Leu Ser Ser Val Lys Asp Arg Phe
            180                 185                 190

Ile Asn Phe Phe Val Gly Asn Thr Ile Asn Ser Ser Tyr Phe Pro Asp
        195                 200                 205

His Pro Leu His Ser Ile Ser Val Arg Arg Leu Lys Glu Thr Lys Asp
    210                 215                 220

Gly Phe Met Phe Leu Thr Asp Gln Ser Tyr Ile Asp Val Leu Pro Glu
225                 230                 235                 240

Phe Arg Asp Ser Tyr Pro Ile Lys Tyr Val His Ala Phe Glu Ser Asn
                245                 250                 255

Asn Phe Ile Tyr Phe Leu Thr Val Gln Arg Glu Thr Leu Asp Ala Gln
            260                 265                 270

Thr Phe His Thr Arg Ile Ile Arg Phe Cys Ser Ile Asn Ser Gly Leu
        275                 280                 285

His Ser Tyr Met Glu Met Pro Leu Glu Cys Ile Leu Thr Glu Lys Arg
    290                 295                 300

Lys Lys Arg Ser Thr Lys Lys Glu Val Phe Asn Ile Leu Gln Ala Ala
305                 310                 315                 320

Tyr Val Ser Lys Pro Gly Ala Gln Leu Ala Arg Gln Ile Gly Ala Ser
                325                 330                 335

Leu Asn Asp Asp Ile Leu Phe Gly Val Phe Ala Gln Ser Lys Pro Asp
            340                 345                 350

Ser Ala Glu Pro Met Asp Arg Ser Ala Met Cys Ala Phe Pro Ile Lys
        355                 360                 365

Tyr Val Asn Asp Phe Phe Asn Lys Ile Val Asn Lys Asn Asn Val Arg
    370                 375                 380

Cys Leu Gln His Phe Tyr Gly Pro Asn His Glu His Cys Phe Asn Arg
385                 390                 395                 400

Thr Leu Leu Arg Asn Ser Ser Gly Cys Glu Ala Arg Arg Asp Glu Tyr
                405                 410                 415

Arg Thr Glu Phe Thr Thr Ala Leu Gln Arg Val Asp Leu Phe Met Gly
            420                 425                 430

Gln Phe Ser Glu Val Leu Leu Thr Ser Ile Ser Thr Phe Ile Lys Gly
        435                 440                 445

Asp Leu Thr Ile Ala Asn Leu Gly Thr Ser Glu Gly Arg Phe Met Gln
    450                 455                 460

Val Val Val Ser Arg Ser Gly Pro Ser Thr Pro His Val Asn Phe Leu
465                 470                 475                 480

Leu Asp Ser His Pro Val Ser Pro Glu Val Ile Val Glu His Thr Leu
                485                 490                 495

Asn Gln Asn Gly Tyr Thr Leu Val Ile Thr Gly Lys Lys Ile Thr Lys
            500                 505                 510

Ile Pro Leu Asn Gly Leu Gly Cys Arg His Phe Gln Ser Cys Ser Gln
        515                 520                 525

Cys Leu Ser Ala Pro Pro Phe Val Gln Cys Gly Trp Cys His Asp Lys
    530                 535                 540

Cys Val Arg Ser Glu Glu Cys Leu Ser Gly Thr Trp Thr Gln Gln Ile
545                 550                 555                 560

Cys Leu Pro Ala Ile Tyr Lys Val Phe Pro Asn Ser Ala Pro Leu Glu
                565                 570                 575

Gly Gly Thr Arg Leu Thr Ile Cys Gly Trp Asp Phe Gly Phe Arg Arg

            580                 585                 590

Asn Asn Lys Phe Asp Leu Lys Lys Thr Arg Val Leu Leu Gly Asn Glu
        595                 600                 605

Ser Cys Thr Leu Thr Leu Ser Glu Ser Thr Met Asn Thr Leu Lys Cys
    610                 615                 620

Thr Val Gly Pro Ala Met Asn Lys His Phe Asn Met Ser Ile Ile Ile
625                 630                 635                 640

Ser Asn Gly His Gly Thr Thr Gln Tyr Ser Thr Phe Ser Tyr Val Asp
                645                 650                 655

Pro Val Ile Thr Ser Ile Ser Pro Lys Tyr Gly Pro Met Ala Gly Gly
            660                 665                 670

Thr Leu Leu Thr Leu Thr Gly Asn Tyr Leu Asn Ser Gly Asn Ser Arg
        675                 680                 685

His Ile Ser Ile Gly Gly Lys Thr Cys Thr Leu Lys Ser Val Ser Asn
    690                 695                 700

Ser Ile Leu Glu Cys Tyr Thr Pro Ala Gln Thr Ile Ser Thr Glu Phe
705                 710                 715                 720

Ala Val Lys Leu Lys Ile Asp Leu Ala Asn Arg Glu Thr Ser Ile Phe
                725                 730                 735

Ser Tyr Arg Glu Asp Pro Ile Val Tyr Glu Ile His Pro Thr Lys Ser
            740                 745                 750

Phe Ile Ser Gly Gly Ser Thr Ile Thr Gly Val Gly Lys Asn Leu Asn
        755                 760                 765

Ser Val Ser Val Pro Arg Met Val Ile Asn Val His Glu Ala Gly Arg
    770                 775                 780

Asn Phe Thr Val Ala Cys Gln His Arg Ser Asn Ser Glu Ile Ile Cys
785                 790                 795                 800

Cys Thr Thr Pro Ser Leu Gln Gln Leu Asn Leu Gln Leu Pro Leu Lys
                805                 810                 815

Thr Lys Ala Phe Phe Met Leu Asp Gly Ile Leu Ser Lys Tyr Phe Asp
            820                 825                 830

Leu Ile Tyr Val His Asn Pro Val Phe Lys Pro Phe Glu Lys Pro Val
        835                 840                 845

Met Ile Ser Met Gly Asn Glu Asn Val Leu Glu Ile Lys Gly Asn Asp
    850                 855                 860

Ile Asp Pro Glu Ala Val Lys Gly Glu Val Leu Lys Val Gly Asn Lys
865                 870                 875                 880

Ser Cys Glu Asn Ile His Leu His Ser Glu Ala Val Leu Cys Thr Val
                885                 890                 895

Pro Asn Asp Leu Leu Lys Leu Asn Ser Glu Leu Asn Ile Glu Trp Lys
            900                 905                 910

Gln Ala Ile Ser Ser Thr Val Leu Gly Lys Val Ile Val Gln Pro Asp
        915                 920                 925

Gln Asn Phe Thr Gly Leu Ile Ala Gly Val Val Ser Ile Ser Thr Ala
    930                 935                 940

Leu Leu Leu Leu Leu Gly Phe Phe Leu Trp Leu Lys Lys Arg Lys Gln
945                 950                 955                 960

Ile Lys Asp Leu Gly Ser Glu Leu Val Arg Tyr Asp Ala Arg Val His
                965                 970                 975

Thr Pro His Leu Asp Arg Leu Val Ser Ala Arg Ser Val Ser Pro Thr
            980                 985                 990

Thr Glu Met Val Ser Asn Glu Ser Val Asp Tyr Arg Ala Thr Phe Pro
        995                 1000                1005

Glu Asp Gln Phe Pro Asn Ser Ser Gln Asn Gly Ser Cys Arg Gln
    1010                1015                1020

Val Gln Tyr Pro Leu Thr Asp Met Ser Pro Ile Leu Thr Ser Gly
    1025                1030                1035

Asp Ser Asp Ile Ser Ser Pro Leu Leu Gln Asn Thr Val His Ile
    1040                1045                1050

Asp Leu Ser Ala Leu Asn Pro Glu Leu Val Gln Ala Val Gln His
    1055                1060                1065

Val Val Ile Gly Pro Ser Ser Leu Ile Val His Phe Asn Glu Val
    1070                1075                1080

Ile Gly Arg Gly His Phe Gly Cys Val Tyr His Gly Thr Leu Leu
    1085                1090                1095

Asp Asn Asp Gly Lys Lys Ile His Cys Ala Val Lys Ser Leu Asn
    1100                1105                1110

Arg Ile Thr Asp Ile Gly Glu Val Ser Gln Phe Leu Thr Glu Gly
    1115                1120                1125

Ile Ile Met Lys Asp Phe Ser His Pro Asn Val Leu Ser Leu Leu
    1130                1135                1140

Gly Ile Cys Leu Arg Ser Glu Gly Ser Pro Leu Val Val Leu Pro
    1145                1150                1155

Tyr Met Lys His Gly Asp Leu Arg Asn Phe Ile Arg Asn Glu Thr
    1160                1165                1170

His Asn Pro Thr Val Lys Asp Leu Ile Gly Phe Gly Leu Gln Val
    1175                1180                1185

Ala Lys Gly Met Lys Tyr Leu Ala Ser Lys Lys Phe Val His Arg
    1190                1195                1200

Asp Leu Ala Ala Arg Asn Cys Met Leu Asp Glu Lys Phe Thr Val
    1205                1210                1215

Lys Val Ala Asp Phe Gly Leu Ala Arg Asp Met Tyr Asp Lys Glu
    1220                1225                1230

Tyr Tyr Ser Val His Asn Lys Thr Gly Ala Lys Leu Pro Val Lys
    1235                1240                1245

Trp Met Ala Leu Glu Ser Leu Gln Thr Gln Lys Phe Thr Thr Lys
    1250                1255                1260

Ser Asp Val Trp Ser Phe Gly Val Leu Leu Trp Glu Leu Met Thr
    1265                1270                1275

Arg Gly Ala Pro Pro Tyr Pro Asp Val Asn Thr Phe Asp Ile Thr
    1280                1285                1290

Val Tyr Leu Leu Gln Gly Arg Arg Leu Leu Gln Pro Glu Tyr Cys
    1295                1300                1305

Pro Asp Pro Leu Tyr Glu Val Met Leu Lys Cys Trp His Pro Lys
    1310                1315                1320

Ala Glu Met Arg Pro Ser Phe Ser Glu Leu Val Ser Arg Ile Ser
    1325                1330                1335

Ala Ile Phe Ser Thr Phe Ile Gly Glu His Tyr Val His Val Asn

1340                1345                1350

Ala Thr Tyr Val Asn Val Lys Cys Val Ala Pro Tyr Pro Ser Leu
    1355                1360                1365

Leu Ser Ser Glu Asp Asn Ala Asp Asp Glu Val Asp Thr Arg Pro
    1370                1375                1380

Ala Ser Phe Trp Glu Thr Ser
    1385                1390

<210> 3
<211> 4173
<212> DNA
<213> Homo sapiens

<220>
<221> CDS
<222> (1)..(4170)

<400> 3
atg aag gcc ccc gct gtg ctt gca cct ggc atc ctc gtg ctc ctg ttt      48
acc ttg gtg cag agg agc aat ggg gag tgt aaa gag gca cta gca aag      96
tcc gag atg aat gtg aat atg aag tat cag ctt ccc aac ttc acc gcg      144
gaa aca ccc atc cag aat gtc att cta cat gag cat cac att ttc ctt      192
ggt gcc act aac tac att tat gtt tta aat gag gaa gac ctt cag aag      240
gtt gct gag tac aag act ggg cct gtg ctg gaa cac cca gat tgt ttc      288
cca tgt cag gac tgc agc agc aaa gcc aat tta tca gga ggt gtt tgg 336
aaa gat aac atc aac atg gct cta gtt gtc gac acc tac tat gat gat 384
caa ctc att agc tgt ggc agc gtc aac aga ggg acc tgc cag cga cat 432
gtc ttt ccc cac aat cat act gct gac ata cag tcg gag gtt cac tgc 480
ata ttc tcc cca cag ata gaa gag ccc agc cag tgt cct gac tgt gtg 528
gtg agc gcc ctg gga gcc aaa gtc ctt tca tct gta aag gac cgg ttc 576
atc aac ttc ttt gta ggc aat acc ata aat tct tct tat ttc cca gat 624
cat cca ttg cat tcg ata tca gtg aga agg cta aag gaa acg aaa gat 672
ggt ttt atg ttt ttg acg gac cag tcc tac att gat gtt tta cct gag 720
ttc aga gat tct tac ccc att aag tat gtc cat gcc ttt gaa agc aac 768
aat ttt att tac ttc ttg acg gtc caa agg gaa act cta gat gct cag 816
act ttt cac aca aga ata atc agg ttc tgt tcc ata aac tct gga ttg 864
cat tcc tac atg gaa atg cct ctg gag tgt att ctc aca gaa aag aga 912
aaa aag aga tcc aca aag aag gaa gtg ttt aat ata ctt cag gct gcg 960
tat gtc agc aag cct ggg gcc cag ctt gct aga caa ata gga gcc agc 1008
ctg aat gat gac att ctt ttc ggg gtg ttc gca caa agc aag cca gat 1056
tct gcc gaa cca atg gat cga tct gcc atg tgt gca ttc cct atc aaa 1104
tat gtc aac gac ttc ttc aac aag atc gtc aac aaa aac aat gtg aga 1152
tgt ctc cag cat ttt tac gga ccc aat cat gag cac tgc ttt aat agg 1200
aca ctt ctg aga aat tca tca ggc tgt gaa gcg cgc cgt gat gaa tat 1248
cga aca gag ttt acc aca gct ttg cag cgc gtt gac tta ttc atg ggt 1296
caa ttc agc gaa gtc ctc tta aca tct ata tcc acc ttc att aaa gga 1344
gac ctc acc ata gct aat ctt ggg aca tca gag ggt cgc ttc atg cag 1392
gtt gtg gtt tct cga tca gga cca tca acc cct cat gtg aat ttt ctc 1440
ctg gac tcc cat cca gtg tct cca gaa gtg att gtg gag cat aca tta 1488
aac caa aat ggc tac aca ctg gtt atc act ggg aag aag atc acg aag 1536
atc cca ttg aat ggc ttg ggc tgc aga cat ttc cag tcc tgc agt caa 1584
tgc ctc tct gcc cca ccc ttt gtt cag tgt ggc tgg tgc cac gac aaa 1632
tgt gtg cga tcg gag gaa tgc ctg agc ggg aca tgg act caa cag atc 1680
tgt ctg cct gca atc tac aag gtt ttc cca aat agt gca ccc ctt gaa 1728
gga ggg aca agg ctg acc ata tgt ggc tgg gac ttt gga ttt cgg agg 1776
aat aat aaa ttt gat tta aag aaa act aga gtt ctc ctt gga aat gag 1824
agc tgc acc ttg act tta agt gag agc acg atg aat aca ttg aaa tgc 1872
aca gtt ggt cct gcc atg aat aag cat ttc aat atg tcc ata att att 1920
tca aat ggc cac ggg aca aca caa tac agt aca ttc tcc tat gtg gat 1968
cct gta ata aca agt att tcg ccg aaa tac ggt cct atg gct ggt ggc 2016
act tta ctt act tta act gga aat tac cta aac agt ggg aat tct aga 2064
cac att tca att ggt gga aaa aca tgt act tta aaa agt gtg tca aac 2112
agt att ctt gaa tgt tat acc cca gcc caa acc att tca act gag ttt 2160
gct gtt aaa ttg aaa att gac tta gcc aac cga gag aca agc atc ttc 2208
agt tac cgt gaa gat ccc att gtc tat gaa att cat cca acc aaa tct 2256
ttt att agt ggt ggg agc aca ata aca ggt gtt ggg aaa aac ctg aat 2304
tca gtt agt gtc ccg aga atg gtc ata aat gtg cat gaa gca gga agg 2352
aac ttt aca gtg gca tgt caa cat cgc tct aat tca gag ata atc tgt 2400
tgt acc act cct tcc ctg caa cag ctg aat ctg caa ctc ccc ctg aaa 2448
acc aaa gcc ttt ttc atg tta gat ggg atc ctt tcc aaa tac ttt gat 2496
ctc att tat gta cat aat cct gtg ttt aag cct ttt gaa aag cca gtg 2544
atg atc tca atg ggc aat gaa aat gta ctg gaa att aag gga aat gat 2592
att gac cct gaa gca gtt aaa ggt gaa gtg tta aaa gtt gga aat aag 2640
agc tgt gag aat ata cac tta cat tct gaa gcc gtt tta tgc acg gtc 2688
ccc aat gac ctg ctg aaa ttg aac agc gag cta aat ata gag tgg aag 2736
caa gca att tct tca acc gtc ctt gga aaa gta ata gtt caa cca gat 2784
cag aat ttc aca gga ttg att gct ggt gtt gtc tca ata tca aca gca 2832
ctg tta tta cta ctt ggg ttt ttc ctg tgg ctg aaa aag aga aag caa 2880
att aaa gat ctg ggc agt gaa tta gtt cgc tac gat gca aga gta cac 2928
act cct cat ttg gat agg ctt gta agt gcc cga agt gta agc cca act 2976
aca gaa atg gtt tca aat gaa tct gta gac tac cga gct act ttt cca 3024
gaa gat cag ttt cct aat tca tct cag aac ggt tca tgc cga caa 3069
gtg cag tat cct ctg aca gac atg tcc ccc atc cta act agt ggg 3114
gac tct gat ata tcc agt cca tta ctg caa aat act gtc cac att 3159
gac ctc agt gct cta aat cca gag ctg gtc cag gca gtg cag cat 3204
gta gtg att ggg ccc agt agc ctg att gtg cat ttc aat gaa gtc 3249
ata gga aga ggg cat ttt ggt tgt gta tat cat ggg act ttg ttg 3294
gac aat gat ggc aag aaa att cac tgt gct gtg aaa tcc ttg aac 3339
aga atc act gac ata gga gaa gtt tcc caa ttt ctg acc gag gga 3384
atc atc atg aaa gat ttt agt cat ccc aat gtc ctc tcg ctc ctg 3429
gga atc tgc ctg cga agt gaa ggg tct ccg ctg gtg gtc cta cca 3474
tac atg aaa cat gga gat ctt cga aat ttc att cga aat gag act 3519
cat aat cca act gta aaa gat ctt att ggc ttt ggt ctt caa gta 3564
gcc aaa ggc atg aaa tat ctt gca agc aaa aag ttt gtc cac aga 3609
gac ttg gct gca aga aac tgt atg ctg gat gaa aaa ttc aca gtc 3654
aag gtt gct gat ttt ggt ctt gcc aga gac atg tat gat aaa gaa 3699
tac tat agt gta cac aac aaa aca ggt gca aag ctg cca gtg aag 3744
tgg atg gct ttg gaa agt ctg caa act caa aag ttt acc acc aag 3789
tca gat gtg tgg tcc ttt ggc gtc gtc ctc tgg gag ctg atg aca 3834
aga gga gcc cca cct tat cct gac gta aac acc ttt gat ata act 3879
gtt tac ttg ttg caa ggg aga aga ctc cta caa ccc gaa tac tgc 3924
cca gac ccc tta tat gaa gta atg cta aaa tgc tgg cac cct aaa 3969
gcc gaa atg cgc cca tcc ttt tct gaa ctg gtg tcc cgg ata tca 4014
gcg atc ttc tct act ttc att ggg gag cac tat gtc cat gtg aac 4059
gct act tat gtg aac gta aaa tgt gtc gct ccg tat cct tct ctg 4104
ttg tca tca gaa gat aac gct gat gat gag gtg gac aca cga cca 4149
gcc tcc ttc tgg gag aca tca tag 4173

<210> 4
<211> 1390
<212> PRT
<213> Homo sapiens

<400> 4

<210> 5
<211> 4146
<212> DNA
<213> Macaca mulatta

<220>
<221> CDS
<222> (1)..(4143)

<400> 5
atg aag gcc ccc gct gtg ctt gta cct ggc atc ctc gtg ctc ctg ttt      48
Met Lys Ala Pro Ala Val Leu Val Pro Gly Ile Leu Val Leu Leu Phe
1               5                   10                  15

acc ttg gtg cag agg agc aat ggg gag tgt aaa gag gca cta gca aag      96
Thr Leu Val Gln Arg Ser Asn Gly Glu Cys Lys Glu Ala Leu Ala Lys
            20                  25                  30

tcc gag atg aat gtg aat atg aag tat cag ctt ccc aac ttc acc gcg      144
Ser Glu Met Asn Val Asn Met Lys Tyr Gln Leu Pro Asn Phe Thr Ala
        35                  40                  45

gaa aca gcc atc cag aat gtc att cta cat gag cat cac att ttc ctc      192
Glu Thr Ala Ile Gln Asn Val Ile Leu His Glu His His Ile Phe Leu
    50                  55                  60

ggt gcc acc aac tac att tat gtt tta aat gag gaa gac ctt cag aag      240
Gly Ala Thr Asn Tyr Ile Tyr Val Leu Asn Glu Glu Asp Leu Gln Lys
65                  70                  75                  80

gtt gct gag tac aag acc ggg cct gtg ctg gaa cac cca gat tgt ttc      288
Val Ala Glu Tyr Lys Thr Gly Pro Val Leu Glu His Pro Asp Cys Phe
                85                  90                  95

ccg tgt cag gac tgc agc agc aaa gcc aat tta tca gga ggc gtt tgg      336
Pro Cys Gln Asp Cys Ser Ser Lys Ala Asn Leu Ser Gly Gly Val Trp
            100                 105                 110

aaa gat aac atc aac atg gct ctg gtt gtc gac acc tac tat gat gat     384
Lys Asp Asn Ile Asn Met Ala Leu Val Val Asp Thr Tyr Tyr Asp Asp
        115                 120                 125

caa ctc att agc tgt ggc agc gtc aac aga ggg acc tgc cag cga cat     432
Gln Leu Ile Ser Cys Gly Ser Val Asn Arg Gly Thr Cys Gln Arg His
    130                 135                 140

gtc ttt ccc cat aat cat act gct gac ata cag tcg gag gtt cac tgc     480
Val Phe Pro His Asn His Thr Ala Asp Ile Gln Ser Glu Val His Cys
145                 150                 155                 160

ata ttc tcc cca cag ata gaa gag ccc aac cag tgc cct gac tgt gtg     528
Ile Phe Ser Pro Gln Ile Glu Glu Pro Asn Gln Cys Pro Asp Cys Val
                165                 170                 175

gtg agc gcc ctg gga gcc aaa gtc ctt tca tct gta aag gac cgg ttc     576
Val Ser Ala Leu Gly Ala Lys Val Leu Ser Ser Val Lys Asp Arg Phe
            180                 185                 190

atc aac ttc ttt gta ggc aat acc ata aat tct tct tat ttc cca cat     624
Ile Asn Phe Phe Val Gly Asn Thr Ile Asn Ser Ser Tyr Phe Pro His
        195                 200                 205

cat ccg ttg cat tcg ata tca gtg aga agg cta aag gaa acg aaa gat     672
His Pro Leu His Ser Ile Ser Val Arg Arg Leu Lys Glu Thr Lys Asp
    210                 215                 220

ggt ttt atg ttt ttg acg gac cag tcc tac att gat gtt tta cct gag     720
Gly Phe Met Phe Leu Thr Asp Gln Ser Tyr Ile Asp Val Leu Pro Glu
225                 230                 235                 240

ttc aga gat tct tac ccc att aag tac atc cat gct ttt gaa agc aac     768
Phe Arg Asp Ser Tyr Pro Ile Lys Tyr Ile His Ala Phe Glu Ser Asn
                245                 250                 255

aat ttt att tac ttc ttg acg gtc caa agg gaa act cta aat gct cag     816
Asn Phe Ile Tyr Phe Leu Thr Val Gln Arg Glu Thr Leu Asn Ala Gln
            260                 265                 270

act ttt cac aca aga ata atc agg ttc tgt tcc tta aac tct gga ttg     864
Thr Phe His Thr Arg Ile Ile Arg Phe Cys Ser Leu Asn Ser Gly Leu
        275                 280                 285

cac tcc tac atg gaa atg cct cta gag tgt att ctc aca gaa aag aga     912
His Ser Tyr Met Glu Met Pro Leu Glu Cys Ile Leu Thr Glu Lys Arg
    290                 295                 300

aaa aag aga tcc aca aag aag gaa gtg ttt aat atc ctt cag gct gca 960
Lys Lys Arg Ser Thr Lys Lys Glu Val Phe Asn Ile Leu Gln Ala Ala
305                 310                 315                 320

tat gtc agc aag ccc ggg gcc cag ctt gct aga caa ata gga gcc agc 1008
Tyr Val Ser Lys Pro Gly Ala Gln Leu Ala Arg Gln Ile Gly Ala Ser
                325                 330                 335

ctg aat gat gac att ctc ttt ggg gtg ttc gca caa agc aag cca gat 1056
Leu Asn Asp Asp Ile Leu Phe Gly Val Phe Ala Gln Ser Lys Pro Asp
            340                 345                 350

tct gcc gaa cca atg gat cga tct gca atg tgt gca ttc cct atc aaa 1104
Ser Ala Glu Pro Met Asp Arg Ser Ala Met Cys Ala Phe Pro Ile Lys
        355                 360                 365

tat gtc aac gac ttc ttc aac aag atc gtc aac aaa aac aat gtg aga 1152
Tyr Val Asn Asp Phe Phe Asn Lys Ile Val Asn Lys Asn Asn Val Arg
    370                 375                 380

tgt ctc cag cat ttt tat gga ccc aat cat gag cac tgt ttt aat agg 1200
Cys Leu Gln His Phe Tyr Gly Pro Asn His Glu His Cys Phe Asn Arg
385                 390                 395                 400

aca ctt ctg aga aat tca tca ggc tgt gaa gcg cgc cgt gat gaa tat 1248
Thr Leu Leu Arg Asn Ser Ser Gly Cys Glu Ala Arg Arg Asp Glu Tyr
                405                 410                 415

cga gca gag ttt acc aca gct ttg cag cgc gtt gac tta ttc atg ggt 1296
Arg Ala Glu Phe Thr Thr Ala Leu Gln Arg Val Asp Leu Phe Met Gly
            420                 425                 430

caa ttc agc gaa gtc ctc tta aca tct ata tcc acc ttc gtg aaa gga 1344
Gln Phe Ser Glu Val Leu Leu Thr Ser Ile Ser Thr Phe Val Lys Gly
        435                 440                 445

gac ctc acc atc gct aat ctt ggg aca tca gag ggt cgc ttc atg cag 1392
Asp Leu Thr Ile Ala Asn Leu Gly Thr Ser Glu Gly Arg Phe Met Gln
    450                 455                 460

gtt gtg gtt tct cga tca gga cca tca acc cct cat gtg aat ttt ctc 1440
Val Val Val Ser Arg Ser Gly Pro Ser Thr Pro His Val Asn Phe Leu
465                 470                 475                 480

ctg gac tct cac ccg gtg tct cca gaa gtg att gtg gag cat cca tta 1488
Leu Asp Ser His Pro Val Ser Pro Glu Val Ile Val Glu His Pro Leu
                485                 490                 495

aac cag aat ggc tac aca ctg gtt gtc acg ggg aag aag atc acc aag 1536

Asn Gln Asn Gly Tyr Thr Leu Val Val Thr Gly Lys Lys Ile Thr Lys
            500                 505                 510

atc cca ttg aat ggc ttg ggc tgc aga cat ttc cag tcc tgc agt cag 1584
Ile Pro Leu Asn Gly Leu Gly Cys Arg His Phe Gln Ser Cys Ser Gln
        515                 520                 525

tgc ctc tct gcc cca ccc ttt gtg cag tgt ggc tgg tgc cac gac aaa 1632
Cys Leu Ser Ala Pro Pro Phe Val Gln Cys Gly Trp Cys His Asp Lys
    530                 535                 540

tgt gtg cga tcg gag gag tgc ccc agt ggg aca tgg act caa cag atc 1680
Cys Val Arg Ser Glu Glu Cys Pro Ser Gly Thr Trp Thr Gln Gln Ile
545                 550                 555                 560

tgt ctg cct gca atc tac aag gtt ttc cca act agt gca ccc ctt gaa 1728
Cys Leu Pro Ala Ile Tyr Lys Val Phe Pro Thr Ser Ala Pro Leu Glu
                565                 570                 575

gga ggg aca aga ctg acc ata tgt ggc tgg gac ttt gga ttt cgg agg 1776
Gly Gly Thr Arg Leu Thr Ile Cys Gly Trp Asp Phe Gly Phe Arg Arg
            580                 585                 590

aat aat aaa ttt gat tta aag aaa act aga gtt ctc ctt gga aat gag 1824
Asn Asn Lys Phe Asp Leu Lys Lys Thr Arg Val Leu Leu Gly Asn Glu
        595                 600                 605

agc tgc acc ttg act tta agt gag agc acg atg aat aca ttg aaa tgc 1872
Ser Cys Thr Leu Thr Leu Ser Glu Ser Thr Met Asn Thr Leu Lys Cys
    610                 615                 620

aca gtt ggt cct gcc atg aat aaa cat ttc aat atg tcc ata att att 1920
Thr Val Gly Pro Ala Met Asn Lys His Phe Asn Met Ser Ile Ile Ile
625                 630                 635                 640

tca aat ggc cat ggg aca aca caa tac agt aca ttt tcc tat gtg gat 1968
Ser Asn Gly His Gly Thr Thr Gln Tyr Ser Thr Phe Ser Tyr Val Asp
                645                 650                 655

cct ata ata aca agt att tcg cca aaa tat ggc cct atg gct ggt ggc 2016
Pro Ile Ile Thr Ser Ile Ser Pro Lys Tyr Gly Pro Met Ala Gly Gly
            660                 665                 670

act tta ctt act tta act gga aat tac cta aac agt ggg aat tct aga 2064
Thr Leu Leu Thr Leu Thr Gly Asn Tyr Leu Asn Ser Gly Asn Ser Arg
        675                 680                 685

cac att tca att ggt gga aaa aca tgt act tta aaa agt gtg tca aac 2112
His Ile Ser Ile Gly Gly Lys Thr Cys Thr Leu Lys Ser Val Ser Asn

690                 695                 700

agt att ctc gaa tgt tat acc cca gcc caa acc att tca act gag ttt      2160
Ser Ile Leu Glu Cys Tyr Thr Pro Ala Gln Thr Ile Ser Thr Glu Phe
705                 710                 715                 720

gct gtt aaa ttg aaa att gac tta gcc aac cga gag aca agc atc ttc      2208
Ala Val Lys Leu Lys Ile Asp Leu Ala Asn Arg Glu Thr Ser Ile Phe
                725                 730                 735

agt tac cgg gaa gac ccc att gtc tat gaa att cat cca acc aaa tct      2256
Ser Tyr Arg Glu Asp Pro Ile Val Tyr Glu Ile His Pro Thr Lys Ser
            740                 745                 750

ttt att agt ggt ggg agc aca ata aca ggt gtt ggg aaa aac ctg cat      2304
Phe Ile Ser Gly Gly Ser Thr Ile Thr Gly Val Gly Lys Asn Leu His
        755                 760                 765

tca gtt agt gtc ccg agg atg gtc ata aat gtg cat gaa gca gga agg      2352
Ser Val Ser Val Pro Arg Met Val Ile Asn Val His Glu Ala Gly Arg
    770                 775                 780

aac ttt aca gtg gca tgt cag cat cgc tct aat tca gag ata atc tgc      2400
Asn Phe Thr Val Ala Cys Gln His Arg Ser Asn Ser Glu Ile Ile Cys
785                 790                 795                 800

tgt acc act cct tcc ctg caa cag ctg aat ctg caa ctc cct ctg aaa      2448
Cys Thr Thr Pro Ser Leu Gln Gln Leu Asn Leu Gln Leu Pro Leu Lys
                805                 810                 815

acc aaa gcc ttt ttc atg tta gat ggg atc ctt tcc aaa tac ttt gat      2496
Thr Lys Ala Phe Phe Met Leu Asp Gly Ile Leu Ser Lys Tyr Phe Asp
            820                 825                 830

ctc att tat gta cat aat cct gtg ttt aag cct ttt gaa aag cca gta      2544
Leu Ile Tyr Val His Asn Pro Val Phe Lys Pro Phe Glu Lys Pro Val
        835                 840                 845

atg atc tca atg ggc aat gaa aat gta ctg gaa att aag gga aat gat      2592
Met Ile Ser Met Gly Asn Glu Asn Val Leu Glu Ile Lys Gly Asn Asp
    850                 855                 860

att gac cct gaa gca gtt aaa ggt gaa gtg tta aaa gtt gga aat aag      2640
Ile Asp Pro Glu Ala Val Lys Gly Glu Val Leu Lys Val Gly Asn Lys
865                 870                 875                 880

agc tgt gag aat ata cac tta cat tct gaa gcc gtt tta tgc acg gtc      2688
Ser Cys Glu Asn Ile His Leu His Ser Glu Ala Val Leu Cys Thr Val
                885                 890                 895

ccc aat gac ctg ctg aaa ttg aac agc gag cta aat ata gag tgg aag 2736
Pro Asn Asp Leu Leu Lys Leu Asn Ser Glu Leu Asn Ile Glu Trp Lys
            900                 905                 910

caa gca att tct tca acc gtc ctt gga aaa gta ata gtt caa cca gat 2784
Gln Ala Ile Ser Ser Thr Val Leu Gly Lys Val Ile Val Gln Pro Asp
        915                 920                 925

cag aat ttc acc gga ttg att gct ggt gtt gtc tca ata tca ata gca 2832
Gln Asn Phe Thr Gly Leu Ile Ala Gly Val Val Ser Ile Ser Ile Ala
    930                 935                 940

ctc tta cta cta ctt ggg ctt ttc ctg tgg ctg aaa aag aga aag caa 2880
Leu Leu Leu Leu Leu Gly Leu Phe Leu Trp Leu Lys Lys Arg Lys Gln
945                 950                 955                 960

att aaa gat ctg ggc agt gaa tta gtt cgc tat gat gca aga gta cac 2928
Ile Lys Asp Leu Gly Ser Glu Leu Val Arg Tyr Asp Ala Arg Val His
                965                 970                 975

act cct cat ttg gat agg ctt gta agt gcc cga agt gta agc cca aca 2976
Thr Pro His Leu Asp Arg Leu Val Ser Ala Arg Ser Val Ser Pro Thr
            980                 985                 990

aca gaa atg gtt tca aat gaa tct gta gac tac cga gct act ttt cca 3024
Thr Glu Met Val Ser Asn Glu Ser Val Asp Tyr Arg Ala Thr Phe Pro
        995                 1000                1005

gaa gat cag ttt cct aat tca tct cag aac ggt tca tgc cga caa 3069
Glu Asp Gln Phe Pro Asn Ser Ser Gln Asn Gly Ser Cys Arg Gln
    1010                1015                1020

gtg cag tat cct ctg aca gac atg tcc ccc atc ctg act agt ggg 3114
Val Gln Tyr Pro Leu Thr Asp Met Ser Pro Ile Leu Thr Ser Gly
    1025                1030                1035

gac tct gat ata tcc agt cca tta ctg caa aac act gtc cac att 3159
Asp Ser Asp Ile Ser Ser Pro Leu Leu Gln Asn Thr Val His Ile
    1040                1045                1050

gac ctc agc gct cta aat cca gag ctg gtc cag gca gtg cag cat 3204
Asp Leu Ser Ala Leu Asn Pro Glu Leu Val Gln Ala Val Gln His
    1055                1060                1065

gta gtg att ggg ccc agt agc ctg att gtg cat ttc aat gaa gtc 3249
Val Val Ile Gly Pro Ser Ser Leu Ile Val His Phe Asn Glu Val
    1070                1075                1080

ata gga aga ggg cat ttt ggt tgt gta tat cat ggg act ttg ttg 3294
Ile Gly Arg Gly His Phe Gly Cys Val Tyr His Gly Thr Leu Leu
    1085                1090                1095

gac aat gat ggc aag aaa att cac tgt gct gtg aaa tcc ttg aac 3339
Asp Asn Asp Gly Lys Lys Ile His Cys Ala Val Lys Ser Leu Asn
    1100                1105                1110

aga atc act gac ata gga gaa gtt tcc cag ttt ctg acc gag gga 3384
Arg Ile Thr Asp Ile Gly Glu Val Ser Gln Phe Leu Thr Glu Gly
    1115                1120                1125

atc atc atg aaa gat ttt agt cat ccc aat gtc ctc tcg ctc ctg 3429
Ile Ile Met Lys Asp Phe Ser His Pro Asn Val Leu Ser Leu Leu
    1130                1135                1140

gga atc tgc ctg cga agt gaa ggg tct ccg ctg gtg gtc cta ccc 3474
Gly Ile Cys Leu Arg Ser Glu Gly Ser Pro Leu Val Val Leu Pro
    1145                1150                1155

tac atg aaa cat gga gat ctt cga aat ttc att cga aat gag act 3519
Tyr Met Lys His Gly Asp Leu Arg Asn Phe Ile Arg Asn Glu Thr
    1160                1165                1170

cat aat cca act gta aaa gat ctt att ggc ttt ggt ctt caa gta 3564
His Asn Pro Thr Val Lys Asp Leu Ile Gly Phe Gly Leu Gln Val
    1175                1180                1185

gcc aaa ggc atg aaa tat ctt gca agc aaa aag ttt gtc cac aga 3609
Ala Lys Gly Met Lys Tyr Leu Ala Ser Lys Lys Phe Val His Arg
    1190                1195                1200

gac ttg gct gca aga aac tgt atg ctg gat gaa aaa ttc aca gtc 3654
Asp Leu Ala Ala Arg Asn Cys Met Leu Asp Glu Lys Phe Thr Val
    1205                1210                1215

aag gtt gct gat ttt ggt ctt gcc aga gac atg tat gat aaa gaa 3699
Lys Val Ala Asp Phe Gly Leu Ala Arg Asp Met Tyr Asp Lys Glu
    1220                1225                1230

tac tat agt gtg cac aac aaa aca ggt gca aag ctg cca gtg aag 3744
Tyr Tyr Ser Val His Asn Lys Thr Gly Ala Lys Leu Pro Val Lys
    1235                1240                1245

tgg atg gct ttg gaa agt ctg caa act caa aag ttt acc acc aag 3789
Trp Met Ala Leu Glu Ser Leu Gln Thr Gln Lys Phe Thr Thr Lys
    1250                1255                1260

tca gat gtg tgg tcc ttt ggt gtg ctc ctc tgg gag ctc atg aca 3834

Ser Asp Val Trp Ser Phe Gly Val Leu Leu Trp Glu Leu Met Thr
    1265                1270                1275

aga gga gcc cca ccc tat cct gac gtg aac acc ttt gat ata act 3879
Arg Gly Ala Pro Pro Tyr Pro Asp Val Asn Thr Phe Asp Ile Thr
    1280                1285                1290

gtt tac ttg ttg caa ggg aga agg ctc cta caa ccc gaa tac tgc 3924
Val Tyr Leu Leu Gln Gly Arg Arg Leu Leu Gln Pro Glu Tyr Cys
    1295                1300                1305

cca gac ccc tta tat gaa gta atg cta aaa tgc tgg cac cct aaa 3969
Pro Asp Pro Leu Tyr Glu Val Met Leu Lys Cys Trp His Pro Lys
    1310                1315                1320

gca gaa atg cgc cca tcc ttt tct gaa ctg gtg tcc cgg ata tca 4014
Ala Glu Met Arg Pro Ser Phe Ser Glu Leu Val Ser Arg Ile Ser
    1325                1330                1335

gcg atc ttc tct act ttc att ggg gag cac tac gtc cat gta aac 4059
Ala Ile Phe Ser Thr Phe Ile Gly Glu His Tyr Val His Val Asn
    1340                1345                1350

gct act tat gtg aac gta aaa tgt gtc gct cca tat cct tct ctg 4104
Ala Thr Tyr Val Asn Val Lys Cys Val Ala Pro Tyr Pro Ser Leu
    1355                1360                1365

ttg tca tca gaa gat aac gct gat gac gag gtg gac aca tga 4146
Leu Ser Ser Glu Asp Asn Ala Asp Asp Glu Val Asp Thr
    1370                1375                1380

<210> 6
<211> 1381
<212> PRT
<213> Macaca mulatta

<400> 6

Met Lys Ala Pro Ala Val Leu Val Pro Gly Ile Leu Val Leu Leu Phe
1               5                   10                  15

Thr Leu Val Gln Arg Ser Asn Gly Glu Cys Lys Glu Ala Leu Ala Lys
            20                  25                  30

Ser Glu Met Asn Val Asn Met Lys Tyr Gln Leu Pro Asn Phe Thr Ala
        35                  40                  45

Glu Thr Ala Ile Gln Asn Val Ile Leu His Glu His His Ile Phe Leu
    50                  55                  60

Gly Ala Thr Asn Tyr Ile Tyr Val Leu Asn Glu Glu Asp Leu Gln Lys
65                  70                  75                  80

Val Ala Glu Tyr Lys Thr Gly Pro Val Leu Glu His Pro Asp Cys Phe
                85                  90                  95

Pro Cys Gln Asp Cys Ser Ser Lys Ala Asn Leu Ser Gly Gly Val Trp
            100                 105                 110

Lys Asp Asn Ile Asn Met Ala Leu Val Val Asp Thr Tyr Tyr Asp Asp
        115                 120                 125

Gln Leu Ile Ser Cys Gly Ser Val Asn Arg Gly Thr Cys Gln Arg His
    130                 135                 140

Val Phe Pro His Asn His Thr Ala Asp Ile Gln Ser Glu Val His Cys
145                 150                 155                 160

Ile Phe Ser Pro Gln Ile Glu Glu Pro Asn Gln Cys Pro Asp Cys Val
                165                 170                 175

Val Ser Ala Leu Gly Ala Lys Val Leu Ser Ser Val Lys Asp Arg Phe
            180                 185                 190

Ile Asn Phe Phe Val Gly Asn Thr Ile Asn Ser Ser Tyr Phe Pro His
        195                 200                 205

His Pro Leu His Ser Ile Ser Val Arg Arg Leu Lys Glu Thr Lys Asp
    210                 215                 220

Gly Phe Met Phe Leu Thr Asp Gln Ser Tyr Ile Asp Val Leu Pro Glu
225                 230                 235                 240

Phe Arg Asp Ser Tyr Pro Ile Lys Tyr Ile His Ala Phe Glu Ser Asn
                245                 250                 255

Asn Phe Ile Tyr Phe Leu Thr Val Gln Arg Glu Thr Leu Asn Ala Gln
            260                 265                 270

Thr Phe His Thr Arg Ile Ile Arg Phe Cys Ser Leu Asn Ser Gly Leu
        275                 280                 285

His Ser Tyr Met Glu Met Pro Leu Glu Cys Ile Leu Thr Glu Lys Arg
    290                 295                 300

Lys Lys Arg Ser Thr Lys Lys Glu Val Phe Asn Ile Leu Gln Ala Ala
305                 310                 315                 320

Tyr Val Ser Lys Pro Gly Ala Gln Leu Ala Arg Gln Ile Gly Ala Ser
                325                 330                 335

Leu Asn Asp Asp Ile Leu Phe Gly Val Phe Ala Gln Ser Lys Pro Asp
            340                 345                 350

Ser Ala Glu Pro Met Asp Arg Ser Ala Met Cys Ala Phe Pro Ile Lys
        355                 360                 365

Tyr Val Asn Asp Phe Phe Asn Lys Ile Val Asn Lys Asn Asn Val Arg
    370                 375                 380

Cys Leu Gln His Phe Tyr Gly Pro Asn His Glu His Cys Phe Asn Arg
385                 390                 395                 400

Thr Leu Leu Arg Asn Ser Ser Gly Cys Glu Ala Arg Arg Asp Glu Tyr
                405                 410                 415

Arg Ala Glu Phe Thr Thr Ala Leu Gln Arg Val Asp Leu Phe Met Gly
            420                 425                 430

435 440 445

Asp Leu Thr Ile Ala Asn Leu Gly Thr Ser Glu Gly Arg Phe Met Gln
450 455 460

Val Val Val Ser Arg Ser Gly Pro Ser Thr Pro His Val Asn Phe Leu
465 470 475 480

Leu Asp Ser His Pro Val Ser Pro Glu Val Ile Val Glu His Pro Leu
485 490 495

Asn Gln Asn Gly Tyr Thr Leu Val Val Thr Gly Lys Lys Ile Thr Lys
500 505 510

Ile Pro Leu Asn Gly Leu Gly Cys Arg His Phe Gln Ser Cys Ser Gln
515 520 525

Cys Leu Ser Ala Pro Pro Phe Val Gln Cys Gly Trp Cys His Asp Lys
530 535 540

Cys Val Arg Ser Glu Glu Cys Pro Ser Gly Thr Trp Thr Gln Gln Ile
545 550 555 560

Cys Leu Pro Ala Ile Tyr Lys Val Phe Pro Thr Ser Ala Pro Leu Glu
565 570 575

Gly Gly Thr Arg Leu Thr Ile Cys Gly Trp Asp Phe Gly Phe Arg Arg
580 585 590

Asn Asn Lys Phe Asp Leu Lys Lys Thr Arg Val Leu Leu Gly Asn Glu
595 600 605

Ser Cys Thr Leu Thr Leu Ser Glu Ser Thr Met Asn Thr Leu Lys Cys
610 615 620

Thr Val Gly Pro Ala Met Asn Lys His Phe Asn Met Ser Ile Ile Ile

625                 630                 635                 640

Ser Asn Gly His Gly Thr Thr Gln Tyr Ser Thr Phe Ser Tyr Val Asp
                645                 650                 655

Pro Ile Ile Thr Ser Ile Ser Pro Lys Tyr Gly Pro Met Ala Gly Gly
            660                 665                 670

Thr Leu Leu Thr Leu Thr Gly Asn Tyr Leu Asn Ser Gly Asn Ser Arg
        675                 680                 685

His Ile Ser Ile Gly Gly Lys Thr Cys Thr Leu Lys Ser Val Ser Asn
    690                 695                 700

Ser Ile Leu Glu Cys Tyr Thr Pro Ala Gln Thr Ile Ser Thr Glu Phe
705                 710                 715                 720

Ala Val Lys Leu Lys Ile Asp Leu Ala Asn Arg Glu Thr Ser Ile Phe
                725                 730                 735

Ser Tyr Arg Glu Asp Pro Ile Val Tyr Glu Ile His Pro Thr Lys Ser
            740                 745                 750

Phe Ile Ser Gly Gly Ser Thr Ile Thr Gly Val Gly Lys Asn Leu His
        755                 760                 765

Ser Val Ser Val Pro Arg Met Val Ile Asn Val His Glu Ala Gly Arg
    770                 775                 780

Asn Phe Thr Val Ala Cys Gln His Arg Ser Asn Ser Glu Ile Ile Cys
785                 790                 795                 800

Cys Thr Thr Pro Ser Leu Gln Gln Leu Asn Leu Gln Leu Pro Leu Lys
                805                 810                 815

Thr Lys Ala Phe Phe Met Leu Asp Gly Ile Leu Ser Lys Tyr Phe Asp
            820                 825                 830

Leu Ile Tyr Val His Asn Pro Val Phe Lys Pro Phe Glu Lys Pro Val
835 840 845

Met Ile Ser Met Gly Asn Glu Asn Val Leu Glu Ile Lys Gly Asn Asp
850 855 860

Ile Asp Pro Glu Ala Val Lys Gly Glu Val Leu Lys Val Gly Asn Lys
865 870 875 880

Ser Cys Glu Asn Ile His Leu His Ser Glu Ala Val Leu Cys Thr Val
885 890 895

Pro Asn Asp Leu Leu Lys Leu Asn Ser Glu Leu Asn Ile Glu Trp Lys
900 905 910

Gln Ala Ile Ser Ser Thr Val Leu Gly Lys Val Ile Val Gln Pro Asp
915 920 925

Gln Asn Phe Thr Gly Leu Ile Ala Gly Val Val Ser Ile Ser Ile Ala
930 935 940

Leu Leu Leu Leu Leu Gly Leu Phe Leu Trp Leu Lys Lys Arg Lys Gln
945 950 955 960

Ile Lys Asp Leu Gly Ser Glu Leu Val Arg Tyr Asp Ala Arg Val His
965 970 975

Thr Pro His Leu Asp Arg Leu Val Ser Ala Arg Ser Val Ser Pro Thr
980 985 990

Thr Glu Met Val Ser Asn Glu Ser Val Asp Tyr Arg Ala Thr Phe Pro
995 1000 1005

Glu Asp Gln Phe Pro Asn Ser Ser Gln Asn Gly Ser Cys Arg Gln
1010 1015 1020

Val Gln Tyr Pro Leu Thr Asp Met Ser Pro Ile Leu Thr Ser Gly
1025 1030 1035

Asp Ser Asp Ile Ser Ser Pro Leu Leu Gln Asn Thr Val His Ile
1040 1045 1050

Asp Leu Ser Ala Leu Asn Pro Glu Leu Val Gln Ala Val Gln His
1055 1060 1065

Val Val Ile Gly Pro Ser Ser Leu Ile Val His Phe Asn Glu Val
1070 1075 1080

Ile Gly Arg Gly His Phe Gly Cys Val Tyr His Gly Thr Leu Leu
1085 1090 1095

Asp Asn Asp Gly Lys Lys Ile His Cys Ala Val Lys Ser Leu Asn
1100 1105 1110

Arg Ile Thr Asp Ile Gly Glu Val Ser Gln Phe Leu Thr Glu Gly
1115 1120 1125

Ile Ile Met Lys Asp Phe Ser His Pro Asn Val Leu Ser Leu Leu
1130 1135 1140

Gly Ile Cys Leu Arg Ser Glu Gly Ser Pro Leu Val Val Leu Pro
1145 1150 1155

Tyr Met Lys His Gly Asp Leu Arg Asn Phe Ile Arg Asn Glu Thr
1160 1165 1170

His Asn Pro Thr Val Lys Asp Leu Ile Gly Phe Gly Leu Gln Val
1175 1180 1185

Ala Lys Gly Met Lys Tyr Leu Ala Ser Lys Lys Phe Val His Arg
1190 1195 1200

Asp Leu Ala Ala Arg Asn Cys Met Leu Asp Glu Lys Phe Thr Val
1205 1210 1215

Lys Val Ala Asp Phe Gly Leu Ala Arg Asp Met Tyr Asp Lys Glu
1220 1225 1230

Tyr Tyr Ser Val His Asn Lys Thr Gly Ala Lys Leu Pro Val Lys
1235 1240 1245

Trp Met Ala Leu Glu Ser Leu Gln Thr Gln Lys Phe Thr Thr Lys
1250 1255 1260

Ser Asp Val Trp Ser Phe Gly Val Leu Leu Trp Glu Leu Met Thr
1265 1270 1275

Arg Gly Ala Pro Pro Tyr Pro Asp Val Asn Thr Phe Asp Ile Thr
1280 1285 1290

Val Tyr Leu Leu Gln Gly Arg Arg Leu Leu Gln Pro Glu Tyr Cys
1295 1300 1305

Pro Asp Pro Leu Tyr Glu Val Met Leu Lys Cys Trp His Pro Lys
1310 1315 1320

Ala Glu Met Arg Pro Ser Phe Ser Glu Leu Val Ser Arg Ile Ser
1325 1330 1335

Ala Ile Phe Ser Thr Phe Ile Gly Glu His Tyr Val His Val Asn
1340 1345 1350

Ala Thr Tyr Val Asn Val Lys Cys Val Ala Pro Tyr Pro Ser Leu
1355 1360 1365

Leu Ser Ser Glu Asp Asn Ala Asp Asp Glu Val Asp Thr
1370 1375 1380

<210> 7

<211> 4173
<212> DNA
<213> Pan troglodytes

<220>
<221> CDS
<222> (1)..(4170)

<400> 7
atg aag gcc ccc gct gtg ctt gca cct ggc atc ctc gtg ctc ctg ttt 48
Met Lys Ala Pro Ala Val Leu Ala Pro Gly Ile Leu Val Leu Leu Phe
1               5                   10                  15

acc ttg gtg cag agg agc aat ggg gag tgt aaa gag gca cta gca aag 96
Thr Leu Val Gln Arg Ser Asn Gly Glu Cys Lys Glu Ala Leu Ala Lys
            20                  25                  30

tcc gag atg aat gtg aat atg aag tat cag ctt ccc aac ttc acc gcg 144
Ser Glu Met Asn Val Asn Met Lys Tyr Gln Leu Pro Asn Phe Thr Ala
        35                  40                  45

gaa aca ccc atc cag aat gtc att cta cat gag cat cac att ttc ctc 192
Glu Thr Pro Ile Gln Asn Val Ile Leu His Glu His His Ile Phe Leu
    50                  55                  60

ggt gcc act aac tac att tat gtt tta aat gag gaa gac ctt caa aag 240
Gly Ala Thr Asn Tyr Ile Tyr Val Leu Asn Glu Glu Asp Leu Gln Lys
65                  70                  75                  80

gtt gct gag tac aag act ggg cct gtg ctg gaa cac cca gat tgt ttc 288
Val Ala Glu Tyr Lys Thr Gly Pro Val Leu Glu His Pro Asp Cys Phe
                85                  90                  95

cca tgt cag gac tgc agc agc aaa gcc aat tta tca gga ggt gtt tgg 336
Pro Cys Gln Asp Cys Ser Ser Lys Ala Asn Leu Ser Gly Gly Val Trp
            100                 105                 110

aaa gat aac atc aac atg gct cta gtt gtc gac acc tac tat gat gat 384
Lys Asp Asn Ile Asn Met Ala Leu Val Val Asp Thr Tyr Tyr Asp Asp
        115                 120                 125

caa ctc att agc tgt ggc agc gtc aac aga ggg acc tgc cag cga cat 432
Gln Leu Ile Ser Cys Gly Ser Val Asn Arg Gly Thr Cys Gln Arg His
    130                 135                 140

gtc ttt ccc cac aat cat act gct gac ata cag tcg gag gtt cac tgc 480
Val Phe Pro His Asn His Thr Ala Asp Ile Gln Ser Glu Val His Cys
145                 150                 155                 160

ata ttc tcc cca cag ata gaa gag ccc agc cag tgt cct gac tgt gtg 528
Ile Phe Ser Pro Gln Ile Glu Glu Pro Ser Gln Cys Pro Asp Cys Val
                165                 170                 175

gtg agc gcc ctg gga gcc aaa gtc ctt tca tct gta aag gac cgg ttc 576
Val Ser Ala Leu Gly Ala Lys Val Leu Ser Ser Val Lys Asp Arg Phe
            180                 185                 190

atc aac ttc ttt gta ggc aat acc ata aat tct tct tat ttc cca gat 624
Ile Asn Phe Phe Val Gly Asn Thr Ile Asn Ser Ser Tyr Phe Pro Asp
        195                 200                 205

cat ccg ttg cat tca ata tca gtg aga agg cta aag gaa acg aaa gat 672
His Pro Leu His Ser Ile Ser Val Arg Arg Leu Lys Glu Thr Lys Asp
    210                 215                 220

ggt ttt atg ttt ttg aca gac cag tcc tac att gat gtt tta cct gag 720
Gly Phe Met Phe Leu Thr Asp Gln Ser Tyr Ile Asp Val Leu Pro Glu
225                 230                 235                 240

ttc aga gat tct tac ccc att aag tat gtc cat gcc ttt gaa agc aac 768
Phe Arg Asp Ser Tyr Pro Ile Lys Tyr Val His Ala Phe Glu Ser Asn
                245                 250                 255

aat ttt att tac ttc ttg acg gtc caa agg gaa act cta gat gct cag 816
Asn Phe Ile Tyr Phe Leu Thr Val Gln Arg Glu Thr Leu Asp Ala Gln
            260                 265                 270

act ttt cac aca aga ata atc agg ttc tgt tcc ata aac tct gga ttg 864
Thr Phe His Thr Arg Ile Ile Arg Phe Cys Ser Ile Asn Ser Gly Leu
        275                 280                 285

cat tcc tac atg gaa atg cct ctg gag tgt att ctc aca gaa aag aga 912
His Ser Tyr Met Glu Met Pro Leu Glu Cys Ile Leu Thr Glu Lys Arg
    290                 295                 300

aaa aag aga tcc aca aag aag gaa gtg ttt aat atc ctt cag gct gcg 960
Lys Lys Arg Ser Thr Lys Lys Glu Val Phe Asn Ile Leu Gln Ala Ala
305                 310                 315                 320

tat gtc agc aag cct ggg gcc cag ctt gct aga caa ata gga gcc agc 1008
Tyr Val Ser Lys Pro Gly Ala Gln Leu Ala Arg Gln Ile Gly Ala Ser
                325                 330                 335

ctg aat gat gac att ctt ttc ggg gtg ttc gca caa agc aag cca gat 1056
Leu Asn Asp Asp Ile Leu Phe Gly Val Phe Ala Gln Ser Lys Pro Asp
            340                 345                 350

tct gcc gaa cca atg gat cga tct gcc atg tgt gca ttc cct atc aaa 1104
Ser Ala Glu Pro Met Asp Arg Ser Ala Met Cys Ala Phe Pro Ile Lys
        355                 360                 365

tat gtc aac gac ttc ttc aac aag atc gtc aac aaa aac aat gtg aga 1152
Tyr Val Asn Asp Phe Phe Asn Lys Ile Val Asn Lys Asn Asn Val Arg
    370                 375                 380

tgt ctc cag cat ttt tac gga ccc aat cat gag cac tgt ttt aat agg 1200
Cys Leu Gln His Phe Tyr Gly Pro Asn His Glu His Cys Phe Asn Arg
385                 390                 395                 400

aca ctt ctg aga aat tca tca agc tgt gaa gcg cgc cgt gat gaa tat 1248
Thr Leu Leu Arg Asn Ser Ser Ser Cys Glu Ala Arg Arg Asp Glu Tyr
                405                 410                 415

cga aca gag ttt acc aca gct ttg cag cgc gtt gac tta ttc atg ggt 1296
Arg Thr Glu Phe Thr Thr Ala Leu Gln Arg Val Asp Leu Phe Met Gly
            420                 425                 430

caa ttc agc gaa gtc ctc tta aca tct ata tcc acc ttc att aaa gga 1344
Gln Phe Ser Glu Val Leu Leu Thr Ser Ile Ser Thr Phe Ile Lys Gly
        435                 440                 445

gac ctc acc ata gct aat ctt ggg aca tca gag ggt cgc ttc atg cag 1392
Asp Leu Thr Ile Ala Asn Leu Gly Thr Ser Glu Gly Arg Phe Met Gln
    450                 455                 460

gtt gtg gtt tct cga tca gga cca tca acc cct cat gtg aat ttt ctc 1440
Val Val Val Ser Arg Ser Gly Pro Ser Thr Pro His Val Asn Phe Leu
465                 470                 475                 480

ctg gac tcc cat cca gtg tct cca gaa gtg att gtg gag cat aca tta 1488
Leu Asp Ser His Pro Val Ser Pro Glu Val Ile Val Glu His Thr Leu
                485                 490                 495

aac cag aat ggc tac aca ctg gtt gtc act ggg aag aag atc acg aag 1536
Asn Gln Asn Gly Tyr Thr Leu Val Val Thr Gly Lys Lys Ile Thr Lys
            500                 505                 510

atc cca ttg aat ggc ttg ggc tgc aga cat ttc cag tcc tgc agt cag 1584
Ile Pro Leu Asn Gly Leu Gly Cys Arg His Phe Gln Ser Cys Ser Gln
        515                 520                 525

tgc ctc tct gcc cca ccc ttt gtt cag tgt ggc tgg tgc cac gac aaa 1632
Cys Leu Ser Ala Pro Pro Phe Val Gln Cys Gly Trp Cys His Asp Lys
    530                 535                 540

tgt gtg cga tcg gag gaa tgc ctg agc ggg aca tgg act caa cag atc 1680

Cys Val Arg Ser Glu Glu Cys Leu Ser Gly Thr Trp Thr Gln Gln Ile
545                 550                 555                 560

tgt ctg cct gca atc tac aag gtt ttc cca aat agt gca ccc ctt gaa      1728
Cys Leu Pro Ala Ile Tyr Lys Val Phe Pro Asn Ser Ala Pro Leu Glu
                565                 570                 575

gga ggg aca agg ctg acc ata tgt ggc tgg gac ttt gga ttt cgg agg      1776
Gly Gly Thr Arg Leu Thr Ile Cys Gly Trp Asp Phe Gly Phe Arg Arg
            580                 585                 590

aat aat aaa ttt gat tta aag aaa act aga gtt ctc ctt gga aat gag      1824
Asn Asn Lys Phe Asp Leu Lys Lys Thr Arg Val Leu Leu Gly Asn Glu
        595                 600                 605

agc tgc acc ttg act tta agt gag agc acg atg aat aca ttg aaa tgc      1872
Ser Cys Thr Leu Thr Leu Ser Glu Ser Thr Met Asn Thr Leu Lys Cys
    610                 615                 620

aca gtt ggt cct gcc atg aat aag cat ttc aat atg tcc ata att att      1920
Thr Val Gly Pro Ala Met Asn Lys His Phe Asn Met Ser Ile Ile Ile
625                 630                 635                 640

tca aat ggc cac ggg aca aca caa tac agt aca ttc tcc tat gtg gat      1968
Ser Asn Gly His Gly Thr Thr Gln Tyr Ser Thr Phe Ser Tyr Val Asp
                645                 650                 655

cct gta ata aca agt att tcg ccg aaa tac ggt cct atg gct ggt ggc      2016
Pro Val Ile Thr Ser Ile Ser Pro Lys Tyr Gly Pro Met Ala Gly Gly
            660                 665                 670

act tta ctt act tta act gga aat tac cta aac agt ggg aat tct aga      2064
Thr Leu Leu Thr Leu Thr Gly Asn Tyr Leu Asn Ser Gly Asn Ser Arg
        675                 680                 685

cac att tca att ggt gga aaa aca tgt act tta aaa agt gtg tca aac      2112
His Ile Ser Ile Gly Gly Lys Thr Cys Thr Leu Lys Ser Val Ser Asn
    690                 695                 700

agt att ctt gaa tgt tat acc cca gcc caa acc att tca act gag ttt      2160
Ser Ile Leu Glu Cys Tyr Thr Pro Ala Gln Thr Ile Ser Thr Glu Phe
705                 710                 715                 720

gct gtt aaa ttg aaa att gac tta gcc aac cga gag aca agc atc ttc      2208
Ala Val Lys Leu Lys Ile Asp Leu Ala Asn Arg Glu Thr Ser Ile Phe
                725                 730                 735

agt tac cgg gaa gat ccc att gtc tat gaa att cat cca acc aaa tct      2256
Ser Tyr Arg Glu Asp Pro Ile Val Tyr Glu Ile His Pro Thr Lys Ser

            740                 745                 750

ttt att agt ggt ggg agc aca ata aca ggt gtt ggg aaa aac ctg aat 2304
Phe Ile Ser Gly Gly Ser Thr Ile Thr Gly Val Gly Lys Asn Leu Asn
        755                 760                 765

tca gtt agt gtc ccg agg atg gtc ata aat gtg cat gaa gca gga agg 2352
Ser Val Ser Val Pro Arg Met Val Ile Asn Val His Glu Ala Gly Arg
    770                 775                 780

aac ttt aca gtg gca tgt caa cat cgc tct aat tca gag ata atc tgt 2400
Asn Phe Thr Val Ala Cys Gln His Arg Ser Asn Ser Glu Ile Ile Cys
785                 790                 795                 800

tgt acc act cct tcc ctg caa cag ctg aat ctg caa ctc ccc ctg aaa 2448
Cys Thr Thr Pro Ser Leu Gln Gln Leu Asn Leu Gln Leu Pro Leu Lys
                805                 810                 815

acc aaa gcc ttt ttc atg tta gat ggg atc ctt tcc aaa tac ttt gat 2496
Thr Lys Ala Phe Phe Met Leu Asp Gly Ile Leu Ser Lys Tyr Phe Asp
            820                 825                 830

ctc att tat gta cat aat cct gtg ttt aag cct ttt gaa aag cca gtg 2544
Leu Ile Tyr Val His Asn Pro Val Phe Lys Pro Phe Glu Lys Pro Val
        835                 840                 845

atg atc tca atg ggc aat gaa aat gta ctg gaa att aag gga aat gat 2592
Met Ile Ser Met Gly Asn Glu Asn Val Leu Glu Ile Lys Gly Asn Asp
    850                 855                 860

att gac cct gaa gca gtt aaa ggt gaa gtg tta aaa gtt gga aat aag 2640
Ile Asp Pro Glu Ala Val Lys Gly Glu Val Leu Lys Val Gly Asn Lys
865                 870                 875                 880

agc tgt gag aat ata cac tta cat tct gaa gcc gtt tta tgc acg gtc 2688
Ser Cys Glu Asn Ile His Leu His Ser Glu Ala Val Leu Cys Thr Val
                885                 890                 895

ccc aat gac ctg ctg aaa ttg aac agc gag cta aat ata gag tgg aag 2736
Pro Asn Asp Leu Leu Lys Leu Asn Ser Glu Leu Asn Ile Glu Trp Lys
            900                 905                 910

caa gca att tct tca acc gtc ctt gga aaa gta ata gtt caa cca gat 2784
Gln Ala Ile Ser Ser Thr Val Leu Gly Lys Val Ile Val Gln Pro Asp
        915                 920                 925

cag aat ttc aca gga ttg att gct ggt gtt gtc tca ata tca ata gca 2832
Gln Asn Phe Thr Gly Leu Ile Ala Gly Val Val Ser Ile Ser Ile Ala
    930                 935                 940

ctg tta tta cta ctt ggg ttt ttc ctg tgg ctg aaa aag aga aag caa 2880
Leu Leu Leu Leu Leu Gly Phe Phe Leu Trp Leu Lys Lys Arg Lys Gln
945                 950                 955                 960

att aaa gat ctg ggc agt gaa tta gtt cgc tac gat gca aga gta cac 2928
Ile Lys Asp Leu Gly Ser Glu Leu Val Arg Tyr Asp Ala Arg Val His
                965                 970                 975

act cct cat ttg gat agg ctc gta agt gcc cga agt gta agc cca act 2976
Thr Pro His Leu Asp Arg Leu Val Ser Ala Arg Ser Val Ser Pro Thr
            980                 985                 990

aca gaa atg gtt tca aat gaa tct gta gac tac cga gct act ttt cca 3024
Thr Glu Met Val Ser Asn Glu Ser Val Asp Tyr Arg Ala Thr Phe Pro
        995                 1000                1005

gaa gat cag ttt cct aat tca tct cag aac ggt tca tgc cga caa 3069
Glu Asp Gln Phe Pro Asn Ser Ser Gln Asn Gly Ser Cys Arg Gln
    1010                1015                1020

gtg cag tat cct ctg aca gac atg tcc ccc atc cta act agt ggg 3114
Val Gln Tyr Pro Leu Thr Asp Met Ser Pro Ile Leu Thr Ser Gly
    1025                1030                1035

gac tct gat ata tcc agt cca tta ctg caa aat act gtc cac att 3159
Asp Ser Asp Ile Ser Ser Pro Leu Leu Gln Asn Thr Val His Ile
    1040                1045                1050

gac ctc agt gct cta aat cca gag ctg gtc cag gca gtg cag cat 3204
Asp Leu Ser Ala Leu Asn Pro Glu Leu Val Gln Ala Val Gln His
    1055                1060                1065

gta gtg att ggg ccc agt agc ctg att gtg cat ttc aat gaa gtc 3249
Val Val Ile Gly Pro Ser Ser Leu Ile Val His Phe Asn Glu Val
    1070                1075                1080

ata gga aga ggg cat ttt ggt tgt gta tat cat ggg act ttg ttg 3294
Ile Gly Arg Gly His Phe Gly Cys Val Tyr His Gly Thr Leu Leu
    1085                1090                1095

gac aat gat ggc aag aaa att cac tgt gct gtg aaa tcc ttg aac 3339
Asp Asn Asp Gly Lys Lys Ile His Cys Ala Val Lys Ser Leu Asn
    1100                1105                1110

aga atc acc gac ata gga gaa gtt tcc caa ttt ctg acc gag gga 3384
Arg Ile Thr Asp Ile Gly Glu Val Ser Gln Phe Leu Thr Glu Gly
    1115                1120                1125

atc atc atg aaa gat ttt agt cat ccc aat gtc ctc tcg ctc ctg 3429
Ile Ile Met Lys Asp Phe Ser His Pro Asn Val Leu Ser Leu Leu
    1130                1135                1140

gga atc tgc ctg cga agt gaa ggg tct ccg ctg gtg gtc cta cca 3474
Gly Ile Cys Leu Arg Ser Glu Gly Ser Pro Leu Val Val Leu Pro
    1145                1150                1155

tac atg aaa cat gga gat ctt cga aat ttc att cga aat gag act 3519
Tyr Met Lys His Gly Asp Leu Arg Asn Phe Ile Arg Asn Glu Thr
    1160                1165                1170

cat aat cca act gta aaa gat ctt att ggc ttt ggt ctt caa gta 3564
His Asn Pro Thr Val Lys Asp Leu Ile Gly Phe Gly Leu Gln Val
    1175                1180                1185

gcc aaa ggc atg aaa tat ctt gca agc aaa aag ttt gtc cac aga 3609
Ala Lys Gly Met Lys Tyr Leu Ala Ser Lys Lys Phe Val His Arg
    1190                1195                1200

gac ttg gct gca aga aac tgt atg ctg gat gaa aaa ttc aca gtc 3654
Asp Leu Ala Ala Arg Asn Cys Met Leu Asp Glu Lys Phe Thr Val
    1205                1210                1215

aag gtt gct gat ttt ggt ctt gcc aga gac atg tat gat aaa gaa 3699
Lys Val Ala Asp Phe Gly Leu Ala Arg Asp Met Tyr Asp Lys Glu
    1220                1225                1230

tac tat agt gta cac aac aaa aca ggt gca aag ctg cca gtg aag 3744
Tyr Tyr Ser Val His Asn Lys Thr Gly Ala Lys Leu Pro Val Lys
    1235                1240                1245

tgg atg gct ttg gaa agt ctg caa act caa aag ttt acc acc aag 3789
Trp Met Ala Leu Glu Ser Leu Gln Thr Gln Lys Phe Thr Thr Lys
    1250                1255                1260

tca gat gtg tgg tcc ttt ggc gtg ctc ctc tgg gag ctg atg aca 3834
Ser Asp Val Trp Ser Phe Gly Val Leu Leu Trp Glu Leu Met Thr
    1265                1270                1275

aga gga gcc cca cct tat cct gat gtg aac acc ttt gat ata act 3879
Arg Gly Ala Pro Pro Tyr Pro Asp Val Asn Thr Phe Asp Ile Thr
    1280                1285                1290

gtt tac ttg ttg caa ggg aga agg ctc cta caa ccc gaa tac tgc 3924
Val Tyr Leu Leu Gln Gly Arg Arg Leu Leu Gln Pro Glu Tyr Cys
    1295                1300                1305

cca gac ccc tta tac gaa gta atg cta aaa tgc tgg cac cct aaa 3969

Pro Asp Pro Leu Tyr Glu Val Met Leu Lys Cys Trp His Pro Lys
    1310                1315                1320

gcc gaa atg cgc cca tcc ttt tct gaa ctg gtg tcc cgg ata tca 4014
Ala Glu Met Arg Pro Ser Phe Ser Glu Leu Val Ser Arg Ile Ser
    1325                1330                1335

gcg atc ttc tct act ttc att ggg gag cac tat gtc cat gtg aac 4059
Ala Ile Phe Ser Thr Phe Ile Gly Glu His Tyr Val His Val Asn
    1340                1345                1350

gct act tat gtg aac gta aaa tgt gtc gct cca tat cct tct ctg 4104
Ala Thr Tyr Val Asn Val Lys Cys Val Ala Pro Tyr Pro Ser Leu
    1355                1360                1365

ttg tca tca gaa gat aac gct gat gat gag gtg gac aca cga cca 4149
Leu Ser Ser Glu Asp Asn Ala Asp Asp Glu Val Asp Thr Arg Pro
    1370                1375                1380

gcc tcc ttc tgg gag aca tca tag 4173
Ala Ser Phe Trp Glu Thr Ser
    1385                1390

<210> 8
<211> 1390
<212> PRT
<213> Pan troglodytes

<400> 8

Met Lys Ala Pro Ala Val Leu Ala Pro Gly Ile Leu Val Leu Leu Phe
1               5                   10                  15

Thr Leu Val Gln Arg Ser Asn Gly Glu Cys Lys Glu Ala Leu Ala Lys
            20                  25                  30

Ser Glu Met Asn Val Asn Met Lys Tyr Gln Leu Pro Asn Phe Thr Ala
        35                  40                  45

Glu Thr Pro Ile Gln Asn Val Ile Leu His Glu His His Ile Phe Leu
    50                  55                  60

Gly Ala Thr Asn Tyr Ile Tyr Val Leu Asn Glu Glu Asp Leu Gln Lys
65                  70                  75                  80

Val Ala Glu Tyr Lys Thr Gly Pro Val Leu Glu His Pro Asp Cys Phe
                85                  90                  95

Pro Cys Gln Asp Cys Ser Ser Lys Ala Asn Leu Ser Gly Gly Val Trp
            100                 105                 110

Lys Asp Asn Ile Asn Met Ala Leu Val Val Asp Thr Tyr Tyr Asp Asp
        115                 120                 125

Gln Leu Ile Ser Cys Gly Ser Val Asn Arg Gly Thr Cys Gln Arg His
    130                 135                 140

Val Phe Pro His Asn His Thr Ala Asp Ile Gln Ser Glu Val His Cys
145                 150                 155                 160

Ile Phe Ser Pro Gln Ile Glu Glu Pro Ser Gln Cys Pro Asp Cys Val
                165                 170                 175

Val Ser Ala Leu Gly Ala Lys Val Leu Ser Ser Val Lys Asp Arg Phe
            180                 185                 190

Ile Asn Phe Phe Val Gly Asn Thr Ile Asn Ser Ser Tyr Phe Pro Asp
        195                 200                 205

His Pro Leu His Ser Ile Ser Val Arg Arg Leu Lys Glu Thr Lys Asp
    210                 215                 220

Gly Phe Met Phe Leu Thr Asp Gln Ser Tyr Ile Asp Val Leu Pro Glu
225                 230                 235                 240

Phe Arg Asp Ser Tyr Pro Ile Lys Tyr Val His Ala Phe Glu Ser Asn
                245                 250                 255

Asn Phe Ile Tyr Phe Leu Thr Val Gln Arg Glu Thr Leu Asp Ala Gln
            260                 265                 270

Thr Phe His Thr Arg Ile Ile Arg Phe Cys Ser Ile Asn Ser Gly Leu
        275                 280                 285

His Ser Tyr Met Glu Met Pro Leu Glu Cys Ile Leu Thr Glu Lys Arg
    290                 295                 300

Lys Lys Arg Ser Thr Lys Lys Glu Val Phe Asn Ile Leu Gln Ala Ala
305                 310                 315                 320

Tyr Val Ser Lys Pro Gly Ala Gln Leu Ala Arg Gln Ile Gly Ala Ser
                325                 330                 335

Leu Asn Asp Asp Ile Leu Phe Gly Val Phe Ala Gln Ser Lys Pro Asp
            340                 345                 350

Ser Ala Glu Pro Met Asp Arg Ser Ala Met Cys Ala Phe Pro Ile Lys
        355                 360                 365

Tyr Val Asn Asp Phe Phe Asn Lys Ile Val Asn Lys Asn Asn Val Arg
    370                 375                 380

Cys Leu Gln His Phe Tyr Gly Pro Asn His Glu His Cys Phe Asn Arg
385                 390                 395                 400

Thr Leu Leu Arg Asn Ser Ser Ser Cys Glu Ala Arg Arg Asp Glu Tyr
                405                 410                 415

Arg Thr Glu Phe Thr Thr Ala Leu Gln Arg Val Asp Leu Phe Met Gly
            420                 425                 430

Gln Phe Ser Glu Val Leu Leu Thr Ser Ile Ser Thr Phe Ile Lys Gly
        435                 440                 445

Asp Leu Thr Ile Ala Asn Leu Gly Thr Ser Glu Gly Arg Phe Met Gln
    450                 455                 460

Val Val Val Ser Arg Ser Gly Pro Ser Thr Pro His Val Asn Phe Leu
465                 470                 475                 480

Leu Asp Ser His Pro Val Ser Pro Glu Val Ile Val Glu His Thr Leu
                485                 490                 495

Asn Gln Asn Gly Tyr Thr Leu Val Val Thr Gly Lys Lys Ile Thr Lys
            500                 505                 510

Ile Pro Leu Asn Gly Leu Gly Cys Arg His Phe Gln Ser Cys Ser Gln
        515                 520                 525

Cys Leu Ser Ala Pro Pro Phe Val Gln Cys Gly Trp Cys His Asp Lys
    530                 535                 540

Cys Val Arg Ser Glu Glu Cys Leu Ser Gly Thr Trp Thr Gln Gln Ile
545                 550                 555                 560

Cys Leu Pro Ala Ile Tyr Lys Val Phe Pro Asn Ser Ala Pro Leu Glu
                565                 570                 575

Gly Gly Thr Arg Leu Thr Ile Cys Gly Trp Asp Phe Gly Phe Arg Arg
            580                 585                 590

Asn Asn Lys Phe Asp Leu Lys Lys Thr Arg Val Leu Leu Gly Asn Glu
        595                 600                 605

Ser Cys Thr Leu Thr Leu Ser Glu Ser Thr Met Asn Thr Leu Lys Cys
    610                 615                 620

Thr Val Gly Pro Ala Met Asn Lys His Phe Asn Met Ser Ile Ile Ile
625                 630                 635                 640

Ser Asn Gly His Gly Thr Thr Gln Tyr Ser Thr Phe Ser Tyr Val Asp
                645                 650                 655

Pro Val Ile Thr Ser Ile Ser Pro Lys Tyr Gly Pro Met Ala Gly Gly

660                 665                 670

Thr Leu Leu Thr Leu Thr Gly Asn Tyr Leu Asn Ser Gly Asn Ser Arg
        675                 680                 685

His Ile Ser Ile Gly Gly Lys Thr Cys Thr Leu Lys Ser Val Ser Asn
    690                 695                 700

Ser Ile Leu Glu Cys Tyr Thr Pro Ala Gln Thr Ile Ser Thr Glu Phe
705                 710                 715                 720

Ala Val Lys Leu Lys Ile Asp Leu Ala Asn Arg Glu Thr Ser Ile Phe
                725                 730                 735

Ser Tyr Arg Glu Asp Pro Ile Val Tyr Glu Ile His Pro Thr Lys Ser
            740                 745                 750

Phe Ile Ser Gly Gly Ser Thr Ile Thr Gly Val Gly Lys Asn Leu Asn
        755                 760                 765

Ser Val Ser Val Pro Arg Met Val Ile Asn Val His Glu Ala Gly Arg
    770                 775                 780

Asn Phe Thr Val Ala Cys Gln His Arg Ser Asn Ser Glu Ile Ile Cys
785                 790                 795                 800

Cys Thr Thr Pro Ser Leu Gln Gln Leu Asn Leu Gln Leu Pro Leu Lys
                805                 810                 815

Thr Lys Ala Phe Phe Met Leu Asp Gly Ile Leu Ser Lys Tyr Phe Asp
            820                 825                 830

Leu Ile Tyr Val His Asn Pro Val Phe Lys Pro Phe Glu Lys Pro Val
        835                 840                 845

Met Ile Ser Met Gly Asn Glu Asn Val Leu Glu Ile Lys Gly Asn Asp
    850                 855                 860

Ile Asp Pro Glu Ala Val Lys Gly Glu Val Leu Lys Val Gly Asn Lys
865                 870                 875                 880

Ser Cys Glu Asn Ile His Leu His Ser Glu Ala Val Leu Cys Thr Val
                885                 890                 895

Pro Asn Asp Leu Leu Lys Leu Asn Ser Glu Leu Asn Ile Glu Trp Lys
            900                 905                 910

Gln Ala Ile Ser Ser Thr Val Leu Gly Lys Val Ile Val Gln Pro Asp
        915                 920                 925

Gln Asn Phe Thr Gly Leu Ile Ala Gly Val Val Ser Ile Ser Ile Ala
    930                 935                 940

Leu Leu Leu Leu Leu Gly Phe Phe Leu Trp Leu Lys Lys Arg Lys Gln
945                 950                 955                 960

Ile Lys Asp Leu Gly Ser Glu Leu Val Arg Tyr Asp Ala Arg Val His
                965                 970                 975

Thr Pro His Leu Asp Arg Leu Val Ser Ala Arg Ser Val Ser Pro Thr
            980                 985                 990

Thr Glu Met Val Ser Asn Glu Ser  Val Asp Tyr Arg Ala  Thr Phe Pro
        995                 1000                1005

Glu Asp  Gln Phe Pro Asn Ser  Ser Gln Asn Gly Ser  Cys Arg Gln
    1010                1015                1020

Val Gln  Tyr Pro Leu Thr Asp  Met Ser Pro Ile Leu  Thr Ser Gly
    1025                1030                1035

Asp Ser  Asp Ile Ser Ser Pro  Leu Leu Gln Asn Thr  Val His Ile
    1040                1045                1050

Asp Leu Ser Ala Leu Asn Pro Glu Leu Val Gln Ala Val Gln His
    1055                1060                1065

Val Val Ile Gly Pro Ser Ser Leu Ile Val His Phe Asn Glu Val
    1070                1075                1080

Ile Gly Arg Gly His Phe Gly Cys Val Tyr His Gly Thr Leu Leu
    1085                1090                1095

Asp Asn Asp Gly Lys Lys Ile His Cys Ala Val Lys Ser Leu Asn
    1100                1105                1110

Arg Ile Thr Asp Ile Gly Glu Val Ser Gln Phe Leu Thr Glu Gly
    1115                1120                1125

Ile Ile Met Lys Asp Phe Ser His Pro Asn Val Leu Ser Leu Leu
    1130                1135                1140

Gly Ile Cys Leu Arg Ser Glu Gly Ser Pro Leu Val Val Leu Pro
    1145                1150                1155

Tyr Met Lys His Gly Asp Leu Arg Asn Phe Ile Arg Asn Glu Thr
    1160                1165                1170

His Asn Pro Thr Val Lys Asp Leu Ile Gly Phe Gly Leu Gln Val
    1175                1180                1185

Ala Lys Gly Met Lys Tyr Leu Ala Ser Lys Lys Phe Val His Arg
    1190                1195                1200

Asp Leu Ala Ala Arg Asn Cys Met Leu Asp Glu Lys Phe Thr Val
    1205                1210                1215

Lys Val Ala Asp Phe Gly Leu Ala Arg Asp Met Tyr Asp Lys Glu
    1220                1225                1230

Tyr Tyr Ser Val His Asn Lys Thr Gly Ala Lys Leu Pro Val Lys
    1235                1240                1245

Trp Met Ala Leu Glu Ser Leu Gln Thr Gln Lys Phe Thr Thr Lys
    1250                1255                1260

Ser Asp Val Trp Ser Phe Gly Val Leu Leu Trp Glu Leu Met Thr
    1265                1270                1275

Arg Gly Ala Pro Pro Tyr Pro Asp Val Asn Thr Phe Asp Ile Thr
    1280                1285                1290

Val Tyr Leu Leu Gln Gly Arg Arg Leu Leu Gln Pro Glu Tyr Cys
    1295                1300                1305

Pro Asp Pro Leu Tyr Glu Val Met Leu Lys Cys Trp His Pro Lys
    1310                1315                1320

Ala Glu Met Arg Pro Ser Phe Ser Glu Leu Val Ser Arg Ile Ser
    1325                1330                1335

Ala Ile Phe Ser Thr Phe Ile Gly Glu His Tyr Val His Val Asn
    1340                1345                1350

Ala Thr Tyr Val Asn Val Lys Cys Val Ala Pro Tyr Pro Ser Leu
    1355                1360                1365

Leu Ser Ser Glu Asp Asn Ala Asp Asp Glu Val Asp Thr Arg Pro
    1370                1375                1380

Ala Ser Phe Trp Glu Thr Ser
    1385                1390

<210> 9
<211> 4149
<212> DNA
<213> Rattus norvegicus

<220>
<221> CDS
<222> (1)..(4146)

<400> 9
atg aag gct ccc acc gcg ctg gca cct ggc att ctg ctg ctg ctg ctg 48
Met Lys Ala Pro Thr Ala Leu Ala Pro Gly Ile Leu Leu Leu Leu Leu
1               5                   10                  15

acc ttg gcg cag agg agc cat ggg gag tgc aag gag gcc cta gtg aag 96
Thr Leu Ala Gln Arg Ser His Gly Glu Cys Lys Glu Ala Leu Val Lys
            20                  25                  30

tct gag atg aac gtg aac atg aag tac cag ctt ccc aac ttc acc gca 144
Ser Glu Met Asn Val Asn Met Lys Tyr Gln Leu Pro Asn Phe Thr Ala
        35                  40                  45

gaa acc ccc atc cac aat gtc gtc ctc cct ggg cac cat att tat ctc 192
Glu Thr Pro Ile His Asn Val Val Leu Pro Gly His His Ile Tyr Leu
    50                  55                  60

gga gcc aca aac tac att tat gtt tta aat gac aaa gac ctt cag aag 240
Gly Ala Thr Asn Tyr Ile Tyr Val Leu Asn Asp Lys Asp Leu Gln Lys
65                  70                  75                  80

gta tct gag ttc aag acc ggg ccc gtg gtg gaa cac cca gat tgt ttt 288
Val Ser Glu Phe Lys Thr Gly Pro Val Val Glu His Pro Asp Cys Phe
                85                  90                  95

cct tgt cag gac tgc agc agc aaa gcc aat gtg tca gga ggt gtt tgg 336
Pro Cys Gln Asp Cys Ser Ser Lys Ala Asn Val Ser Gly Gly Val Trp
            100                 105                 110

aaa gac aac gtc aac atg gcg ctg ctt gtt gac act tac tat gac gac 384
Lys Asp Asn Val Asn Met Ala Leu Leu Val Asp Thr Tyr Tyr Asp Asp
        115                 120                 125

cag ctc atc agc tgt ggc agc gtc aac aga ggg acc tgc caa agg cat 432
Gln Leu Ile Ser Cys Gly Ser Val Asn Arg Gly Thr Cys Gln Arg His
    130                 135                 140

gtc ctt cct cct gac aat gct gcc gac att cag tcc gag gtt cac tgc 480
Val Leu Pro Pro Asp Asn Ala Ala Asp Ile Gln Ser Glu Val His Cys
145                 150                 155                 160

atg ttc tcc cca ctt gcg gag gaa gag tca ggc cag tgt ccc gac tgt 528
Met Phe Ser Pro Leu Ala Glu Glu Glu Ser Gly Gln Cys Pro Asp Cys
                165                 170                 175

gta gtg agt gcc ctg gga gcc aaa gtc ctc ctg tct gaa aag gac cgg 576
Val Val Ser Ala Leu Gly Ala Lys Val Leu Leu Ser Glu Lys Asp Arg
            180                 185                 190

ttc atc aat ttc ttc gtg ggg aat acg ata aac tct tcc tac cct ccc 624
Phe Ile Asn Phe Phe Val Gly Asn Thr Ile Asn Ser Ser Tyr Pro Pro
        195                 200                 205

gat tat tca ttg cat tca ata tcg gtg agg cgg ctg aag gaa acc cag 672
Asp Tyr Ser Leu His Ser Ile Ser Val Arg Arg Leu Lys Glu Thr Gln
    210                 215                 220

gac ggt ttt aag ttt ttg aca gac cag tcc tac att gat gtc ctg cca 720
Asp Gly Phe Lys Phe Leu Thr Asp Gln Ser Tyr Ile Asp Val Leu Pro
225                 230                 235                 240

gaa ttc cga gat tcc tac ccc ata aag tac ata cat gcc ttc gaa agc 768
Glu Phe Arg Asp Ser Tyr Pro Ile Lys Tyr Ile His Ala Phe Glu Ser
                245                 250                 255

aac cat ttt atc tac ttt ctg act gtc cag aag gaa acc cta gat gct 816
Asn His Phe Ile Tyr Phe Leu Thr Val Gln Lys Glu Thr Leu Asp Ala
            260                 265                 270

cag act ttc cat aca aga ata atc agg ttc tgt tct gta gac tct ggg 864
Gln Thr Phe His Thr Arg Ile Ile Arg Phe Cys Ser Val Asp Ser Gly
        275                 280                 285

ttg cac tcc tac atg gaa atg cct ctg gag tgc att ctg acg gaa aaa 912
Leu His Ser Tyr Met Glu Met Pro Leu Glu Cys Ile Leu Thr Glu Lys
    290                 295                 300

aga aga aag aga tcc aca agg gaa gaa gtg ttt aat atc ctc caa gcc 960
Arg Arg Lys Arg Ser Thr Arg Glu Glu Val Phe Asn Ile Leu Gln Ala
305                 310                 315                 320

gca tat gtc agt aaa ccg ggg gcc aat ctt gct aag caa ata ggg gct 1008
Ala Tyr Val Ser Lys Pro Gly Ala Asn Leu Ala Lys Gln Ile Gly Ala
                325                 330                 335

agc ccg tat gat gac att ctc tac ggg gtg ttt gca caa agc aag cca 1056
Ser Pro Tyr Asp Asp Ile Leu Tyr Gly Val Phe Ala Gln Ser Lys Pro
            340                 345                 350

gat tct gct gag ccc atg aac cga tca gcg gtc tgt gcg ttc ccc atc 1104
Asp Ser Ala Glu Pro Met Asn Arg Ser Ala Val Cys Ala Phe Pro Ile
        355                 360                 365

aaa tat gtc aat gac ttc ttc aac aag att gtc aac aaa aac aac gta     1152
Lys Tyr Val Asn Asp Phe Phe Asn Lys Ile Val Asn Lys Asn Asn Val
    370                 375                 380

cgg tgt ctc cag cat ttt tat gga ccc aac cac gag cac tgt ttc aat     1200
Arg Cys Leu Gln His Phe Tyr Gly Pro Asn His Glu His Cys Phe Asn
385                 390                 395                 400

agg acc ctg ctg aga aat tca tcg ggc tgc gaa gtg cgc agt gac gag     1248
Arg Thr Leu Leu Arg Asn Ser Ser Gly Cys Glu Val Arg Ser Asp Glu
                405                 410                 415

tac cgg acg gag ttt acc acg gcg ctg cag cgt gtg gat tta ttc atg     1296
Tyr Arg Thr Glu Phe Thr Thr Ala Leu Gln Arg Val Asp Leu Phe Met
            420                 425                 430

ggc cgg ctc aac cat gta ctc ttg acg tct atc tct acc ttc atc aaa     1344
Gly Arg Leu Asn His Val Leu Leu Thr Ser Ile Ser Thr Phe Ile Lys
        435                 440                 445

ggt gac ctc acc att gct aat cta ggg aca tca gaa ggt cgc ttc atg     1392
Gly Asp Leu Thr Ile Ala Asn Leu Gly Thr Ser Glu Gly Arg Phe Met
    450                 455                 460

cag gtg gtg ctc tct cgc aca gca cat ttc acc ccc cat gtg aat ttc     1440
Gln Val Val Leu Ser Arg Thr Ala His Phe Thr Pro His Val Asn Phe
465                 470                 475                 480

ctc ctg gat tcc tat cct gtg tct ccg gaa gtt att gtc gaa cat cca     1488
Leu Leu Asp Ser Tyr Pro Val Ser Pro Glu Val Ile Val Glu His Pro
                485                 490                 495

tca aat caa aat ggc tat acc ttg gtg gtc aca ggg aag aag atc acc     1536
Ser Asn Gln Asn Gly Tyr Thr Leu Val Val Thr Gly Lys Lys Ile Thr
            500                 505                 510

aag att cca ctg aat ggc cta ggc tgt ggg cat ttc cag tcc tgc agt     1584
Lys Ile Pro Leu Asn Gly Leu Gly Cys Gly His Phe Gln Ser Cys Ser
        515                 520                 525

cag tgt ctc tct ccc ccc tac ttt ata cag tgt ggc tgg tgc cac aat     1632
Gln Cys Leu Ser Pro Pro Tyr Phe Ile Gln Cys Gly Trp Cys His Asn
    530                 535                 540

cgg tgt gtg cat tcc aat gaa tgc ccc agc ggt aca tgg act caa gag     1680
Arg Cys Val His Ser Asn Glu Cys Pro Ser Gly Thr Trp Thr Gln Glu
545                 550                 555                 560

atc tgt ctg cca gca gtt tat aag gtt ttc ccc act agt gca ccc ctc     1728

Ile Cys Leu Pro Ala Val Tyr Lys Val Phe Pro Thr Ser Ala Pro Leu
                565                 570                 575

gaa gga gga aca atg ctg acc ata tgt ggc tgg gac ttt gga ttc aag      1776
Glu Gly Gly Thr Met Leu Thr Ile Cys Gly Trp Asp Phe Gly Phe Lys
            580                 585                 590

aag aat aat aaa ttt gat tta agg aaa acc aaa gtt ctg ctt ggc aac      1824
Lys Asn Asn Lys Phe Asp Leu Arg Lys Thr Lys Val Leu Leu Gly Asn
        595                 600                 605

gag agc tgt acc ttg acc tta agc gag agt acg aca aat acg ttg aaa      1872
Glu Ser Cys Thr Leu Thr Leu Ser Glu Ser Thr Thr Asn Thr Leu Lys
    610                 615                 620

tgc aca gtt ggc ccc gcg atg agt gag cac ttc aat gtg tct gtg atc      1920
Cys Thr Val Gly Pro Ala Met Ser Glu His Phe Asn Val Ser Val Ile
625                 630                 635                 640

gtc tca aac agt cga gag aca aca cag tac agt gcg ttt tcc tat gtg      1968
Val Ser Asn Ser Arg Glu Thr Thr Gln Tyr Ser Ala Phe Ser Tyr Val
                645                 650                 655

gat cct gta ata aca agt att tct cca agg tat ggt cct cat gcc gga      2016
Asp Pro Val Ile Thr Ser Ile Ser Pro Arg Tyr Gly Pro His Ala Gly
            660                 665                 670

ggc acc tta ctc act ttg act gga aaa tac ctc aac agc ggc aat tct      2064
Gly Thr Leu Leu Thr Leu Thr Gly Lys Tyr Leu Asn Ser Gly Asn Ser
        675                 680                 685

aga cac att tca atc gga ggg aaa aca tgt act tta aaa agt gta tca      2112
Arg His Ile Ser Ile Gly Gly Lys Thr Cys Thr Leu Lys Ser Val Ser
    690                 695                 700

gat agc att ctc gaa tgc tac acc cca ggc cac acc gtc tct gcc gag      2160
Asp Ser Ile Leu Glu Cys Tyr Thr Pro Gly His Thr Val Ser Ala Glu
705                 710                 715                 720

ttt ccc gtg aaa ttg aaa atc gac ctg gct gac cga gtg aca agc agc      2208
Phe Pro Val Lys Leu Lys Ile Asp Leu Ala Asp Arg Val Thr Ser Ser
                725                 730                 735

ttc agt tac cgg gaa gac ccg gtt gtc tct gaa atc cac ccg acc aaa      2256
Phe Ser Tyr Arg Glu Asp Pro Val Val Ser Glu Ile His Pro Thr Lys
            740                 745                 750

tct ttt atc agt ggt gga agc aca ata acg ggg att gga aag aac ctg      2304
Ser Phe Ile Ser Gly Gly Ser Thr Ile Thr Gly Ile Gly Lys Asn Leu

        755                 760                 765

aat tca gtt agc acc cca aag ctg gta ata gaa gtg cat gac gtg ggc 2352
Asn Ser Val Ser Thr Pro Lys Leu Val Ile Glu Val His Asp Val Gly
    770                 775                 780

gtg aac tac acc gtg gcg tgc caa cat cgc tcg agt tca gag atc atc 2400
Val Asn Tyr Thr Val Ala Cys Gln His Arg Ser Ser Ser Glu Ile Ile
785                 790                 795                 800

tgc tgc acc act cct tcc ctg cga cag ctg gac ctg caa ctc ccc ctg 2448
Cys Cys Thr Thr Pro Ser Leu Arg Gln Leu Asp Leu Gln Leu Pro Leu
                805                 810                 815

aag acc aaa gcc ttc ttc ctg ctg gac ggg atc ctt tcc aaa cac ttt 2496
Lys Thr Lys Ala Phe Phe Leu Leu Asp Gly Ile Leu Ser Lys His Phe
            820                 825                 830

gat ctc act tat gta cat gat cct atg ttt aag cct ttt gaa aag cca 2544
Asp Leu Thr Tyr Val His Asp Pro Met Phe Lys Pro Phe Glu Lys Pro
        835                 840                 845

gta atg atc tcc atg ggc aat gag aat gta gtg gaa att aag gga gac 2592
Val Met Ile Ser Met Gly Asn Glu Asn Val Val Glu Ile Lys Gly Asp
    850                 855                 860

gat att gac cct gaa gca gtt aaa ggt gaa gtg tta aaa gtc ggg aat 2640
Asp Ile Asp Pro Glu Ala Val Lys Gly Glu Val Leu Lys Val Gly Asn
865                 870                 875                 880

aag agc tgt gag aat ctc cac tgg cat tct gaa gct ttg ttg tgt acg 2688
Lys Ser Cys Glu Asn Leu His Trp His Ser Glu Ala Leu Leu Cys Thr
                885                 890                 895

gtc ccc agt gac ctg ctg aag ctg aac ggc ggc gag cta aat ata gag 2736
Val Pro Ser Asp Leu Leu Lys Leu Asn Gly Gly Glu Leu Asn Ile Glu
            900                 905                 910

tgg aag caa gca gtc tct tca act gtc ctt gga aaa gtg atc gtt caa 2784
Trp Lys Gln Ala Val Ser Ser Thr Val Leu Gly Lys Val Ile Val Gln
        915                 920                 925

ccg gat cag aat ttt gca gga ttg atc att ggt gcg gtc tca ata tca 2832
Pro Asp Gln Asn Phe Ala Gly Leu Ile Ile Gly Ala Val Ser Ile Ser
    930                 935                 940

gtg gta gtt ttg tta gta tcc ggg ctc ttc ctg tgg ctg aga aag aga 2880
Val Val Val Leu Leu Val Ser Gly Leu Phe Leu Trp Leu Arg Lys Arg
945                 950                 955                 960

aag cat aaa gat ctg ggc agt gaa tta gtt cgc tat gac gca aga gta     2928
Lys His Lys Asp Leu Gly Ser Glu Leu Val Arg Tyr Asp Ala Arg Val
                965                 970                 975

cac act cct cat ttg gat agg ctt gta agt gcc cga agt gta agc cca     2976
His Thr Pro His Leu Asp Arg Leu Val Ser Ala Arg Ser Val Ser Pro
            980                 985                 990

act aca gag atg gtc tca aat gag  tct gta gac tac aga  gct act ttt   3024
Thr Thr Glu Met Val Ser Asn Glu  Ser Val Asp Tyr Arg  Ala Thr Phe
        995                 1000                 1005

cca gaa  gac cag ttt ccc aac  tcc tct cag aat gga  gcc tgc aga     3069
Pro Glu  Asp Gln Phe Pro Asn  Ser Ser Gln Asn Gly  Ala Cys Arg
    1010                 1015                 1020

caa gtg  cag tat ctt ctg aca  gat ctg tcc ccc atc  ctg acg agt     3114
Gln Val  Gln Tyr Leu Leu Thr  Asp Leu Ser Pro Ile  Leu Thr Ser
    1025                 1030                 1035

gga gac  tct gat ata tcc agc  cca tta cta caa aac  act gtt cac     3159
Gly Asp  Ser Asp Ile Ser Ser  Pro Leu Leu Gln Asn  Thr Val His
    1040                 1045                 1050

att gac  ctc agc gct cta aat  cca gag ctg gtc caa  gcg gtg ccg     3204
Ile Asp  Leu Ser Ala Leu Asn  Pro Glu Leu Val Gln  Ala Val Pro
    1055                 1060                 1065

cac gta  gtg att gga ccc agt  agc ctg att gtg cat  ttc aat gaa     3249
His Val  Val Ile Gly Pro Ser  Ser Leu Ile Val His  Phe Asn Glu
    1070                 1075                 1080

gtc ata  gga aga ggg cat ttt  ggc tgt gtc tat cat  ggg act ttg     3294
Val Ile  Gly Arg Gly His Phe  Gly Cys Val Tyr His  Gly Thr Leu
    1085                 1090                 1095

ttg gac  agt gac gga aag aaa  att cac tgt gct gtg  aaa tcc ttg     3339
Leu Asp  Ser Asp Gly Lys Lys  Ile His Cys Ala Val  Lys Ser Leu
    1100                 1105                 1110

aac aga  atc aca gat ata gaa  gaa gtc tcc cag ttt  ctg act gag     3384
Asn Arg  Ile Thr Asp Ile Glu  Glu Val Ser Gln Phe  Leu Thr Glu
    1115                 1120                 1125

gga atc  atc atg aaa gat ttc  agc cac ccc aat gtt  ctc tca ctc     3429
Gly Ile  Ile Met Lys Asp Phe  Ser His Pro Asn Val  Leu Ser Leu
    1130                 1135                 1140

ttg gga atc tgc ctg cgg agt gaa ggg tcc cct ctg gtg gtt ctg 3474
Leu Gly Ile Cys Leu Arg Ser Glu Gly Ser Pro Leu Val Val Leu
    1145                1150                1155

ccc tat atg aag cac gga gat ctt cgc aat ttc att cga aat gag 3519
Pro Tyr Met Lys His Gly Asp Leu Arg Asn Phe Ile Arg Asn Glu
    1160                1165                1170

act cat aac cca act gtg aaa gat ctt ata gga ttc ggt ctt caa 3564
Thr His Asn Pro Thr Val Lys Asp Leu Ile Gly Phe Gly Leu Gln
    1175                1180                1185

gta gcc aag ggc atg aaa tat ctt gtc agc aaa aag ttt gtc cac 3609
Val Ala Lys Gly Met Lys Tyr Leu Val Ser Lys Lys Phe Val His
    1190                1195                1200

aga gac tta gct gca aga aac tgc atg ttg gat gaa aaa ttc act 3654
Arg Asp Leu Ala Ala Arg Asn Cys Met Leu Asp Glu Lys Phe Thr
    1205                1210                1215

gtc aag gtt gct gat ttc ggt ctt gcc aga gac atg tac gac aaa 3699
Val Lys Val Ala Asp Phe Gly Leu Ala Arg Asp Met Tyr Asp Lys
    1220                1225                1230

gag tat tat agc gtc cac aac aaa acg ggt gcg aaa cta ccg gtg 3744
Glu Tyr Tyr Ser Val His Asn Lys Thr Gly Ala Lys Leu Pro Val
    1235                1240                1245

aag tgg atg gct tta gag agt ctg cag acg caa aag ttc acc acc 3789
Lys Trp Met Ala Leu Glu Ser Leu Gln Thr Gln Lys Phe Thr Thr
    1250                1255                1260

aag tca gac gtg tgg tcc ttc ggt gtg ctt ctc tgg gag ctc atg 3834
Lys Ser Asp Val Trp Ser Phe Gly Val Leu Leu Trp Glu Leu Met
    1265                1270                1275

acg aga gga gcc cct cct tat cct gac gtg aac aca ttt gat atc 3879
Thr Arg Gly Ala Pro Pro Tyr Pro Asp Val Asn Thr Phe Asp Ile
    1280                1285                1290

act ata tac ctg ttg caa ggc aga aga ctc ttg caa cca gag tac 3924
Thr Ile Tyr Leu Leu Gln Gly Arg Arg Leu Leu Gln Pro Glu Tyr
    1295                1300                1305

tgt cca gac gcc ttg tat gaa gtg atg cta aaa tgc tgg cac ccc 3969
Cys Pro Asp Ala Leu Tyr Glu Val Met Leu Lys Cys Trp His Pro
    1310                1315                1320

aaa gca gaa atg cgc cca tcc gtt tct gaa ctg gtc tcc agg ata 4014

Lys Ala Glu Met Arg Pro Ser Val Ser Glu Leu Val Ser Arg Ile
    1325                1330                1335

tcc tca atc ttc tcc act ttc att ggc gag cac tat gtc cat gtg 4059
Ser Ser Ile Phe Ser Thr Phe Ile Gly Glu His Tyr Val His Val
    1340                1345                1350

aac gct act tat gtg aat gta aaa tgt gtt gct cca tat cct tct 4104
Asn Ala Thr Tyr Val Asn Val Lys Cys Val Ala Pro Tyr Pro Ser
    1355                1360                1365

ctg ttg cca tcc caa gac aac att gac ggc gaa gcg aac aca tga 4149
Leu Leu Pro Ser Gln Asp Asn Ile Asp Gly Glu Ala Asn Thr
    1370                1375                1380

<210> 10
<211> 1382
<212> PRT
<213> Rattus norvegicus

<400> 10
   Met Lys Ala Pro Thr Ala Leu Ala Pro Gly Ile Leu Leu Leu Leu Leu
   1 5 10 15
   Thr Leu Ala Gln Arg Ser His Gly Glu Cys Lys Glu Ala Leu Val Lys
   20 25 30
   Ser Glu Met Asn Val Asn Met Lys Tyr Gln Leu Pro Asn Phe Thr Ala
   35 40 45
   Glu Thr Pro Ile His Asn Val Val Leu Pro Gly His His Ile Tyr Leu
   50 55 60
   Gly Ala Thr Asn Tyr Ile Tyr Val Leu Asn Asp Lys Asp Leu Gln Lys
   65 70 75 80
   Val Ser Glu Phe Lys Thr Gly Pro Val Val Glu His Pro Asp Cys Phe
   85 90 95
   Pro Cys Gln Asp Cys Ser Ser Lys Ala Asn Val Ser Gly Gly Val Trp
   100 105 110
   Lys Asp Asn Val Asn Met Ala Leu Leu Val Asp Thr Tyr Tyr Asp Asp
   115 120 125
   Gln Leu Ile Ser Cys Gly Ser Val Asn Arg Gly Thr Cys Gln Arg His
   130 135 140
   Val Leu Pro Pro Asp Asn Ala Ala Asp Ile Gln Ser Glu Val His Cys
   145 150 155 160
   Met Phe Ser Pro Leu Ala Glu Glu Glu Ser Gly Gln Cys Pro Asp Cys
   165 170 175
   Val Val Ser Ala Leu Gly Ala Lys Val Leu Leu Ser Glu Lys Asp Arg
   180 185 190
   Phe Ile Asn Phe Phe Val Gly Asn Thr Ile Asn Ser Ser Tyr Pro Pro
   195 200 205
   Asp Tyr Ser Leu His Ser Ile Ser Val Arg Arg Leu Lys Glu Thr Gln
   210 215 220
   Asp Gly Phe Lys Phe Leu Thr Asp Gln Ser Tyr Ile Asp Val Leu Pro
   225 230 235 240
   Glu Phe Arg Asp Ser Tyr Pro Ile Lys Tyr Ile His Ala Phe Glu Ser
   245 250 255
   Asn His Phe Ile Tyr Phe Leu Thr Val Gln Lys Glu Thr Leu Asp Ala
   260 265 270
   Gln Thr Phe His Thr Arg Ile Ile Arg Phe Cys Ser Val Asp Ser Gly
   275 280 285
   Leu His Ser Tyr Met Glu Met Pro Leu Glu Cys Ile Leu Thr Glu Lys
   290 295 300
   Arg Arg Lys Arg Ser Thr Arg Glu Glu Val Phe Asn Ile Leu Gln Ala
   305                 310                 315                 320
   Ala Tyr Val Ser Lys Pro Gly Ala Asn Leu Ala Lys Gln Ile Gly Ala
                   325                 330                 335
   Ser Pro Tyr Asp Asp Ile Leu Tyr Gly Val Phe Ala Gln Ser Lys Pro
               340                 345                 350
   Asp Ser Ala Glu Pro Met Asn Arg Ser Ala Val Cys Ala Phe Pro Ile
           355                 360                 365
   Lys Tyr Val Asn Asp Phe Phe Asn Lys Ile Val Asn Lys Asn Asn Val
       370                 375                 380
   Arg Cys Leu Gln His Phe Tyr Gly Pro Asn His Glu His Cys Phe Asn
   385                 390                 395                 400
   Arg Thr Leu Leu Arg Asn Ser Ser Gly Cys Glu Val Arg Ser Asp Glu
                   405                 410                 415
   Tyr Arg Thr Glu Phe Thr Thr Ala Leu Gln Arg Val Asp Leu Phe Met
               420                 425                 430
   Gly Arg Leu Asn His Val Leu Leu Thr Ser Ile Ser Thr Phe Ile Lys
           435                 440                 445
   Gly Asp Leu Thr Ile Ala Asn Leu Gly Thr Ser Glu Gly Arg Phe Met
       450                 455                 460
   Gln Val Val Leu Ser Arg Thr Ala His Phe Thr Pro His Val Asn Phe
   465                 470                 475                 480
   Leu Leu Asp Ser Tyr Pro Val Ser Pro Glu Val Ile Val Glu His Pro
                   485                 490                 495
   Ser Asn Gln Asn Gly Tyr Thr Leu Val Val Thr Gly Lys Lys Ile Thr
               500                 505                 510
   Lys Ile Pro Leu Asn Gly Leu Gly Cys Gly His Phe Gln Ser Cys Ser
           515                 520                 525
   Gln Cys Leu Ser Pro Pro Tyr Phe Ile Gln Cys Gly Trp Cys His Asn
       530                 535                 540
   Arg Cys Val His Ser Asn Glu Cys Pro Ser Gly Thr Trp Thr Gln Glu
   545                 550                 555                 560
   Ile Cys Leu Pro Ala Val Tyr Lys Val Phe Pro Thr Ser Ala Pro Leu
                   565                 570                 575
   Glu Gly Gly Thr Met Leu Thr Ile Cys Gly Trp Asp Phe Gly Phe Lys
               580                 585                 590
   Lys Asn Asn Lys Phe Asp Leu Arg Lys Thr Lys Val Leu Leu Gly Asn
           595                 600                 605
   Glu Ser Cys Thr Leu Thr Leu Ser Glu Ser Thr Thr Asn Thr Leu Lys
       610                 615                 620
   Cys Thr Val Gly Pro Ala Met Ser Glu His Phe Asn Val Ser Val Ile
   625                 630                 635                 640
   Val Ser Asn Ser Arg Glu Thr Thr Gln Tyr Ser Ala Phe Ser Tyr Val
                   645                 650                 655
   Asp Pro Val Ile Thr Ser Ile Ser Pro Arg Tyr Gly Pro His Ala Gly
               660                 665                 670
   Gly Thr Leu Leu Thr Leu Thr Gly Lys Tyr Leu Asn Ser Gly Asn Ser
           675                 680                 685
   Arg His Ile Ser Ile Gly Gly Lys Thr Cys Thr Leu Lys Ser Val Ser
   690                 695                 700
   Asp Ser Ile Leu Glu Cys Tyr Thr Pro Gly His Thr Val Ser Ala Glu
   705                 710                 715                 720
   Phe Pro Val Lys Leu Lys Ile Asp Leu Ala Asp Arg Val Thr Ser Ser
                   725                 730                 735
   Phe Ser Tyr Arg Glu Asp Pro Val Val Ser Glu Ile His Pro Thr Lys
               740                 745                 750
   Ser Phe Ile Ser Gly Gly Ser Thr Ile Thr Gly Ile Gly Lys Asn Leu
           755                 760                 765
   Asn Ser Val Ser Thr Pro Lys Leu Val Ile Glu Val His Asp Val Gly
       770                 775                 780
   Val Asn Tyr Thr Val Ala Cys Gln His Arg Ser Ser Ser Glu Ile Ile
   785                 790                 795                 800
   Cys Cys Thr Thr Pro Ser Leu Arg Gln Leu Asp Leu Gln Leu Pro Leu
                   805                 810                 815
   Lys Thr Lys Ala Phe Phe Leu Leu Asp Gly Ile Leu Ser Lys His Phe
               820                 825                 830
   Asp Leu Thr Tyr Val His Asp Pro Met Phe Lys Pro Phe Glu Lys Pro
           835                 840                 845
   Val Met Ile Ser Met Gly Asn Glu Asn Val Val Glu Ile Lys Gly Asp
       850                 855                 860
   Asp Ile Asp Pro Glu Ala Val Lys Gly Glu Val Leu Lys Val Gly Asn
   865                 870                 875                 880
   Lys Ser Cys Glu Asn Leu His Trp His Ser Glu Ala Leu Leu Cys Thr
                   885                 890                 895
   Val Pro Ser Asp Leu Leu Lys Leu Asn Gly Gly Glu Leu Asn Ile Glu
   900 905 910
   Trp Lys Gln Ala Val Ser Ser Thr Val Leu Gly Lys Val Ile Val Gln
   915 920 925
   Pro Asp Gln Asn Phe Ala Gly Leu Ile Ile Gly Ala Val Ser Ile Ser
   930 935 940
   Val Val Val Leu Leu Val Ser Gly Leu Phe Leu Trp Leu Arg Lys Arg
   945 950 955 960
   Lys His Lys Asp Leu Gly Ser Glu Leu Val Arg Tyr Asp Ala Arg Val
   965 970 975
   His Thr Pro His Leu Asp Arg Leu Val Ser Ala Arg Ser Val Ser Pro
   980 985 990
   Thr Thr Glu Met Val Ser Asn Glu Ser Val Asp Tyr Arg Ala Thr Phe
   995 1000 1005
   Pro Glu Asp Gln Phe Pro Asn Ser Ser Gln Asn Gly Ala Cys Arg
   1010 1015 1020
   Gln Val Gln Tyr Leu Leu Thr Asp Leu Ser Pro Ile Leu Thr Ser
   1025 1030 1035
   Gly Asp Ser Asp Ile Ser Ser Pro Leu Leu Gln Asn Thr Val His
   1040 1045 1050
   Ile Asp Leu Ser Ala Leu Asn Pro Glu Leu Val Gln Ala Val Pro
   1055 1060 1065
   His Val Val Ile Gly Pro Ser Ser Leu Ile Val His Phe Asn Glu
   1070 1075 1080
   Val Ile Gly Arg Gly His Phe Gly Cys Val Tyr His Gly Thr Leu
   1085 1090 1095
   Leu Asp Ser Asp Gly Lys Lys Ile His Cys Ala Val Lys Ser Leu
   1100 1105 1110
   Asn Arg Ile Thr Asp Ile Glu Glu Val Ser Gln Phe Leu Thr Glu
   1115 1120 1125
   Gly Ile Ile Met Lys Asp Phe Ser His Pro Asn Val Leu Ser Leu
   1130 1135 1140
   Leu Gly Ile Cys Leu Arg Ser Glu Gly Ser Pro Leu Val Val Leu
   1145 1150 1155
   Pro Tyr Met Lys His Gly Asp Leu Arg Asn Phe Ile Arg Asn Glu
   1160 1165 1170
   Thr His Asn Pro Thr Val Lys Asp Leu Ile Gly Phe Gly Leu Gln
   1175 1180 1185
   Val Ala Lys Gly Met Lys Tyr Leu Val Ser Lys Lys Phe Val His
   1190 1195 1200
   Arg Asp Leu Ala Ala Arg Asn Cys Met Leu Asp Glu Lys Phe Thr
   1205 1210 1215
   Val Lys Val Ala Asp Phe Gly Leu Ala Arg Asp Met Tyr Asp Lys
   1220 1225 1230
   Glu Tyr Tyr Ser Val His Asn Lys Thr Gly Ala Lys Leu Pro Val
   1235 1240 1245
   Lys Trp Met Ala Leu Glu Ser Leu Gln Thr Gln Lys Phe Thr Thr
   1250 1255 1260
   Lys Ser Asp Val Trp Ser Phe Gly Val Leu Leu Trp Glu Leu Met
   1265 1270 1275
   Thr Arg Gly Ala Pro Pro Tyr Pro Asp Val Asn Thr Phe Asp Ile
   1280 1285 1290
   Thr Ile Tyr Leu Leu Gln Gly Arg Arg Leu Leu Gln Pro Glu Tyr
   1295 1300 1305
   Cys Pro Asp Ala Leu Tyr Glu Val Met Leu Lys Cys Trp His Pro
   1310 1315 1320
   Lys Ala Glu Met Arg Pro Ser Val Ser Glu Leu Val Ser Arg Ile
   1325 1330 1335
   Ser Ser Ile Phe Ser Thr Phe Ile Gly Glu His Tyr Val His Val
   1340 1345 1350
   Asn Ala Thr Tyr Val Asn Val Lys Cys Val Ala Pro Tyr Pro Ser
   1355 1360 1365
   Leu Leu Pro Ser Gln Asp Asn Ile Asp Gly Glu Ala Asn Thr
   1370 1375 1380

<210> 11
<211> 4149
<212> DNA
<213> Rattus norvegicus

<220>
<221> CDS
<222> (1)..(4146)

<400> 11
atg aag gct ccc acc gcg ctg gca cct ggc att ctg ctg ctg ctg ctg      48
Met Lys Ala Pro Thr Ala Leu Ala Pro Gly Ile Leu Leu Leu Leu Leu
1               5                   10                  15

acc ttg gcg cag agg agc cat ggg gag tgc aag gag gcc cta gtg aag      96
Thr Leu Ala Gln Arg Ser His Gly Glu Cys Lys Glu Ala Leu Val Lys

20                       25                       30

tct gag atg aac gtg aac atg aag tac cag ctt ccc aac ttc acc gca          144
Ser Glu Met Asn Val Asn Met Lys Tyr Gln Leu Pro Asn Phe Thr Ala
        35                      40                      45

gaa acc ccc atc cag aat gtc gtc ctc cat ggg cac cat att tat ctc          192
Glu Thr Pro Ile Gln Asn Val Val Leu His Gly His His Ile Tyr Leu
    50                      55                      60

gga gcc aca aac tac att tat gtt tta aat gac aaa gac ctt cag aag          240
Gly Ala Thr Asn Tyr Ile Tyr Val Leu Asn Asp Lys Asp Leu Gln Lys
65                      70                      75                      80

gta tct gag ttc aag acc ggg ccc gtg gtg gaa cac cca gat tgt ttt          288
Val Ser Glu Phe Lys Thr Gly Pro Val Val Glu His Pro Asp Cys Phe
                85                      90                      95

cct tgt cag gac tgc agc agc aaa gcc aat gtg tca gga ggt gtt tgg          336
Pro Cys Gln Asp Cys Ser Ser Lys Ala Asn Val Ser Gly Gly Val Trp
            100                     105                     110

aaa gac aac gtc aac atg gcg ctg ctt gtt gac act tac tat gac gac          384
Lys Asp Asn Val Asn Met Ala Leu Leu Val Asp Thr Tyr Tyr Asp Asp
        115                     120                     125

cag ctc atc agc tgt ggc agc gtc aac aga ggg acc tgc caa agg cat          432
Gln Leu Ile Ser Cys Gly Ser Val Asn Arg Gly Thr Cys Gln Arg His
    130                     135                     140

gtc ctt cct cct gac aat gct gcc gac att cag tcc gag gtt cac tgc          480
Val Leu Pro Pro Asp Asn Ala Ala Asp Ile Gln Ser Glu Val His Cys
145                     150                     155                     160

atg ttc tcc cca ctt gcg gag gaa gag tca ggc cag tgt ccc gac tgt          528
Met Phe Ser Pro Leu Ala Glu Glu Glu Ser Gly Gln Cys Pro Asp Cys
                165                     170                     175

gta gtg agt gcc ctg gga gcc aaa gtc ctc ctg tct gaa aag gac cgg          576
Val Val Ser Ala Leu Gly Ala Lys Val Leu Leu Ser Glu Lys Asp Arg
            180                     185                     190

ttc atc aat ttc ttc gtg ggg aat acg ata aac tct tcc tac cct ccc          624
Phe Ile Asn Phe Phe Val Gly Asn Thr Ile Asn Ser Ser Tyr Pro Pro
        195                     200                     205

gat tat tca ttg cat tca ata tcg gtg agg cgg ctg aag gaa acc cag          672
Asp Tyr Ser Leu His Ser Ile Ser Val Arg Arg Leu Lys Glu Thr Gln
    210                     215                     220

gac ggt ttt aag ttt ttg aca gac cag tcc tac att gat gtc ctg gga 720
Asp Gly Phe Lys Phe Leu Thr Asp Gln Ser Tyr Ile Asp Val Leu Gly
225                 230                 235                 240

gaa ttc cga gat tcc tac ccc atc aag tac ata cat gcc ttc gaa agc 768
Glu Phe Arg Asp Ser Tyr Pro Ile Lys Tyr Ile His Ala Phe Glu Ser
                245                 250                 255

aac cat ttt atc tac ttt ctg act gtc cag aag gaa acc cta gat gct 816
Asn His Phe Ile Tyr Phe Leu Thr Val Gln Lys Glu Thr Leu Asp Ala
            260                 265                 270

cag act ttc cat aca aga ata atc agg ttc tgt tct gta gac tct ggg 864
Gln Thr Phe His Thr Arg Ile Ile Arg Phe Cys Ser Val Asp Ser Gly
        275                 280                 285

ttg cac tcc tac atg gaa atg cct ctg gag tgc att ctg acg gaa aaa 912
Leu His Ser Tyr Met Glu Met Pro Leu Glu Cys Ile Leu Thr Glu Lys
    290                 295                 300

aga aga aag aga tcc aca agg gaa gaa gtg ttt aat atc ctc caa gcc 960
Arg Arg Lys Arg Ser Thr Arg Glu Glu Val Phe Asn Ile Leu Gln Ala
305                 310                 315                 320

gcg tat gtc agt aaa cca ggg gcc aat ctt gct aag caa ata ggg gcc 1008
Ala Tyr Val Ser Lys Pro Gly Ala Asn Leu Ala Lys Gln Ile Gly Ala
                325                 330                 335

agc ccg tat gat gac att ctc tac ggg gtg ttt gca caa agc aag cca 1056
Ser Pro Tyr Asp Asp Ile Leu Tyr Gly Val Phe Ala Gln Ser Lys Pro
            340                 345                 350

gat tct gct gag ccc atg aac cga tca gcg gtc tgt gca ttc ccc atc 1104
Asp Ser Ala Glu Pro Met Asn Arg Ser Ala Val Cys Ala Phe Pro Ile
        355                 360                 365

aaa tat gtc aat gac ttc ttc aac aag att gtc aac aaa aac aac gta 1152
Lys Tyr Val Asn Asp Phe Phe Asn Lys Ile Val Asn Lys Asn Asn Val
    370                 375                 380

cgg tgt ctc cag cat ttt tat gga ccc aac cac gag cac tgt ttc aat 1200
Arg Cys Leu Gln His Phe Tyr Gly Pro Asn His Glu His Cys Phe Asn
385                 390                 395                 400

agg acc ctg ctg aga aat tca tcg ggc tgc gaa gtg cgc agt gac gag 1248
Arg Thr Leu Leu Arg Asn Ser Ser Gly Cys Glu Val Arg Ser Asp Glu
                405                 410                 415

tac cgg acg gag ttt acc aca gcg ctg cag gct gtg gat tta ttc atg 1296
Tyr Arg Thr Glu Phe Thr Thr Ala Leu Gln Ala Val Asp Leu Phe Met
            420                 425                 430

ggc cgg ctc aac cat gta ctc ttg acg tct atc tct acc ttc atc aaa 1344
Gly Arg Leu Asn His Val Leu Leu Thr Ser Ile Ser Thr Phe Ile Lys
        435                 440                 445

ggt gac ctc acc att gct aat cta ggg aca tca gaa ggt cgc ttc atg 1392
Gly Asp Leu Thr Ile Ala Asn Leu Gly Thr Ser Glu Gly Arg Phe Met
    450                 455                 460

cag gtg gtg ctc tct cgc aca gca cat ttc acc ccc cat gtg aat ttc 1440
Gln Val Val Leu Ser Arg Thr Ala His Phe Thr Pro His Val Asn Phe
465                 470                 475                 480

ctc ctg gat tcc cat cct gtg tct ccg gaa gtt att gtc gaa cat cca 1488
Leu Leu Asp Ser His Pro Val Ser Pro Glu Val Ile Val Glu His Pro
                485                 490                 495

tca aat caa aat ggc tat acc ctg gtg gtc aca ggg aag aag atc acc 1536
Ser Asn Gln Asn Gly Tyr Thr Leu Val Val Thr Gly Lys Lys Ile Thr
            500                 505                 510

aag att cca ctg aat ggc cta ggc tgt ggg cat ttc cag tcc tgc agt 1584
Lys Ile Pro Leu Asn Gly Leu Gly Cys Gly His Phe Gln Ser Cys Ser
        515                 520                 525

cag tgt ctc tct gcc ccc tac ttt ata cag tgt ggc tgg tgc cac aat 1632
Gln Cys Leu Ser Ala Pro Tyr Phe Ile Gln Cys Gly Trp Cys His Asn
    530                 535                 540

cgg tgt gtg cat tcc aat gaa tgc ccc agc ggt aca tgg act caa gag 1680
Arg Cys Val His Ser Asn Glu Cys Pro Ser Gly Thr Trp Thr Gln Glu
545                 550                 555                 560

atc tgt ctg cca gca gtt tat aag gtt ttc ccc act agt gca ccc ctc 1728
Ile Cys Leu Pro Ala Val Tyr Lys Val Phe Pro Thr Ser Ala Pro Leu
                565                 570                 575

gaa gga gga aca atg ctg acc ata tgt ggc tgg gac ttt gga ttc aag 1776
Glu Gly Gly Thr Met Leu Thr Ile Cys Gly Trp Asp Phe Gly Phe Lys
            580                 585                 590

aag aat aat aaa ttt gat tta agg aaa acc aaa gtt ctg ctt ggc aac 1824
Lys Asn Asn Lys Phe Asp Leu Arg Lys Thr Lys Val Leu Leu Gly Asn
        595                 600                 605

gag agc tgt acc ttg acc tta agc gag agc acg aca aat acg ttg aaa 1872

Glu Ser Cys Thr Leu Thr Leu Ser Glu Ser Thr Thr Asn Thr Leu Lys
    610                 615                 620

tgc aca gtt ggc ccc gcg atg agt gag cac ttc aat gtg tct gtg atc      1920
Cys Thr Val Gly Pro Ala Met Ser Glu His Phe Asn Val Ser Val Ile
625                 630                 635                 640

gtc tca aac agt cga gag aca aca cag tac agt gcg ttt tcc tat gtg      1968
Val Ser Asn Ser Arg Glu Thr Thr Gln Tyr Ser Ala Phe Ser Tyr Val
                645                 650                 655

gat cct gta ata aca agt att tct cca agg tat ggt cct cat gcc gga      2016
Asp Pro Val Ile Thr Ser Ile Ser Pro Arg Tyr Gly Pro His Ala Gly
            660                 665                 670

ggc acc tta ctc act ttg act gga aaa tac ctc aac agc ggc aat tct      2064
Gly Thr Leu Leu Thr Leu Thr Gly Lys Tyr Leu Asn Ser Gly Asn Ser
        675                 680                 685

aga cac att tca atc gga ggg aaa aca tgt act tta aaa agt gta tca      2112
Arg His Ile Ser Ile Gly Gly Lys Thr Cys Thr Leu Lys Ser Val Ser
    690                 695                 700

gat agc att ctc gaa tgc tac acc cca ggc cac acc gtc tct gcc gag      2160
Asp Ser Ile Leu Glu Cys Tyr Thr Pro Gly His Thr Val Ser Ala Glu
705                 710                 715                 720

ttt ccc gtg aaa ttg aaa atc gac ctg gct gac cga gtg aca agc agc      2208
Phe Pro Val Lys Leu Lys Ile Asp Leu Ala Asp Arg Val Thr Ser Ser
                725                 730                 735

ttc agt tac ggg gaa gac ccg ttt gtc tct gaa atc cac ccg acc aaa      2256
Phe Ser Tyr Gly Glu Asp Pro Phe Val Ser Glu Ile His Pro Thr Lys
            740                 745                 750

tct ttt atc agt ggt gga agc aca ata acg ggg att gga aag aac ctg      2304
Ser Phe Ile Ser Gly Gly Ser Thr Ile Thr Gly Ile Gly Lys Asn Leu
        755                 760                 765

aat tca gtt agc acc cca aag ctg gta ata gaa gtg cat gac gtg ggc      2352
Asn Ser Val Ser Thr Pro Lys Leu Val Ile Glu Val His Asp Val Gly
    770                 775                 780

gtg aac tac acc gtg gcg tgc caa cat cgc tcg agt tca gag atc atc      2400
Val Asn Tyr Thr Val Ala Cys Gln His Arg Ser Ser Ser Glu Ile Ile
785                 790                 795                 800

tgc tgc acc act cct tcc ctg caa cag ctg gac ctg caa ctc ccc ctg      2448
Cys Cys Thr Thr Pro Ser Leu Gln Gln Leu Asp Leu Gln Leu Pro Leu

                805                 810                 815

aag acc aaa gcc ttc ttc ctg ctg gac ggg atc ctt tcc aaa cac ttt 2496
Lys Thr Lys Ala Phe Phe Leu Leu Asp Gly Ile Leu Ser Lys His Phe
            820                 825                 830

gat ctc act tat gta cat gat cct atg ttt aag cct ttt gaa aag cca 2544
Asp Leu Thr Tyr Val His Asp Pro Met Phe Lys Pro Phe Glu Lys Pro
        835                 840                 845

gta atg atc tcc atg ggc aat gag aat gta gtg gaa att aag gga gac 2592
Val Met Ile Ser Met Gly Asn Glu Asn Val Val Glu Ile Lys Gly Asp
    850                 855                 860

gat att gac cct gaa gca gtt aaa ggt gaa gtg tta aaa gtc ggg aat 2640
Asp Ile Asp Pro Glu Ala Val Lys Gly Glu Val Leu Lys Val Gly Asn
865                 870                 875                 880

aag agc tgt gag aat ctc cac tgg cat tct gaa gct ttg ttg tgt acg 2688
Lys Ser Cys Glu Asn Leu His Trp His Ser Glu Ala Leu Leu Cys Thr
                885                 890                 895

gtc ccc agt gac ctg ctg aag ctg aac ggc ggc gag cta aat ata gag 2736
Val Pro Ser Asp Leu Leu Lys Leu Asn Gly Gly Glu Leu Asn Ile Glu
            900                 905                 910

tgg aag caa gca gtc tct tca act gtc ctt gga aaa gtg atc gtt caa 2784
Trp Lys Gln Ala Val Ser Ser Thr Val Leu Gly Lys Val Ile Val Gln
        915                 920                 925

ccg gat cag aat ttt gca gga ttg atc att ggt gcg gtc tca ata tca 2832
Pro Asp Gln Asn Phe Ala Gly Leu Ile Ile Gly Ala Val Ser Ile Ser
    930                 935                 940

gtg gta gtt ttg tta gta tcc ggg ctc ttc ctg tgg ctg aga aag aga 2880
Val Val Val Leu Leu Val Ser Gly Leu Phe Leu Trp Leu Arg Lys Arg
945                 950                 955                 960

aag cat aaa gat ctg ggc agt gaa tta gtt cgc tat gac gca aga gta 2928
Lys His Lys Asp Leu Gly Ser Glu Leu Val Arg Tyr Asp Ala Arg Val
                965                 970                 975

cac act cct cat ttg gat agg ctt gta agt gcc cga agt gta agc cca 2976
His Thr Pro His Leu Asp Arg Leu Val Ser Ala Arg Ser Val Ser Pro
            980                 985                 990

act aca gag atg gtc tca aat gag tct gta gac tac aga gct act ttt 3024
Thr Thr Glu Met Val Ser Asn Glu Ser Val Asp Tyr Arg Ala Thr Phe
        995                 1000                1005

cca gaa gac cag ttt ccc aac tcc tct cag aat gga gcc tgc aga 3069
Pro Glu Asp Gln Phe Pro Asn Ser Ser Gln Asn Gly Ala Cys Arg
    1010                1015                1020

caa gtg cag tat cca ctg aca gat ctg tcc ccc atc ctg acg agt 3114
Gln Val Gln Tyr Pro Leu Thr Asp Leu Ser Pro Ile Leu Thr Ser
    1025                1030                1035

gga gac tct gat ata tcc agc cca tta cta caa aac act gtt cac 3159
Gly Asp Ser Asp Ile Ser Ser Pro Leu Leu Gln Asn Thr Val His
    1040                1045                1050

att gac ctc agc gct cta aat cca gag ctg gtc caa gcg gtg cag 3204
Ile Asp Leu Ser Ala Leu Asn Pro Glu Leu Val Gln Ala Val Gln
    1055                1060                1065

cac gta gtg att gga ccc agt agc ctg att gtg cat ttc aat gaa 3249
His Val Val Ile Gly Pro Ser Ser Leu Ile Val His Phe Asn Glu
    1070                1075                1080

gtc ata gga aga ggg cat ttt ggc tgt gtc tat cat ggg act ttg 3294
Val Ile Gly Arg Gly His Phe Gly Cys Val Tyr His Gly Thr Leu
    1085                1090                1095

ttg gac agt gac gga aag aaa att cac tgt gct gtg aaa tcc ttg 3339
Leu Asp Ser Asp Gly Lys Lys Ile His Cys Ala Val Lys Ser Leu
    1100                1105                1110

aat aga atc aca gat ata gaa gaa gtc tcc cag ttt ctg act gag 3384
Asn Arg Ile Thr Asp Ile Glu Glu Val Ser Gln Phe Leu Thr Glu
    1115                1120                1125

gga atc atc atg aaa gat ttc agc cac ccc aat gtt ctc tca ctc 3429
Gly Ile Ile Met Lys Asp Phe Ser His Pro Asn Val Leu Ser Leu
    1130                1135                1140

ttg gga atc tgc ctg cgg agt gaa ggg tcc cct ctg gtg gtt ctg 3474
Leu Gly Ile Cys Leu Arg Ser Glu Gly Ser Pro Leu Val Val Leu
    1145                1150                1155

ccc tat atg aag cac gga gat ctt cgc aat ttc att cga aac gag 3519
Pro Tyr Met Lys His Gly Asp Leu Arg Asn Phe Ile Arg Asn Glu
    1160                1165                1170

act cat aac cca act gtg aaa gat ctt ata gga ttc ggt ctt caa 3564
Thr His Asn Pro Thr Val Lys Asp Leu Ile Gly Phe Gly Leu Gln
    1175                1180                1185

gta gcc aag ggc atg aaa tat ctt gcc agc aaa aag ttt gtc cac 3609
Val Ala Lys Gly Met Lys Tyr Leu Ala Ser Lys Lys Phe Val His
    1190                1195                1200

aga gac tta gct gca aga aac tgc atg ttg gat gaa aaa ttc act 3654
Arg Asp Leu Ala Ala Arg Asn Cys Met Leu Asp Glu Lys Phe Thr
    1205                1210                1215

gtc aag gtt gct gat ttc ggt ctt gcc aga gac atg tac gac aaa 3699
Val Lys Val Ala Asp Phe Gly Leu Ala Arg Asp Met Tyr Asp Lys
    1220                1225                1230

gag tat tat agc gtc cac aac aaa acg ggt gcg aaa cta ccg gtg 3744
Glu Tyr Tyr Ser Val His Asn Lys Thr Gly Ala Lys Leu Pro Val
    1235                1240                1245

aag tgg atg gct tta gag agt ctg cag acg caa aag ttc acc acc 3789
Lys Trp Met Ala Leu Glu Ser Leu Gln Thr Gln Lys Phe Thr Thr
    1250                1255                1260

aag tca gac gtg tgg tcc ttc ggt gtg ctt ctc tgg gag ctc atg 3834
Lys Ser Asp Val Trp Ser Phe Gly Val Leu Leu Trp Glu Leu Met
    1265                1270                1275

acg aga gga gcc cct cct tat cct gac gtg aac aca ttt gat atc 3879
Thr Arg Gly Ala Pro Pro Tyr Pro Asp Val Asn Thr Phe Asp Ile
    1280                1285                1290

act ata tac ctg ttg caa ggc aga aga ctc ttg caa cca gag tac 3924
Thr Ile Tyr Leu Leu Gln Gly Arg Arg Leu Leu Gln Pro Glu Tyr
    1295                1300                1305

tgt cca gac gcc ttg tat gaa gtg atg cta aaa tgc tgg cac ccc 3969
Cys Pro Asp Ala Leu Tyr Glu Val Met Leu Lys Cys Trp His Pro
    1310                1315                1320

aaa gca gaa atg cgc cca tcg ttt tct gaa ctg gtc tcc aga ata 4014
Lys Ala Glu Met Arg Pro Ser Phe Ser Glu Leu Val Ser Arg Ile
    1325                1330                1335

tcc tca atc ttc tcc act ttc att ggc gag cac tat gtc cat gtg 4059
Ser Ser Ile Phe Ser Thr Phe Ile Gly Glu His Tyr Val His Val
    1340                1345                1350

aac gct act tat gtg aat gta aaa tgt gtt gct cca tat cct tct 4104
Asn Ala Thr Tyr Val Asn Val Lys Cys Val Ala Pro Tyr Pro Ser
    1355                1360                1365

ctg ttg cca tcc caa gac aac att gac ggc gaa gcg aac aca tga 4149

Leu Leu Pro Ser Gln Asp Asn Ile Asp Gly Glu Ala Asn Thr
    1370                1375                1380

<210> 12
<211> 1382
<212> PRT
<213> Rattus norvegicus

<400> 12

Met Lys Ala Pro Thr Ala Leu Ala Pro Gly Ile Leu Leu Leu Leu Leu
1               5                   10                  15

Thr Leu Ala Gln Arg Ser His Gly Glu Cys Lys Glu Ala Leu Val Lys
            20                  25                  30

Ser Glu Met Asn Val Asn Met Lys Tyr Gln Leu Pro Asn Phe Thr Ala
        35                  40                  45

Glu Thr Pro Ile Gln Asn Val Val Leu His Gly His His Ile Tyr Leu
    50                  55                  60

Gly Ala Thr Asn Tyr Ile Tyr Val Leu Asn Asp Lys Asp Leu Gln Lys
65                  70                  75                  80

Val Ser Glu Phe Lys Thr Gly Pro Val Val Glu His Pro Asp Cys Phe
                85                  90                  95

Pro Cys Gln Asp Cys Ser Ser Lys Ala Asn Val Ser Gly Gly Val Trp
            100                 105                 110

Lys Asp Asn Val Asn Met Ala Leu Leu Val Asp Thr Tyr Tyr Asp Asp
        115                 120                 125

Gln Leu Ile Ser Cys Gly Ser Val Asn Arg Gly Thr Cys Gln Arg His
    130                 135                 140

Val Leu Pro Pro Asp Asn Ala Ala Asp Ile Gln Ser Glu Val His Cys
145                 150                 155                 160

Met Phe Ser Pro Leu Ala Glu Glu Glu Ser Gly Gln Cys Pro Asp Cys
                165                 170                 175

Val Val Ser Ala Leu Gly Ala Lys Val Leu Leu Ser Glu Lys Asp Arg
            180                 185                 190

Phe Ile Asn Phe Phe Val Gly Asn Thr Ile Asn Ser Ser Tyr Pro Pro
        195                 200                 205

Asp Tyr Ser Leu His Ser Ile Ser Val Arg Arg Leu Lys Glu Thr Gln
    210                 215                 220

Asp Gly Phe Lys Phe Leu Thr Asp Gln Ser Tyr Ile Asp Val Leu Gly
225                 230                 235                 240

Glu Phe Arg Asp Ser Tyr Pro Ile Lys Tyr Ile His Ala Phe Glu Ser
                245                 250                 255

Asn His Phe Ile Tyr Phe Leu Thr Val Gln Lys Glu Thr Leu Asp Ala
            260                 265                 270

Gln Thr Phe His Thr Arg Ile Ile Arg Phe Cys Ser Val Asp Ser Gly
        275                 280                 285

Leu His Ser Tyr Met Glu Met Pro Leu Glu Cys Ile Leu Thr Glu Lys
    290                 295                 300

Arg Arg Lys Arg Ser Thr Arg Glu Glu Val Phe Asn Ile Leu Gln Ala
305                 310                 315                 320

Ala Tyr Val Ser Lys Pro Gly Ala Asn Leu Ala Lys Gln Ile Gly Ala
                325                 330                 335

Ser Pro Tyr Asp Asp Ile Leu Tyr Gly Val Phe Ala Gln Ser Lys Pro
            340                 345                 350

Asp Ser Ala Glu Pro Met Asn Arg Ser Ala Val Cys Ala Phe Pro Ile
        355                 360                 365

Lys Tyr Val Asn Asp Phe Phe Asn Lys Ile Val Asn Lys Asn Asn Val
    370                 375                 380

Arg Cys Leu Gln His Phe Tyr Gly Pro Asn His Glu His Cys Phe Asn
385                 390                 395                 400

Arg Thr Leu Leu Arg Asn Ser Ser Gly Cys Glu Val Arg Ser Asp Glu
                405                 410                 415

Tyr Arg Thr Glu Phe Thr Thr Ala Leu Gln Ala Val Asp Leu Phe Met
            420                 425                 430

Gly Arg Leu Asn His Val Leu Leu Thr Ser Ile Ser Thr Phe Ile Lys
        435                 440                 445

Gly Asp Leu Thr Ile Ala Asn Leu Gly Thr Ser Glu Gly Arg Phe Met
    450                 455                 460

Gln Val Val Leu Ser Arg Thr Ala His Phe Thr Pro His Val Asn Phe
465                 470                 475                 480

Leu Leu Asp Ser His Pro Val Ser Pro Glu Val Ile Val Glu His Pro
                485                 490                 495

Ser Asn Gln Asn Gly Tyr Thr Leu Val Val Thr Gly Lys Lys Ile Thr
            500                 505                 510

Lys Ile Pro Leu Asn Gly Leu Gly Cys Gly His Phe Gln Ser Cys Ser
        515                 520                 525

Gln Cys Leu Ser Ala Pro Tyr Phe Ile Gln Cys Gly Trp Cys His Asn
    530                 535                 540

Arg Cys Val His Ser Asn Glu Cys Pro Ser Gly Thr Trp Thr Gln Glu
545                 550                 555                 560

Ile Cys Leu Pro Ala Val Tyr Lys Val Phe Pro Thr Ser Ala Pro Leu
                565                 570                 575

Glu Gly Gly Thr Met Leu Thr Ile Cys Gly Trp Asp Phe Gly Phe Lys
            580                 585                 590

Lys Asn Asn Lys Phe Asp Leu Arg Lys Thr Lys Val Leu Leu Gly Asn
        595                 600                 605

Glu Ser Cys Thr Leu Thr Leu Ser Glu Ser Thr Thr Asn Thr Leu Lys
    610                 615                 620

Cys Thr Val Gly Pro Ala Met Ser Glu His Phe Asn Val Ser Val Ile
625                 630                 635                 640

Val Ser Asn Ser Arg Glu Thr Thr Gln Tyr Ser Ala Phe Ser Tyr Val
                645                 650                 655

Asp Pro Val Ile Thr Ser Ile Ser Pro Arg Tyr Gly Pro His Ala Gly
            660                 665                 670

Gly Thr Leu Leu Thr Leu Thr Gly Lys Tyr Leu Asn Ser Gly Asn Ser
        675                 680                 685

Arg His Ile Ser Ile Gly Gly Lys Thr Cys Thr Leu Lys Ser Val Ser
    690                 695                 700

Asp Ser Ile Leu Glu Cys Tyr Thr Pro Gly His Thr Val Ser Ala Glu
705                 710                 715                 720

Phe Pro Val Lys Leu Lys Ile Asp Leu Ala Asp Arg Val Thr Ser Ser
                725                 730                 735

Phe Ser Tyr Gly Glu Asp Pro Phe Val Ser Glu Ile His Pro Thr Lys

            740                 745                 750

Ser Phe Ile Ser Gly Gly Ser Thr Ile Thr Gly Ile Gly Lys Asn Leu
        755                 760                 765

Asn Ser Val Ser Thr Pro Lys Leu Val Ile Glu Val His Asp Val Gly
    770                 775                 780

Val Asn Tyr Thr Val Ala Cys Gln His Arg Ser Ser Ser Glu Ile Ile
785                 790                 795                 800

Cys Cys Thr Thr Pro Ser Leu Gln Gln Leu Asp Leu Gln Leu Pro Leu
                805                 810                 815

Lys Thr Lys Ala Phe Phe Leu Leu Asp Gly Ile Leu Ser Lys His Phe
            820                 825                 830

Asp Leu Thr Tyr Val His Asp Pro Met Phe Lys Pro Phe Glu Lys Pro
        835                 840                 845

Val Met Ile Ser Met Gly Asn Glu Asn Val Val Glu Ile Lys Gly Asp
    850                 855                 860

Asp Ile Asp Pro Glu Ala Val Lys Gly Glu Val Leu Lys Val Gly Asn
865                 870                 875                 880

Lys Ser Cys Glu Asn Leu His Trp His Ser Glu Ala Leu Leu Cys Thr
                885                 890                 895

Val Pro Ser Asp Leu Leu Lys Leu Asn Gly Gly Glu Leu Asn Ile Glu
            900                 905                 910

Trp Lys Gln Ala Val Ser Ser Thr Val Leu Gly Lys Val Ile Val Gln
        915                 920                 925

Pro Asp Gln Asn Phe Ala Gly Leu Ile Ile Gly Ala Val Ser Ile Ser
    930                 935                 940

Val Val Val Leu Leu Val Ser Gly Leu Phe Leu Trp Leu Arg Lys Arg
945                 950                 955                 960

Lys His Lys Asp Leu Gly Ser Glu Leu Val Arg Tyr Asp Ala Arg Val
                965                 970                 975

His Thr Pro His Leu Asp Arg Leu Val Ser Ala Arg Ser Val Ser Pro
            980                 985                 990

Thr Thr Glu Met Val Ser Asn Glu  Ser Val Asp Tyr Arg  Ala Thr Phe
        995                 1000                 1005

Pro Glu  Asp Gln Phe Pro Asn  Ser Ser Gln Asn Gly  Ala Cys Arg
    1010                 1015                 1020

Gln Val  Gln Tyr Pro Leu Thr  Asp Leu Ser Pro Ile  Leu Thr Ser
    1025                 1030                 1035

Gly Asp  Ser Asp Ile Ser Ser  Pro Leu Leu Gln Asn  Thr Val His
    1040                 1045                 1050

Ile Asp  Leu Ser Ala Leu Asn  Pro Glu Leu Val Gln  Ala Val Gln
    1055                 1060                 1065

His Val  Val Ile Gly Pro Ser  Ser Leu Ile Val His  Phe Asn Glu
    1070                 1075                 1080

Val Ile  Gly Arg Gly His Phe  Gly Cys Val Tyr His  Gly Thr Leu
    1085                 1090                 1095

Leu Asp  Ser Asp Gly Lys Lys  Ile His Cys Ala Val  Lys Ser Leu
    1100                 1105                 1110

Asn Arg  Ile Thr Asp Ile Glu  Glu Val Ser Gln Phe  Leu Thr Glu
    1115                 1120                 1125

Gly Ile Ile Met Lys Asp Phe Ser His Pro Asn Val Leu Ser Leu
    1130                1135                1140

Leu Gly Ile Cys Leu Arg Ser Glu Gly Ser Pro Leu Val Val Leu
    1145                1150                1155

Pro Tyr Met Lys His Gly Asp Leu Arg Asn Phe Ile Arg Asn Glu
    1160                1165                1170

Thr His Asn Pro Thr Val Lys Asp Leu Ile Gly Phe Gly Leu Gln
    1175                1180                1185

Val Ala Lys Gly Met Lys Tyr Leu Ala Ser Lys Lys Phe Val His
    1190                1195                1200

Arg Asp Leu Ala Ala Arg Asn Cys Met Leu Asp Glu Lys Phe Thr
    1205                1210                1215

Val Lys Val Ala Asp Phe Gly Leu Ala Arg Asp Met Tyr Asp Lys
    1220                1225                1230

Glu Tyr Tyr Ser Val His Asn Lys Thr Gly Ala Lys Leu Pro Val
    1235                1240                1245

Lys Trp Met Ala Leu Glu Ser Leu Gln Thr Gln Lys Phe Thr Thr
    1250                1255                1260

Lys Ser Asp Val Trp Ser Phe Gly Val Leu Leu Trp Glu Leu Met
    1265                1270                1275

Thr Arg Gly Ala Pro Pro Tyr Pro Asp Val Asn Thr Phe Asp Ile
    1280                1285                1290

Thr Ile Tyr Leu Leu Gln Gly Arg Arg Leu Leu Gln Pro Glu Tyr
    1295                1300                1305

Cys Pro Asp Ala Leu Tyr Glu Val Met Leu Lys Cys Trp His Pro
    1310                1315                1320

Lys Ala Glu Met Arg Pro Ser Phe Ser Glu Leu Val Ser Arg Ile
    1325                1330                1335

Ser Ser Ile Phe Ser Thr Phe Ile Gly Glu His Tyr Val His Val
    1340                1345                1350

Asn Ala Thr Tyr Val Asn Val Lys Cys Val Ala Pro Tyr Pro Ser
    1355                1360                1365

Leu Leu Pro Ser Gln Asp Asn Ile Asp Gly Glu Ala Asn Thr
    1370                1375                1380

<210> 13
<211> 4188
<212> DNA
<213> Rattus norvegicus

<220>
<221> CDS
<222> (1)..(4185)

<400> 13
   atg aag gct ccc acc gcg ctg gca cct ggc att ctg ctg ctg ctg ctg      48
   Met Lys Ala Pro Thr Ala Leu Ala Pro Gly Ile Leu Leu Leu Leu Leu
   1               5                   10                  15
   acc ttg gcg cag agg agc cat ggg gag tgc aag gag gcc cta gtg aag 96
   Thr Leu Ala Gln Arg Ser His Gly Glu Cys Lys Glu Ala Leu Val Lys
   20 25 30
   tct gag atg aac gtg aac atg aag tac cag ctt ccc aac ttc acc gca 144
   Ser Glu Met Asn Val Asn Met Lys Tyr Gln Leu Pro Asn Phe Thr Ala
   35 40 45
   gaa acc ccc att cac aat gtc gtc ctc cat ggg cac cat att tat ctc 192
   Glu Thr Pro Ile His Asn Val Val Leu His Gly His His Ile Tyr Leu
   50 55 60
   gga gcc aca aac tac att tat gtt tta aat gac aaa gac ctt cag aag 240
   Gly Ala Thr Asn Tyr Ile Tyr Val Leu Asn Asp Lys Asp Leu Gln Lys
   65                  70                  75                  80
   gta tct gag ttc aag acc ggg ccc gtg gtg gaa cac cca gat tgt ttt      288
   Val Ser Glu Phe Lys Thr Gly Pro Val Val Glu His Pro Asp Cys Phe
                   85                  90                  95
   cct tgt cag gac tgc agc agc aaa gcc aat gtg tca gga ggt gtt tgg      336
   Pro Cys Gln Asp Cys Ser Ser Lys Ala Asn Val Ser Gly Gly Val Trp
               100                 105                 110
   aaa gac aac gtc aac atg gcg ctg ctt gtt gac act tac tat gac gac      384
   Lys Asp Asn Val Asn Met Ala Leu Leu Val Asp Thr Tyr Tyr Asp Asp
           115                 120                 125
   cag ctc atc agc tgt ggc agc gtc aac aga ggg acc tgc caa agg cat      432
   Gln Leu Ile Ser Cys Gly Ser Val Asn Arg Gly Thr Cys Gln Arg His
       130                 135                 140
   gtc ctt cct cct gac aat gct gcc gac att cag tcc gag gtt cac tgc      480
   Val Leu Pro Pro Asp Asn Ala Ala Asp Ile Gln Ser Glu Val His Cys
   145                 150                 155                 160
   atg ttc tcc cca ctt gcg gag gaa gag tca ggc cag tgt ccc gac tgt      528
   Met Phe Ser Pro Leu Ala Glu Glu Glu Ser Gly Gln Cys Pro Asp Cys
                   165                 170                 175
   gta gtg agt gcc ctg gga gcc aaa gtc ctc ctg tct gaa aag gac cgg      576
   Val Val Ser Ala Leu Gly Ala Lys Val Leu Leu Ser Glu Lys Asp Arg
               180                 185                 190
   ttc atc aat ttc ttc gtg ggg aat acg ata aac tct tcc tac cct ccc      624
   Phe Ile Asn Phe Phe Val Gly Asn Thr Ile Asn Ser Ser Tyr Pro Pro
           195                 200                 205
   gat tat tca ttg cat tca ata tcg gtg agg cgg ctg aag gaa acc cag      672
   Asp Tyr Ser Leu His Ser Ile Ser Val Arg Arg Leu Lys Glu Thr Gln
       210                 215                 220
   gac ggt ttt aag ttt ttg aca gac cag tcc tac att gat gtc ctg cca      720
   Asp Gly Phe Lys Phe Leu Thr Asp Gln Ser Tyr Ile Asp Val Leu Pro
   225                 230                 235                 240
   gaa ttc cga gat tcc tac ccc atc aag tac ata cat gcc ttc gaa agc      768
   Glu Phe Arg Asp Ser Tyr Pro Ile Lys Tyr Ile His Ala Phe Glu Ser
                   245                 250                 255
   aac cat ttt atc tac ttt ctg act gtc cag aag gaa acc cta gat gct      816
   Asn His Phe Ile Tyr Phe Leu Thr Val Gln Lys Glu Thr Leu Asp Ala
               260                 265                 270
   cag act ttc cat aca aga ata atc agg ttc tgt tct gta gac tct ggg 864
   Gln Thr Phe His Thr Arg Ile Ile Arg Phe Cys Ser Val Asp Ser Gly
   275 280 285
   ttg cac tcc tac atg gaa atg cct ctg gag tgc att ctg acg gaa aaa 912
   Leu His Ser Tyr Met Glu Met Pro Leu Glu Cys Ile Leu Thr Glu Lys
   290 295 300
   aga aga aag aga tcc aca agg gaa gaa gtg ttt aat atc ctc caa gcc 960
   Arg Arg Lys Arg Ser Thr Arg Glu Glu Val Phe Asn Ile Leu Gln Ala
   305 310 315 320
   gca tat gtc agt aaa ccg ggg gcc aat ctt gct aag caa ata ggg gct 1008
   Ala Tyr Val Ser Lys Pro Gly Ala Asn Leu Ala Lys Gln Ile Gly Ala
   325 330 335
   agc ccg tat gat gac att ctc tac ggg gtg ttt gca caa agc aag cca 1056
   Ser Pro Tyr Asp Asp Ile Leu Tyr Gly Val Phe Ala Gln Ser Lys Pro
   340 345 350
   gat tct gct gag ccc atg aac cga tca gcg gtc tgt gca ttc ccc atc 1104
   Asp Ser Ala Glu Pro Met Asn Arg Ser Ala Val Cys Ala Phe Pro Ile
   355 360 365
   aaa tat gtc aat gac ttc ttc aac aag att gtc aac aaa aac aac gta 1152
   Lys Tyr Val Asn Asp Phe Phe Asn Lys Ile Val Asn Lys Asn Asn Val
   370 375 380
   cgg tgt ctc cag cat ttt tat gga ccc aac cac gag cac tgt ttc aat 1200
   Arg Cys Leu Gln His Phe Tyr Gly Pro Asn His Glu His Cys Phe Asn
   385 390 395 400
   agg acc ctg ctg aga aat tca tcg ggc tgc gaa gtg cgc agt gac gag 1248
   Arg Thr Leu Leu Arg Asn Ser Ser Gly Cys Glu Val Arg Ser Asp Glu
   405 410 415
   tac cgg acg gag ttt acc aca gcg ctg cag cgt gtg gat tta ttc atg 1296
   Tyr Arg Thr Glu Phe Thr Thr Ala Leu Gln Arg Val Asp Leu Phe Met
   420 425 430
   ggc cgg ctc aac cat gta ctc ttg acg tct atc tct acc ttc atc aaa 1344
   Gly Arg Leu Asn His Val Leu Leu Thr Ser Ile Ser Thr Phe Ile Lys
   435 440 445
   ggt gac ctc acc att gct aat cta ggg aca tca gaa ggt cgc ttc atg 1392
   Gly Asp Leu Thr Ile Ala Asn Leu Gly Thr Ser Glu Gly Arg Phe Met
   450 455 460
   cag gtg gtg ctc tct cgc aca gca cat ttc acc ccc cat gtg aat ttc 1440
   Gln Val Val Leu Ser Arg Thr Ala His Phe Thr Pro His Val Asn Phe
   465 470 475 480
   ctc ctg gat tcc tat cct gtg tct ccg gaa gtt att gtc gaa cat cca 1488
   Leu Leu Asp Ser Tyr Pro Val Ser Pro Glu Val Ile Val Glu His Pro
   485 490 495
   tca aat caa aat ggc tat acc ttg gtg gtc aca ggg aag aag atc acc 1536
   Ser Asn Gln Asn Gly Tyr Thr Leu Val Val Thr Gly Lys Lys Ile Thr
   500 505 510
   aag att cca ctg aat ggc cta ggc tgt ggg cat ttc cag tcc tgc agt 1584
   Lys Ile Pro Leu Asn Gly Leu Gly Cys Gly His Phe Gln Ser Cys Ser
   515 520 525
   cag tgt ctc tct gcc ccc tac ttt ata cag tgt ggc tgg tgc cac aat 1632
   Gln Cys Leu Ser Ala Pro Tyr Phe Ile Gln Cys Gly Trp Cys His Asn
   530 535 540
   cgg tgt gtg cat tcc aat gaa tgc ccc agc ggt aca tgg act caa gag 1680
   Arg Cys Val His Ser Asn Glu Cys Pro Ser Gly Thr Trp Thr Gln Glu
   545 550 555 560
   atc tgt ctg cca gca gtt tat aag gtt ttc ccc act agt gca ccc ctc 1728
   Ile Cys Leu Pro Ala Val Tyr Lys Val Phe Pro Thr Ser Ala Pro Leu
   565 570 575
   gaa gga gga aca atg ctg acc ata tgt ggc tgg gac ttt gga ttc aag 1776
   Glu Gly Gly Thr Met Leu Thr Ile Cys Gly Trp Asp Phe Gly Phe Lys
   580 585 590
   aag aat aat aaa ttt gat tta agg aaa acc aaa gtt ctg ctt ggc aac 1824
   Lys Asn Asn Lys Phe Asp Leu Arg Lys Thr Lys Val Leu Leu Gly Asn
   595 600 605
   gag agc tgt acc ttg acc tta agc gag agc acg aca aat acg ttg aaa 1872
   Glu Ser Cys Thr Leu Thr Leu Ser Glu Ser Thr Thr Asn Thr Leu Lys
   610 615 620
   tgc aca gtt ggc ccc gcg atg agt gag cac ttc aat gtg tct gtg atc 1920
   Cys Thr Val Gly Pro Ala Met Ser Glu His Phe Asn Val Ser Val Ile
   625 630 635 640
   gtc tca aac agt cga gag aca aca cag tac agt gcg ttt tcc tat gtg 1968
   Val Ser Asn Ser Arg Glu Thr Thr Gln Tyr Ser Ala Phe Ser Tyr Val
   645 650 655
   gat cct gta ata aca agt att tct cca agg tat ggt cct cat gcc gga 2016
   Asp Pro Val Ile Thr Ser Ile Ser Pro Arg Tyr Gly Pro His Ala Gly
   660 665 670
   ggc acc tta ctc act ttg act gga aaa tac ctc aac agc ggc aat tct 2064
   Gly Thr Leu Leu Thr Leu Thr Gly Lys Tyr Leu Asn Ser Gly Asn Ser
   675 680 685
   aga cac att tca atc gga ggg aaa aca tgt act tta aaa agt gta tca 2112
   Arg His Ile Ser Ile Gly Gly Lys Thr Cys Thr Leu Lys Ser Val Ser
   690 695 700
   gat agc att ctc gaa tgc tac acc cca ggc cac acc gtc tct gcc gag 2160
   Asp Ser Ile Leu Glu Cys Tyr Thr Pro Gly His Thr Val Ser Ala Glu
   705 710 715 720
   ttt ccc gtg aaa ttg aaa atc gac ctg gtt gac cga gtg aca agc agc 2208
   Phe Pro Val Lys Leu Lys Ile Asp Leu Val Asp Arg Val Thr Ser Ser
   725 730 735
   ttc agt tac cgg gaa gac ccg gtt gtc tct gaa atc cac ccg acc aaa 2256
   Phe Ser Tyr Arg Glu Asp Pro Val Val Ser Glu Ile His Pro Thr Lys
   740 745 750
   tct ttt atc agt ggt gga agc aca ata acg ggg att gga aag aac ctg 2304
   Ser Phe Ile Ser Gly Gly Ser Thr Ile Thr Gly Ile Gly Lys Asn Leu
   755 760 765
   aat tca gtt agc acc cca aag ctg gta ata gaa gtg cat gac gtg ggc 2352
   Asn Ser Val Ser Thr Pro Lys Leu Val Ile Glu Val His Asp Val Gly
   770 775 780
   gtg aac tac acc gtg gcg tgc caa cat cgc tcg agt tca gag atc atc 2400
   Val Asn Tyr Thr Val Ala Cys Gln His Arg Ser Ser Ser Glu Ile Ile
   785 790 795 800
   tgc tgc acc act cct tcc ctg caa cag ctg gac ctg caa ctc ccc ctg 2448
   Cys Cys Thr Thr Pro Ser Leu Gln Gln Leu Asp Leu Gln Leu Pro Leu
   805 810 815
   aag acc aaa gcc ttc ttc ctg ctg gac ggg atc ctt tcc aaa cac ttt 2496
   Lys Thr Lys Ala Phe Phe Leu Leu Asp Gly Ile Leu Ser Lys His Phe
   820 825 830
   gat ctc act tat gta cat gat cct atg ttt aag cct ttt gaa aag cca 2544
   Asp Leu Thr Tyr Val His Asp Pro Met Phe Lys Pro Phe Glu Lys Pro
   835 840 845
   gta atg atc tcc atg ggc aat gag aat gta gtg gaa att aag gga gac 2592
   Val Met Ile Ser Met Gly Asn Glu Asn Val Val Glu Ile Lys Gly Asp
   850                 855                 860
   gat att gac cct gaa gca gtt aaa ggt gaa gtg tta aaa gtc ggg aat     2640
   Asp Ile Asp Pro Glu Ala Val Lys Gly Glu Val Leu Lys Val Gly Asn
   865                 870                 875                 880
   aag agc tgt gag aat ctc cac tgg cat tct gaa gct ttg ttg tgt acg     2688
   Lys Ser Cys Glu Asn Leu His Trp His Ser Glu Ala Leu Leu Cys Thr
                   885                 890                 895
   gtc ccc agt gac ctg ctg aag ctg aac ggc ggc gag cta aat ata gag     2736
   Val Pro Ser Asp Leu Leu Lys Leu Asn Gly Gly Glu Leu Asn Ile Glu
               900                 905                 910
   tgg aag caa gca gtc tct tca act gtc ctt gga aaa gtg atc gtt caa     2784
   Trp Lys Gln Ala Val Ser Ser Thr Val Leu Gly Lys Val Ile Val Gln
           915                 920                 925
   ccg gat cag aat ttt gca gga ttg atc att ggt gcg gtc tca ata tca     2832
   Pro Asp Gln Asn Phe Ala Gly Leu Ile Ile Gly Ala Val Ser Ile Ser
       930                 935                 940
   gtg gta gtt tgg tta gta tcc ggg ctc ttc ctg tgg ctg aga aag aga     2880
   Val Val Val Trp Leu Val Ser Gly Leu Phe Leu Trp Leu Arg Lys Arg
   945                 950                 955                 960
   aag cat aaa gat ctg ggc agt gaa tta gtt cgc tat gac gca aga gta     2928
   Lys His Lys Asp Leu Gly Ser Glu Leu Val Arg Tyr Asp Ala Arg Val
                   965                 970                 975
   cac act cct cat ttg gat agg ctt gta agt gcc cga agt gta agc cca     2976
   His Thr Pro His Leu Asp Arg Leu Val Ser Ala Arg Ser Val Ser Pro
               980                 985                 990
   act aca gag atg gtc tca aat gag  tct gta gac tac aga  gct act ttt     3024
   Thr Thr Glu Met Val Ser Asn Glu  Ser Val Asp Tyr Arg  Ala Thr Phe
           995                 1000                1005
   cca gaa  gac cag ttt ccc aac  tcc tct cag aat gga  gcc tgc aga     3069
   Pro Glu  Asp Gln Phe Pro Asn  Ser Ser Gln Asn Gly  Ala Cys Arg
       1010                1015                1020
   caa gtg  cag tat cct ctg aca  gat ctg tcc ccc atc  ctg acg agt     3114
   Gln Val  Gln Tyr Pro Leu Thr  Asp Leu Ser Pro Ile  Leu Thr Ser
       1025                1030                1035
   gga gac  tct gat ata tcc agc  cca tta cta caa aac  act gtt cac     3159
   Gly Asp  Ser Asp Ile Ser Ser  Pro Leu Leu Gln Asn  Thr Val His
       1040                1045                1050
   att gac ctc agc gct cta aat cca gag ctg gtc caa gcg gtg cag 3204
   Ile Asp Leu Ser Ala Leu Asn Pro Glu Leu Val Gln Ala Val Gln
   1055 1060 1065
   cac gta gtg att gga ccc agt agc ctg att gtg cat ttc aat gaa 3249
   His Val Val Ile Gly Pro Ser Ser Leu Ile Val His Phe Asn Glu
   1070 1075 1080
   gtc ata gga aga ggg cat ttt ggc tgt gtc tat cat ggg act ttg 3294
   Val Ile Gly Arg Gly His Phe Gly Cys Val Tyr His Gly Thr Leu
   1085 1090 1095
   ttg gac agt gac gga aag aaa att cac tgt gct gtg aaa tcc ttg 3339
   Leu Asp Ser Asp Gly Lys Lys Ile His Cys Ala Val Lys Ser Leu
   1100 1105 1110
   aac aga atc aca gat ata gaa gaa gtc tcc cag ttt ctg act gag 3384
   Asn Arg Ile Thr Asp Ile Glu Glu Val Ser Gln Phe Leu Thr Glu
   1115 1120 1125
   gga atc atc atg aaa gat ttc agc cac ccc aat gtt ctc tca ctc 3429
   Gly Ile Ile Met Lys Asp Phe Ser His Pro Asn Val Leu Ser Leu
   1130 1135 1140
   ttg gga atc tgc ctg cgg agt gaa ggg tcc cct ctg gtg gtt ctg 3474
   Leu Gly Ile Cys Leu Arg Ser Glu Gly Ser Pro Leu Val Val Leu
   1145 1150 1155
   ccc tat atg aag cac gga gat ctt cgc aat ttc att cga aat gag 3519
   Pro Tyr Met Lys His Gly Asp Leu Arg Asn Phe Ile Arg Asn Glu
   1160 1165 1170
   act cat aac cca act gtg aaa gat ctt ata gga ttc ggt ctt caa 3564
   Thr His Asn Pro Thr Val Lys Asp Leu Ile Gly Phe Gly Leu Gln
   1175 1180 1185
   gta gcc aag ggc atg aaa tat ctt gcc agc aaa aag ttt gtc cac 3609
   Val Ala Lys Gly Met Lys Tyr Leu Ala Ser Lys Lys Phe Val His
   1190 1195 1200
   aga gac tta gct gca aga aac tgc atg ttg gat gaa aaa ttc act 3654
   Arg Asp Leu Ala Ala Arg Asn Cys Met Leu Asp Glu Lys Phe Thr
   1205 1210 1215
   gtc aag gtt gct gat ttc ggt ctt gcc aga gac atg tac gac aaa 3699
   Val Lys Val Ala Asp Phe Gly Leu Ala Arg Asp Met Tyr Asp Lys
   1220 1225 1230
   gag tat tat agc gtc cac aac aaa acg ggt gcg aaa cta ccg gtg 3744
   Glu Tyr Tyr Ser Val His Asn Lys Thr Gly Ala Lys Leu Pro Val
   1235 1240 1245
   aag tgg atg gct tta gag agt ctg cag acg caa aag ttc acc acc 3789
   Lys Trp Met Ala Leu Glu Ser Leu Gln Thr Gln Lys Phe Thr Thr
   1250 1255 1260
   aag tca gac gtg tgg tcc ttc ggt gtg ctt ctc tgg gag ctc atg 3834
   Lys Ser Asp Val Trp Ser Phe Gly Val Leu Leu Trp Glu Leu Met
   1265 1270 1275
   acg aga gga gcc cct cct tat cct gac gtg aac aca ttt gat atc 3879
   Thr Arg Gly Ala Pro Pro Tyr Pro Asp Val Asn Thr Phe Asp Ile
   1280 1285 1290
   act ata tac ctg ttg caa ggc aga aga ctc ttg caa cca gag tac 3924
   Thr Ile Tyr Leu Leu Gln Gly Arg Arg Leu Leu Gln Pro Glu Tyr
   1295 1300 1305
   tgt cca gac gcc ttg tat gaa gtg atg cta aaa tgc tgg cac ccc 3969
   Cys Pro Asp Ala Leu Tyr Glu Val Met Leu Lys Cys Trp His Pro
   1310 1315 1320
   aaa gca gaa atg cgc cca tcc cgt tct gaa ctg gtc tcc agg ata 4014
   Lys Ala Glu Met Arg Pro Ser Arg Ser Glu Leu Val Ser Arg Ile
   1325 1330 1335
   tcc tca atc ttc tcc act ttc att ggc gag cac tat gtc cat gtg 4059
   Ser Ser Ile Phe Ser Thr Phe Ile Gly Glu His Tyr Val His Val
   1340 1345 1350
   aac gct act tat gtg aat gta aaa tgt gtt gct cca tat cct tct 4104
   Asn Ala Thr Tyr Val Asn Val Lys Cys Val Ala Pro Tyr Pro Ser
   1355 1360 1365
   ctg ttg cca tcc caa gac aac att gac ggc gaa gcg aac aca gcg 4149
   Leu Leu Pro Ser Gln Asp Asn Ile Asp Gly Glu Ala Asn Thr Ala
   1370 1375 1380
   gcc gca gac tac cca tac gac gtc cca gac tac gct tag 4188
   Ala Ala Asp Tyr Pro Tyr Asp Val Pro Asp Tyr Ala
   1385 1390 1395

<210> 14
<211> 1395
<212> PRT
<213> Rattus norvegicus

<400> 14

Met Lys Ala Pro Thr Ala Leu Ala Pro Gly Ile Leu Leu Leu Leu Leu
1               5                   10                  15

Thr Leu Ala Gln Arg Ser His Gly Glu Cys Lys Glu Ala Leu Val Lys
            20                  25                  30

Ser Glu Met Asn Val Asn Met Lys Tyr Gln Leu Pro Asn Phe Thr Ala
        35                  40                  45

Glu Thr Pro Ile His Asn Val Val Leu His Gly His His Ile Tyr Leu
    50                  55                  60

Gly Ala Thr Asn Tyr Ile Tyr Val Leu Asn Asp Lys Asp Leu Gln Lys
65                  70                  75                  80

Val Ser Glu Phe Lys Thr Gly Pro Val Val Glu His Pro Asp Cys Phe
                85                  90                  95

Pro Cys Gln Asp Cys Ser Ser Lys Ala Asn Val Ser Gly Gly Val Trp
            100                 105                 110

Lys Asp Asn Val Asn Met Ala Leu Leu Val Asp Thr Tyr Tyr Asp Asp
        115                 120                 125

Gln Leu Ile Ser Cys Gly Ser Val Asn Arg Gly Thr Cys Gln Arg His
    130                 135                 140

Val Leu Pro Pro Asp Asn Ala Ala Asp Ile Gln Ser Glu Val His Cys
145                 150                 155                 160

Met Phe Ser Pro Leu Ala Glu Glu Glu Ser Gly Gln Cys Pro Asp Cys
                165                 170                 175

Val Val Ser Ala Leu Gly Ala Lys Val Leu Leu Ser Glu Lys Asp Arg
            180                 185                 190

Phe Ile Asn Phe Phe Val Gly Asn Thr Ile Asn Ser Ser Tyr Pro Pro
        195                 200                 205

Asp Tyr Ser Leu His Ser Ile Ser Val Arg Arg Leu Lys Glu Thr Gln
    210                 215                 220

Asp Gly Phe Lys Phe Leu Thr Asp Gln Ser Tyr Ile Asp Val Leu Pro
225                 230                 235                 240

Glu Phe Arg Asp Ser Tyr Pro Ile Lys Tyr Ile His Ala Phe Glu Ser
                245                 250                 255

Asn His Phe Ile Tyr Phe Leu Thr Val Gln Lys Glu Thr Leu Asp Ala
            260                 265                 270

Gln Thr Phe His Thr Arg Ile Ile Arg Phe Cys Ser Val Asp Ser Gly
        275                 280                 285

Leu His Ser Tyr Met Glu Met Pro Leu Glu Cys Ile Leu Thr Glu Lys
    290                 295                 300

Arg Arg Lys Arg Ser Thr Arg Glu Glu Val Phe Asn Ile Leu Gln Ala
305                 310                 315                 320

Ala Tyr Val Ser Lys Pro Gly Ala Asn Leu Ala Lys Gln Ile Gly Ala
                325                 330                 335

Ser Pro Tyr Asp Asp Ile Leu Tyr Gly Val Phe Ala Gln Ser Lys Pro
            340                 345                 350

Asp Ser Ala Glu Pro Met Asn Arg Ser Ala Val Cys Ala Phe Pro Ile
        355                 360                 365

Lys Tyr Val Asn Asp Phe Phe Asn Lys Ile Val Asn Lys Asn Asn Val
    370                 375                 380

Arg Cys Leu Gln His Phe Tyr Gly Pro Asn His Glu His Cys Phe Asn
385                 390                 395                 400

Arg Thr Leu Leu Arg Asn Ser Ser Gly Cys Glu Val Arg Ser Asp Glu
                405                 410                 415

Tyr Arg Thr Glu Phe Thr Thr Ala Leu Gln Arg Val Asp Leu Phe Met
            420                 425                 430

Gly Arg Leu Asn His Val Leu Leu Thr Ser Ile Ser Thr Phe Ile Lys
        435                 440                 445

Gly Asp Leu Thr Ile Ala Asn Leu Gly Thr Ser Glu Gly Arg Phe Met
    450                 455                 460

Gln Val Val Leu Ser Arg Thr Ala His Phe Thr Pro His Val Asn Phe
465                 470                 475                 480

Leu Leu Asp Ser Tyr Pro Val Ser Pro Glu Val Ile Val Glu His Pro
                485                 490                 495

Ser Asn Gln Asn Gly Tyr Thr Leu Val Val Thr Gly Lys Lys Ile Thr
            500                 505                 510

Lys Ile Pro Leu Asn Gly Leu Gly Cys Gly His Phe Gln Ser Cys Ser
        515                 520                 525

Gln Cys Leu Ser Ala Pro Tyr Phe Ile Gln Cys Gly Trp Cys His Asn
    530                 535                 540

Arg Cys Val His Ser Asn Glu Cys Pro Ser Gly Thr Trp Thr Gln Glu
545                 550                 555                 560

Ile Cys Leu Pro Ala Val Tyr Lys Val Phe Pro Thr Ser Ala Pro Leu
                565                 570                 575

Glu Gly Gly Thr Met Leu Thr Ile Cys Gly Trp Asp Phe Gly Phe Lys
            580                 585                 590

Lys Asn Asn Lys Phe Asp Leu Arg Lys Thr Lys Val Leu Leu Gly Asn
        595                 600                 605

Glu Ser Cys Thr Leu Thr Leu Ser Glu Ser Thr Thr Asn Thr Leu Lys
    610                 615                 620

Cys Thr Val Gly Pro Ala Met Ser Glu His Phe Asn Val Ser Val Ile
625                 630                 635                 640

Val Ser Asn Ser Arg Glu Thr Thr Gln Tyr Ser Ala Phe Ser Tyr Val
                645                 650                 655

Asp Pro Val Ile Thr Ser Ile Ser Pro Arg Tyr Gly Pro His Ala Gly
            660                 665                 670

Gly Thr Leu Leu Thr Leu Thr Gly Lys Tyr Leu Asn Ser Gly Asn Ser
        675                 680                 685

Arg His Ile Ser Ile Gly Gly Lys Thr Cys Thr Leu Lys Ser Val Ser
    690                 695                 700

Asp Ser Ile Leu Glu Cys Tyr Thr Pro Gly His Thr Val Ser Ala Glu
705                 710                 715                 720

Phe Pro Val Lys Leu Lys Ile Asp Leu Val Asp Arg Val Thr Ser Ser
                725                 730                 735

Phe Ser Tyr Arg Glu Asp Pro Val Val Ser Glu Ile His Pro Thr Lys
            740                 745                 750

Ser Phe Ile Ser Gly Gly Ser Thr Ile Thr Gly Ile Gly Lys Asn Leu
        755                 760                 765

Asn Ser Val Ser Thr Pro Lys Leu Val Ile Glu Val His Asp Val Gly

770                 775                 780

Val Asn Tyr Thr Val Ala Cys Gln His Arg Ser Ser Ser Glu Ile Ile
785                 790                 795                 800

Cys Cys Thr Thr Pro Ser Leu Gln Gln Leu Asp Leu Gln Leu Pro Leu
                805                 810                 815

Lys Thr Lys Ala Phe Phe Leu Leu Asp Gly Ile Leu Ser Lys His Phe
            820                 825                 830

Asp Leu Thr Tyr Val His Asp Pro Met Phe Lys Pro Phe Glu Lys Pro
        835                 840                 845

Val Met Ile Ser Met Gly Asn Glu Asn Val Val Glu Ile Lys Gly Asp
    850                 855                 860

Asp Ile Asp Pro Glu Ala Val Lys Gly Glu Val Leu Lys Val Gly Asn
865                 870                 875                 880

Lys Ser Cys Glu Asn Leu His Trp His Ser Glu Ala Leu Leu Cys Thr
                885                 890                 895

Val Pro Ser Asp Leu Leu Lys Leu Asn Gly Gly Glu Leu Asn Ile Glu
            900                 905                 910

Trp Lys Gln Ala Val Ser Ser Thr Val Leu Gly Lys Val Ile Val Gln
        915                 920                 925

Pro Asp Gln Asn Phe Ala Gly Leu Ile Ile Gly Ala Val Ser Ile Ser
    930                 935                 940

Val Val Val Trp Leu Val Ser Gly Leu Phe Leu Trp Leu Arg Lys Arg
945                 950                 955                 960

Lys His Lys Asp Leu Gly Ser Glu Leu Val Arg Tyr Asp Ala Arg Val
                965                 970                 975

His Thr Pro His Leu Asp Arg Leu Val Ser Ala Arg Ser Val Ser Pro
            980                 985                 990

Thr Thr Glu Met Val Ser Asn Glu  Ser Val Asp Tyr Arg  Ala Thr Phe
        995                 1000                1005

Pro Glu  Asp Gln Phe Pro Asn  Ser Ser Gln Asn Gly  Ala Cys Arg
    1010                 1015                 1020

Gln Val  Gln Tyr Pro Leu Thr  Asp Leu Ser Pro Ile  Leu Thr Ser
    1025                 1030                 1035

Gly Asp  Ser Asp Ile Ser Ser  Pro Leu Leu Gln Asn  Thr Val His
    1040                 1045                 1050

Ile Asp  Leu Ser Ala Leu Asn  Pro Glu Leu Val Gln  Ala Val Gln
    1055                 1060                 1065

His Val  Val Ile Gly Pro Ser  Ser Leu Ile Val His  Phe Asn Glu
    1070                 1075                 1080

Val Ile  Gly Arg Gly His Phe  Gly Cys Val Tyr His  Gly Thr Leu
    1085                 1090                 1095

Leu Asp  Ser Asp Gly Lys Lys  Ile His Cys Ala Val  Lys Ser Leu
    1100                 1105                 1110

Asn Arg  Ile Thr Asp Ile Glu  Glu Val Ser Gln Phe  Leu Thr Glu
    1115                 1120                 1125

Gly Ile  Ile Met Lys Asp Phe  Ser His Pro Asn Val  Leu Ser Leu
    1130                 1135                 1140

Leu Gly  Ile Cys Leu Arg Ser  Glu Gly Ser Pro Leu  Val Val Leu
    1145                 1150                 1155

Pro Tyr Met Lys His Gly Asp Leu Arg Asn Phe Ile Arg Asn Glu
    1160                1165                1170

Thr His Asn Pro Thr Val Lys Asp Leu Ile Gly Phe Gly Leu Gln
    1175                1180                1185

Val Ala Lys Gly Met Lys Tyr Leu Ala Ser Lys Lys Phe Val His
    1190                1195                1200

Arg Asp Leu Ala Ala Arg Asn Cys Met Leu Asp Glu Lys Phe Thr
    1205                1210                1215

Val Lys Val Ala Asp Phe Gly Leu Ala Arg Asp Met Tyr Asp Lys
    1220                1225                1230

Glu Tyr Tyr Ser Val His Asn Lys Thr Gly Ala Lys Leu Pro Val
    1235                1240                1245

Lys Trp Met Ala Leu Glu Ser Leu Gln Thr Gln Lys Phe Thr Thr
    1250                1255                1260

Lys Ser Asp Val Trp Ser Phe Gly Val Leu Leu Trp Glu Leu Met
    1265                1270                1275

Thr Arg Gly Ala Pro Pro Tyr Pro Asp Val Asn Thr Phe Asp Ile
    1280                1285                1290

Thr Ile Tyr Leu Leu Gln Gly Arg Arg Leu Leu Gln Pro Glu Tyr
    1295                1300                1305

Cys Pro Asp Ala Leu Tyr Glu Val Met Leu Lys Cys Trp His Pro
    1310                1315                1320

Lys Ala Glu Met Arg Pro Ser Arg Ser Glu Leu Val Ser Arg Ile
    1325                1330                1335

Ser Ser Ile Phe Ser Thr Phe Ile Gly Glu His Tyr Val His Val
    1340                1345                1350

Asn Ala Thr Tyr Val Asn Val Lys Cys Val Ala Pro Tyr Pro Ser
    1355                1360                1365

Leu Leu Pro Ser Gln Asp Asn Ile Asp Gly Glu Ala Asn Thr Ala
    1370                1375                1380

Ala Ala Asp Tyr Pro Tyr Asp Val Pro Asp Tyr Ala
    1385                1390                1395

<210> 15
<211> 4140
<212> DNA
<213> Mus musculus

<220>
<221> CDS
<222> (1)..(4137)

<400> 15
atg aag gct ccc acc gtg ctg gca cct ggc att ctg gtg ctg ctg ttg 48
Met Lys Ala Pro Thr Val Leu Ala Pro Gly Ile Leu Val Leu Leu Leu
1               5                   10                  15

tcc ttg gtg cag agg agc cat ggg gag tgc aag gag gcc cta gtg aag 96
Ser Leu Val Gln Arg Ser His Gly Glu Cys Lys Glu Ala Leu Val Lys
            20                  25                  30

tct gag atg aac gtg aac atg aag tat cag ctc ccc aac ttc acg gca 144
Ser Glu Met Asn Val Asn Met Lys Tyr Gln Leu Pro Asn Phe Thr Ala
        35                  40                  45

gaa acc ccc atc cag aat gtc gtc cta cac ggc cat cat att tat ctc 192
Glu Thr Pro Ile Gln Asn Val Val Leu His Gly His His Ile Tyr Leu
    50                  55                  60

gga gcc aca aac tac att tat gtt tta aat gac aaa gac ctt cag aag 240
Gly Ala Thr Asn Tyr Ile Tyr Val Leu Asn Asp Lys Asp Leu Gln Lys
65                  70                  75                  80

gta tcc gaa ttc aag acc ggg ccc gtg ttg gaa cac cca gat tgt tta 288
Val Ser Glu Phe Lys Thr Gly Pro Val Leu Glu His Pro Asp Cys Leu

85                  90                  95

cct tgt cgg gac tgc agc agc aaa gcc aat tca tca gga ggg gtt tgg      336
Pro Cys Arg Asp Cys Ser Ser Lys Ala Asn Ser Ser Gly Gly Val Trp
            100                 105                 110

aaa gac aac atc aac atg gct ctg ctt gtt gac aca tac tat gat gat      384
Lys Asp Asn Ile Asn Met Ala Leu Leu Val Asp Thr Tyr Tyr Asp Asp
        115                 120                 125

caa ctc att agc tgt ggc agt gtc aac aga ggg act tgc cag cgg cat      432
Gln Leu Ile Ser Cys Gly Ser Val Asn Arg Gly Thr Cys Gln Arg His
    130                 135                 140

gtc ctt cct cct gac aat tct gct gac atc cag tct gag gtc cac tgc      480
Val Leu Pro Pro Asp Asn Ser Ala Asp Ile Gln Ser Glu Val His Cys
145                 150                 155                 160

atg ttc tcc cca gaa gag gag tca ggg cag tgt cct gac tgt gta gtg      528
Met Phe Ser Pro Glu Glu Glu Ser Gly Gln Cys Pro Asp Cys Val Val
                165                 170                 175

agt gcc ctc gga gcc aaa gtc ctc ctg tcg gaa aag gac cgg ttc atc      576
Ser Ala Leu Gly Ala Lys Val Leu Leu Ser Glu Lys Asp Arg Phe Ile
            180                 185                 190

aat ttc ttt gtg ggg aat acg atc aat tcc tcc tat cct cct ggt tat      624
Asn Phe Phe Val Gly Asn Thr Ile Asn Ser Ser Tyr Pro Pro Gly Tyr
        195                 200                 205

tca ctg cat tcg ata tcg gtg aga cgg ctg aag gaa acc caa gat ggt      672
Ser Leu His Ser Ile Ser Val Arg Arg Leu Lys Glu Thr Gln Asp Gly
    210                 215                 220

ttt aag ttt ttg aca gac cag tcc tat att gat gtc tta cca gaa ttc      720
Phe Lys Phe Leu Thr Asp Gln Ser Tyr Ile Asp Val Leu Pro Glu Phe
225                 230                 235                 240

ctt gat tcc tac ccc ata aag tac ata cat gcc ttc gaa agc aac cat      768
Leu Asp Ser Tyr Pro Ile Lys Tyr Ile His Ala Phe Glu Ser Asn His
                245                 250                 255

ttt att tac ttt ctg act gtc caa aag gaa act cta gat gct cag act      816
Phe Ile Tyr Phe Leu Thr Val Gln Lys Glu Thr Leu Asp Ala Gln Thr
            260                 265                 270

ttt cat aca aga ata atc agg ttc tgt tcc gta gac tct ggg ttg cac      864
Phe His Thr Arg Ile Ile Arg Phe Cys Ser Val Asp Ser Gly Leu His
        275                 280                 285

tcc tac atg gaa atg ccc ctg gaa tgc atc ctg aca gaa aaa aga agg 912
Ser Tyr Met Glu Met Pro Leu Glu Cys Ile Leu Thr Glu Lys Arg Arg
    290                 295                 300

aag aga tcc aca agg gaa gaa gtg ttt aat atc ctc caa gcc gcg tat 960
Lys Arg Ser Thr Arg Glu Glu Val Phe Asn Ile Leu Gln Ala Ala Tyr
305                 310                 315                 320

gtc agt aaa cca ggg gcc aat ctt gct aag caa ata gga gct agc cct 1008
Val Ser Lys Pro Gly Ala Asn Leu Ala Lys Gln Ile Gly Ala Ser Pro
                325                 330                 335

tct gat gac att ctc ttc ggg gtg ttt gca caa agc aag cca gat tct 1056
Ser Asp Asp Ile Leu Phe Gly Val Phe Ala Gln Ser Lys Pro Asp Ser
            340                 345                 350

gct gaa cct gtg aat cga tca gca gtc tgt gca ttc ccc atc aaa tat 1104
Ala Glu Pro Val Asn Arg Ser Ala Val Cys Ala Phe Pro Ile Lys Tyr
        355                 360                 365

gtc aat gac ttc ttc aac aag att gtc aac aaa aac aac gtg aga tgt 1152
Val Asn Asp Phe Phe Asn Lys Ile Val Asn Lys Asn Asn Val Arg Cys
    370                 375                 380

ctc cag cat ttt tac gga ccc aac cat gag cac tgt ttc aat agg acc 1200
Leu Gln His Phe Tyr Gly Pro Asn His Glu His Cys Phe Asn Arg Thr
385                 390                 395                 400

ctg ctg aga aac tct tcg ggc tgt gaa gcg cgc agt gac gag tat cgg 1248
Leu Leu Arg Asn Ser Ser Gly Cys Glu Ala Arg Ser Asp Glu Tyr Arg
                405                 410                 415

aca gag ttt acc acg gct ttg cag cgc gtc gac tta ttc atg ggc cgg 1296
Thr Glu Phe Thr Thr Ala Leu Gln Arg Val Asp Leu Phe Met Gly Arg
            420                 425                 430

ctt aac caa gtg ctc ctg aca tcc atc tcc acc ttc atc aaa ggt gac 1344
Leu Asn Gln Val Leu Leu Thr Ser Ile Ser Thr Phe Ile Lys Gly Asp
        435                 440                 445

ctc acc att gct aat cta ggg acg tca gaa ggt cgc ttc atg cag gtg 1392
Leu Thr Ile Ala Asn Leu Gly Thr Ser Glu Gly Arg Phe Met Gln Val
    450                 455                 460

gtg ctc tct cga aca gca cac ctc act cct cat gtg aac ttc ctc ctg 1440
Val Leu Ser Arg Thr Ala His Leu Thr Pro His Val Asn Phe Leu Leu
465                 470                 475                 480

gac tcc cat cct gta tct cca gaa gtt att gtt gag cat cca tca aat 1488
Asp Ser His Pro Val Ser Pro Glu Val Ile Val Glu His Pro Ser Asn
                485                 490                 495

caa aat ggc tat aca ttg gtt gtc aca gga aag aag atc acc aag att 1536
Gln Asn Gly Tyr Thr Leu Val Val Thr Gly Lys Lys Ile Thr Lys Ile
            500                 505                 510

cca ttg aat ggc ctg ggc tgt gga cat ttc caa tcc tgc agt cag tgc 1584
Pro Leu Asn Gly Leu Gly Cys Gly His Phe Gln Ser Cys Ser Gln Cys
        515                 520                 525

ctc tct gcc cct tac ttt ata cag tgt ggc tgg tgc cac aat caa tgt 1632
Leu Ser Ala Pro Tyr Phe Ile Gln Cys Gly Trp Cys His Asn Gln Cys
    530                 535                 540

gtg cgt ttt gat gaa tgc ccc agc ggt aca tgg act caa gag atc tgt 1680
Val Arg Phe Asp Glu Cys Pro Ser Gly Thr Trp Thr Gln Glu Ile Cys
545                 550                 555                 560

ctg cca gcg gtt tat aag gtg ttc ccc acc agc gcg ccc ctt gaa gga 1728
Leu Pro Ala Val Tyr Lys Val Phe Pro Thr Ser Ala Pro Leu Glu Gly
                565                 570                 575

gga aca gtg ttg acc ata tgt ggc tgg gac ttt gga ttc agg aag aat 1776
Gly Thr Val Leu Thr Ile Cys Gly Trp Asp Phe Gly Phe Arg Lys Asn
            580                 585                 590

aat aaa ttt gat tta agg aaa acc aaa gtt ctg ctt ggc aac gag agc 1824
Asn Lys Phe Asp Leu Arg Lys Thr Lys Val Leu Leu Gly Asn Glu Ser
        595                 600                 605

tgt acc ttg acc tta agc gag agc acg aca aat acg ttg aaa tgc aca 1872
Cys Thr Leu Thr Leu Ser Glu Ser Thr Thr Asn Thr Leu Lys Cys Thr
    610                 615                 620

gtt ggt ccc gcg atg agt gag cac ttc aat gtg tct gta att atc tca 1920
Val Gly Pro Ala Met Ser Glu His Phe Asn Val Ser Val Ile Ile Ser
625                 630                 635                 640

aac agt cga gag aca aca caa tac agt gca ttc tcc tat gta gat cct 1968
Asn Ser Arg Glu Thr Thr Gln Tyr Ser Ala Phe Ser Tyr Val Asp Pro
                645                 650                 655

gta ata aca agc att tct ccg agg tac ggc cct cag gct gga ggc acc 2016
Val Ile Thr Ser Ile Ser Pro Arg Tyr Gly Pro Gln Ala Gly Gly Thr
            660                 665                 670

tta ctc act ctt act ggg aaa tac ctc aac agt ggc aat tct aga cac 2064

Leu Leu Thr Leu Thr Gly Lys Tyr Leu Asn Ser Gly Asn Ser Arg His
        675                 680                 685

att tca att gga ggg aaa aca tgt act tta aaa agt gta tca gat agt 2112
Ile Ser Ile Gly Gly Lys Thr Cys Thr Leu Lys Ser Val Ser Asp Ser
    690                 695                 700

att ctt gaa tgc tac acc cca gcc caa act acc tct gat gag ttt cct 2160
Ile Leu Glu Cys Tyr Thr Pro Ala Gln Thr Thr Ser Asp Glu Phe Pro
705                 710                 715                 720

gtg aaa ttg aag att gac ttg gct aac cga gag acc agc agc ttc agt 2208
Val Lys Leu Lys Ile Asp Leu Ala Asn Arg Glu Thr Ser Ser Phe Ser
                725                 730                 735

tac cgg gaa gac ccc gtt gtc tat gaa atc cac cca acc aaa tct ttt 2256
Tyr Arg Glu Asp Pro Val Val Tyr Glu Ile His Pro Thr Lys Ser Phe
            740                 745                 750

att agt ggt gga agc aca ata acg ggt att ggg aag acc ctg aat tcg 2304
Ile Ser Gly Gly Ser Thr Ile Thr Gly Ile Gly Lys Thr Leu Asn Ser
        755                 760                 765

gtt agc ctc cca aag ctg gta ata gat gtg cat gaa gtg ggt gtg aac 2352
Val Ser Leu Pro Lys Leu Val Ile Asp Val His Glu Val Gly Val Asn
    770                 775                 780

tac aca gtg gca tgt cag cat cgc tca aat tca gag atc atc tgc tgc 2400
Tyr Thr Val Ala Cys Gln His Arg Ser Asn Ser Glu Ile Ile Cys Cys
785                 790                 795                 800

act act cct tca ctg aaa cag ctg ggc ctg caa ctc ccc ctg aag acc 2448
Thr Thr Pro Ser Leu Lys Gln Leu Gly Leu Gln Leu Pro Leu Lys Thr
                805                 810                 815

aaa gcc ttc ttc ctg tta gac ggg att ctt tcc aaa cac ttt gat ctc 2496
Lys Ala Phe Phe Leu Leu Asp Gly Ile Leu Ser Lys His Phe Asp Leu
            820                 825                 830

act tat gtg cat aat cct gtg ttt gag cct ttt gaa aag cca gta atg 2544
Thr Tyr Val His Asn Pro Val Phe Glu Pro Phe Glu Lys Pro Val Met
        835                 840                 845

atc tca atg ggc aat gaa aat gta gtg gaa att aag gga aac aat att 2592
Ile Ser Met Gly Asn Glu Asn Val Val Glu Ile Lys Gly Asn Asn Ile
    850                 855                 860

gac cct gaa gca gtt aaa ggt gaa gtg tta aaa gtt gga aat cag agc 2640
Asp Pro Glu Ala Val Lys Gly Glu Val Leu Lys Val Gly Asn Gln Ser

865                 870                 875                 880

tgc gag agt ctc cac tgg cac tct gga gct gtg ttg tgt aca gtc ccc      2688
Cys Glu Ser Leu His Trp His Ser Gly Ala Val Leu Cys Thr Val Pro
                885                 890                 895

agt gac ctg ctc aaa ctg aac agc gag cta aat ata gag tgg aag caa      2736
Ser Asp Leu Leu Lys Leu Asn Ser Glu Leu Asn Ile Glu Trp Lys Gln
            900                 905                 910

gca gtc tct tca act gtt ctt gga aaa gtg atc gtt caa ccg gat cag      2784
Ala Val Ser Ser Thr Val Leu Gly Lys Val Ile Val Gln Pro Asp Gln
        915                 920                 925

aat ttt gca gga ttg atc att ggt gcg gtc tca ata tca gta gta gtt      2832
Asn Phe Ala Gly Leu Ile Ile Gly Ala Val Ser Ile Ser Val Val Val
    930                 935                 940

ttg tta tta tcc ggg ctc ttc ctg tgg atg aga aag aga aag cat aaa      2880
Leu Leu Leu Ser Gly Leu Phe Leu Trp Met Arg Lys Arg Lys His Lys
945                 950                 955                 960

gat ctg ggc agt gaa tta gtt cgc tat gac gca aga gta cac act cct      2928
Asp Leu Gly Ser Glu Leu Val Arg Tyr Asp Ala Arg Val His Thr Pro
                965                 970                 975

cat ttg gat agg ctt gta agt gcc cga agt gta agt cca act aca gag      2976
His Leu Asp Arg Leu Val Ser Ala Arg Ser Val Ser Pro Thr Thr Glu
            980                 985                 990

atg gtt tca aat gag tct gta gac  tac aga gct act ttt  cca gaa gac      3024
Met Val Ser Asn Glu Ser Val Asp  Tyr Arg Ala Thr Phe  Pro Glu Asp
        995                 1000                 1005

cag ttt  ccc aac tcc tct cag  aat gga gca tgc aga  caa gtg caa      3069
Gln Phe  Pro Asn Ser Ser Gln  Asn Gly Ala Cys Arg  Gln Val Gln
    1010                 1015                 1020

tat cct  ctg aca gac ctg tcc  cct atc ctg acg agt  gga gac tct      3114
Tyr Pro  Leu Thr Asp Leu Ser  Pro Ile Leu Thr Ser  Gly Asp Ser
    1025                 1030                 1035

gat ata  tcc agc cca tta cta  caa aat act gtt cac  att gac ctc      3159
Asp Ile  Ser Ser Pro Leu Leu  Gln Asn Thr Val His  Ile Asp Leu
    1040                 1045                 1050

agt gct  cta aat cca gag ctg  gtc caa gca gtt cag  cac gta gtg      3204
Ser Ala  Leu Asn Pro Glu Leu  Val Gln Ala Val Gln  His Val Val
    1055                 1060                 1065

att gga ccc agc agc ctg att gtg cat ttc aat gaa gtc ata gga 3249
Ile Gly Pro Ser Ser Leu Ile Val His Phe Asn Glu Val Ile Gly
    1070                1075                1080

aga ggg cat ttt ggc tgt gtc tat cat ggg act ttg ctg gac aat 3294
Arg Gly His Phe Gly Cys Val Tyr His Gly Thr Leu Leu Asp Asn
    1085                1090                1095

gac gga aag aaa att cac tgt gct gtg aaa tcc ttg aat aga atc 3339
Asp Gly Lys Lys Ile His Cys Ala Val Lys Ser Leu Asn Arg Ile
    1100                1105                1110

aca gat ata gaa gag gtc tcc cag ttt ctg act gag gga atc atc 3384
Thr Asp Ile Glu Glu Val Ser Gln Phe Leu Thr Glu Gly Ile Ile
    1115                1120                1125

atg aaa gac ttc agc cat ccc aat gtt ctc tca ctc ttg gga atc 3429
Met Lys Asp Phe Ser His Pro Asn Val Leu Ser Leu Leu Gly Ile
    1130                1135                1140

tgc ctg agg agt gaa ggg tct cct ctg gtg gtc ctg ccc tat atg 3474
Cys Leu Arg Ser Glu Gly Ser Pro Leu Val Val Leu Pro Tyr Met
    1145                1150                1155

aag cat gga gat ctg cga aat ttc att cga aac gag act cat aat 3519
Lys His Gly Asp Leu Arg Asn Phe Ile Arg Asn Glu Thr His Asn
    1160                1165                1170

cca act gtg aaa gat ctt ata gga ttt ggc ctt caa gta gcc aaa 3564
Pro Thr Val Lys Asp Leu Ile Gly Phe Gly Leu Gln Val Ala Lys
    1175                1180                1185

ggc atg aaa tat ctt gcc agc aaa aag ttt gtc cac aga gac tta 3609
Gly Met Lys Tyr Leu Ala Ser Lys Lys Phe Val His Arg Asp Leu
    1190                1195                1200

gct gca aga aac tgc atg ttg gat gaa aaa ttc act gtc aag gtt 3654
Ala Ala Arg Asn Cys Met Leu Asp Glu Lys Phe Thr Val Lys Val
    1205                1210                1215

gct gat ttc ggt ctt gcc aga gac atg tac gat aaa gag tac tat 3699
Ala Asp Phe Gly Leu Ala Arg Asp Met Tyr Asp Lys Glu Tyr Tyr
    1220                1225                1230

agt gtc cac aac aag acg ggt gcc aag cta cca gta aag tgg atg 3744
Ser Val His Asn Lys Thr Gly Ala Lys Leu Pro Val Lys Trp Met
    1235                1240                1245

gct tta gag agt ctg caa acg cag aag ttc acc acc aag tca gat 3789
Ala Leu Glu Ser Leu Gln Thr Gln Lys Phe Thr Thr Lys Ser Asp
    1250                1255                1260

gtg tgg tcc ttt ggt gtg ctc ctc tgg gag ctc atg acg aga gga 3834
Val Trp Ser Phe Gly Val Leu Leu Trp Glu Leu Met Thr Arg Gly
    1265                1270                1275

gcc cct cct tat ccc gac gtg aac aca ttt gat atc act atc tac 3879
Ala Pro Pro Tyr Pro Asp Val Asn Thr Phe Asp Ile Thr Ile Tyr
    1280                1285                1290

ctg ttg caa ggc aga aga ctc ttg caa cca gaa tac tgt cca gac 3924
Leu Leu Gln Gly Arg Arg Leu Leu Gln Pro Glu Tyr Cys Pro Asp
    1295                1300                1305

gcc ttg tac gaa gtg atg cta aaa tgc tgg cac ccc aaa gcg gaa 3969
Ala Leu Tyr Glu Val Met Leu Lys Cys Trp His Pro Lys Ala Glu
    1310                1315                1320

atg cgc ccg tcc ttt tcc gaa ctg gtc tcc agg ata tcc tca atc 4014
Met Arg Pro Ser Phe Ser Glu Leu Val Ser Arg Ile Ser Ser Ile
    1325                1330                1335

ttc tcc acg ttc att ggg gaa cac tac gtc cac gtg aac gct act 4059
Phe Ser Thr Phe Ile Gly Glu His Tyr Val His Val Asn Ala Thr
    1340                1345                1350

tat gtg aat gta aaa tgt gtt gct cca tat cct tct ctg ttg cca 4104
Tyr Val Asn Val Lys Cys Val Ala Pro Tyr Pro Ser Leu Leu Pro
    1355                1360                1365

tcc caa gac aac att gat ggc gag ggg aac aca tga 4140
Ser Gln Asp Asn Ile Asp Gly Glu Gly Asn Thr
    1370                1375

<210> 16
<211> 1379
<212> PRT
<213> Mus musculus

<400> 16

Met Lys Ala Pro Thr Val Leu Ala Pro Gly Ile Leu Val Leu Leu Leu
1               5                   10                  15

Ser Leu Val Gln Arg Ser His Gly Glu Cys Lys Glu Ala Leu Val Lys

20                  25                  30

Ser Glu Met Asn Val Asn Met Lys Tyr Gln Leu Pro Asn Phe Thr Ala
        35                  40                  45

Glu Thr Pro Ile Gln Asn Val Val Leu His Gly His His Ile Tyr Leu
    50                  55                  60

Gly Ala Thr Asn Tyr Ile Tyr Val Leu Asn Asp Lys Asp Leu Gln Lys
65                  70                  75                  80

Val Ser Glu Phe Lys Thr Gly Pro Val Leu Glu His Pro Asp Cys Leu
                85                  90                  95

Pro Cys Arg Asp Cys Ser Ser Lys Ala Asn Ser Ser Gly Gly Val Trp
            100                 105                 110

Lys Asp Asn Ile Asn Met Ala Leu Leu Val Asp Thr Tyr Tyr Asp Asp
        115                 120                 125

Gln Leu Ile Ser Cys Gly Ser Val Asn Arg Gly Thr Cys Gln Arg His
    130                 135                 140

Val Leu Pro Pro Asp Asn Ser Ala Asp Ile Gln Ser Glu Val His Cys
145                 150                 155                 160

Met Phe Ser Pro Glu Glu Glu Ser Gly Gln Cys Pro Asp Cys Val Val
                165                 170                 175

Ser Ala Leu Gly Ala Lys Val Leu Leu Ser Glu Lys Asp Arg Phe Ile
            180                 185                 190

Asn Phe Phe Val Gly Asn Thr Ile Asn Ser Ser Tyr Pro Pro Gly Tyr
        195                 200                 205

Ser Leu His Ser Ile Ser Val Arg Arg Leu Lys Glu Thr Gln Asp Gly
    210                 215                 220

Phe Lys Phe Leu Thr Asp Gln Ser Tyr Ile Asp Val Leu Pro Glu Phe
225                 230                 235                 240

Leu Asp Ser Tyr Pro Ile Lys Tyr Ile His Ala Phe Glu Ser Asn His
                245                 250                 255

Phe Ile Tyr Phe Leu Thr Val Gln Lys Glu Thr Leu Asp Ala Gln Thr
            260                 265                 270

Phe His Thr Arg Ile Ile Arg Phe Cys Ser Val Asp Ser Gly Leu His
        275                 280                 285

Ser Tyr Met Glu Met Pro Leu Glu Cys Ile Leu Thr Glu Lys Arg Arg
    290                 295                 300

Lys Arg Ser Thr Arg Glu Glu Val Phe Asn Ile Leu Gln Ala Ala Tyr
305                 310                 315                 320

Val Ser Lys Pro Gly Ala Asn Leu Ala Lys Gln Ile Gly Ala Ser Pro
                325                 330                 335

Ser Asp Asp Ile Leu Phe Gly Val Phe Ala Gln Ser Lys Pro Asp Ser
            340                 345                 350

Ala Glu Pro Val Asn Arg Ser Ala Val Cys Ala Phe Pro Ile Lys Tyr
        355                 360                 365

Val Asn Asp Phe Phe Asn Lys Ile Val Asn Lys Asn Asn Val Arg Cys
    370                 375                 380

Leu Gln His Phe Tyr Gly Pro Asn His Glu His Cys Phe Asn Arg Thr
385                 390                 395                 400

Leu Leu Arg Asn Ser Ser Gly Cys Glu Ala Arg Ser Asp Glu Tyr Arg
                405                 410                 415

Thr Glu Phe Thr Thr Ala Leu Gln Arg Val Asp Leu Phe Met Gly Arg
            420                 425                 430

Leu Asn Gln Val Leu Leu Thr Ser Ile Ser Thr Phe Ile Lys Gly Asp
        435                 440                 445

Leu Thr Ile Ala Asn Leu Gly Thr Ser Glu Gly Arg Phe Met Gln Val
    450                 455                 460

Val Leu Ser Arg Thr Ala His Leu Thr Pro His Val Asn Phe Leu Leu
465                 470                 475                 480

Asp Ser His Pro Val Ser Pro Glu Val Ile Val Glu His Pro Ser Asn
                485                 490                 495

Gln Asn Gly Tyr Thr Leu Val Val Thr Gly Lys Lys Ile Thr Lys Ile
            500                 505                 510

Pro Leu Asn Gly Leu Gly Cys Gly His Phe Gln Ser Cys Ser Gln Cys
        515                 520                 525

Leu Ser Ala Pro Tyr Phe Ile Gln Cys Gly Trp Cys His Asn Gln Cys
    530                 535                 540

Val Arg Phe Asp Glu Cys Pro Ser Gly Thr Trp Thr Gln Glu Ile Cys
545                 550                 555                 560

Leu Pro Ala Val Tyr Lys Val Phe Pro Thr Ser Ala Pro Leu Glu Gly
                565                 570                 575

Gly Thr Val Leu Thr Ile Cys Gly Trp Asp Phe Gly Phe Arg Lys Asn
            580                 585                 590

Asn Lys Phe Asp Leu Arg Lys Thr Lys Val Leu Leu Gly Asn Glu Ser
        595                 600                 605

Cys Thr Leu Thr Leu Ser Glu Ser Thr Thr Asn Thr Leu Lys Cys Thr
    610                 615                 620

Val Gly Pro Ala Met Ser Glu His Phe Asn Val Ser Val Ile Ile Ser
625                 630                 635                 640

Asn Ser Arg Glu Thr Thr Gln Tyr Ser Ala Phe Ser Tyr Val Asp Pro
                645                 650                 655

Val Ile Thr Ser Ile Ser Pro Arg Tyr Gly Pro Gln Ala Gly Gly Thr
            660                 665                 670

Leu Leu Thr Leu Thr Gly Lys Tyr Leu Asn Ser Gly Asn Ser Arg His
        675                 680                 685

Ile Ser Ile Gly Gly Lys Thr Cys Thr Leu Lys Ser Val Ser Asp Ser
    690                 695                 700

Ile Leu Glu Cys Tyr Thr Pro Ala Gln Thr Thr Ser Asp Glu Phe Pro
705                 710                 715                 720

Val Lys Leu Lys Ile Asp Leu Ala Asn Arg Glu Thr Ser Ser Phe Ser
                725                 730                 735

Tyr Arg Glu Asp Pro Val Val Tyr Glu Ile His Pro Thr Lys Ser Phe
            740                 745                 750

Ile Ser Gly Gly Ser Thr Ile Thr Gly Ile Gly Lys Thr Leu Asn Ser
        755                 760                 765

Val Ser Leu Pro Lys Leu Val Ile Asp Val His Glu Val Gly Val Asn
    770                 775                 780

Tyr Thr Val Ala Cys Gln His Arg Ser Asn Ser Glu Ile Ile Cys Cys
785                 790                 795                 800

Thr Thr Pro Ser Leu Lys Gln Leu Gly Leu Gln Leu Pro Leu Lys Thr

                805                 810                 815

Lys Ala Phe Phe Leu Leu Asp Gly Ile Leu Ser Lys His Phe Asp Leu
            820                 825                 830

Thr Tyr Val His Asn Pro Val Phe Glu Pro Phe Glu Lys Pro Val Met
        835                 840                 845

Ile Ser Met Gly Asn Glu Asn Val Val Glu Ile Lys Gly Asn Asn Ile
    850                 855                 860

Asp Pro Glu Ala Val Lys Gly Glu Val Leu Lys Val Gly Asn Gln Ser
865                 870                 875                 880

Cys Glu Ser Leu His Trp His Ser Gly Ala Val Leu Cys Thr Val Pro
                885                 890                 895

Ser Asp Leu Leu Lys Leu Asn Ser Glu Leu Asn Ile Glu Trp Lys Gln
            900                 905                 910

Ala Val Ser Ser Thr Val Leu Gly Lys Val Ile Val Gln Pro Asp Gln
        915                 920                 925

Asn Phe Ala Gly Leu Ile Ile Gly Ala Val Ser Ile Ser Val Val Val
    930                 935                 940

Leu Leu Leu Ser Gly Leu Phe Leu Trp Met Arg Lys Arg Lys His Lys
945                 950                 955                 960

Asp Leu Gly Ser Glu Leu Val Arg Tyr Asp Ala Arg Val His Thr Pro
                965                 970                 975

His Leu Asp Arg Leu Val Ser Ala Arg Ser Val Ser Pro Thr Thr Glu
            980                 985                 990

Met Val Ser Asn Glu Ser Val Asp Tyr Arg Ala Thr Phe Pro Glu Asp
        995                 1000                1005

Gln Phe Pro Asn Ser Ser Gln Asn Gly Ala Cys Arg Gln Val Gln
    1010                1015                1020

Tyr Pro Leu Thr Asp Leu Ser Pro Ile Leu Thr Ser Gly Asp Ser
    1025                1030                1035

Asp Ile Ser Ser Pro Leu Leu Gln Asn Thr Val His Ile Asp Leu
    1040                1045                1050

Ser Ala Leu Asn Pro Glu Leu Val Gln Ala Val Gln His Val Val
    1055                1060                1065

Ile Gly Pro Ser Ser Leu Ile Val His Phe Asn Glu Val Ile Gly
    1070                1075                1080

Arg Gly His Phe Gly Cys Val Tyr His Gly Thr Leu Leu Asp Asn
    1085                1090                1095

Asp Gly Lys Lys Ile His Cys Ala Val Lys Ser Leu Asn Arg Ile
    1100                1105                1110

Thr Asp Ile Glu Glu Val Ser Gln Phe Leu Thr Glu Gly Ile Ile
    1115                1120                1125

Met Lys Asp Phe Ser His Pro Asn Val Leu Ser Leu Leu Gly Ile
    1130                1135                1140

Cys Leu Arg Ser Glu Gly Ser Pro Leu Val Val Leu Pro Tyr Met
    1145                1150                1155

Lys His Gly Asp Leu Arg Asn Phe Ile Arg Asn Glu Thr His Asn
    1160                1165                1170

Pro Thr Val Lys Asp Leu Ile Gly Phe Gly Leu Gln Val Ala Lys
    1175                1180                1185

Gly Met Lys Tyr Leu Ala Ser Lys Lys Phe Val His Arg Asp Leu
    1190                1195                1200

Ala Ala Arg Asn Cys Met Leu Asp Glu Lys Phe Thr Val Lys Val
    1205                1210                1215

Ala Asp Phe Gly Leu Ala Arg Asp Met Tyr Asp Lys Glu Tyr Tyr
    1220                1225                1230

Ser Val His Asn Lys Thr Gly Ala Lys Leu Pro Val Lys Trp Met
    1235                1240                1245

Ala Leu Glu Ser Leu Gln Thr Gln Lys Phe Thr Thr Lys Ser Asp
    1250                1255                1260

Val Trp Ser Phe Gly Val Leu Leu Trp Glu Leu Met Thr Arg Gly
    1265                1270                1275

Ala Pro Pro Tyr Pro Asp Val Asn Thr Phe Asp Ile Thr Ile Tyr
    1280                1285                1290

Leu Leu Gln Gly Arg Arg Leu Leu Gln Pro Glu Tyr Cys Pro Asp
    1295                1300                1305

Ala Leu Tyr Glu Val Met Leu Lys Cys Trp His Pro Lys Ala Glu
    1310                1315                1320

Met Arg Pro Ser Phe Ser Glu Leu Val Ser Arg Ile Ser Ser Ile
    1325                1330                1335

Phe Ser Thr Phe Ile Gly Glu His Tyr Val His Val Asn Ala Thr
    1340                1345                1350

Tyr Val Asn Val Lys Cys Val Ala Pro Tyr Pro Ser Leu Leu Pro
    1355                1360                1365

Ser Gln Asp Asn Ile Asp Gly Glu Gly Asn Thr
    1370                1375

<210> 17
<211> 4137
<212> DNA
<213> Mus musculus

<220>
<221> CDS
<222> (1)..(4134)

<400> 17
aag gct ccc acc gtg ctg gta cct ggc att ctg gtg ctg ctg ttg tcc 48
Lys Ala Pro Thr Val Leu Val Pro Gly Ile Leu Val Leu Leu Leu Ser
1               5                   10                  15

ttg gtg cag agg agc cat ggg gag tgc aag gag gcc cta gtg aag tct 96
Leu Val Gln Arg Ser His Gly Glu Cys Lys Glu Ala Leu Val Lys Ser
            20                  25                  30

gag atg aac gtg aac atg aag tat cag ctc ccc aac ttc acg gca gaa 144
Glu Met Asn Val Asn Met Lys Tyr Gln Leu Pro Asn Phe Thr Ala Glu
        35                  40                  45

acc ccc atc cag aat gtc gtc cta cac ggc cat cat att tat ctc gga 192
Thr Pro Ile Gln Asn Val Val Leu His Gly His His Ile Tyr Leu Gly
    50                  55                  60

gcc aca aac tac att tat gtt tta aat gac aaa gac ctt cag aag gta 240
Ala Thr Asn Tyr Ile Tyr Val Leu Asn Asp Lys Asp Leu Gln Lys Val
65                  70                  75                  80

tcc gaa ttc aag acc ggg ccc gtg ttg gaa cac cca gat tgt tta cct 288
Ser Glu Phe Lys Thr Gly Pro Val Leu Glu His Pro Asp Cys Leu Pro
                85                  90                  95

tgt cgg gac tgc agc agc aaa gcc aat tca tca gga ggg gtt tgg aaa 336
Cys Arg Asp Cys Ser Ser Lys Ala Asn Ser Ser Gly Gly Val Trp Lys
            100                 105                 110

gac aac atc aac atg gct ctg ctt gtt gac aca tac tat gat gat caa 384
Asp Asn Ile Asn Met Ala Leu Leu Val Asp Thr Tyr Tyr Asp Asp Gln
        115                 120                 125

ctc att agc tgt ggc agt gtc aac aga ggg act tgc cag cgg cat gtc 432
Leu Ile Ser Cys Gly Ser Val Asn Arg Gly Thr Cys Gln Arg His Val

130                 135                 140

ctt cct cct gac aat tct gct gac atc cag tct gag gtc cac tgc atg      480
Leu Pro Pro Asp Asn Ser Ala Asp Ile Gln Ser Glu Val His Cys Met
145                 150                 155                 160

ttc tcc cca gaa gag gag tca ggg cag tgt cct gac tgt gta gtg agt      528
Phe Ser Pro Glu Glu Glu Ser Gly Gln Cys Pro Asp Cys Val Val Ser
                165                 170                 175

gcc ctc gga gcc aaa gtc ctc ctg tcg gaa aag gac cgg ttc atc aat      576
Ala Leu Gly Ala Lys Val Leu Leu Ser Glu Lys Asp Arg Phe Ile Asn
            180                 185                 190

ttc ttt gtg ggg aat acg atc aat tcc tcc tat cct cct ggt tat tca      624
Phe Phe Val Gly Asn Thr Ile Asn Ser Ser Tyr Pro Pro Gly Tyr Ser
        195                 200                 205

ctg cat tcg ata tcg gtg aga cgg ctg aag gaa acc caa gat ggt ttt      672
Leu His Ser Ile Ser Val Arg Arg Leu Lys Glu Thr Gln Asp Gly Phe
    210                 215                 220

aag ttt ttg aca gac cag tcc tat att gat gtc tta cca gaa ttc caa      720
Lys Phe Leu Thr Asp Gln Ser Tyr Ile Asp Val Leu Pro Glu Phe Gln
225                 230                 235                 240

gat tcc tac ccc ata aag tac ata cat gcc ttc gaa agc aac cat ttt      768
Asp Ser Tyr Pro Ile Lys Tyr Ile His Ala Phe Glu Ser Asn His Phe
                245                 250                 255

att tac ttt ctg act gtc caa aag gaa act cta gat gct cag act ttt      816
Ile Tyr Phe Leu Thr Val Gln Lys Glu Thr Leu Asp Ala Gln Thr Phe
            260                 265                 270

cat aca aga ata atc agg ttc tgt tcc gta gac tct ggg ttg cac tcc      864
His Thr Arg Ile Ile Arg Phe Cys Ser Val Asp Ser Gly Leu His Ser
        275                 280                 285

tac atg gaa atg ccc ctg gaa tgc atc ctg aca gaa aaa aga agg aag      912
Tyr Met Glu Met Pro Leu Glu Cys Ile Leu Thr Glu Lys Arg Arg Lys
    290                 295                 300

aga tcc aca agg gaa gaa gtg ttt aat atc ctc caa gcc gcg tat gtc      960
Arg Ser Thr Arg Glu Glu Val Phe Asn Ile Leu Gln Ala Ala Tyr Val
305                 310                 315                 320

agt aaa cca ggg gcc aat ctt gct aag caa ata gga gct agc cct tct      1008
Ser Lys Pro Gly Ala Asn Leu Ala Lys Gln Ile Gly Ala Ser Pro Ser
                325                 330                 335

gat gac att ctc ttc ggg gtg ttt gca caa agc aag cca gat tct gct 1056
Asp Asp Ile Leu Phe Gly Val Phe Ala Gln Ser Lys Pro Asp Ser Ala
            340                 345                 350

gaa cct gtg aat cga tca gca gtc tgt gca ttc ccc atc aaa tat gtc 1104
Glu Pro Val Asn Arg Ser Ala Val Cys Ala Phe Pro Ile Lys Tyr Val
        355                 360                 365

aat gac ttc ttc aac aag att gtc aac aaa aac aac gtg aga tgt ctc 1152
Asn Asp Phe Phe Asn Lys Ile Val Asn Lys Asn Asn Val Arg Cys Leu
    370                 375                 380

cag cat ttt tac gga ccc aac cat gag cac tgt ttc aat agg acc ctg 1200
Gln His Phe Tyr Gly Pro Asn His Glu His Cys Phe Asn Arg Thr Leu
385                 390                 395                 400

ctg aga aac tct tcg ggc tgt gaa gcg cgc agt gac gag tat cgg aca 1248
Leu Arg Asn Ser Ser Gly Cys Glu Ala Arg Ser Asp Glu Tyr Arg Thr
                405                 410                 415

gag ttt acc acg gct ttg cag cgc gtc gac tta ttc atg ggc cgg ctt 1296
Glu Phe Thr Thr Ala Leu Gln Arg Val Asp Leu Phe Met Gly Arg Leu
            420                 425                 430

aac caa gtg ctc ctg aca tcc atc tcc acc ttc atc aaa ggt gac ctc 1344
Asn Gln Val Leu Leu Thr Ser Ile Ser Thr Phe Ile Lys Gly Asp Leu
        435                 440                 445

acc att gct aat cta ggg acg tca gaa ggt cgc ttc atg cag gtg gtg 1392
Thr Ile Ala Asn Leu Gly Thr Ser Glu Gly Arg Phe Met Gln Val Val
    450                 455                 460

ctc tct cga aca gca cac ctc act cct cat gtg aac ttc ctc ctg gac 1440
Leu Ser Arg Thr Ala His Leu Thr Pro His Val Asn Phe Leu Leu Asp
465                 470                 475                 480

tcc cat cct gta tct cca gaa gtt att gtt gag cat cca tca aat caa 1488
Ser His Pro Val Ser Pro Glu Val Ile Val Glu His Pro Ser Asn Gln
                485                 490                 495

aat ggc tat aca ttg gtt gtc aca gga aag aag atc acc aag att cca 1536
Asn Gly Tyr Thr Leu Val Val Thr Gly Lys Lys Ile Thr Lys Ile Pro
            500                 505                 510

ttg aat ggc ctg ggc tgt gga cat ttc caa tcc tgc agt cag tgc ctc 1584
Leu Asn Gly Leu Gly Cys Gly His Phe Gln Ser Cys Ser Gln Cys Leu
        515                 520                 525

tct gcc cct tac ttt ata cag tgt ggc tgg tgc cac aat caa tgt gtg 1632
Ser Ala Pro Tyr Phe Ile Gln Cys Gly Trp Cys His Asn Gln Cys Val
    530                 535                 540

cgt ttt gat gaa tgc ccc agc ggt aca tgg act caa gag atc tgt ctg 1680
Arg Phe Asp Glu Cys Pro Ser Gly Thr Trp Thr Gln Glu Ile Cys Leu
545                 550                 555                 560

cca gcg gtt tat aag gtg ttc ccc acc agc gcg ccc ctt gaa gga gga 1728
Pro Ala Val Tyr Lys Val Phe Pro Thr Ser Ala Pro Leu Glu Gly Gly
                565                 570                 575

aca gtg ttg acc ata tgt ggc tgg gac ttt gga ttc agg aag aat aat 1776
Thr Val Leu Thr Ile Cys Gly Trp Asp Phe Gly Phe Arg Lys Asn Asn
            580                 585                 590

aaa ttt gat tta agg aaa acc aaa gtt ctg ctt ggc aac gag agc tgt 1824
Lys Phe Asp Leu Arg Lys Thr Lys Val Leu Leu Gly Asn Glu Ser Cys
        595                 600                 605

acc ttg acc tta agc gag agc acg aca aat acg ttg aaa tgc aca gtt 1872
Thr Leu Thr Leu Ser Glu Ser Thr Thr Asn Thr Leu Lys Cys Thr Val
    610                 615                 620

ggt ccc gcg atg agt gag cac ttc aat gtg tct gta att atc tca aac 1920
Gly Pro Ala Met Ser Glu His Phe Asn Val Ser Val Ile Ile Ser Asn
625                 630                 635                 640

agt cga gag aca aca caa tac agt gca ttc tcc tat gta gat cct gta 1968
Ser Arg Glu Thr Thr Gln Tyr Ser Ala Phe Ser Tyr Val Asp Pro Val
                645                 650                 655

ata aca agc att tct ccg agg tac ggc cct cag gct gga ggc acc tta 2016
Ile Thr Ser Ile Ser Pro Arg Tyr Gly Pro Gln Ala Gly Gly Thr Leu
            660                 665                 670

ctc act ctt act ggg aaa tac ctc aac agt ggc aat tct aga cac att 2064
Leu Thr Leu Thr Gly Lys Tyr Leu Asn Ser Gly Asn Ser Arg His Ile
        675                 680                 685

tca att gga ggg aaa aca tgt act tta aaa agt gta tca gat agt att 2112
Ser Ile Gly Gly Lys Thr Cys Thr Leu Lys Ser Val Ser Asp Ser Ile
    690                 695                 700

ctt gaa tgc tac acc cca gcc caa act acc tct gat gag ttt cct gtg 2160
Leu Glu Cys Tyr Thr Pro Ala Gln Thr Thr Ser Asp Glu Phe Pro Val
705                 710                 715                 720

aaa ttg aag att gac ttg gct aac cga gag acc agc agc ttc agt tac 2208

Lys Leu Lys Ile Asp Leu Ala Asn Arg Glu Thr Ser Ser Phe Ser Tyr
                725                 730                 735

cgg gaa gac ccc gtt gtc tat gaa atc cac cca acc aaa tct ttt att 2256
Arg Glu Asp Pro Val Val Tyr Glu Ile His Pro Thr Lys Ser Phe Ile
            740                 745                 750

agt ggt gga agc aca ata acg ggt att ggg aag acc ctg aat tcg gtt 2304
Ser Gly Gly Ser Thr Ile Thr Gly Ile Gly Lys Thr Leu Asn Ser Val
        755                 760                 765

agc ctc cca aag ctg gta ata gat gtg cat gaa gtg ggt gtg aac tac 2352
Ser Leu Pro Lys Leu Val Ile Asp Val His Glu Val Gly Val Asn Tyr
    770                 775                 780

aca gtg gca tgt cag cat cgc tca aat tca gag atc atc tgc tgc act 2400
Thr Val Ala Cys Gln His Arg Ser Asn Ser Glu Ile Ile Cys Cys Thr
785                 790                 795                 800

act cct tca ctg aaa cag ctg ggc ctg caa ctc ccc ctg aag acc aaa 2448
Thr Pro Ser Leu Lys Gln Leu Gly Leu Gln Leu Pro Leu Lys Thr Lys
                805                 810                 815

gcc ttc ttc ctg tta gac ggg att ctt tcc aaa cac ttt gat ctc act 2496
Ala Phe Phe Leu Leu Asp Gly Ile Leu Ser Lys His Phe Asp Leu Thr
            820                 825                 830

tat gtg cat aat cct gtg ttt gag cct ttt gaa aag cca gta atg atc 2544
Tyr Val His Asn Pro Val Phe Glu Pro Phe Glu Lys Pro Val Met Ile
        835                 840                 845

tca atg ggc aat gaa aat gta gtg gaa att aag gga aac aat att gac 2592
Ser Met Gly Asn Glu Asn Val Val Glu Ile Lys Gly Asn Asn Ile Asp
    850                 855                 860

cct gaa gca gtt aaa ggt gaa gtg tta aaa gtt gga aat cag agc tgc 2640
Pro Glu Ala Val Lys Gly Glu Val Leu Lys Val Gly Asn Gln Ser Cys
865                 870                 875                 880

gag agt ctc cac tgg cac tct gga gct gtg ttg tgt aca gtc ccc agt 2688
Glu Ser Leu His Trp His Ser Gly Ala Val Leu Cys Thr Val Pro Ser
                885                 890                 895

gac ctg ctc aaa ctg aac agc gag cta aat ata gag tgg aag caa gca 2736
Asp Leu Leu Lys Leu Asn Ser Glu Leu Asn Ile Glu Trp Lys Gln Ala
            900                 905                 910

gtc tct tca act gtt ctt gga aaa gtg atc gtt caa ccg gat cag aat 2784
Val Ser Ser Thr Val Leu Gly Lys Val Ile Val Gln Pro Asp Gln Asn

915                    920                    925

ttt gca gga ttg atc att ggt gcg gtc tca ata tca gta gta gtt ttg     2832
Phe Ala Gly Leu Ile Ile Gly Ala Val Ser Ile Ser Val Val Val Leu
    930                    935                    940

tta tta tcc ggg ctc ttc ctg tgg atg aga aag aga aag cat aaa gat     2880
Leu Leu Ser Gly Leu Phe Leu Trp Met Arg Lys Arg Lys His Lys Asp
945                    950                    955                    960

ctg ggc agt gaa tta gtt cgc tat gac gca aga gta cac act cct cat     2928
Leu Gly Ser Glu Leu Val Arg Tyr Asp Ala Arg Val His Thr Pro His
                965                    970                    975

ttg gat agg ctt gta agt gcc cga agt gta agt cca act aca gag atg     2976
Leu Asp Arg Leu Val Ser Ala Arg Ser Val Ser Pro Thr Thr Glu Met
            980                    985                    990

gtt tca aat gag tct gta gac tac  aga gct act ttt cca  gaa gac cag     3024
Val Ser Asn Glu Ser Val Asp Tyr  Arg Ala Thr Phe Pro  Glu Asp Gln
        995                    1000                    1005

ttt ccc  aac tcc tct cag aat  gga gca tgc aga caa  gtg caa tat     3069
Phe Pro  Asn Ser Ser Gln Asn  Gly Ala Cys Arg Gln  Val Gln Tyr
    1010                    1015                    1020

cct ctg  aca gac ctg tcc cct  atc ctg acg agt gga  gac tct gat     3114
Pro Leu  Thr Asp Leu Ser Pro  Ile Leu Thr Ser Gly  Asp Ser Asp
    1025                    1030                    1035

ata tcc  agc cca tta cta caa  aat act gtt cac att  gac ctc agt     3159
Ile Ser  Ser Pro Leu Leu Gln  Asn Thr Val His Ile  Asp Leu Ser
    1040                    1045                    1050

gct cta  aat cca gag ctg gtc  caa gca gtt cag cac  gtt gtg att     3204
Ala Leu  Asn Pro Glu Leu Val  Gln Ala Val Gln His  Val Val Ile
    1055                    1060                    1065

gga ccc  agc agc ctg att gtg  cat ttc aat gaa gtc  ata gga aga     3249
Gly Pro  Ser Ser Leu Ile Val  His Phe Asn Glu Val  Ile Gly Arg
    1070                    1075                    1080

ggg cat  ttt ggc tgt gtc tat  cat ggg act ttg ctg  gac aat gac     3294
Gly His  Phe Gly Cys Val Tyr  His Gly Thr Leu Leu  Asp Asn Asp
    1085                    1090                    1095

gga aag  aaa att cac tgt gct  gtg aaa tcc ttg aat  aga atc aca     3339
Gly Lys  Lys Ile His Cys Ala  Val Lys Ser Leu Asn  Arg Ile Thr
    1100                    1105                    1110

gat ata gaa gag gtc tcc cag ttt ctg act gag gga atc atc atg 3384
Asp Ile Glu Glu Val Ser Gln Phe Leu Thr Glu Gly Ile Ile Met
    1115                1120                1125

aaa gac ttc agc cat ccc aat gtt ctc tca ctc ttg gga atc tgc 3429
Lys Asp Phe Ser His Pro Asn Val Leu Ser Leu Leu Gly Ile Cys
    1130                1135                1140

ctg agg agt gaa ggg tct cct ctg gtg gtc ctg ccc tat atg aag 3474
Leu Arg Ser Glu Gly Ser Pro Leu Val Val Leu Pro Tyr Met Lys
    1145                1150                1155

cat gga gat ctg cga aat ttc att cga aac gag act cat aat cca 3519
His Gly Asp Leu Arg Asn Phe Ile Arg Asn Glu Thr His Asn Pro
    1160                1165                1170

act gtg aaa gat ctt ata gga ttt ggc ctt caa gta gcc aaa ggc 3564
Thr Val Lys Asp Leu Ile Gly Phe Gly Leu Gln Val Ala Lys Gly
    1175                1180                1185

atg aaa tat ctt gcc agc aaa aag ttt gtc cac aga gac tta gct 3609
Met Lys Tyr Leu Ala Ser Lys Lys Phe Val His Arg Asp Leu Ala
    1190                1195                1200

gca aga aac tgc atg ttg gat gaa aaa ttc act gtc aag gtt gct 3654
Ala Arg Asn Cys Met Leu Asp Glu Lys Phe Thr Val Lys Val Ala
    1205                1210                1215

gat ttc ggt ctt gcc aga gac atg tac gat aaa gag tac tat agt 3699
Asp Phe Gly Leu Ala Arg Asp Met Tyr Asp Lys Glu Tyr Tyr Ser
    1220                1225                1230

gtc cac aac aag acg ggt gcc aag cta cca gtg aag tgg atg gct 3744
Val His Asn Lys Thr Gly Ala Lys Leu Pro Val Lys Trp Met Ala
    1235                1240                1245

tta gag agt ctg caa acg cag aag ttc acc acc aag tca gat gtg 3789
Leu Glu Ser Leu Gln Thr Gln Lys Phe Thr Thr Lys Ser Asp Val
    1250                1255                1260

tgg tcc ttt ggt gtg ctc ctc tgg gag ctc atg acg aga gga gcc 3834
Trp Ser Phe Gly Val Leu Leu Trp Glu Leu Met Thr Arg Gly Ala
    1265                1270                1275

cct cct tat ccc gac gtg aac aca ttt gat atc act atc tac ctg 3879
Pro Pro Tyr Pro Asp Val Asn Thr Phe Asp Ile Thr Ile Tyr Leu
    1280                1285                1290

ttg caa ggc aga aga ctc ttg caa cca gaa tac tgt cca gac gcc 3924
Leu Gln Gly Arg Arg Leu Leu Gln Pro Glu Tyr Cys Pro Asp Ala
    1295                1300                1305

ttg tac gaa gtg atg cta aaa tgc tgg cac ccc aaa gcg gaa atg 3969
Leu Tyr Glu Val Met Leu Lys Cys Trp His Pro Lys Ala Glu Met
    1310                1315                1320

cgc ccg tcc ttt tcc gaa ctg gtc tcc agg ata tcc tca atc ttc 4014
Arg Pro Ser Phe Ser Glu Leu Val Ser Arg Ile Ser Ser Ile Phe
    1325                1330                1335

tcc acg ttc att ggg gaa cac tac gtc cac gtg aac gct act tat 4059
Ser Thr Phe Ile Gly Glu His Tyr Val His Val Asn Ala Thr Tyr
    1340                1345                1350

gtg aat gta aaa tgt gtt gct cca tat cct tct ctg ttg cca tcc 4104
Val Asn Val Lys Cys Val Ala Pro Tyr Pro Ser Leu Leu Pro Ser
    1355                1360                1365

caa gac aac att gat ggc gag ggg aac aca tga 4137
Gln Asp Asn Ile Asp Gly Glu Gly Asn Thr
    1370                1375

<210> 18
<211> 1378
<212> PRT
<213> Mus musculus

<400> 18

Lys Ala Pro Thr Val Leu Val Pro Gly Ile Leu Val Leu Leu Leu Ser
1               5                   10                  15

Leu Val Gln Arg Ser His Gly Glu Cys Lys Glu Ala Leu Val Lys Ser
            20                  25                  30

Glu Met Asn Val Asn Met Lys Tyr Gln Leu Pro Asn Phe Thr Ala Glu
        35                  40                  45

Thr Pro Ile Gln Asn Val Val Leu His Gly His His Ile Tyr Leu Gly
    50                  55                  60

Ala Thr Asn Tyr Ile Tyr Val Leu Asn Asp Lys Asp Leu Gln Lys Val

65                  70                  75                  80

Ser Glu Phe Lys Thr Gly Pro Val Leu Glu His Pro Asp Cys Leu Pro
                85                  90                  95

Cys Arg Asp Cys Ser Ser Lys Ala Asn Ser Ser Gly Gly Val Trp Lys
            100                 105                 110

Asp Asn Ile Asn Met Ala Leu Leu Val Asp Thr Tyr Tyr Asp Asp Gln
        115                 120                 125

Leu Ile Ser Cys Gly Ser Val Asn Arg Gly Thr Cys Gln Arg His Val
    130                 135                 140

Leu Pro Pro Asp Asn Ser Ala Asp Ile Gln Ser Glu Val His Cys Met
145                 150                 155                 160

Phe Ser Pro Glu Glu Glu Ser Gly Gln Cys Pro Asp Cys Val Val Ser
                165                 170                 175

Ala Leu Gly Ala Lys Val Leu Leu Ser Glu Lys Asp Arg Phe Ile Asn
            180                 185                 190

Phe Phe Val Gly Asn Thr Ile Asn Ser Ser Tyr Pro Pro Gly Tyr Ser
        195                 200                 205

Leu His Ser Ile Ser Val Arg Arg Leu Lys Glu Thr Gln Asp Gly Phe
    210                 215                 220

Lys Phe Leu Thr Asp Gln Ser Tyr Ile Asp Val Leu Pro Glu Phe Gln
225                 230                 235                 240

Asp Ser Tyr Pro Ile Lys Tyr Ile His Ala Phe Glu Ser Asn His Phe
                245                 250                 255

Ile Tyr Phe Leu Thr Val Gln Lys Glu Thr Leu Asp Ala Gln Thr Phe
            260                 265                 270

His Thr Arg Ile Ile Arg Phe Cys Ser Val Asp Ser Gly Leu His Ser
        275                 280                 285

Tyr Met Glu Met Pro Leu Glu Cys Ile Leu Thr Glu Lys Arg Arg Lys
    290                 295                 300

Arg Ser Thr Arg Glu Glu Val Phe Asn Ile Leu Gln Ala Ala Tyr Val
305                 310                 315                 320

Ser Lys Pro Gly Ala Asn Leu Ala Lys Gln Ile Gly Ala Ser Pro Ser
                325                 330                 335

Asp Asp Ile Leu Phe Gly Val Phe Ala Gln Ser Lys Pro Asp Ser Ala
            340                 345                 350

Glu Pro Val Asn Arg Ser Ala Val Cys Ala Phe Pro Ile Lys Tyr Val
        355                 360                 365

Asn Asp Phe Phe Asn Lys Ile Val Asn Lys Asn Asn Val Arg Cys Leu
    370                 375                 380

Gln His Phe Tyr Gly Pro Asn His Glu His Cys Phe Asn Arg Thr Leu
385                 390                 395                 400

Leu Arg Asn Ser Ser Gly Cys Glu Ala Arg Ser Asp Glu Tyr Arg Thr
                405                 410                 415

Glu Phe Thr Thr Ala Leu Gln Arg Val Asp Leu Phe Met Gly Arg Leu
            420                 425                 430

Asn Gln Val Leu Leu Thr Ser Ile Ser Thr Phe Ile Lys Gly Asp Leu
        435                 440                 445

Thr Ile Ala Asn Leu Gly Thr Ser Glu Gly Arg Phe Met Gln Val Val
    450                 455                 460

Leu Ser Arg Thr Ala His Leu Thr Pro His Val Asn Phe Leu Leu Asp
465                 470                 475                 480

Ser His Pro Val Ser Pro Glu Val Ile Val Glu His Pro Ser Asn Gln
                485                 490                 495

Asn Gly Tyr Thr Leu Val Val Thr Gly Lys Lys Ile Thr Lys Ile Pro
            500                 505                 510

Leu Asn Gly Leu Gly Cys Gly His Phe Gln Ser Cys Ser Gln Cys Leu
        515                 520                 525

Ser Ala Pro Tyr Phe Ile Gln Cys Gly Trp Cys His Asn Gln Cys Val
    530                 535                 540

Arg Phe Asp Glu Cys Pro Ser Gly Thr Trp Thr Gln Glu Ile Cys Leu
545                 550                 555                 560

Pro Ala Val Tyr Lys Val Phe Pro Thr Ser Ala Pro Leu Glu Gly Gly
                565                 570                 575

Thr Val Leu Thr Ile Cys Gly Trp Asp Phe Gly Phe Arg Lys Asn Asn
            580                 585                 590

Lys Phe Asp Leu Arg Lys Thr Lys Val Leu Leu Gly Asn Glu Ser Cys
        595                 600                 605

Thr Leu Thr Leu Ser Glu Ser Thr Thr Asn Thr Leu Lys Cys Thr Val
    610                 615                 620

Gly Pro Ala Met Ser Glu His Phe Asn Val Ser Val Ile Ile Ser Asn
625                 630                 635                 640

Ser Arg Glu Thr Thr Gln Tyr Ser Ala Phe Ser Tyr Val Asp Pro Val
                645                 650                 655

Ile Thr Ser Ile Ser Pro Arg Tyr Gly Pro Gln Ala Gly Gly Thr Leu
            660                 665                 670

Leu Thr Leu Thr Gly Lys Tyr Leu Asn Ser Gly Asn Ser Arg His Ile
        675                 680                 685

Ser Ile Gly Gly Lys Thr Cys Thr Leu Lys Ser Val Ser Asp Ser Ile
    690                 695                 700

Leu Glu Cys Tyr Thr Pro Ala Gln Thr Thr Ser Asp Glu Phe Pro Val
705                 710                 715                 720

Lys Leu Lys Ile Asp Leu Ala Asn Arg Glu Thr Ser Ser Phe Ser Tyr
                725                 730                 735

Arg Glu Asp Pro Val Val Tyr Glu Ile His Pro Thr Lys Ser Phe Ile
            740                 745                 750

Ser Gly Gly Ser Thr Ile Thr Gly Ile Gly Lys Thr Leu Asn Ser Val
        755                 760                 765

Ser Leu Pro Lys Leu Val Ile Asp Val His Glu Val Gly Val Asn Tyr
    770                 775                 780

Thr Val Ala Cys Gln His Arg Ser Asn Ser Glu Ile Ile Cys Cys Thr
785                 790                 795                 800

Thr Pro Ser Leu Lys Gln Leu Gly Leu Gln Leu Pro Leu Lys Thr Lys
                805                 810                 815

Ala Phe Phe Leu Leu Asp Gly Ile Leu Ser Lys His Phe Asp Leu Thr
            820                 825                 830

Tyr Val His Asn Pro Val Phe Glu Pro Phe Glu Lys Pro Val Met Ile
        835                 840                 845

Ser Met Gly Asn Glu Asn Val Val Glu Ile Lys Gly Asn Asn Ile Asp

850                 855                 860

Pro Glu Ala Val Lys Gly Glu Val Leu Lys Val Gly Asn Gln Ser Cys
865                 870                 875                 880

Glu Ser Leu His Trp His Ser Gly Ala Val Leu Cys Thr Val Pro Ser
                885                 890                 895

Asp Leu Leu Lys Leu Asn Ser Glu Leu Asn Ile Glu Trp Lys Gln Ala
            900                 905                 910

Val Ser Ser Thr Val Leu Gly Lys Val Ile Val Gln Pro Asp Gln Asn
        915                 920                 925

Phe Ala Gly Leu Ile Ile Gly Ala Val Ser Ile Ser Val Val Val Leu
    930                 935                 940

Leu Leu Ser Gly Leu Phe Leu Trp Met Arg Lys Arg Lys His Lys Asp
945                 950                 955                 960

Leu Gly Ser Glu Leu Val Arg Tyr Asp Ala Arg Val His Thr Pro His
                965                 970                 975

Leu Asp Arg Leu Val Ser Ala Arg Ser Val Ser Pro Thr Thr Glu Met
            980                 985                 990

Val Ser Asn Glu Ser Val Asp Tyr Arg Ala Thr Phe Pro Glu Asp Gln
        995                 1000                1005

Phe Pro Asn Ser Ser Gln Asn Gly Ala Cys Arg Gln Val Gln Tyr
    1010                1015                1020

Pro Leu Thr Asp Leu Ser Pro Ile Leu Thr Ser Gly Asp Ser Asp
    1025                1030                1035

Ile Ser Ser Pro Leu Leu Gln Asn Thr Val His Ile Asp Leu Ser
    1040                1045                1050

Ala Leu Asn Pro Glu Leu Val Gln Ala Val Gln His Val Val Ile
    1055                1060                1065

Gly Pro Ser Ser Leu Ile Val His Phe Asn Glu Val Ile Gly Arg
    1070                1075                1080

Gly His Phe Gly Cys Val Tyr His Gly Thr Leu Leu Asp Asn Asp
    1085                1090                1095

Gly Lys Lys Ile His Cys Ala Val Lys Ser Leu Asn Arg Ile Thr
    1100                1105                1110

Asp Ile Glu Glu Val Ser Gln Phe Leu Thr Glu Gly Ile Ile Met
    1115                1120                1125

Lys Asp Phe Ser His Pro Asn Val Leu Ser Leu Leu Gly Ile Cys
    1130                1135                1140

Leu Arg Ser Glu Gly Ser Pro Leu Val Val Leu Pro Tyr Met Lys
    1145                1150                1155

His Gly Asp Leu Arg Asn Phe Ile Arg Asn Glu Thr His Asn Pro
    1160                1165                1170

Thr Val Lys Asp Leu Ile Gly Phe Gly Leu Gln Val Ala Lys Gly
    1175                1180                1185

Met Lys Tyr Leu Ala Ser Lys Lys Phe Val His Arg Asp Leu Ala
    1190                1195                1200

Ala Arg Asn Cys Met Leu Asp Glu Lys Phe Thr Val Lys Val Ala
    1205                1210                1215

Asp Phe Gly Leu Ala Arg Asp Met Tyr Asp Lys Glu Tyr Tyr Ser
    1220                1225                1230

Val His Asn Lys Thr Gly Ala Lys Leu Pro Val Lys Trp Met Ala
    1235                1240                1245

Leu Glu Ser Leu Gln Thr Gln Lys Phe Thr Thr Lys Ser Asp Val
    1250                1255                1260

Trp Ser Phe Gly Val Leu Leu Trp Glu Leu Met Thr Arg Gly Ala
    1265                1270                1275

Pro Pro Tyr Pro Asp Val Asn Thr Phe Asp Ile Thr Ile Tyr Leu
    1280                1285                1290

Leu Gln Gly Arg Arg Leu Leu Gln Pro Glu Tyr Cys Pro Asp Ala
    1295                1300                1305

Leu Tyr Glu Val Met Leu Lys Cys Trp His Pro Lys Ala Glu Met
    1310                1315                1320

Arg Pro Ser Phe Ser Glu Leu Val Ser Arg Ile Ser Ser Ile Phe
    1325                1330                1335

Ser Thr Phe Ile Gly Glu His Tyr Val His Val Asn Ala Thr Tyr
    1340                1345                1350

Val Asn Val Lys Cys Val Ala Pro Tyr Pro Ser Leu Leu Pro Ser
    1355                1360                1365

Gln Asp Asn Ile Asp Gly Glu Gly Asn Thr
    1370                1375

<210> 19
<211> 4140
<212> DNA
<213> Mus musculus

<220>
<221> CDS

<222> (1)..(4137)

<400> 19
atg aag gct ccc acc gtg ctg gca cct ggc att ctg gtg ctg ctg ttg 48
Met Lys Ala Pro Thr Val Leu Ala Pro Gly Ile Leu Val Leu Leu Leu
1               5                   10                  15

tcc ttg gtg cag agg agc cat ggg gag tgc aag gag gcc cta gtg aag 96
Ser Leu Val Gln Arg Ser His Gly Glu Cys Lys Glu Ala Leu Val Lys
            20                  25                  30

tct gag atg aac gtg aac atg aag tat cag ctc ccc aac ttc acg gca 144
Ser Glu Met Asn Val Asn Met Lys Tyr Gln Leu Pro Asn Phe Thr Ala
        35                  40                  45

gaa acc ccc atc cag aat gtc gtc cta cac ggc cat cat att tat ctc 192
Glu Thr Pro Ile Gln Asn Val Val Leu His Gly His His Ile Tyr Leu
    50                  55                  60

gga gcc aca aac tac att tat gtt tta aat gac aaa gac ctt cag aag 240
Gly Ala Thr Asn Tyr Ile Tyr Val Leu Asn Asp Lys Asp Leu Gln Lys
65                  70                  75                  80

gta tcc gaa ttc aag acc ggg ccc gtg ttg gaa cac cca gat tgt tta 288
Val Ser Glu Phe Lys Thr Gly Pro Val Leu Glu His Pro Asp Cys Leu
                85                  90                  95

cct tgt cgg gac tgc agc agc aaa gcc aat tca tca gga ggg gtt tgg 336
Pro Cys Arg Asp Cys Ser Ser Lys Ala Asn Ser Ser Gly Gly Val Trp
            100                 105                 110

aaa gac aac atc aac atg gct ctg ctt gtt gac aca tac tat gat gat 384
Lys Asp Asn Ile Asn Met Ala Leu Leu Val Asp Thr Tyr Tyr Asp Asp
        115                 120                 125

caa ctc att agc tgt ggc agt gtc aac aga ggg act tgc cag cgg cat 432
Gln Leu Ile Ser Cys Gly Ser Val Asn Arg Gly Thr Cys Gln Arg His
    130                 135                 140

gtc ctt cct cct gac aat tct gct gac atc cag tct gag gtc cac tgc 480
Val Leu Pro Pro Asp Asn Ser Ala Asp Ile Gln Ser Glu Val His Cys
145                 150                 155                 160

atg ttc tcc cca gaa gag gag tca ggg cag tgt cct gac tgt gta gtg 528
Met Phe Ser Pro Glu Glu Glu Ser Gly Gln Cys Pro Asp Cys Val Val
                165                 170                 175

agt gcc ctc gga gcc aaa gtc ctc ctg tcg gaa aag gac cgg ttc atc 576
Ser Ala Leu Gly Ala Lys Val Leu Leu Ser Glu Lys Asp Arg Phe Ile

            180                 185                 190

aat ttc ttt gtg ggg aat acg atc aat tcc tcc tat cct cct ggt tat 624
Asn Phe Phe Val Gly Asn Thr Ile Asn Ser Ser Tyr Pro Pro Gly Tyr
        195                 200                 205

tca ctg cat tcg ata tcg gtg aga cgg ctg aag gaa acc caa gat ggt 672
Ser Leu His Ser Ile Ser Val Arg Arg Leu Lys Glu Thr Gln Asp Gly
    210                 215                 220

ttt aag ttt ttg aca gac cag tcc tat att gat gtc tta cca gaa ttc 720
Phe Lys Phe Leu Thr Asp Gln Ser Tyr Ile Asp Val Leu Pro Glu Phe
225                 230                 235                 240

caa gat tcc tac ccc ata aag tac ata cat gcc ttc gaa agc aac cat 768
Gln Asp Ser Tyr Pro Ile Lys Tyr Ile His Ala Phe Glu Ser Asn His
                245                 250                 255

ttt att tac ttt ctg act gtc caa aag gaa act cta gat gct cag act 816
Phe Ile Tyr Phe Leu Thr Val Gln Lys Glu Thr Leu Asp Ala Gln Thr
            260                 265                 270

ttt cat aca aga ata atc agg ttc tgt tcc gta gac tct ggg ttg cac 864
Phe His Thr Arg Ile Ile Arg Phe Cys Ser Val Asp Ser Gly Leu His
        275                 280                 285

tcc tac atg gaa atg ccc ctg gaa tgc atc ctg aca gaa aaa aga agg 912
Ser Tyr Met Glu Met Pro Leu Glu Cys Ile Leu Thr Glu Lys Arg Arg
    290                 295                 300

aag aga tcc aca agg gaa gaa gtg ttt aat atc ctc caa gcc gcg tat 960
Lys Arg Ser Thr Arg Glu Glu Val Phe Asn Ile Leu Gln Ala Ala Tyr
305                 310                 315                 320

gtc agt aaa cca ggg gcc aat ctt gct aag caa ata gga gct agc cct 1008
Val Ser Lys Pro Gly Ala Asn Leu Ala Lys Gln Ile Gly Ala Ser Pro
                325                 330                 335

tct gat gac att ctc ttc ggg gtg ttt gca caa agc aag cca gat tct 1056
Ser Asp Asp Ile Leu Phe Gly Val Phe Ala Gln Ser Lys Pro Asp Ser
            340                 345                 350

gct gaa cct gtg aat cga tca gca gtc tgt gca ttc ccc atc aaa tat 1104
Ala Glu Pro Val Asn Arg Ser Ala Val Cys Ala Phe Pro Ile Lys Tyr
        355                 360                 365

gtc aat gac ttc ttc aac aag att gtc aac aaa aac aac gtg aga tgt 1152
Val Asn Asp Phe Phe Asn Lys Ile Val Asn Lys Asn Asn Val Arg Cys
    370                 375                 380

ctc cag cat ttt tac gga ccc aac cat gag cac tgt ttc aat agg acc 1200
Leu Gln His Phe Tyr Gly Pro Asn His Glu His Cys Phe Asn Arg Thr
385                 390                 395                 400

ctg ctg aga aac tct tcc ggc tgt gaa gcg cgc agt gac gag tat cgg 1248
Leu Leu Arg Asn Ser Ser Gly Cys Glu Ala Arg Ser Asp Glu Tyr Arg
                405                 410                 415

aca gag ttt acc acg gct ttg cag cgc gtc gac tta ttc atg ggc cgg 1296
Thr Glu Phe Thr Thr Ala Leu Gln Arg Val Asp Leu Phe Met Gly Arg
            420                 425                 430

ctt aac caa gtg ctc ctg aca tcc atc tcc acc ttc atc aaa ggt gac 1344
Leu Asn Gln Val Leu Leu Thr Ser Ile Ser Thr Phe Ile Lys Gly Asp
        435                 440                 445

ctc acc att gct aat cta ggg acg tca gaa ggt cgc ttc atg cag gtg 1392
Leu Thr Ile Ala Asn Leu Gly Thr Ser Glu Gly Arg Phe Met Gln Val
    450                 455                 460

gtg ctc tct cga aca gca cac ctc act cct cat gtg aac ttc ctc ctg 1440
Val Leu Ser Arg Thr Ala His Leu Thr Pro His Val Asn Phe Leu Leu
465                 470                 475                 480

gac tcc cat cct gta tct cca gaa gtt att gtt gag cat cca tca aat 1488
Asp Ser His Pro Val Ser Pro Glu Val Ile Val Glu His Pro Ser Asn
                485                 490                 495

caa aat ggc tat aca ttg gtt gtc aca gga aag aag atc acc aag att 1536
Gln Asn Gly Tyr Thr Leu Val Val Thr Gly Lys Lys Ile Thr Lys Ile
            500                 505                 510

cca ttg aat ggc ctg ggc tgt gga cat ttc caa tcc tgc agt cag tgc 1584
Pro Leu Asn Gly Leu Gly Cys Gly His Phe Gln Ser Cys Ser Gln Cys
        515                 520                 525

ctc tct gcc cct tac ttt ata cag tgt ggc tgg tgc cac aat caa tgt 1632
Leu Ser Ala Pro Tyr Phe Ile Gln Cys Gly Trp Cys His Asn Gln Cys
    530                 535                 540

gtg cgt ttt gat gaa tgc ccc agc ggt aca tgg act caa gag atc tgt 1680
Val Arg Phe Asp Glu Cys Pro Ser Gly Thr Trp Thr Gln Glu Ile Cys
545                 550                 555                 560

ctg ccg gcg gtt tat aag gtg ttc ccc acc agc gcg ccc ctt gaa gga 1728
Leu Pro Ala Val Tyr Lys Val Phe Pro Thr Ser Ala Pro Leu Glu Gly
                565                 570                 575

gga aca gtg ttg acc ata tgt ggc tgg gac ttt gga ttc agg aag aat 1776
Gly Thr Val Leu Thr Ile Cys Gly Trp Asp Phe Gly Phe Arg Lys Asn
            580                 585                 590

aat aaa ttt gat tta agg aaa acc aaa gtt ctg ctt ggc aac gag agc 1824
Asn Lys Phe Asp Leu Arg Lys Thr Lys Val Leu Leu Gly Asn Glu Ser
        595                 600                 605

tgt acc ttg acc tta agc gag agc acg aca aat acg ttg aaa tgc aca 1872
Cys Thr Leu Thr Leu Ser Glu Ser Thr Thr Asn Thr Leu Lys Cys Thr
    610                 615                 620

gtt ggt ccc gcg atg agt gag cac ttc aat gtg tct gta att atc tca 1920
Val Gly Pro Ala Met Ser Glu His Phe Asn Val Ser Val Ile Ile Ser
625                 630                 635                 640

aac agt cga gag acg acg caa tac agt gca ttc tcc tat gta gat cct 1968
Asn Ser Arg Glu Thr Thr Gln Tyr Ser Ala Phe Ser Tyr Val Asp Pro
                645                 650                 655

gta ata aca agc att tct ccg agg tac ggc cct cag gct gga ggc acc 2016
Val Ile Thr Ser Ile Ser Pro Arg Tyr Gly Pro Gln Ala Gly Gly Thr
            660                 665                 670

tta ctc act ctt act ggg aaa tac ctc aac agt ggc aat tct aga cac 2064
Leu Leu Thr Leu Thr Gly Lys Tyr Leu Asn Ser Gly Asn Ser Arg His
        675                 680                 685

att tca att gga ggg aaa aca tgt act tta aaa agt gta tca gat agt 2112
Ile Ser Ile Gly Gly Lys Thr Cys Thr Leu Lys Ser Val Ser Asp Ser
    690                 695                 700

att ctt gaa tgc tac acc cca gcc caa act acc tct gat gag ttt cct 2160
Ile Leu Glu Cys Tyr Thr Pro Ala Gln Thr Thr Ser Asp Glu Phe Pro
705                 710                 715                 720

gtg aaa ttg aag att gac ttg gct aac cga gag acc agc agc ttc agt 2208
Val Lys Leu Lys Ile Asp Leu Ala Asn Arg Glu Thr Ser Ser Phe Ser
                725                 730                 735

tac cgg gaa gac ccc gtt gtc tat gaa atc cac ccg acc aaa tct ttt 2256
Tyr Arg Glu Asp Pro Val Val Tyr Glu Ile His Pro Thr Lys Ser Phe
            740                 745                 750

att agt ggt gga agc aca ata acg ggt att ggg aag acc ctg aac tcg 2304
Ile Ser Gly Gly Ser Thr Ile Thr Gly Ile Gly Lys Thr Leu Asn Ser
        755                 760                 765

gtt agc ctc cca aag ctg gta ata gat gtg cat gaa gtg ggt gtg aac 2352

Val Ser Leu Pro Lys Leu Val Ile Asp Val His Glu Val Gly Val Asn
    770                 775                 780

tac aca gtg gca tgt cag cat cgc tca aat tca gag atc atc tgc tgc     2400
Tyr Thr Val Ala Cys Gln His Arg Ser Asn Ser Glu Ile Ile Cys Cys
785                 790                 795                 800

act act cct tca ctg aaa cag ctg ggc ctg caa ctc ccc ctg aag acc     2448
Thr Thr Pro Ser Leu Lys Gln Leu Gly Leu Gln Leu Pro Leu Lys Thr
                805                 810                 815

aaa gcc ttc ttc ctg tta gac ggg att ctt tcc aaa cac ttt gat ctc     2496
Lys Ala Phe Phe Leu Leu Asp Gly Ile Leu Ser Lys His Phe Asp Leu
            820                 825                 830

act tat gtg cat aat cct gtg ttt gag cct ttt gaa aag cca gta atg     2544
Thr Tyr Val His Asn Pro Val Phe Glu Pro Phe Glu Lys Pro Val Met
        835                 840                 845

atc tca ata ggc aat gaa aat gta gtg gaa att aag gga aac aat att     2592
Ile Ser Ile Gly Asn Glu Asn Val Val Glu Ile Lys Gly Asn Asn Ile
    850                 855                 860

gac cct gaa gca gtt aaa ggt gaa gtg tta aaa gtt gga aat cag agc     2640
Asp Pro Glu Ala Val Lys Gly Glu Val Leu Lys Val Gly Asn Gln Ser
865                 870                 875                 880

tgc gag agt ctc cac tgg cac tct gga gct gtg ttg tgt aca gtc ccc     2688
Cys Glu Ser Leu His Trp His Ser Gly Ala Val Leu Cys Thr Val Pro
                885                 890                 895

agt gac ctg ctc aaa ctg aac agc gag cta aat ata gag tgg aag caa     2736
Ser Asp Leu Leu Lys Leu Asn Ser Glu Leu Asn Ile Glu Trp Lys Gln
            900                 905                 910

gca gtc tct tca act gtt ctt gga aaa gtg atc gtt caa ccg gat cag     2784
Ala Val Ser Ser Thr Val Leu Gly Lys Val Ile Val Gln Pro Asp Gln
        915                 920                 925

aat ttt gca gga ttg atc att ggt gcg gtc tca ata tca gta gta gtt     2832
Asn Phe Ala Gly Leu Ile Ile Gly Ala Val Ser Ile Ser Val Val Val
    930                 935                 940

ttg tta tta tcc ggg ctc ttc ctg tgg atg aga aag aga aag cat aaa     2880
Leu Leu Leu Ser Gly Leu Phe Leu Trp Met Arg Lys Arg Lys His Lys
945                 950                 955                 960

gat ctg ggc agt gaa tta gtt cgc tat gac gca aga gta cac act cct     2928
Asp Leu Gly Ser Glu Leu Val Arg Tyr Asp Ala Arg Val His Thr Pro

                965                 970                 975

cat ttg gat agg ctt gta agt gcc cga agt gta agt cca act aca gag 2976
His Leu Asp Arg Leu Val Ser Ala Arg Ser Val Ser Pro Thr Thr Glu
            980                 985                 990

atg gtt tca aat gag tct gta gac tac aga gct act ttt cca gaa gac 3024
Met Val Ser Asn Glu Ser Val Asp Tyr Arg Ala Thr Phe Pro Glu Asp
        995                 1000                1005

cag ttt ccc aac tcc tct cag aat gga gca tgc aga caa gtg caa 3069
Gln Phe Pro Asn Ser Ser Gln Asn Gly Ala Cys Arg Gln Val Gln
    1010                1015                1020

tac cct ctg aca gac ctg tcc cct atc ctg aca agt gga gac tct 3114
Tyr Pro Leu Thr Asp Leu Ser Pro Ile Leu Thr Ser Gly Asp Ser
    1025                1030                1035

gat ata tcc agc cca tta cta caa aat act gtt cac att gac ctc 3159
Asp Ile Ser Ser Pro Leu Leu Gln Asn Thr Val His Ile Asp Leu
    1040                1045                1050

agt gct cta aat cca gag ctg gtc caa gca gtt cag cac gta gtg 3204
Ser Ala Leu Asn Pro Glu Leu Val Gln Ala Val Gln His Val Val
    1055                1060                1065

att gga ccc agc agc ctg att gtg cat ttc aat gaa gtc ata gga 3249
Ile Gly Pro Ser Ser Leu Ile Val His Phe Asn Glu Val Ile Gly
    1070                1075                1080

aga ggg cat ttt ggc tgt gtc tat cat ggg act ttg ctg gac aat 3294
Arg Gly His Phe Gly Cys Val Tyr His Gly Thr Leu Leu Asp Asn
    1085                1090                1095

gac gga aag aaa att cac tgt gct gtg aaa tcc tta aat aga atc 3339
Asp Gly Lys Lys Ile His Cys Ala Val Lys Ser Leu Asn Arg Ile
    1100                1105                1110

aca gat ata gaa gag gtc tcc cag ttt ctg act gag gga atc atc 3384
Thr Asp Ile Glu Glu Val Ser Gln Phe Leu Thr Glu Gly Ile Ile
    1115                1120                1125

atg aaa gac ttc agc cat ccc aat gtt ctc tca ctc ttg gga atc 3429
Met Lys Asp Phe Ser His Pro Asn Val Leu Ser Leu Leu Gly Ile
    1130                1135                1140

tgc ctg agg agt gaa ggg tct cct ctg gtg gtc ctg ccc tat atg 3474
Cys Leu Arg Ser Glu Gly Ser Pro Leu Val Val Leu Pro Tyr Met
    1145                1150                1155

aag cat gga gat ctg cga aat ttc att cga aac gag act cat aat 3519
Lys His Gly Asp Leu Arg Asn Phe Ile Arg Asn Glu Thr His Asn
    1160                1165                1170

cca act gtg aaa gat ctt ata gga ttt ggc ctt caa gta gcc aaa 3564
Pro Thr Val Lys Asp Leu Ile Gly Phe Gly Leu Gln Val Ala Lys
    1175                1180                1185

ggc atg aaa tat ctt gcc agc aaa aag ttt gtc cac aga gac tta 3609
Gly Met Lys Tyr Leu Ala Ser Lys Lys Phe Val His Arg Asp Leu
    1190                1195                1200

gct gca aga aac tgc atg ttg gat gaa aaa ttc act gtc aag gtt 3654
Ala Ala Arg Asn Cys Met Leu Asp Glu Lys Phe Thr Val Lys Val
    1205                1210                1215

gct gat ttc ggt ctt gcc aga gac atg tac gat aaa gag tac tat 3699
Ala Asp Phe Gly Leu Ala Arg Asp Met Tyr Asp Lys Glu Tyr Tyr
    1220                1225                1230

agt gtc cac aac aag acg ggt gcc aag cta cca gta aag tgg atg 3744
Ser Val His Asn Lys Thr Gly Ala Lys Leu Pro Val Lys Trp Met
    1235                1240                1245

gct tta gag agt ctg caa acg cag aag ttc acc acc aag tca gat 3789
Ala Leu Glu Ser Leu Gln Thr Gln Lys Phe Thr Thr Lys Ser Asp
    1250                1255                1260

gtg tgg tcc ttt ggt gtg ctc ctc tgg gag ctc atg acg aga gga 3834
Val Trp Ser Phe Gly Val Leu Leu Trp Glu Leu Met Thr Arg Gly
    1265                1270                1275

gcc cct cct tat ccc gac gtg aac aca ttt gat atc act atc tac 3879
Ala Pro Pro Tyr Pro Asp Val Asn Thr Phe Asp Ile Thr Ile Tyr
    1280                1285                1290

ctg ttg caa ggc aga aga ctc ttg caa cca gaa tac tgt cca gac 3924
Leu Leu Gln Gly Arg Arg Leu Leu Gln Pro Glu Tyr Cys Pro Asp
    1295                1300                1305

gcc ttg tac gaa gtg atg cta aaa tgc tgg cac ccc aaa gcg gaa 3969
Ala Leu Tyr Glu Val Met Leu Lys Cys Trp His Pro Lys Ala Glu
    1310                1315                1320

atg cgc ccg tcc ttt tcc gaa ctg gtc tcc agg ata tcc tca atc 4014
Met Arg Pro Ser Phe Ser Glu Leu Val Ser Arg Ile Ser Ser Ile
    1325                1330                1335

ttc tcc acg ttc att ggg gaa cac tac gtc cac gtg aac gct act 4059
Phe Ser Thr Phe Ile Gly Glu His Tyr Val His Val Asn Ala Thr
    1340                1345                1350

tat gtg aat gta aaa tgt gtt gct cca tat cct tct ctg ttg cca 4104
Tyr Val Asn Val Lys Cys Val Ala Pro Tyr Pro Ser Leu Leu Pro
    1355                1360                1365

tcc caa gac aac att gat ggc gag ggg aac aca tga 4140
Ser Gln Asp Asn Ile Asp Gly Glu Gly Asn Thr
    1370                1375

<210> 20
<211> 1379
<212> PRT
<213> Mus musculus

<400> 20

Met Lys Ala Pro Thr Val Leu Ala Pro Gly Ile Leu Val Leu Leu Leu
1               5                   10                  15

Ser Leu Val Gln Arg Ser His Gly Glu Cys Lys Glu Ala Leu Val Lys
            20                  25                  30

Ser Glu Met Asn Val Asn Met Lys Tyr Gln Leu Pro Asn Phe Thr Ala
        35                  40                  45

Glu Thr Pro Ile Gln Asn Val Val Leu His Gly His His Ile Tyr Leu
    50                  55                  60

Gly Ala Thr Asn Tyr Ile Tyr Val Leu Asn Asp Lys Asp Leu Gln Lys
65                  70                  75                  80

Val Ser Glu Phe Lys Thr Gly Pro Val Leu Glu His Pro Asp Cys Leu
                85                  90                  95

Pro Cys Arg Asp Cys Ser Ser Lys Ala Asn Ser Ser Gly Gly Val Trp
100 .105 110

Lys Asp Asn Ile Asn Met Ala Leu Leu Val Asp Thr Tyr Tyr Asp Asp

115                 120                 125

Gln Leu Ile Ser Cys Gly Ser Val Asn Arg Gly Thr Cys Gln Arg His
    130                 135                 140

Val Leu Pro Pro Asp Asn Ser Ala Asp Ile Gln Ser Glu Val His Cys
145                 150                 155                 160

Met Phe Ser Pro Glu Glu Glu Ser Gly Gln Cys Pro Asp Cys Val Val
                165                 170                 175

Ser Ala Leu Gly Ala Lys Val Leu Leu Ser Glu Lys Asp Arg Phe Ile
            180                 185                 190

Asn Phe Phe Val Gly Asn Thr Ile Asn Ser Ser Tyr Pro Pro Gly Tyr
        195                 200                 205

Ser Leu His Ser Ile Ser Val Arg Arg Leu Lys Glu Thr Gln Asp Gly
    210                 215                 220

Phe Lys Phe Leu Thr Asp Gln Ser Tyr Ile Asp Val Leu Pro Glu Phe
225                 230                 235                 240

Gln Asp Ser Tyr Pro Ile Lys Tyr Ile His Ala Phe Glu Ser Asn His
                245                 250                 255

Phe Ile Tyr Phe Leu Thr Val Gln Lys Glu Thr Leu Asp Ala Gln Thr
            260                 265                 270

Phe His Thr Arg Ile Ile Arg Phe Cys Ser Val Asp Ser Gly Leu His
        275                 280                 285

Ser Tyr Met Glu Met Pro Leu Glu Cys Ile Leu Thr Glu Lys Arg Arg
    290                 295                 300

Lys Arg Ser Thr Arg Glu Glu Val Phe Asn Ile Leu Gln Ala Ala Tyr
305                 310                 315                 320

Val Ser Lys Pro Gly Ala Asn Leu Ala Lys Gln Ile Gly Ala Ser Pro
325 330 335

Ser Asp Asp Ile Leu Phe Gly Val Phe Ala Gln Ser Lys Pro Asp Ser
340 345 350

Ala Glu Pro Val Asn Arg Ser Ala Val Cys Ala Phe Pro Ile Lys Tyr
355 360 365

Val Asn Asp Phe Phe Asn Lys Ile Val Asn Lys Asn Asn Val Arg Cys
370 375 380

Leu Gln His Phe Tyr Gly Pro Asn His Glu His Cys Phe Asn Arg Thr
385 390 395 400

Leu Leu Arg Asn Ser Ser Gly Cys Glu Ala Arg Ser Asp Glu Tyr Arg
405 410 415

Thr Glu Phe Thr Thr Ala Leu Gln Arg Val Asp Leu Phe Met Gly Arg
420 425 430

Leu Asn Gln Val Leu Leu Thr Ser Ile Ser Thr Phe Ile Lys Gly Asp
435 440 445

Leu Thr Ile Ala Asn Leu Gly Thr Ser Glu Gly Arg Phe Met Gln Val
450 455 460

Val Leu Ser Arg Thr Ala His Leu Thr Pro His Val Asn Phe Leu Leu
465 470 475 480

Asp Ser His Pro Val Ser Pro Glu Val Ile Val Glu His Pro Ser Asn
485 490 495

Gln Asn Gly Tyr Thr Leu Val Val Thr Gly Lys Lys Ile Thr Lys Ile
500 505 510

Pro Leu Asn Gly Leu Gly Cys Gly His Phe Gln Ser Cys Ser Gln Cys
515 520 525

Leu Ser Ala Pro Tyr Phe Ile Gln Cys Gly Trp Cys His Asn Gln Cys
530 535 540

Val Arg Phe Asp Glu Cys Pro Ser Gly Thr Trp Thr Gln Glu Ile Cys
545 550 555 560

Leu Pro Ala Val Tyr Lys Val Phe Pro Thr Ser Ala Pro Leu Glu Gly
565 570 575

Gly Thr Val Leu Thr Ile Cys Gly Trp Asp Phe Gly Phe Arg Lys Asn
580 585 590

Asn Lys Phe Asp Leu Arg Lys Thr Lys Val Leu Leu Gly Asn Glu Ser
595 600 605

Cys Thr Leu Thr Leu Ser Glu Ser Thr Thr Asn Thr Leu Lys Cys Thr
610 615 620

Val Gly Pro Ala Met Ser Glu His Phe Asn Val Ser Val Ile Ile Ser
625 630 635 640

Asn Ser Arg Glu Thr Thr Gln Tyr Ser Ala Phe Ser Tyr Val Asp Pro
645 650 655

Val Ile Thr Ser Ile Ser Pro Arg Tyr Gly Pro Gln Ala Gly Gly Thr
660 665 670

Leu Leu Thr Leu Thr Gly Lys Tyr Leu Asn Ser Gly Asn Ser Arg His
675 680 685

Ile Ser Ile Gly Gly Lys Thr Cys Thr Leu Lys Ser Val Ser Asp Ser
690 695 700

Ile Leu Glu Cys Tyr Thr Pro Ala Gln Thr Thr Ser Asp Glu Phe Pro
705                 710                 715                 720

Val Lys Leu Lys Ile Asp Leu Ala Asn Arg Glu Thr Ser Ser Phe Ser
                725                 730                 735

Tyr Arg Glu Asp Pro Val Val Tyr Glu Ile His Pro Thr Lys Ser Phe
            740                 745                 750

Ile Ser Gly Gly Ser Thr Ile Thr Gly Ile Gly Lys Thr Leu Asn Ser
        755                 760                 765

Val Ser Leu Pro Lys Leu Val Ile Asp Val His Glu Val Gly Val Asn
    770                 775                 780

Tyr Thr Val Ala Cys Gln His Arg Ser Asn Ser Glu Ile Ile Cys Cys
785                 790                 795                 800

Thr Thr Pro Ser Leu Lys Gln Leu Gly Leu Gln Leu Pro Leu Lys Thr
                805                 810                 815

Lys Ala Phe Phe Leu Leu Asp Gly Ile Leu Ser Lys His Phe Asp Leu
            820                 825                 830

Thr Tyr Val His Asn Pro Val Phe Glu Pro Phe Glu Lys Pro Val Met
        835                 840                 845

Ile Ser Ile Gly Asn Glu Asn Val Val Glu Ile Lys Gly Asn Asn Ile
    850                 855                 860

Asp Pro Glu Ala Val Lys Gly Glu Val Leu Lys Val Gly Asn Gln Ser
865                 870                 875                 880

Cys Glu Ser Leu His Trp His Ser Gly Ala Val Leu Cys Thr Val Pro
                885                 890                 895

Ser Asp Leu Leu Lys Leu Asn Ser Glu Leu Asn Ile Glu Trp Lys Gln

900                 905                 910

Ala Val Ser Ser Thr Val Leu Gly Lys Val Ile Val Gln Pro Asp Gln
        915                 920                 925

Asn Phe Ala Gly Leu Ile Ile Gly Ala Val Ser Ile Ser Val Val Val
    930                 935                 940

Leu Leu Leu Ser Gly Leu Phe Leu Trp Met Arg Lys Arg Lys His Lys
945                 950                 955                 960

Asp Leu Gly Ser Glu Leu Val Arg Tyr Asp Ala Arg Val His Thr Pro
                965                 970                 975

His Leu Asp Arg Leu Val Ser Ala Arg Ser Val Ser Pro Thr Thr Glu
            980                 985                 990

Met Val Ser Asn Glu Ser Val Asp  Tyr Arg Ala Thr Phe  Pro Glu Asp
        995                 1000                1005

Gln Phe  Pro Asn Ser Ser Gln  Asn Gly Ala Cys Arg  Gln Val Gln
    1010                1015                1020

Tyr Pro  Leu Thr Asp Leu Ser  Pro Ile Leu Thr Ser  Gly Asp Ser
    1025                1030                1035

Asp Ile  Ser Ser Pro Leu Leu  Gln Asn Thr Val His  Ile Asp Leu
    1040                1045                1050

Ser Ala  Leu Asn Pro Glu Leu  Val Gln Ala Val Gln  His Val Val
    1055                1060                1065

Ile Gly  Pro Ser Ser Leu Ile  Val His Phe Asn Glu  Val Ile Gly
    1070                1075                1080

Arg Gly  His Phe Gly Cys Val  Tyr His Gly Thr Leu  Leu Asp Asn
    1085                1090                1095

Asp Gly Lys Lys Ile His Cys Ala Val Lys Ser Leu Asn Arg Ile
1100 1105 1110

Thr Asp Ile Glu Glu Val Ser Gln Phe Leu Thr Glu Gly Ile Ile
1115 1120 1125

Met Lys Asp Phe Ser His Pro Asn Val Leu Ser Leu Leu Gly Ile
1130 1135 1140

Cys Leu Arg Ser Glu Gly Ser Pro Leu Val Val Leu Pro Tyr Met
1145 1150 1155

Lys His Gly Asp Leu Arg Asn Phe Ile Arg Asn Glu Thr His Asn
1160 1165 1170

Pro Thr Val Lys Asp Leu Ile Gly Phe Gly Leu Gln Val Ala Lys
1175 1180 1185

Gly Met Lys Tyr Leu Ala Ser Lys Lys Phe Val His Arg Asp Leu
1190 1195 1200

Ala Ala Arg Asn Cys Met Leu Asp Glu Lys Phe Thr Val Lys Val
1205 1210 1215

Ala Asp Phe Gly Leu Ala Arg Asp Met Tyr Asp Lys Glu Tyr Tyr
1220 1225 1230

Ser Val His Asn Lys Thr Gly Ala Lys Leu Pro Val Lys Trp Met
1235 1240 1245

Ala Leu Glu Ser Leu Gln Thr Gln Lys Phe Thr Thr Lys Ser Asp
1250 1255 1260

Val Trp Ser Phe Gly Val Leu Leu Trp Glu Leu Met Thr Arg Gly
1265 1270 1275

Ala Pro Pro Tyr Pro Asp Val Asn Thr Phe Asp Ile Thr Ile Tyr
1280 1285 1290

Leu Leu Gln Gly Arg Arg Leu Leu Gln Pro Glu Tyr Cys Pro Asp
1295 1300 1305

Ala Leu Tyr Glu Val Met Leu Lys Cys Trp His Pro Lys Ala Glu
1310 1315 1320

Met Arg Pro Ser Phe Ser Glu Leu Val Ser Arg Ile Ser Ser Ile
1325 1330 1335

Phe Ser Thr Phe Ile Gly Glu His Tyr Val His Val Asn Ala Thr
1340 1345 1350

Tyr Val Asn Val Lys Cys Val Ala Pro Tyr Pro Ser Leu Leu Pro
1355 1360 1365

Ser Gln Asp Asn Ile Asp Gly Glu Gly Asn Thr
1370 1375

<210> 21
<211> 4146
<212> DNA
<213> Equus caballus

<220>
<221> CDS
<222> (1)..(4143)

<400> 21
atg aag gca cct gct gtg ctt gca cct ggc atc ctt gtg ctt ctg ttt 48
Met Lys Ala Pro Ala Val Leu Ala Pro Gly Ile Leu Val Leu Leu Phe
1               5                   10                  15

acc ttg gtg cag aag agc gat ggg gag tgt aaa gag gcg cta gta aag 96
Thr Leu Val Gln Lys Ser Asp Gly Glu Cys Lys Glu Ala Leu Val Lys
            20                  25                  30

tcc gag atg aat gtg aac atg aag tat cag ctt ccc aac ttc act gca 144

Ser Glu Met Asn Val Asn Met Lys Tyr Gln Leu Pro Asn Phe Thr Ala
        35                  40                  45

gaa aca ccc atc cag aac gtc gtt tta cac aag cat cac att tac ctc 192
Glu Thr Pro Ile Gln Asn Val Val Leu His Lys His His Ile Tyr Leu
    50                  55                  60

ggg gcc act aac tac att tat gtt tta aat gac aaa gat ctt cag aaa 240
Gly Ala Thr Asn Tyr Ile Tyr Val Leu Asn Asp Lys Asp Leu Gln Lys
65                  70                  75                  80

gtt gct gag tac aag act ggg ccc gtg ctg gaa cac cca gat tgt ttc 288
Val Ala Glu Tyr Lys Thr Gly Pro Val Leu Glu His Pro Asp Cys Phe
                85                  90                  95

cca tgt cag gac tgc agc cgc aaa gcc aat tta tca ggt ggc gct tgg 336
Pro Cys Gln Asp Cys Ser Arg Lys Ala Asn Leu Ser Gly Gly Ala Trp
            100                 105                 110

aaa gat aac atc aac atg gct ctg ctt gtt gac aca tac tat gac gat 384
Lys Asp Asn Ile Asn Met Ala Leu Leu Val Asp Thr Tyr Tyr Asp Asp
        115                 120                 125

caa ctc att agt tgc ggc agc gtc cac aga ggg acc tgc cag cgg cac 432
Gln Leu Ile Ser Cys Gly Ser Val His Arg Gly Thr Cys Gln Arg His
    130                 135                 140

gtc ctt cca ctc aac aat gtt gct gac ata cag tca gaa gtt tat tgc 480
Val Leu Pro Leu Asn Asn Val Ala Asp Ile Gln Ser Glu Val Tyr Cys
145                 150                 155                 160

atg tac tct cca caa gca gaa gag ccc cac cag tgt cct gac tgt gtg 528
Met Tyr Ser Pro Gln Ala Glu Glu Pro His Gln Cys Pro Asp Cys Val
                165                 170                 175

gtg agt gcc ctg gga acc aaa gtc ctg ctg tct gaa aag gac cga ttt 576
Val Ser Ala Leu Gly Thr Lys Val Leu Leu Ser Glu Lys Asp Arg Phe
            180                 185                 190

gtc acc ttc ttc gtg ggc aat acc ata aat tct tct tac ctt cca gat 624
Val Thr Phe Phe Val Gly Asn Thr Ile Asn Ser Ser Tyr Leu Pro Asp
        195                 200                 205

cat tca ttg cat tcg ata tcg gtg aga agg cta aag gaa acg caa gat 672
His Ser Leu His Ser Ile Ser Val Arg Arg Leu Lys Glu Thr Gln Asp
    210                 215                 220

ggt ttt aag ttt ttg aca gat cag tcc tat att gat gtt cta cct gag 720
Gly Phe Lys Phe Leu Thr Asp Gln Ser Tyr Ile Asp Val Leu Pro Glu

225                 230                 235                 240

ttc cga gat tct tac ccc att aag tat atc cac gcc ttt gaa agc aac       768
Phe Arg Asp Ser Tyr Pro Ile Lys Tyr Ile His Ala Phe Glu Ser Asn
                245                 250                 255

cat ttt att tac ttt tta acg gtc caa cgg gaa act cta gat gct cag       816
His Phe Ile Tyr Phe Leu Thr Val Gln Arg Glu Thr Leu Asp Ala Gln
            260                 265                 270

act ttt cac aca aga ata atc agg ttc tgt tcc gtg gac tct gga ttg       864
Thr Phe His Thr Arg Ile Ile Arg Phe Cys Ser Val Asp Ser Gly Leu
        275                 280                 285

cat tcc tac atg gaa atg cct ctg gag tgt att ctc aca gaa aag aga       912
His Ser Tyr Met Glu Met Pro Leu Glu Cys Ile Leu Thr Glu Lys Arg
    290                 295                 300

aga aag aga tcc aca agt gag gaa gtg ttt aat atc ctc caa gct gcg       960
Arg Lys Arg Ser Thr Ser Glu Glu Val Phe Asn Ile Leu Gln Ala Ala
305                 310                 315                 320

tat gtc agt aag cct ggg gct cat ctt gct aag caa ata ggt gcc aac      1008
Tyr Val Ser Lys Pro Gly Ala His Leu Ala Lys Gln Ile Gly Ala Asn
                325                 330                 335

ctg aat gat gac att ctc tat ggg gtg ttt gca cag agc aag cca gat      1056
Leu Asn Asp Asp Ile Leu Tyr Gly Val Phe Ala Gln Ser Lys Pro Asp
            340                 345                 350

tct gct gaa cca atg aat cgc tct gcc gtc tgt gca ttc cct gtc aaa      1104
Ser Ala Glu Pro Met Asn Arg Ser Ala Val Cys Ala Phe Pro Val Lys
        355                 360                 365

tat gtc aat gaa ttc ttc aac aag att gtc aac aaa aac aac gtg aga      1152
Tyr Val Asn Glu Phe Phe Asn Lys Ile Val Asn Lys Asn Asn Val Arg
    370                 375                 380

tgt ctc cag cat ttt tat gga ccc cac cat gaa cac tgc ttc aat agg      1200
Cys Leu Gln His Phe Tyr Gly Pro His His Glu His Cys Phe Asn Arg
385                 390                 395                 400

aca ctt ttg aga aat tca tca ggc tgt gaa gtg cgc aat gat gaa tat      1248
Thr Leu Leu Arg Asn Ser Ser Gly Cys Glu Val Arg Asn Asp Glu Tyr
                405                 410                 415

cga acg gag ttt acc aca gct ttg cag cgc gtt gac ttg ttc atg ggc      1296
Arg Thr Glu Phe Thr Thr Ala Leu Gln Arg Val Asp Leu Phe Met Gly
            420                 425                 430

caa ttc aac caa gtc ctc ttg aca tct ata tcc acc ttc att aaa gga 1344
Gln Phe Asn Gln Val Leu Leu Thr Ser Ile Ser Thr Phe Ile Lys Gly
        435                 440                 445

aac ctc act ata gct aat ctt ggg aca tca gaa ggt cgc ttc atg cag 1392
Asn Leu Thr Ile Ala Asn Leu Gly Thr Ser Glu Gly Arg Phe Met Gln
    450                 455                 460

gtt gtg gtt tct cga tca gga tcg tca acc cct cat gtg aat ttt cac 1440
Val Val Val Ser Arg Ser Gly Ser Ser Thr Pro His Val Asn Phe His
465                 470                 475                 480

ctg gat tcc cac cct gtg tct cca gaa gtg att gtg gaa cac cca tta 1488
Leu Asp Ser His Pro Val Ser Pro Glu Val Ile Val Glu His Pro Leu
                485                 490                 495

aac caa aat ggt tac aca ctg gtt gtc acc ggg aaa aag atc acc aag 1536
Asn Gln Asn Gly Tyr Thr Leu Val Val Thr Gly Lys Lys Ile Thr Lys
            500                 505                 510

atc cca ctg aat ggc ttg ggc tgt gaa cat ttc cag tcc tgc agt cag 1584
Ile Pro Leu Asn Gly Leu Gly Cys Glu His Phe Gln Ser Cys Ser Gln
        515                 520                 525

tgt ctc tcc gcc cct ccc ttt gtg cag tgt ggc tgg tgc cac gac aaa 1632
Cys Leu Ser Ala Pro Pro Phe Val Gln Cys Gly Trp Cys His Asp Lys
    530                 535                 540

tgt gtg cga ttg gag gaa tgc cac aac gga aca tgg act caa gag atc 1680
Cys Val Arg Leu Glu Glu Cys His Asn Gly Thr Trp Thr Gln Glu Ile
545                 550                 555                 560

tgt ctg cct aca atc tac aag gtt ttc cca acc agt gcc ccc ctc gaa 1728
Cys Leu Pro Thr Ile Tyr Lys Val Phe Pro Thr Ser Ala Pro Leu Glu
                565                 570                 575

gga ggg aca acg ctg aca gta tgt ggc tgg gac ttt gga ttc agg aag 1776
Gly Gly Thr Thr Leu Thr Val Cys Gly Trp Asp Phe Gly Phe Arg Lys
            580                 585                 590

aat aat aaa ctt gat tca aag aaa acc aaa gtt ctc ctt gga aat gag 1824
Asn Asn Lys Leu Asp Ser Lys Lys Thr Lys Val Leu Leu Gly Asn Glu
        595                 600                 605

agc tgc acc ttg act tta agt gag agc acg tca aat acg ttg aaa tgc 1872
Ser Cys Thr Leu Thr Leu Ser Glu Ser Thr Ser Asn Thr Leu Lys Cys
    610                 615                 620

aca gtt ggt cct gca atg aat gag cgt ttc aat ata tcc ata act gtt 1920
Thr Val Gly Pro Ala Met Asn Glu Arg Phe Asn Ile Ser Ile Thr Val
625                 630                 635                 640

tcc aac agt cga ggg aca gca cga tat agt aca ttt tcc tat gtg gat 1968
Ser Asn Ser Arg Gly Thr Ala Arg Tyr Ser Thr Phe Ser Tyr Val Asp
                645                 650                 655

cct ata ata aca agt att tct cca agt tat ggt cct aag act ggt ggc 2016
Pro Ile Ile Thr Ser Ile Ser Pro Ser Tyr Gly Pro Lys Thr Gly Gly
            660                 665                 670

act tta ctt act tta act gga aag tac tta aac agt ggg aat tct aga 2064
Thr Leu Leu Thr Leu Thr Gly Lys Tyr Leu Asn Ser Gly Asn Ser Arg
        675                 680                 685

cac att tca att ggt gga aaa aca tgt acc tta aaa agt gtg tca gat 2112
His Ile Ser Ile Gly Gly Lys Thr Cys Thr Leu Lys Ser Val Ser Asp
    690                 695                 700

agt att ctt gaa tgt tat act cca gcc caa acc act cca act gag ttt 2160
Ser Ile Leu Glu Cys Tyr Thr Pro Ala Gln Thr Thr Pro Thr Glu Phe
705                 710                 715                 720

ccc gtt aaa ctg aaa att gac tta gcc aac cga gag atg aac agc ttc 2208
Pro Val Lys Leu Lys Ile Asp Leu Ala Asn Arg Glu Met Asn Ser Phe
                725                 730                 735

agt tac cgg gaa gac ccc att gtc tat gaa att cat cca acc aaa tct 2256
Ser Tyr Arg Glu Asp Pro Ile Val Tyr Glu Ile His Pro Thr Lys Ser
            740                 745                 750

ttt att agt ggt ggg agc aca att aca ggt gtt gga aaa aat ctg aat 2304
Phe Ile Ser Gly Gly Ser Thr Ile Thr Gly Val Gly Lys Asn Leu Asn
        755                 760                 765

tcg gtt agt gtc ctg agg atg gtg ata aat gtg cgt gaa gca gga agg 2352
Ser Val Ser Val Leu Arg Met Val Ile Asn Val Arg Glu Ala Gly Arg
    770                 775                 780

aac ttt aca gtg gca tgt cag cat cgc tct aat tca gag ata atc tgc 2400
Asn Phe Thr Val Ala Cys Gln His Arg Ser Asn Ser Glu Ile Ile Cys
785                 790                 795                 800

tgt aca act cct tca cta caa cag ctg aat ttg caa ctc cct ctg aaa 2448
Cys Thr Thr Pro Ser Leu Gln Gln Leu Asn Leu Gln Leu Pro Leu Lys
                805                 810                 815

acc aaa gcc ttc ttc atg ttg gac ggg atc cat tcc aaa tac ttt gat 2496

Thr Lys Ala Phe Phe Met Leu Asp Gly Ile His Ser Lys Tyr Phe Asp
            820                 825                 830

ctc att tat gta cat aat cct gtg ttt aag cct ttt gaa aag cca gtg 2544
Leu Ile Tyr Val His Asn Pro Val Phe Lys Pro Phe Glu Lys Pro Val
        835                 840                 845

atg atc tca ata ggc aat gaa aat gta ctg gaa att aag gga aat gat 2592
Met Ile Ser Ile Gly Asn Glu Asn Val Leu Glu Ile Lys Gly Asn Asp
    850                 855                 860

att gac cct gaa gcc gtt aaa ggt gaa gtg tta aaa gtt gga aat aag 2640
Ile Asp Pro Glu Ala Val Lys Gly Glu Val Leu Lys Val Gly Asn Lys
865                 870                 875                 880

agc tgt gag aat ata cac tca cat tct gaa gcc gtt tta tgt aca gtc 2688
Ser Cys Glu Asn Ile His Ser His Ser Glu Ala Val Leu Cys Thr Val
                885                 890                 895

ccc agt gac ctg ctg aaa ttg aac agc gag cta aat ata gag tgg aag 2736
Pro Ser Asp Leu Leu Lys Leu Asn Ser Glu Leu Asn Ile Glu Trp Lys
            900                 905                 910

caa gca gtt tct tca acc atc ctt gga aaa gta ata gtt caa cca gat 2784
Gln Ala Val Ser Ser Thr Ile Leu Gly Lys Val Ile Val Gln Pro Asp
        915                 920                 925

cag aat ttc aca gga ttg att gtt ggt gtt gtc tca ata tca ata ata 2832
Gln Asn Phe Thr Gly Leu Ile Val Gly Val Val Ser Ile Ser Ile Ile
    930                 935                 940

ctc tta tta tta ctc ggg ctt ttc ctg tgg ctg aaa agg aga aag caa 2880
Leu Leu Leu Leu Leu Gly Leu Phe Leu Trp Leu Lys Arg Arg Lys Gln
945                 950                 955                 960

att aaa gat ctg ggc agt gaa tta gtt cgc tat gat gca aga gta cac 2928
Ile Lys Asp Leu Gly Ser Glu Leu Val Arg Tyr Asp Ala Arg Val His
                965                 970                 975

act cct cat ttg gat agg ctt gta agt gcc cga agt gta agc cca act 2976
Thr Pro His Leu Asp Arg Leu Val Ser Ala Arg Ser Val Ser Pro Thr
            980                 985                 990

aca gaa atg gtt tca aat gaa tct gta gac tac cga gct act ttt cca 3024
Thr Glu Met Val Ser Asn Glu Ser Val Asp Tyr Arg Ala Thr Phe Pro
        995                 1000                1005

gaa gac cag ttt cct aac tca tct cag aat gga tca tgc aga caa 3069
Glu Asp Gln Phe Pro Asn Ser Ser Gln Asn Gly Ser Cys Arg Gln

1010                1015                1020

gtg cag tat cct ctg acg gac cta tcc ccc atc cta act agt ggg        3114
Val Gln Tyr Pro Leu Thr Asp Leu Ser Pro Ile Leu Thr Ser Gly
    1025                1030                1035

gac tct gat ata tcc agt cca tta ctg caa aat acc gtc cac att        3159
Asp Ser Asp Ile Ser Ser Pro Leu Leu Gln Asn Thr Val His Ile
    1040                1045                1050

gac ctc agt gct cta aat cca gag ctg gtg cag gca gtc cag cac        3204
Asp Leu Ser Ala Leu Asn Pro Glu Leu Val Gln Ala Val Gln His
    1055                1060                1065

gta gtg att ggg cct agt agt ctg att gtg cat ttc aat gaa gtc        3249
Val Val Ile Gly Pro Ser Ser Leu Ile Val His Phe Asn Glu Val
    1070                1075                1080

ata gga aga gga cat ttt ggg tgt gta tat cat ggg act ttg ttg        3294
Ile Gly Arg Gly His Phe Gly Cys Val Tyr His Gly Thr Leu Leu
    1085                1090                1095

gac aat gat gac aag aaa att cac tgt gct gtg aaa tcc ttg aat        3339
Asp Asn Asp Asp Lys Lys Ile His Cys Ala Val Lys Ser Leu Asn
    1100                1105                1110

aga atc act gac ata gga gaa gtt tcc cag ttt ctc acc gag gga        3384
Arg Ile Thr Asp Ile Gly Glu Val Ser Gln Phe Leu Thr Glu Gly
    1115                1120                1125

att atc atg aaa gat ttt agt cat ccc aac gtt ctc tcg ctc ttg        3429
Ile Ile Met Lys Asp Phe Ser His Pro Asn Val Leu Ser Leu Leu
    1130                1135                1140

gga atc tgc ctt cga agt gag gga tct cca ctg gtg gtt cta ccg        3474
Gly Ile Cys Leu Arg Ser Glu Gly Ser Pro Leu Val Val Leu Pro
    1145                1150                1155

tac atg aaa cat gga gac ctt cga aat ttc att aga aat gag acc        3519
Tyr Met Lys His Gly Asp Leu Arg Asn Phe Ile Arg Asn Glu Thr
    1160                1165                1170

cac aac cca act gta aaa gat ctt att ggc ttt ggt ctt caa gta        3564
His Asn Pro Thr Val Lys Asp Leu Ile Gly Phe Gly Leu Gln Val
    1175                1180                1185

gcc aaa ggc atg aaa tat ctt gca agc aaa aag ttt gtc cac aga        3609
Ala Lys Gly Met Lys Tyr Leu Ala Ser Lys Lys Phe Val His Arg
    1190                1195                1200

gac ttg gct gca aga aac tgt atg ctg gat gaa aaa ttc act gtc 3654
Asp Leu Ala Ala Arg Asn Cys Met Leu Asp Glu Lys Phe Thr Val
    1205                1210                1215

aag gtt gct gat ttt ggg ctt gcc aga gac atg tat gat aaa gaa 3699
Lys Val Ala Asp Phe Gly Leu Ala Arg Asp Met Tyr Asp Lys Glu
    1220                1225                1230

tac tat agt gtg cac aac aaa aca ggt gca aaa ctg cct gtg aag 3744
Tyr Tyr Ser Val His Asn Lys Thr Gly Ala Lys Leu Pro Val Lys
    1235                1240                1245

tgg atg gct tta gag agt ctg caa act cag aag ttt acc acc aag 3789
Trp Met Ala Leu Glu Ser Leu Gln Thr Gln Lys Phe Thr Thr Lys
    1250                1255                1260

tca gat gtg tgg tcc ttt ggc gtg ctc ctc tgg gag ctg atg aca 3834
Ser Asp Val Trp Ser Phe Gly Val Leu Leu Trp Glu Leu Met Thr
    1265                1270                1275

aga gga gca cca cct tat ccc gat gtc aac acc ttt gat ata aca 3879
Arg Gly Ala Pro Pro Tyr Pro Asp Val Asn Thr Phe Asp Ile Thr
    1280                1285                1290

gtt tac ttg ttg caa ggc aga agg ctc tta caa cca gag tac tgc 3924
Val Tyr Leu Leu Gln Gly Arg Arg Leu Leu Gln Pro Glu Tyr Cys
    1295                1300                1305

cca gat ccc ttg tat gaa gtg atg cta aaa tgc tgg cac cct aaa 3969
Pro Asp Pro Leu Tyr Glu Val Met Leu Lys Cys Trp His Pro Lys
    1310                1315                1320

gct gaa ctg cgc cca tcc ttt tct gaa ctg gtc tcc agg ata tca 4014
Ala Glu Leu Arg Pro Ser Phe Ser Glu Leu Val Ser Arg Ile Ser
    1325                1330                1335

gcg ata ttc tct act ttc atc ggg gag cac tac gtc cat gtg aac 4059
Ala Ile Phe Ser Thr Phe Ile Gly Glu His Tyr Val His Val Asn
    1340                1345                1350

gct act tat gtg aat gtg aaa tgt gtt gct cca tat cct tct ctg 4104
Ala Thr Tyr Val Asn Val Lys Cys Val Ala Pro Tyr Pro Ser Leu
    1355                1360                1365

ttg tca tca caa gat aac gtt gat ggc gag gta gat aca tga 4146
Leu Ser Ser Gln Asp Asn Val Asp Gly Glu Val Asp Thr
    1370                1375                1380

<210> 22
<211> 1381
<212> PRT
<213> Equus caballus

<400> 22

Met Lys Ala Pro Ala Val Leu Ala Pro Gly Ile Leu Val Leu Leu Phe
1               5                   10                  15

Thr Leu Val Gln Lys Ser Asp Gly Glu Cys Lys Glu Ala Leu Val Lys
            20                  25                  30

Ser Glu Met Asn Val Asn Met Lys Tyr Gln Leu Pro Asn Phe Thr Ala
        35                  40                  45

Glu Thr Pro Ile Gln Asn Val Val Leu His Lys His His Ile Tyr Leu
    50                  55                  60

Gly Ala Thr Asn Tyr Ile Tyr Val Leu Asn Asp Lys Asp Leu Gln Lys
65                  70                  75                  80

Val Ala Glu Tyr Lys Thr Gly Pro Val Leu Glu His Pro Asp Cys Phe
                85                  90                  95

Pro Cys Gln Asp Cys Ser Arg Lys Ala Asn Leu Ser Gly Gly Ala Trp
            100                 105                 110

Lys Asp Asn Ile Asn Met Ala Leu Leu Val Asp Thr Tyr Tyr Asp Asp
        115                 120                 125

Gln Leu Ile Ser Cys Gly Ser Val His Arg Gly Thr Cys Gln Arg His
    130                 135                 140

Val Leu Pro Leu Asn Asn Val Ala Asp Ile Gln Ser Glu Val Tyr Cys
145                 150                 155                 160

Met Tyr Ser Pro Gln Ala Glu Glu Pro His Gln Cys Pro Asp Cys Val

                165                 170                 175

Val Ser Ala Leu Gly Thr Lys Val Leu Leu Ser Glu Lys Asp Arg Phe
            180                 185                 190

Val Thr Phe Phe Val Gly Asn Thr Ile Asn Ser Ser Tyr Leu Pro Asp
        195                 200                 205

His Ser Leu His Ser Ile Ser Val Arg Arg Leu Lys Glu Thr Gln Asp
    210                 215                 220

Gly Phe Lys Phe Leu Thr Asp Gln Ser Tyr Ile Asp Val Leu Pro Glu
225                 230                 235                 240

Phe Arg Asp Ser Tyr Pro Ile Lys Tyr Ile His Ala Phe Glu Ser Asn
                245                 250                 255

His Phe Ile Tyr Phe Leu Thr Val Gln Arg Glu Thr Leu Asp Ala Gln
            260                 265                 270

Thr Phe His Thr Arg Ile Ile Arg Phe Cys Ser Val Asp Ser Gly Leu
        275                 280                 285

His Ser Tyr Met Glu Met Pro Leu Glu Cys Ile Leu Thr Glu Lys Arg
    290                 295                 300

Arg Lys Arg Ser Thr Ser Glu Glu Val Phe Asn Ile Leu Gln Ala Ala
305                 310                 315                 320

Tyr Val Ser Lys Pro Gly Ala His Leu Ala Lys Gln Ile Gly Ala Asn
                325                 330                 335

Leu Asn Asp Asp Ile Leu Tyr Gly Val Phe Ala Gln Ser Lys Pro Asp
            340                 345                 350

Ser Ala Glu Pro Met Asn Arg Ser Ala Val Cys Ala Phe Pro Val Lys
        355                 360                 365

Tyr Val Asn Glu Phe Phe Asn Lys Ile Val Asn Lys Asn Asn Val Arg
    370                 375                 380

Cys Leu Gln His Phe Tyr Gly Pro His His Glu His Cys Phe Asn Arg
385                 390                 395                 400

Thr Leu Leu Arg Asn Ser Ser Gly Cys Glu Val Arg Asn Asp Glu Tyr
                405                 410                 415

Arg Thr Glu Phe Thr Thr Ala Leu Gln Arg Val Asp Leu Phe Met Gly
            420                 425                 430

Gln Phe Asn Gln Val Leu Leu Thr Ser Ile Ser Thr Phe Ile Lys Gly
        435                 440                 445

Asn Leu Thr Ile Ala Asn Leu Gly Thr Ser Glu Gly Arg Phe Met Gln
    450                 455                 460

Val Val Val Ser Arg Ser Gly Ser Ser Thr Pro His Val Asn Phe His
465                 470                 475                 480

Leu Asp Ser His Pro Val Ser Pro Glu Val Ile Val Glu His Pro Leu
                485                 490                 495

Asn Gln Asn Gly Tyr Thr Leu Val Val Thr Gly Lys Lys Ile Thr Lys
            500                 505                 510

Ile Pro Leu Asn Gly Leu Gly Cys Glu His Phe Gln Ser Cys Ser Gln
        515                 520                 525

Cys Leu Ser Ala Pro Pro Phe Val Gln Cys Gly Trp Cys His Asp Lys
    530                 535                 540

Cys Val Arg Leu Glu Glu Cys His Asn Gly Thr Trp Thr Gln Glu Ile
545                 550                 555                 560

Cys Leu Pro Thr Ile Tyr Lys Val Phe Pro Thr Ser Ala Pro Leu Glu
                565                 570                 575

Gly Gly Thr Thr Leu Thr Val Cys Gly Trp Asp Phe Gly Phe Arg Lys
            580                 585                 590

Asn Asn Lys Leu Asp Ser Lys Lys Thr Lys Val Leu Leu Gly Asn Glu
        595                 600                 605

Ser Cys Thr Leu Thr Leu Ser Glu Ser Thr Ser Asn Thr Leu Lys Cys
    610                 615                 620

Thr Val Gly Pro Ala Met Asn Glu Arg Phe Asn Ile Ser Ile Thr Val
625                 630                 635                 640

Ser Asn Ser Arg Gly Thr Ala Arg Tyr Ser Thr Phe Ser Tyr Val Asp
                645                 650                 655

Pro Ile Ile Thr Ser Ile Ser Pro Ser Tyr Gly Pro Lys Thr Gly Gly
            660                 665                 670

Thr Leu Leu Thr Leu Thr Gly Lys Tyr Leu Asn Ser Gly Asn Ser Arg
        675                 680                 685

His Ile Ser Ile Gly Gly Lys Thr Cys Thr Leu Lys Ser Val Ser Asp
    690                 695                 700

Ser Ile Leu Glu Cys Tyr Thr Pro Ala Gln Thr Thr Pro Thr Glu Phe
705                 710                 715                 720

Pro Val Lys Leu Lys Ile Asp Leu Ala Asn Arg Glu Met Asn Ser Phe
                725                 730                 735

Ser Tyr Arg Glu Asp Pro Ile Val Tyr Glu Ile His Pro Thr Lys Ser
            740                 745                 750

Phe Ile Ser Gly Gly Ser Thr Ile Thr Gly Val Gly Lys Asn Leu Asn
        755                 760                 765

Ser Val Ser Val Leu Arg Met Val Ile Asn Val Arg Glu Ala Gly Arg
    770                 775                 780

Asn Phe Thr Val Ala Cys Gln His Arg Ser Asn Ser Glu Ile Ile Cys
785                 790                 795                 800

Cys Thr Thr Pro Ser Leu Gln Gln Leu Asn Leu Gln Leu Pro Leu Lys
                805                 810                 815

Thr Lys Ala Phe Phe Met Leu Asp Gly Ile His Ser Lys Tyr Phe Asp
            820                 825                 830

Leu Ile Tyr Val His Asn Pro Val Phe Lys Pro Phe Glu Lys Pro Val
        835                 840                 845

Met Ile Ser Ile Gly Asn Glu Asn Val Leu Glu Ile Lys Gly Asn Asp
    850                 855                 860

Ile Asp Pro Glu Ala Val Lys Gly Glu Val Leu Lys Val Gly Asn Lys
865                 870                 875                 880

Ser Cys Glu Asn Ile His Ser His Ser Glu Ala Val Leu Cys Thr Val
                885                 890                 895

Pro Ser Asp Leu Leu Lys Leu Asn Ser Glu Leu Asn Ile Glu Trp Lys
            900                 905                 910

Gln Ala Val Ser Ser Thr Ile Leu Gly Lys Val Ile Val Gln Pro Asp
        915                 920                 925

Gln Asn Phe Thr Gly Leu Ile Val Gly Val Val Ser Ile Ser Ile Ile
    930                 935                 940

Leu Leu Leu Leu Leu Gly Leu Phe Leu Trp Leu Lys Arg Arg Lys Gln

945                 950                 955                 960

Ile Lys Asp Leu Gly Ser Glu Leu Val Arg Tyr Asp Ala Arg Val His
                965                 970                 975

Thr Pro His Leu Asp Arg Leu Val Ser Ala Arg Ser Val Ser Pro Thr
            980                 985                 990

Thr Glu Met Val Ser Asn Glu Ser  Val Asp Tyr Arg Ala  Thr Phe Pro
        995                 1000                 1005

Glu Asp  Gln Phe Pro Asn Ser  Ser Gln Asn Gly Ser  Cys Arg Gln
    1010                 1015                 1020

Val Gln  Tyr Pro Leu Thr Asp  Leu Ser Pro Ile Leu  Thr Ser Gly
    1025                 1030                 1035

Asp Ser  Asp Ile Ser Ser Pro  Leu Leu Gln Asn Thr  Val His Ile
    1040                 1045                 1050

Asp Leu  Ser Ala Leu Asn Pro  Glu Leu Val Gln Ala  Val Gln His
    1055                 1060                 1065

Val Val  Ile Gly Pro Ser Ser  Leu Ile Val His Phe  Asn Glu Val
    1070                 1075                 1080

Ile Gly  Arg Gly His Phe Gly  Cys Val Tyr His Gly  Thr Leu Leu
    1085                 1090                 1095

Asp Asn  Asp Asp Lys Lys Ile  His Cys Ala Val Lys  Ser Leu Asn
    1100                 1105                 1110

Arg Ile  Thr Asp Ile Gly Glu  Val Ser Gln Phe Leu  Thr Glu Gly
    1115                 1120                 1125

Ile Ile  Met Lys Asp Phe Ser  His Pro Asn Val Leu  Ser Leu Leu
    1130                 1135                 1140

Gly Ile Cys Leu Arg Ser Glu Gly Ser Pro Leu Val Val Leu Pro
    1145                1150                1155

Tyr Met Lys His Gly Asp Leu Arg Asn Phe Ile Arg Asn Glu Thr
    1160                1165                1170

His Asn Pro Thr Val Lys Asp Leu Ile Gly Phe Gly Leu Gln Val
    1175                1180                1185

Ala Lys Gly Met Lys Tyr Leu Ala Ser Lys Lys Phe Val His Arg
    1190                1195                1200

Asp Leu Ala Ala Arg Asn Cys Met Leu Asp Glu Lys Phe Thr Val
    1205                1210                1215

Lys Val Ala Asp Phe Gly Leu Ala Arg Asp Met Tyr Asp Lys Glu
    1220                1225                1230

Tyr Tyr Ser Val His Asn Lys Thr Gly Ala Lys Leu Pro Val Lys
    1235                1240                1245

Trp Met Ala Leu Glu Ser Leu Gln Thr Gln Lys Phe Thr Thr Lys
    1250                1255                1260

Ser Asp Val Trp Ser Phe Gly Val Leu Leu Trp Glu Leu Met Thr
    1265                1270                1275

Arg Gly Ala Pro Pro Tyr Pro Asp Val Asn Thr Phe Asp Ile Thr
    1280                1285                1290

Val Tyr Leu Leu Gln Gly Arg Arg Leu Leu Gln Pro Glu Tyr Cys
    1295                1300                1305

Pro Asp Pro Leu Tyr Glu Val Met Leu Lys Cys Trp His Pro Lys
    1310                1315                1320

Ala Glu Leu Arg Pro Ser Phe Ser Glu Leu Val Ser Arg Ile Ser
    1325                1330                1335

Ala Ile Phe Ser Thr Phe Ile Gly Glu His Tyr Val His Val Asn
    1340                1345                1350

Ala Thr Tyr Val Asn Val Lys Cys Val Ala Pro Tyr Pro Ser Leu
    1355                1360                1365

Leu Ser Ser Gln Asp Asn Val Asp Gly Glu Val Asp Thr
    1370                1375                1380

<210> 23
<211> 4146
<212> DNA
<213> Sus scrofa

<220>
<221> CDS
<222> (1)..(4143)

<220>
<221> misc_feature
<222> (3564)..(3564)
<223> n is a, c, g, or t

<400> 23
atg aag gcc cct gct gtg ctt gca cct ggc atc ctt gtg ctt ctg ttt 48
Met Lys Ala Pro Ala Val Leu Ala Pro Gly Ile Leu Val Leu Leu Phe
1               5                   10                  15

acc ttg gtg cag aag agc aag gga gag tgt aaa gag gca ctg gta aag 96
Thr Leu Val Gln Lys Ser Lys Gly Glu Cys Lys Glu Ala Leu Val Lys
            20                  25                  30

tcc acg atg aat ttg aac atg caa tat cag ctt cct aac ttc act gcg 144
Ser Thr Met Asn Leu Asn Met Gln Tyr Gln Leu Pro Asn Phe Thr Ala
        35                  40                  45

gaa aca ccc att cag aat gtc gtt tta cac aag cat cac att tac ctg 192
Glu Thr Pro Ile Gln Asn Val Val Leu His Lys His His Ile Tyr Leu
    50                  55                  60

ggt gcc ctt aac tac att tat gtt tta aat gac ata gac ctt cgg aag 240
Gly Ala Leu Asn Tyr Ile Tyr Val Leu Asn Asp Ile Asp Leu Arg Lys
65                  70                  75                  80

gtt gct gag tac aaa act ggg ccc gtg ctg gaa cac cca gat tgt ttc 288
Val Ala Glu Tyr Lys Thr Gly Pro Val Leu Glu His Pro Asp Cys Phe
                85                  90                  95

cca tgt cag gac tgc agc cac aaa gcc aat tta tca ggt ggc att tgg 336
Pro Cys Gln Asp Cys Ser His Lys Ala Asn Leu Ser Gly Gly Ile Trp
            100                 105                 110

aga gat aac atc aac atg gct ctg ctt gtt gac acg tat tat gat gac 384
Arg Asp Asn Ile Asn Met Ala Leu Leu Val Asp Thr Tyr Tyr Asp Asp
        115                 120                 125

caa ctc att agc tgc ggc agt gtc cac aga ggg acc tgc cag cgg cat 432
Gln Leu Ile Ser Cys Gly Ser Val His Arg Gly Thr Cys Gln Arg His
    130                 135                 140

gtc ctt cca cct gat aat atc gct gac atc aag tca gag gtt cac tgc 480
Val Leu Pro Pro Asp Asn Ile Ala Asp Ile Lys Ser Glu Val His Cys
145                 150                 155                 160

atg tac tcc cca cag gta gat gaa gag ccc agc cag tgt cct gac tgt 528
Met Tyr Ser Pro Gln Val Asp Glu Glu Pro Ser Gln Cys Pro Asp Cys
                165                 170                 175

gtg gtg agt gcg ctg gga acc aaa gtc ctg ctg tct gaa aag gac cgg 576
Val Val Ser Ala Leu Gly Thr Lys Val Leu Leu Ser Glu Lys Asp Arg
            180                 185                 190

ttc atc aac ttc ttc gtg ggt aat acc ata aat tct tct tac ctt ccg 624
Phe Ile Asn Phe Phe Val Gly Asn Thr Ile Asn Ser Ser Tyr Leu Pro
        195                 200                 205

gat cat tca ttg cat tca ata tca gtg aga agg cta aag gaa acg caa 672
Asp His Ser Leu His Ser Ile Ser Val Arg Arg Leu Lys Glu Thr Gln
    210                 215                 220

gat ggt ttt aag ttt ttg aca gac cag tcc tat att gat gtt cta cct 720
Asp Gly Phe Lys Phe Leu Thr Asp Gln Ser Tyr Ile Asp Val Leu Pro
225                 230                 235                 240

gag ttc cga gat tcc tac ccc att aag tat gtc cat gcc ttt gaa agc 768
Glu Phe Arg Asp Ser Tyr Pro Ile Lys Tyr Val His Ala Phe Glu Ser
                245                 250                 255

aac cat ttt att tac ttt ttg aca gtc caa cgg gaa act ctc gac gct 816

Asn His Phe Ile Tyr Phe Leu Thr Val Gln Arg Glu Thr Leu Asp Ala
            260                 265                 270

cag act ttt cac aca aga ata atc agg ttc tgt tct gta gac tct gga 864
Gln Thr Phe His Thr Arg Ile Ile Arg Phe Cys Ser Val Asp Ser Gly
        275                 280                 285

ttg cat tcc tac atg gaa atg cct ctg gag tgt att ctc aca gaa aag 912
Leu His Ser Tyr Met Glu Met Pro Leu Glu Cys Ile Leu Thr Glu Lys
    290                 295                 300

aga aga aag aga tcc aca agg gag gaa gtg ttc aat att ctc caa gct 960
Arg Arg Lys Arg Ser Thr Arg Glu Glu Val Phe Asn Ile Leu Gln Ala
305                 310                 315                 320

gca tat gtc agt aag cct ggg act cag ctc gct aag caa ata ggt gcc 1008
Ala Tyr Val Ser Lys Pro Gly Thr Gln Leu Ala Lys Gln Ile Gly Ala
                325                 330                 335

aac ctg aat gat gac att ctc tat ggg gta ttt gca caa agc aag cca 1056
Asn Leu Asn Asp Asp Ile Leu Tyr Gly Val Phe Ala Gln Ser Lys Pro
            340                 345                 350

gat tct gcc gaa cca atg aat cgc tct gcc gtc tgt gca ttc cct gtg 1104
Asp Ser Ala Glu Pro Met Asn Arg Ser Ala Val Cys Ala Phe Pro Val
        355                 360                 365

aaa tat gtc aat gaa ttc ttc aac aag att gtc aac aaa aac aat gtg 1152
Lys Tyr Val Asn Glu Phe Phe Asn Lys Ile Val Asn Lys Asn Asn Val
    370                 375                 380

aga tgt ctc cag cat ttt tat ggc ccc aac cat gaa cac tgc ttc aac 1200
Arg Cys Leu Gln His Phe Tyr Gly Pro Asn His Glu His Cys Phe Asn
385                 390                 395                 400

agg aca ctt ttg aga aat tca tca ggc tgt gaa gtg cgc agt gat gaa 1248
Arg Thr Leu Leu Arg Asn Ser Ser Gly Cys Glu Val Arg Ser Asp Glu
                405                 410                 415

tat cga aca gag ttt acc aca gct ttg cag cga att gac tta ttc atg 1296
Tyr Arg Thr Glu Phe Thr Thr Ala Leu Gln Arg Ile Asp Leu Phe Met
            420                 425                 430

ggc cag ttc aac caa gtc ctc tta aca tcc ata tcc acc ttc att aaa 1344
Gly Gln Phe Asn Gln Val Leu Leu Thr Ser Ile Ser Thr Phe Ile Lys
        435                 440                 445

gga gac ctc acc atc gct aac ctc ggg acg tca gag ggt cga ttc atg 1392
Gly Asp Leu Thr Ile Ala Asn Leu Gly Thr Ser Glu Gly Arg Phe Met

450                 455                 460

cag gtt gtg gtt tct cga cca gga ttg tca acc cct cac gtg aat ttt      1440
Gln Val Val Val Ser Arg Pro Gly Leu Ser Thr Pro His Val Asn Phe
465                 470                 475                 480

cac ctg gat tcc cac cct gta tct cca gaa gtg atc gtg gag cca cta      1488
His Leu Asp Ser His Pro Val Ser Pro Glu Val Ile Val Glu Pro Leu
                485                 490                 495

aac caa aat ggc tat acc ctg gta gtc act ggg aag aag atc acc aag      1536
Asn Gln Asn Gly Tyr Thr Leu Val Val Thr Gly Lys Lys Ile Thr Lys
            500                 505                 510

atc ccg ctg aac ggc ttg ggc tgt gaa cat ttc cag tcc tgc agt cag      1584
Ile Pro Leu Asn Gly Leu Gly Cys Glu His Phe Gln Ser Cys Ser Gln
        515                 520                 525

tgc ctc tct gcc cct ccc ttt gta cag tgt ggc tgg tgc cag gat aaa      1632
Cys Leu Ser Ala Pro Pro Phe Val Gln Cys Gly Trp Cys Gln Asp Lys
    530                 535                 540

tgt gtg caa ttg gag gaa tgc ccc agt ggg aca tgg act caa gag atc      1680
Cys Val Gln Leu Glu Glu Cys Pro Ser Gly Thr Trp Thr Gln Glu Ile
545                 550                 555                 560

tgt ctg cct aca gtc tac aag gtt ttc cct act agt gcc ccc ctt gaa      1728
Cys Leu Pro Thr Val Tyr Lys Val Phe Pro Thr Ser Ala Pro Leu Glu
                565                 570                 575

gga ggg aca acg ctg acc ata tgt ggc tgg gac ttt gga ttc agg aga      1776
Gly Gly Thr Thr Leu Thr Ile Cys Gly Trp Asp Phe Gly Phe Arg Arg
            580                 585                 590

aat aat aaa ttt gac tta agg aaa acc aaa gtt ctc ctt ggg aat gag      1824
Asn Asn Lys Phe Asp Leu Arg Lys Thr Lys Val Leu Leu Gly Asn Glu
        595                 600                 605

agc tgt acc ttg acc tta agt gag agc acg aca aac acg ttg aaa tgc      1872
Ser Cys Thr Leu Thr Leu Ser Glu Ser Thr Thr Asn Thr Leu Lys Cys
    610                 615                 620

aca gtt ggt cct gca atg aat gaa cat ttc aat atg tcc ata gtt att      1920
Thr Val Gly Pro Ala Met Asn Glu His Phe Asn Met Ser Ile Val Ile
625                 630                 635                 640

tca aat ggt cga ggg aca aca caa tat agg acg ttt tcc tat gtg gat      1968
Ser Asn Gly Arg Gly Thr Thr Gln Tyr Arg Thr Phe Ser Tyr Val Asp
                645                 650                 655

cct gta ata aca aat att tct cca agt ttt ggt cct aaa act ggt ggt 2016
Pro Val Ile Thr Asn Ile Ser Pro Ser Phe Gly Pro Lys Thr Gly Gly
            660                 665                 670

act tta ctt aca tta act gga atg cac cta aac agt ggg aac tct aga 2064
Thr Leu Leu Thr Leu Thr Gly Met His Leu Asn Ser Gly Asn Ser Arg
        675                 680                 685

cac att tca att ggt gga aaa acg tgt act ttg aaa agt gtg tca gat 2112
His Ile Ser Ile Gly Gly Lys Thr Cys Thr Leu Lys Ser Val Ser Asp
    690                 695                 700

agt gtt ctt gaa tgt tat acc cca gcc caa acc act cca act gaa ttc 2160
Ser Val Leu Glu Cys Tyr Thr Pro Ala Gln Thr Thr Pro Thr Glu Phe
705                 710                 715                 720

cct att aag ttg aaa att gac tta gcc aac cga gag ata aac agc ttc 2208
Pro Ile Lys Leu Lys Ile Asp Leu Ala Asn Arg Glu Ile Asn Ser Phe
                725                 730                 735

act tac cgg gaa gac ccc att gtc tat gaa ata cat ccc acc aaa tct 2256
Thr Tyr Arg Glu Asp Pro Ile Val Tyr Glu Ile His Pro Thr Lys Ser
            740                 745                 750

ttt att agt gga ggg agc aca ata aca ggt gtt gga aga aac ctg aat 2304
Phe Ile Ser Gly Gly Ser Thr Ile Thr Gly Val Gly Arg Asn Leu Asn
        755                 760                 765

tca gtt agt gtc ctg agg atg gta ata aat gtg cat gaa gca gaa aga 2352
Ser Val Ser Val Leu Arg Met Val Ile Asn Val His Glu Ala Glu Arg
    770                 775                 780

aat ttt aca gtg gca tgc cag cat cac tct aat tca gag ata atc tgc 2400
Asn Phe Thr Val Ala Cys Gln His His Ser Asn Ser Glu Ile Ile Cys
785                 790                 795                 800

tgt aca act cct tca cta caa cag ctg aac cta caa ctc ccc ctg aaa 2448
Cys Thr Thr Pro Ser Leu Gln Gln Leu Asn Leu Gln Leu Pro Leu Lys
                805                 810                 815

acc aag gca ttt ttc atg tta gat ggg att cat tcc aaa tac ttt gat 2496
Thr Lys Ala Phe Phe Met Leu Asp Gly Ile His Ser Lys Tyr Phe Asp
            820                 825                 830

ctc att tat gta cat aac cct gtg ttt aag cct ttt gaa aag cca gtg 2544
Leu Ile Tyr Val His Asn Pro Val Phe Lys Pro Phe Glu Lys Pro Val
        835                 840                 845

atg atc tca ata ggc aat gaa aac gta ctg gaa att aag gga aat gat 2592
Met Ile Ser Ile Gly Asn Glu Asn Val Leu Glu Ile Lys Gly Asn Asp
    850                 855                 860

att gac cct gaa gca gtt aaa ggt gaa gtg tta aaa gtt gga aat aag 2640
Ile Asp Pro Glu Ala Val Lys Gly Glu Val Leu Lys Val Gly Asn Lys
865                 870                 875                 880

agt tgt gag aat ata cat tca cat tct gaa gcc gtt tta tgt aca gtc 2688
Ser Cys Glu Asn Ile His Ser His Ser Glu Ala Val Leu Cys Thr Val
                885                 890                 895

ccc aat gac ctg ctg aaa ttg aac agc gag cta aat ata gag tgg aag 2736
Pro Asn Asp Leu Leu Lys Leu Asn Ser Glu Leu Asn Ile Glu Trp Lys
            900                 905                 910

caa gca att tct tca act gtc ctt gga aaa gta ata gtt caa cca gat 2784
Gln Ala Ile Ser Ser Thr Val Leu Gly Lys Val Ile Val Gln Pro Asp
        915                 920                 925

cag aat ttc aca gga ttg att gtt ggt gtt gtc tca ata tca ata ata 2832
Gln Asn Phe Thr Gly Leu Ile Val Gly Val Val Ser Ile Ser Ile Ile
    930                 935                 940

ctc tta tta ctg ctc ggg ctt ttc ctg tgg ctg aaa aag aga aag caa 2880
Leu Leu Leu Leu Leu Gly Leu Phe Leu Trp Leu Lys Lys Arg Lys Gln
945                 950                 955                 960

att aaa gat cta ggc agt gaa cta gtt cgc tat gat gca aga gta cac 2928
Ile Lys Asp Leu Gly Ser Glu Leu Val Arg Tyr Asp Ala Arg Val His
                965                 970                 975

act cct cat ttg gat agg ctt gta agt gcc cga agt gta agc cca act 2976
Thr Pro His Leu Asp Arg Leu Val Ser Ala Arg Ser Val Ser Pro Thr
            980                 985                 990

aca gaa atg gtt tca agt gaa cct gta gac tac cga gct act ttt cca 3024
Thr Glu Met Val Ser Ser Glu Pro Val Asp Tyr Arg Ala Thr Phe Pro
        995                 1000                1005

gaa gac cag ttc cct aac tca tct cag aat gga tca tgc aga caa 3069
Glu Asp Gln Phe Pro Asn Ser Ser Gln Asn Gly Ser Cys Arg Gln
    1010                1015                1020

gtg cag tat cct ctg aca gac cta tcc ccc atc cta act agt gga 3114
Val Gln Tyr Pro Leu Thr Asp Leu Ser Pro Ile Leu Thr Ser Gly
    1025                1030                1035

gac tct gat ata tct agt cca tta ctg caa aat acc gtc cac att 3159

Asp Ser Asp Ile Ser Ser Pro Leu Leu Gln Asn Thr Val His Ile
    1040                1045                1050

gac ctc agt gct cta aat cca gag ctg gtc cag gca gtt cag cat 3204
Asp Leu Ser Ala Leu Asn Pro Glu Leu Val Gln Ala Val Gln His
    1055                1060                1065

gta gtg att ggg cca agc agc ctg att gtg cat ttc aat gaa gtc 3249
Val Val Ile Gly Pro Ser Ser Leu Ile Val His Phe Asn Glu Val
    1070                1075                1080

ata gga aga gga cat ttt ggg tgt gta tac cat ggg act tta ttg 3294
Ile Gly Arg Gly His Phe Gly Cys Val Tyr His Gly Thr Leu Leu
    1085                1090                1095

gat aat gat gat aag aaa att cac tgt gct gtg aaa tcc ttg aat 3339
Asp Asn Asp Asp Lys Lys Ile His Cys Ala Val Lys Ser Leu Asn
    1100                1105                1110

aga atc act gac ata gga gaa gtt tcc caa ttt ctg act gag gga 3384
Arg Ile Thr Asp Ile Gly Glu Val Ser Gln Phe Leu Thr Glu Gly
    1115                1120                1125

atc atc atg aaa gat ttt agt cat ccc aat gtt ctc tcg ctc ttg 3429
Ile Ile Met Lys Asp Phe Ser His Pro Asn Val Leu Ser Leu Leu
    1130                1135                1140

gga atc tgc ctt cga agt gag ggg tct cca ctg gtg gtc cta cca 3474
Gly Ile Cys Leu Arg Ser Glu Gly Ser Pro Leu Val Val Leu Pro
    1145                1150                1155

tac atg aaa cat ggg gat ctt cga aat ttc att aga aat gag act 3519
Tyr Met Lys His Gly Asp Leu Arg Asn Phe Ile Arg Asn Glu Thr
    1160                1165                1170

cat aac cca act gta aaa gat ctt att ggc ttt ggc ctt caa gtn 3564
His Asn Pro Thr Val Lys Asp Leu Ile Gly Phe Gly Leu Gln Val
    1175                1180                1185

gcc aaa ggc atg aaa tat ctt gca agc aaa aag ttt gtc cac aga 3609
Ala Lys Gly Met Lys Tyr Leu Ala Ser Lys Lys Phe Val His Arg
    1190                1195                1200

gac ttg gct gca aga aac tgt atg ctg gat gaa aaa ttc act gtc 3654
Asp Leu Ala Ala Arg Asn Cys Met Leu Asp Glu Lys Phe Thr Val
    1205                1210                1215

aag gtt gct gat ttt gga ctt gcc agg gat gtg tat gat aaa gaa 3699
Lys Val Ala Asp Phe Gly Leu Ala Arg Asp Val Tyr Asp Lys Glu

1220                    1225                    1230

tac tat agt gta cac aac aaa aca ggt gca aaa ctg cca gtg aag        3744
Tyr Tyr Ser Val His Asn Lys Thr Gly Ala Lys Leu Pro Val Lys
    1235                    1240                    1245

tgg atg gct tta gaa agc ctg caa act cag aag ttt acc acc aag        3789
Trp Met Ala Leu Glu Ser Leu Gln Thr Gln Lys Phe Thr Thr Lys
    1250                    1255                    1260

tca gat gtg tgg tcc ttt ggc gtg ctc ctc tgg gaa ctg atg aca        3834
Ser Asp Val Trp Ser Phe Gly Val Leu Leu Trp Glu Leu Met Thr
    1265                    1270                    1275

aga gga gca cca cct tat cct gac gtc aac acc ttt gat ata aca        3879
Arg Gly Ala Pro Pro Tyr Pro Asp Val Asn Thr Phe Asp Ile Thr
    1280                    1285                    1290

gtg tac ttg ttg caa ggc aga agg ctc cta caa cct gaa tac tgc        3924
Val Tyr Leu Leu Gln Gly Arg Arg Leu Leu Gln Pro Glu Tyr Cys
    1295                    1300                    1305

cca gat ccc ttg tac gaa gtg atg ctg aaa tgc tgg cat cct aga        3969
Pro Asp Pro Leu Tyr Glu Val Met Leu Lys Cys Trp His Pro Arg
    1310                    1315                    1320

gct gaa ctt cgc cca tcc ttt tct gaa ctg gtc tcc agg ata tca        4014
Ala Glu Leu Arg Pro Ser Phe Ser Glu Leu Val Ser Arg Ile Ser
    1325                    1330                    1335

gct atc ttc tct act ttc att ggg gag cac tat gtc cac gtg aat        4059
Ala Ile Phe Ser Thr Phe Ile Gly Glu His Tyr Val His Val Asn
    1340                    1345                    1350

gct act tac gtg aat gtc aaa tgt gtt gct cca tat cct tct ctt        4104
Ala Thr Tyr Val Asn Val Lys Cys Val Ala Pro Tyr Pro Ser Leu
    1355                    1360                    1365

ttg tca tca caa gat aat gtt gat ggc gag ggg gac aca tga            4146
Leu Ser Ser Gln Asp Asn Val Asp Gly Glu Gly Asp Thr
    1370                    1375                    1380

<210>  24
<211>  1381
<212>  PRT
<213>  Sus scrofa

<400>  24

Met Lys Ala Pro Ala Val Leu Ala Pro Gly Ile Leu Val Leu Leu Phe
1               5                   10                  15

Thr Leu Val Gln Lys Ser Lys Gly Glu Cys Lys Glu Ala Leu Val Lys
            20                  25                  30

Ser Thr Met Asn Leu Asn Met Gln Tyr Gln Leu Pro Asn Phe Thr Ala
        35                  40                  45

Glu Thr Pro Ile Gln Asn Val Val Leu His Lys His His Ile Tyr Leu
    50                  55                  60

Gly Ala Leu Asn Tyr Ile Tyr Val Leu Asn Asp Ile Asp Leu Arg Lys
65                  70                  75                  80

Val Ala Glu Tyr Lys Thr Gly Pro Val Leu Glu His Pro Asp Cys Phe
                85                  90                  95

Pro Cys Gln Asp Cys Ser His Lys Ala Asn Leu Ser Gly Gly Ile Trp
            100                 105                 110

Arg Asp Asn Ile Asn Met Ala Leu Leu Val Asp Thr Tyr Tyr Asp Asp
        115                 120                 125

Gln Leu Ile Ser Cys Gly Ser Val His Arg Gly Thr Cys Gln Arg His
    130                 135                 140

Val Leu Pro Pro Asp Asn Ile Ala Asp Ile Lys Ser Glu Val His Cys
145                 150                 155                 160

Met Tyr Ser Pro Gln Val Asp Glu Glu Pro Ser Gln Cys Pro Asp Cys
                165                 170                 175

Val Val Ser Ala Leu Gly Thr Lys Val Leu Leu Ser Glu Lys Asp Arg
            180                 185                 190

Phe Ile Asn Phe Phe Val Gly Asn Thr Ile Asn Ser Ser Tyr Leu Pro
        195                 200                 205

Asp His Ser Leu His Ser Ile Ser Val Arg Arg Leu Lys Glu Thr Gln
    210                 215                 220

Asp Gly Phe Lys Phe Leu Thr Asp Gln Ser Tyr Ile Asp Val Leu Pro
225                 230                 235                 240

Glu Phe Arg Asp Ser Tyr Pro Ile Lys Tyr Val His Ala Phe Glu Ser
                245                 250                 255

Asn His Phe Ile Tyr Phe Leu Thr Val Gln Arg Glu Thr Leu Asp Ala
            260                 265                 270

Gln Thr Phe His Thr Arg Ile Ile Arg Phe Cys Ser Val Asp Ser Gly
        275                 280                 285

Leu His Ser Tyr Met Glu Met Pro Leu Glu Cys Ile Leu Thr Glu Lys
    290                 295                 300

Arg Arg Lys Arg Ser Thr Arg Glu Glu Val Phe Asn Ile Leu Gln Ala
305                 310                 315                 320

Ala Tyr Val Ser Lys Pro Gly Thr Gln Leu Ala Lys Gln Ile Gly Ala
                325                 330                 335

Asn Leu Asn Asp Asp Ile Leu Tyr Gly Val Phe Ala Gln Ser Lys Pro
            340                 345                 350

Asp Ser Ala Glu Pro Met Asn Arg Ser Ala Val Cys Ala Phe Pro Val
        355                 360                 365

Lys Tyr Val Asn Glu Phe Phe Asn Lys Ile Val Asn Lys Asn Asn Val
    370                 375                 380

Arg Cys Leu Gln His Phe Tyr Gly Pro Asn His Glu His Cys Phe Asn

385                 390                 395                 400

Arg Thr Leu Leu Arg Asn Ser Ser Gly Cys Glu Val Arg Ser Asp Glu
                405                 410                 415

Tyr Arg Thr Glu Phe Thr Thr Ala Leu Gln Arg Ile Asp Leu Phe Met
            420                 425                 430

Gly Gln Phe Asn Gln Val Leu Leu Thr Ser Ile Ser Thr Phe Ile Lys
        435                 440                 445

Gly Asp Leu Thr Ile Ala Asn Leu Gly Thr Ser Glu Gly Arg Phe Met
    450                 455                 460

Gln Val Val Val Ser Arg Pro Gly Leu Ser Thr Pro His Val Asn Phe
465                 470                 475                 480

His Leu Asp Ser His Pro Val Ser Pro Glu Val Ile Val Glu Pro Leu
                485                 490                 495

Asn Gln Asn Gly Tyr Thr Leu Val Val Thr Gly Lys Lys Ile Thr Lys
            500                 505                 510

Ile Pro Leu Asn Gly Leu Gly Cys Glu His Phe Gln Ser Cys Ser Gln
        515                 520                 525

Cys Leu Ser Ala Pro Pro Phe Val Gln Cys Gly Trp Cys Gln Asp Lys
    530                 535                 540

Cys Val Gln Leu Glu Glu Cys Pro Ser Gly Thr Trp Thr Gln Glu Ile
545                 550                 555                 560

Cys Leu Pro Thr Val Tyr Lys Val Phe Pro Thr Ser Ala Pro Leu Glu
                565                 570                 575

Gly Gly Thr Thr Leu Thr Ile Cys Gly Trp Asp Phe Gly Phe Arg Arg
            580                 585                 590

Asn Asn Lys Phe Asp Leu Arg Lys Thr Lys Val Leu Leu Gly Asn Glu
        595                 600                 605

Ser Cys Thr Leu Thr Leu Ser Glu Ser Thr Thr Asn Thr Leu Lys Cys
    610                 615                 620

Thr Val Gly Pro Ala Met Asn Glu His Phe Asn Met Ser Ile Val Ile
625                 630                 635                 640

Ser Asn Gly Arg Gly Thr Thr Gln Tyr Arg Thr Phe Ser Tyr Val Asp
                645                 650                 655

Pro Val Ile Thr Asn Ile Ser Pro Ser Phe Gly Pro Lys Thr Gly Gly
            660                 665                 670

Thr Leu Leu Thr Leu Thr Gly Met His Leu Asn Ser Gly Asn Ser Arg
        675                 680                 685

His Ile Ser Ile Gly Gly Lys Thr Cys Thr Leu Lys Ser Val Ser Asp
    690                 695                 700

Ser Val Leu Glu Cys Tyr Thr Pro Ala Gln Thr Thr Pro Thr Glu Phe
705                 710                 715                 720

Pro Ile Lys Leu Lys Ile Asp Leu Ala Asn Arg Glu Ile Asn Ser Phe
                725                 730                 735

Thr Tyr Arg Glu Asp Pro Ile Val Tyr Glu Ile His Pro Thr Lys Ser
            740                 745                 750

Phe Ile Ser Gly Gly Ser Thr Ile Thr Gly Val Gly Arg Asn Leu Asn
        755                 760                 765

Ser Val Ser Val Leu Arg Met Val Ile Asn Val His Glu Ala Glu Arg
    770                 775                 780

Asn Phe Thr Val Ala Cys Gln His His Ser Asn Ser Glu Ile Ile Cys
785                 790                 795                 800

Cys Thr Thr Pro Ser Leu Gln Gln Leu Asn Leu Gln Leu Pro Leu Lys
                805                 810                 815

Thr Lys Ala Phe Phe Met Leu Asp Gly Ile His Ser Lys Tyr Phe Asp
            820                 825                 830

Leu Ile Tyr Val His Asn Pro Val Phe Lys Pro Phe Glu Lys Pro Val
        835                 840                 845

Met Ile Ser Ile Gly Asn Glu Asn Val Leu Glu Ile Lys Gly Asn Asp
    850                 855                 860

Ile Asp Pro Glu Ala Val Lys Gly Glu Val Leu Lys Val Gly Asn Lys
865                 870                 875                 880

Ser Cys Glu Asn Ile His Ser His Ser Glu Ala Val Leu Cys Thr Val
                885                 890                 895

Pro Asn Asp Leu Leu Lys Leu Asn Ser Glu Leu Asn Ile Glu Trp Lys
            900                 905                 910

Gln Ala Ile Ser Ser Thr Val Leu Gly Lys Val Ile Val Gln Pro Asp
        915                 920                 925

Gln Asn Phe Thr Gly Leu Ile Val Gly Val Val Ser Ile Ser Ile Ile
    930                 935                 940

Leu Leu Leu Leu Leu Gly Leu Phe Leu Trp Leu Lys Lys Arg Lys Gln
945                 950                 955                 960

Ile Lys Asp Leu Gly Ser Glu Leu Val Arg Tyr Asp Ala Arg Val His
                965                 970                 975

Thr Pro His Leu Asp Arg Leu Val Ser Ala Arg Ser Val Ser Pro Thr
            980                 985                 990

Thr Glu Met Val Ser Ser Glu Pro Val Asp Tyr Arg Ala Thr Phe Pro
        995                 1000                1005

Glu Asp Gln Phe Pro Asn Ser Ser Gln Asn Gly Ser Cys Arg Gln
    1010                1015                1020

Val Gln Tyr Pro Leu Thr Asp Leu Ser Pro Ile Leu Thr Ser Gly
    1025                1030                1035

Asp Ser Asp Ile Ser Ser Pro Leu Leu Gln Asn Thr Val His Ile
    1040                1045                1050

Asp Leu Ser Ala Leu Asn Pro Glu Leu Val Gln Ala Val Gln His
    1055                1060                1065

Val Val Ile Gly Pro Ser Ser Leu Ile Val His Phe Asn Glu Val
    1070                1075                1080

Ile Gly Arg Gly His Phe Gly Cys Val Tyr His Gly Thr Leu Leu
    1085                1090                1095

Asp Asn Asp Asp Lys Lys Ile His Cys Ala Val Lys Ser Leu Asn
    1100                1105                1110

Arg Ile Thr Asp Ile Gly Glu Val Ser Gln Phe Leu Thr Glu Gly
    1115                1120                1125

Ile Ile Met Lys Asp Phe Ser His Pro Asn Val Leu Ser Leu Leu
    1130                1135                1140

Gly Ile Cys Leu Arg Ser Glu Gly Ser Pro Leu Val Val Leu Pro
    1145                1150                1155

Tyr Met Lys His Gly Asp Leu Arg Asn Phe Ile Arg Asn Glu Thr

1160                    1165                    1170

His Asn  Pro Thr Val Lys Asp  Leu Ile Gly Phe Gly  Leu Gln Val
    1175                    1180                    1185

Ala Lys  Gly Met Lys Tyr Leu  Ala Ser Lys Lys Phe  Val His Arg
    1190                    1195                    1200

Asp Leu  Ala Ala Arg Asn Cys  Met Leu Asp Glu Lys  Phe Thr Val
    1205                    1210                    1215

Lys Val  Ala Asp Phe Gly Leu  Ala Arg Asp Val Tyr  Asp Lys Glu
    1220                    1225                    1230

Tyr Tyr  Ser Val His Asn Lys  Thr Gly Ala Lys Leu  Pro Val Lys
    1235                    1240                    1245

Trp Met  Ala Leu Glu Ser Leu  Gln Thr Gln Lys Phe  Thr Thr Lys
    1250                    1255                    1260

Ser Asp  Val Trp Ser Phe Gly  Val Leu Leu Trp Glu  Leu Met Thr
    1265                    1270                    1275

Arg Gly  Ala Pro Pro Tyr Pro  Asp Val Asn Thr Phe  Asp Ile Thr
    1280                    1285                    1290

Val Tyr  Leu Leu Gln Gly Arg  Arg Leu Leu Gln Pro  Glu Tyr Cys
    1295                    1300                    1305

Pro Asp  Pro Leu Tyr Glu Val  Met Leu Lys Cys Trp  His Pro Arg
    1310                    1315                    1320

Ala Glu  Leu Arg Pro Ser Phe  Ser Glu Leu Val Ser  Arg Ile Ser
    1325                    1330                    1335

Ala Ile  Phe Ser Thr Phe Ile  Gly Glu His Tyr Val  His Val Asn
    1340                    1345                    1350

Ala Thr Tyr Val Asn Val Lys Cys Val Ala Pro Tyr Pro Ser Leu
    1355                1360                1365

Leu Ser Ser Gln Asp Asn Val Asp Gly Glu Gly Asp Thr
    1370                1375                1380

<210> 25
<211> 4149
<212> DNA
<213> Canis lupus familiaris

<220>
<221> CDS
<222> (1)..(4146)

<400> 25
atg aag gct cct gct gtg ctt gca cct ggc atc ctt gtg ctt ctg ttt 48
Met Lys Ala Pro Ala Val Leu Ala Pro Gly Ile Leu Val Leu Leu Phe
1               5                   10                  15

acc ttg gtg cag aag agc tat ggg gag tgc aaa gag gca cta gta aag 96
Thr Leu Val Gln Lys Ser Tyr Gly Glu Cys Lys Glu Ala Leu Val Lys
            20                  25                  30

tct gag atg aat gtg aac atg aag tat cag ctt ccc aac ttc act gcc 144
Ser Glu Met Asn Val Asn Met Lys Tyr Gln Leu Pro Asn Phe Thr Ala
        35                  40                  45

gaa aca ccc atc cag aat gtt gtt tta cac aag cat cat att tac ctt 192
Glu Thr Pro Ile Gln Asn Val Val Leu His Lys His His Ile Tyr Leu
    50                  55                  60

ggt gca gtt aac tat att tac gtt tta aat gac aaa gac ctt cag aag 240
Gly Ala Val Asn Tyr Ile Tyr Val Leu Asn Asp Lys Asp Leu Gln Lys
65                  70                  75                  80

gtt gct gag tac aag act ggg ccc gtg ctg gaa cac cca gat tgt tcc 288
Val Ala Glu Tyr Lys Thr Gly Pro Val Leu Glu His Pro Asp Cys Ser
                85                  90                  95

cca tgt cag gac tgc agc cac aaa gcc aat tta tca ggt ggt gtt tgg 336
Pro Cys Gln Asp Cys Ser His Lys Ala Asn Leu Ser Gly Gly Val Trp
            100                 105                 110

gaa gat aac atc aac atg gct ctg ctt gtt gac aca tac tac gat gac 384
Glu Asp Asn Ile Asn Met Ala Leu Leu Val Asp Thr Tyr Tyr Asp Asp
        115                 120                 125

caa ctc att agc tgt ggc agt gtc cac aga ggg acc tgc cag cga cat 432
Gln Leu Ile Ser Cys Gly Ser Val His Arg Gly Thr Cys Gln Arg His
    130                 135                 140

atc ctt cca ccc agc aat att gct gac ata cag tcg gaa gtg cat tgc 480
Ile Leu Pro Pro Ser Asn Ile Ala Asp Ile Gln Ser Glu Val His Cys
145                 150                 155                 160

atg tac tcc tca cag gca gac gaa gag ccc agc cag tgc cct gac tgt 528
Met Tyr Ser Ser Gln Ala Asp Glu Glu Pro Ser Gln Cys Pro Asp Cys
                165                 170                 175

gtg gtg agt gct cta gga acc aaa gtc ctg ata tca gaa aag gac cgg 576
Val Val Ser Ala Leu Gly Thr Lys Val Leu Ile Ser Glu Lys Asp Arg
            180                 185                 190

ttc atc aac ttc ttc gta ggc aat acc ata aat tcc tcg gac cat cca 624
Phe Ile Asn Phe Phe Val Gly Asn Thr Ile Asn Ser Ser Asp His Pro
        195                 200                 205

gat cat tca ttg cat tcg ata tcg gtg aga agg cta aag gaa acg caa 672
Asp His Ser Leu His Ser Ile Ser Val Arg Arg Leu Lys Glu Thr Gln
    210                 215                 220

gat ggg ttc aag ttt ttg aca gac cag tct tac att gat gtt cta ccg 720
Asp Gly Phe Lys Phe Leu Thr Asp Gln Ser Tyr Ile Asp Val Leu Pro
225                 230                 235                 240

gag ttc aga gac tcc tac ccc att aaa tat gtc cac gcc ttt gaa agc 768
Glu Phe Arg Asp Ser Tyr Pro Ile Lys Tyr Val His Ala Phe Glu Ser
                245                 250                 255

aac cac ttt att tac ttt ttg aca gtc cag cga gaa act cta gat gct 816
Asn His Phe Ile Tyr Phe Leu Thr Val Gln Arg Glu Thr Leu Asp Ala
            260                 265                 270

cag act ttt cac acg aga ata atc agg ttc tgt tct gta gac tct gga 864
Gln Thr Phe His Thr Arg Ile Ile Arg Phe Cys Ser Val Asp Ser Gly
        275                 280                 285

ttg cat tcc tac atg gaa atg cct ctg gag tgt att ctc acg gaa aag 912
Leu His Ser Tyr Met Glu Met Pro Leu Glu Cys Ile Leu Thr Glu Lys
    290                 295                 300

aga aga aag aga tcc aca agg gag gaa gtg ttt aat att ctc caa gct 960

Arg Arg Lys Arg Ser Thr Arg Glu Glu Val Phe Asn Ile Leu Gln Ala
305                 310                 315                 320

gca tat gtc agt aag cct ggg gcc cat ctc gct aaa caa ata ggt gcc      1008
Ala Tyr Val Ser Lys Pro Gly Ala His Leu Ala Lys Gln Ile Gly Ala
                325                 330                 335

aac ctg aat gat gac att ctc tat gga gtg ttc gca caa agc aag cca      1056
Asn Leu Asn Asp Asp Ile Leu Tyr Gly Val Phe Ala Gln Ser Lys Pro
            340                 345                 350

gat tct gct gaa cca atg aat cgc tct gcc gtc tgt gcg ttc cct atc      1104
Asp Ser Ala Glu Pro Met Asn Arg Ser Ala Val Cys Ala Phe Pro Ile
        355                 360                 365

aaa tat gtc aat gaa ttc ttc aac aag atc gtc aac aaa aac aat gtg      1152
Lys Tyr Val Asn Glu Phe Phe Asn Lys Ile Val Asn Lys Asn Asn Val
    370                 375                 380

aga tgt ctt cag cac ttt tat gga ccc aac cac gaa cac tgc ttt aat      1200
Arg Cys Leu Gln His Phe Tyr Gly Pro Asn His Glu His Cys Phe Asn
385                 390                 395                 400

agg aca ctt ttg aga aat tca tcg ggc tgt gaa gcg cgc aat gat gaa      1248
Arg Thr Leu Leu Arg Asn Ser Ser Gly Cys Glu Ala Arg Asn Asp Glu
                405                 410                 415

tat cga acg gag ttc act aca gct ttg cag cgc gtt gac tta ttc atg      1296
Tyr Arg Thr Glu Phe Thr Thr Ala Leu Gln Arg Val Asp Leu Phe Met
            420                 425                 430

ggc cag ttc aac caa gtc ctc tta acg tct ata tcc acc ttc atc aaa      1344
Gly Gln Phe Asn Gln Val Leu Leu Thr Ser Ile Ser Thr Phe Ile Lys
        435                 440                 445

gga gac ctc acc att gct aat ctt ggg acg tcc gag ggt cgc ttc atg      1392
Gly Asp Leu Thr Ile Ala Asn Leu Gly Thr Ser Glu Gly Arg Phe Met
    450                 455                 460

cag gtc gtg gtt tct cga tca gga ttg tcg acc cct cac gtg aac ttc      1440
Gln Val Val Val Ser Arg Ser Gly Leu Ser Thr Pro His Val Asn Phe
465                 470                 475                 480

cgc ctg gac tcc cac ccc gtg tct cca gaa gca att gtg gag cac cca      1488
Arg Leu Asp Ser His Pro Val Ser Pro Glu Ala Ile Val Glu His Pro
                485                 490                 495

cta aac caa aac ggc tac aca ctc gtt gtc act ggg aag aag atc acc      1536
Leu Asn Gln Asn Gly Tyr Thr Leu Val Val Thr Gly Lys Lys Ile Thr

            500                 505                 510

agg atc cca ctg aat ggc tta ggc tgt gag cat ttt cag tcc tgc agt 1584
Arg Ile Pro Leu Asn Gly Leu Gly Cys Glu His Phe Gln Ser Cys Ser
        515                 520                 525

cag tgt ctc tcc gcc cct ccc ttt gtg cag tgt ggc tgg tgc cac gat 1632
Gln Cys Leu Ser Ala Pro Pro Phe Val Gln Cys Gly Trp Cys His Asp
    530                 535                 540

aga tgt gtg cac ctg gag gaa tgt ccc act gga gcg tgg act cag gag 1680
Arg Cys Val His Leu Glu Glu Cys Pro Thr Gly Ala Trp Thr Gln Glu
545                 550                 555                 560

gtc tgt ctg cct gca atc tat gag gtt ttc cca act agt gca ccc ctg 1728
Val Cys Leu Pro Ala Ile Tyr Glu Val Phe Pro Thr Ser Ala Pro Leu
                565                 570                 575

gaa gga ggg aca gtg ctg act gta tgt ggc tgg gac ttc gga ttc agg 1776
Glu Gly Gly Thr Val Leu Thr Val Cys Gly Trp Asp Phe Gly Phe Arg
            580                 585                 590

agg aat aat aaa ttt gat tta aag aaa acc aaa gtt ttc ctt gga aat 1824
Arg Asn Asn Lys Phe Asp Leu Lys Lys Thr Lys Val Phe Leu Gly Asn
        595                 600                 605

gag agc tgc acc ttg acc tta agt gag agc aca aca aat atg ctg aaa 1872
Glu Ser Cys Thr Leu Thr Leu Ser Glu Ser Thr Thr Asn Met Leu Lys
    610                 615                 620

tgc aca gtt ggc cct gca gtg aac gag cat ttc aat ata tcc ata att 1920
Cys Thr Val Gly Pro Ala Val Asn Glu His Phe Asn Ile Ser Ile Ile
625                 630                 635                 640

att tca aat ggt cga ggg aca gca caa tat agt aca ttt tcg tat gtg 1968
Ile Ser Asn Gly Arg Gly Thr Ala Gln Tyr Ser Thr Phe Ser Tyr Val
                645                 650                 655

gat cct att ata aca agt att tct cca agt tat ggt ccc aag aat ggt 2016
Asp Pro Ile Ile Thr Ser Ile Ser Pro Ser Tyr Gly Pro Lys Asn Gly
            660                 665                 670

ggc acc ttg ctc act tta act gga aaa tac ctc aac agt ggg aat tct 2064
Gly Thr Leu Leu Thr Leu Thr Gly Lys Tyr Leu Asn Ser Gly Asn Ser
        675                 680                 685

aga cac att tca atg ggt gga aaa aca tgt act tta aaa agt gtg tca 2112
Arg His Ile Ser Met Gly Gly Lys Thr Cys Thr Leu Lys Ser Val Ser
    690                 695                 700

gat agt att ctc gaa tgt tat acc cca gct caa gcc act gca act gag 2160
Asp Ser Ile Leu Glu Cys Tyr Thr Pro Ala Gln Ala Thr Ala Thr Glu
705                 710                 715                 720

ttt cct att aaa ttg aaa att gac cta gcc aac cga gag atg aac agc 2208
Phe Pro Ile Lys Leu Lys Ile Asp Leu Ala Asn Arg Glu Met Asn Ser
                725                 730                 735

ttc agt tac cag gaa gac ccc att gtc tat gca att cat cca acg aaa 2256
Phe Ser Tyr Gln Glu Asp Pro Ile Val Tyr Ala Ile His Pro Thr Lys
            740                 745                 750

tct ttt att agt ggt ggg agc aca ata aca gct gtt gga aaa aac ctg 2304
Ser Phe Ile Ser Gly Gly Ser Thr Ile Thr Ala Val Gly Lys Asn Leu
        755                 760                 765

aat tca gtg agt gtc ctg agg atg gta ata gat gtc cat gaa aca aga 2352
Asn Ser Val Ser Val Leu Arg Met Val Ile Asp Val His Glu Thr Arg
    770                 775                 780

agg aac ttt aca gtg gca tgt caa cat cgc tct aat tca gag ata atc 2400
Arg Asn Phe Thr Val Ala Cys Gln His Arg Ser Asn Ser Glu Ile Ile
785                 790                 795                 800

tgt tgt acg act cct tca ctg caa cag ctg aat ctg caa ctc cct ctg 2448
Cys Cys Thr Thr Pro Ser Leu Gln Gln Leu Asn Leu Gln Leu Pro Leu
                805                 810                 815

aaa acc aaa gcc ttt ttc atg tta gat ggg atc cat tcc aaa tac ttt 2496
Lys Thr Lys Ala Phe Phe Met Leu Asp Gly Ile His Ser Lys Tyr Phe
            820                 825                 830

gat ctc att tat gta cat aat cct gtg ttt aag cct ttt gaa aag cca 2544
Asp Leu Ile Tyr Val His Asn Pro Val Phe Lys Pro Phe Glu Lys Pro
        835                 840                 845

gtg atg atc tca ata ggc aat gaa aat gta ctg gaa att aag gga aat 2592
Val Met Ile Ser Ile Gly Asn Glu Asn Val Leu Glu Ile Lys Gly Asn
    850                 855                 860

gat att gac cct gaa gca gtt aaa ggc gaa gtg tta aaa gtt gga aat 2640
Asp Ile Asp Pro Glu Ala Val Lys Gly Glu Val Leu Lys Val Gly Asn
865                 870                 875                 880

aag agc tgt gag act atc tac tca gat tct aaa gcc gtt tta tgc aag 2688
Lys Ser Cys Glu Thr Ile Tyr Ser Asp Ser Lys Ala Val Leu Cys Lys
                885                 890                 895

gtc ccc aat gac ctg ctg aaa ttg aac aac gag cta aat ata gag tgg 2736
Val Pro Asn Asp Leu Leu Lys Leu Asn Asn Glu Leu Asn Ile Glu Trp
            900                 905                 910

aag caa gca gtt tct tca acc gtc ctt gga aaa gta ata gtt caa cca 2784
Lys Gln Ala Val Ser Ser Thr Val Leu Gly Lys Val Ile Val Gln Pro
        915                 920                 925

gat cag aat ttc aca gga ttg att gct ggt gtt atc tca ata tca aca 2832
Asp Gln Asn Phe Thr Gly Leu Ile Ala Gly Val Ile Ser Ile Ser Thr
    930                 935                 940

ata gtc tta tta tta ctc gga ctt ttc ctg tgg ctg aaa agg aaa aag 2880
Ile Val Leu Leu Leu Leu Gly Leu Phe Leu Trp Leu Lys Arg Lys Lys
945                 950                 955                 960

caa att aaa gat ctg ggc agt gaa tta gtt cgc tat gat gca aga gta 2928
Gln Ile Lys Asp Leu Gly Ser Glu Leu Val Arg Tyr Asp Ala Arg Val
                965                 970                 975

cac act cct cat ttg gat agg ctt gta agt gcc cga agt gta agc cca 2976
His Thr Pro His Leu Asp Arg Leu Val Ser Ala Arg Ser Val Ser Pro
            980                 985                 990

act aca gaa atg gtt tca aat gaa tct gta gac tac cga gct act ttt 3024
Thr Thr Glu Met Val Ser Asn Glu Ser Val Asp Tyr Arg Ala Thr Phe
        995                 1000                1005

cca gaa gac cag ttt cct aat tca tct cag aat gga tca tgc aga 3069
Pro Glu Asp Gln Phe Pro Asn Ser Ser Gln Asn Gly Ser Cys Arg
    1010                1015                1020

caa gta caa tat cct ctg acg gac ctg tcc ccc atg ctt act agt 3114
Gln Val Gln Tyr Pro Leu Thr Asp Leu Ser Pro Met Leu Thr Ser
    1025                1030                1035

ggg gac tct gat ata tcc agt cca tta ttg caa aat act gtc cac 3159
Gly Asp Ser Asp Ile Ser Ser Pro Leu Leu Gln Asn Thr Val His
    1040                1045                1050

att gac ctc agt gct cta aat cca gag ctg gtg cag gca gtc cag 3204
Ile Asp Leu Ser Ala Leu Asn Pro Glu Leu Val Gln Ala Val Gln
    1055                1060                1065

cat gta gtg att ggg ccc agt agc ctg att gtg cat ttc aat gaa 3249
His Val Val Ile Gly Pro Ser Ser Leu Ile Val His Phe Asn Glu
    1070                1075                1080

gtc ata gga aga gga cat ttt ggg tgt gta tac cat ggg act ttg 3294

Val Ile Gly Arg Gly His Phe Gly Cys Val Tyr His Gly Thr Leu
    1085                1090                1095

ttg gac aat gac gac aaa aaa att cac tgt gct gtg aaa tcc ctg 3339
Leu Asp Asn Asp Asp Lys Lys Ile His Cys Ala Val Lys Ser Leu
    1100                1105                1110

aat aga atc act gac ata gga gaa gtt tcc cag ttt ctg acc gag 3384
Asn Arg Ile Thr Asp Ile Gly Glu Val Ser Gln Phe Leu Thr Glu
    1115                1120                1125

gga atc atc atg aaa gat ttt agt cat cca aac gta ctc tca ctc 3429
Gly Ile Ile Met Lys Asp Phe Ser His Pro Asn Val Leu Ser Leu
    1130                1135                1140

ttg gga atc tgc ctt cga agt gag ggg tct cca ctg gtg gtc cta 3474
Leu Gly Ile Cys Leu Arg Ser Glu Gly Ser Pro Leu Val Val Leu
    1145                1150                1155

cca tac atg aaa cat gga gat ctt cga aat ttc att aga aat gag 3519
Pro Tyr Met Lys His Gly Asp Leu Arg Asn Phe Ile Arg Asn Glu
    1160                1165                1170

act cat aac cca act gta aaa gat ctt att ggc ttt ggt ctt caa 3564
Thr His Asn Pro Thr Val Lys Asp Leu Ile Gly Phe Gly Leu Gln
    1175                1180                1185

gta gcc aaa ggc atg aaa tat ctt gca agc aaa aag ttt gtc cac 3609
Val Ala Lys Gly Met Lys Tyr Leu Ala Ser Lys Lys Phe Val His
    1190                1195                1200

aga gac ttg gct gca aga aac tgt atg ctg gat gaa aaa ttc act 3654
Arg Asp Leu Ala Ala Arg Asn Cys Met Leu Asp Glu Lys Phe Thr
    1205                1210                1215

gtc aag gtt gct gat ttt ggt ctt gcc aga gac atg tat gat aaa 3699
Val Lys Val Ala Asp Phe Gly Leu Ala Arg Asp Met Tyr Asp Lys
    1220                1225                1230

gaa tac tac agc gta cac aac aaa aca ggc gcc aaa cta cca gtg 3744
Glu Tyr Tyr Ser Val His Asn Lys Thr Gly Ala Lys Leu Pro Val
    1235                1240                1245

aag tgg atg gct tta gaa agt ctg caa act cag aag ttt acc acc 3789
Lys Trp Met Ala Leu Glu Ser Leu Gln Thr Gln Lys Phe Thr Thr
    1250                1255                1260

aag tca gat gtg tgg tcc ttt ggc gtg ctc ctc tgg gaa ctg atg 3834
Lys Ser Asp Val Trp Ser Phe Gly Val Leu Leu Trp Glu Leu Met

1265                 1270                 1275

aca aga gga gca cca cct tat cct gac gtc aac acc ttt gat ata      3879
Thr Arg Gly Ala Pro Pro Tyr Pro Asp Val Asn Thr Phe Asp Ile
    1280                 1285                 1290

aca gtt tac ttg ttg caa ggc aga agg ctc ctg caa ccc gaa tac      3924
Thr Val Tyr Leu Leu Gln Gly Arg Arg Leu Leu Gln Pro Glu Tyr
    1295                 1300                 1305

tgc cca gat ccc tta tac gaa gtg atg cta aaa tgc tgg cac cct      3969
Cys Pro Asp Pro Leu Tyr Glu Val Met Leu Lys Cys Trp His Pro
    1310                 1315                 1320

aga gct gaa ctg cgc cca tct ttt tct gaa ctg gtc tcc agg ata      4014
Arg Ala Glu Leu Arg Pro Ser Phe Ser Glu Leu Val Ser Arg Ile
    1325                 1330                 1335

tca gca ata ttc tct act ttc att ggg gag cac tat gtc cat gtg      4059
Ser Ala Ile Phe Ser Thr Phe Ile Gly Glu His Tyr Val His Val
    1340                 1345                 1350

aac gcc act tat gtg aat gtc aaa tgt gtt gct cca tac cct tct      4104
Asn Ala Thr Tyr Val Asn Val Lys Cys Val Ala Pro Tyr Pro Ser
    1355                 1360                 1365

ctc ttg tca tca caa gat aac att gat ggc gag ggg gac aca tga      4149
Leu Leu Ser Ser Gln Asp Asn Ile Asp Gly Glu Gly Asp Thr
    1370                 1375                 1380

<210>  26
<211>  1382
<212>  PRT
<213>  Canis lupus familiaris

<400>  26

Met Lys Ala Pro Ala Val Leu Ala Pro Gly Ile Leu Val Leu Leu Phe
1               5                   10                  15

Thr Leu Val Gln Lys Ser Tyr Gly Glu Cys Lys Glu Ala Leu Val Lys
            20                  25                  30

Ser Glu Met Asn Val Asn Met Lys Tyr Gln Leu Pro Asn Phe Thr Ala
        35                  40                  45

Glu Thr Pro Ile Gln Asn Val Val Leu His Lys His His Ile Tyr Leu
    50                  55                  60

Gly Ala Val Asn Tyr Ile Tyr Val Leu Asn Asp Lys Asp Leu Gln Lys
65                  70                  75                  80

Val Ala Glu Tyr Lys Thr Gly Pro Val Leu Glu His Pro Asp Cys Ser
                85                  90                  95

Pro Cys Gln Asp Cys Ser His Lys Ala Asn Leu Ser Gly Gly Val Trp
            100                 105                 110

Glu Asp Asn Ile Asn Met Ala Leu Leu Val Asp Thr Tyr Tyr Asp Asp
        115                 120                 125

Gln Leu Ile Ser Cys Gly Ser Val His Arg Gly Thr Cys Gln Arg His
    130                 135                 140

Ile Leu Pro Pro Ser Asn Ile Ala Asp Ile Gln Ser Glu Val His Cys
145                 150                 155                 160

Met Tyr Ser Ser Gln Ala Asp Glu Glu Pro Ser Gln Cys Pro Asp Cys
                165                 170                 175

Val Val Ser Ala Leu Gly Thr Lys Val Leu Ile Ser Glu Lys Asp Arg
            180                 185                 190

Phe Ile Asn Phe Phe Val Gly Asn Thr Ile Asn Ser Ser Asp His Pro
        195                 200                 205

Asp His Ser Leu His Ser Ile Ser Val Arg Arg Leu Lys Glu Thr Gln
    210                 215                 220

Asp Gly Phe Lys Phe Leu Thr Asp Gln Ser Tyr Ile Asp Val Leu Pro
225                 230                 235                 240

Glu Phe Arg Asp Ser Tyr Pro Ile Lys Tyr Val His Ala Phe Glu Ser
                245                 250                 255

Asn His Phe Ile Tyr Phe Leu Thr Val Gln Arg Glu Thr Leu Asp Ala
            260                 265                 270

Gln Thr Phe His Thr Arg Ile Ile Arg Phe Cys Ser Val Asp Ser Gly
        275                 280                 285

Leu His Ser Tyr Met Glu Met Pro Leu Glu Cys Ile Leu Thr Glu Lys
    290                 295                 300

Arg Arg Lys Arg Ser Thr Arg Glu Glu Val Phe Asn Ile Leu Gln Ala
305                 310                 315                 320

Ala Tyr Val Ser Lys Pro Gly Ala His Leu Ala Lys Gln Ile Gly Ala
                325                 330                 335

Asn Leu Asn Asp Asp Ile Leu Tyr Gly Val Phe Ala Gln Ser Lys Pro
            340                 345                 350

Asp Ser Ala Glu Pro Met Asn Arg Ser Ala Val Cys Ala Phe Pro Ile
        355                 360                 365

Lys Tyr Val Asn Glu Phe Phe Asn Lys Ile Val Asn Lys Asn Asn Val
    370                 375                 380

Arg Cys Leu Gln His Phe Tyr Gly Pro Asn His Glu His Cys Phe Asn
385                 390                 395                 400

Arg Thr Leu Leu Arg Asn Ser Ser Gly Cys Glu Ala Arg Asn Asp Glu
                405                 410                 415

Tyr Arg Thr Glu Phe Thr Thr Ala Leu Gln Arg Val Asp Leu Phe Met
            420                 425                 430

Gly Gln Phe Asn Gln Val Leu Leu Thr Ser Ile Ser Thr Phe Ile Lys

        435                 440                 445

Gly Asp Leu Thr Ile Ala Asn Leu Gly Thr Ser Glu Gly Arg Phe Met
    450                 455                 460

Gln Val Val Val Ser Arg Ser Gly Leu Ser Thr Pro His Val Asn Phe
465                 470                 475                 480

Arg Leu Asp Ser His Pro Val Ser Pro Glu Ala Ile Val Glu His Pro
                485                 490                 495

Leu Asn Gln Asn Gly Tyr Thr Leu Val Val Thr Gly Lys Lys Ile Thr
            500                 505                 510

Arg Ile Pro Leu Asn Gly Leu Gly Cys Glu His Phe Gln Ser Cys Ser
        515                 520                 525

Gln Cys Leu Ser Ala Pro Pro Phe Val Gln Cys Gly Trp Cys His Asp
    530                 535                 540

Arg Cys Val His Leu Glu Glu Cys Pro Thr Gly Ala Trp Thr Gln Glu
545                 550                 555                 560

Val Cys Leu Pro Ala Ile Tyr Glu Val Phe Pro Thr Ser Ala Pro Leu
                565                 570                 575

Glu Gly Gly Thr Val Leu Thr Val Cys Gly Trp Asp Phe Gly Phe Arg
            580                 585                 590

Arg Asn Asn Lys Phe Asp Leu Lys Lys Thr Lys Val Phe Leu Gly Asn
        595                 600                 605

Glu Ser Cys Thr Leu Thr Leu Ser Glu Ser Thr Thr Asn Met Leu Lys
    610                 615                 620

Cys Thr Val Gly Pro Ala Val Asn Glu His Phe Asn Ile Ser Ile Ile
625                 630                 635                 640

Ile Ser Asn Gly Arg Gly Thr Ala Gln Tyr Ser Thr Phe Ser Tyr Val
                645                 650                 655

Asp Pro Ile Ile Thr Ser Ile Ser Pro Ser Tyr Gly Pro Lys Asn Gly
            660                 665                 670

Gly Thr Leu Leu Thr Leu Thr Gly Lys Tyr Leu Asn Ser Gly Asn Ser
        675                 680                 685

Arg His Ile Ser Met Gly Gly Lys Thr Cys Thr Leu Lys Ser Val Ser
    690                 695                 700

Asp Ser Ile Leu Glu Cys Tyr Thr Pro Ala Gln Ala Thr Ala Thr Glu
705                 710                 715                 720

Phe Pro Ile Lys Leu Lys Ile Asp Leu Ala Asn Arg Glu Met Asn Ser
                725                 730                 735

Phe Ser Tyr Gln Glu Asp Pro Ile Val Tyr Ala Ile His Pro Thr Lys
            740                 745                 750

Ser Phe Ile Ser Gly Gly Ser Thr Ile Thr Ala Val Gly Lys Asn Leu
        755                 760                 765

Asn Ser Val Ser Val Leu Arg Met Val Ile Asp Val His Glu Thr Arg
    770                 775                 780

Arg Asn Phe Thr Val Ala Cys Gln His Arg Ser Asn Ser Glu Ile Ile
785                 790                 795                 800

Cys Cys Thr Thr Pro Ser Leu Gln Gln Leu Asn Leu Gln Leu Pro Leu
                805                 810                 815

Lys Thr Lys Ala Phe Phe Met Leu Asp Gly Ile His Ser Lys Tyr Phe
            820                 825                 830

Asp Leu Ile Tyr Val His Asn Pro Val Phe Lys Pro Phe Glu Lys Pro
        835                 840                 845

Val Met Ile Ser Ile Gly Asn Glu Asn Val Leu Glu Ile Lys Gly Asn
    850                 855                 860

Asp Ile Asp Pro Glu Ala Val Lys Gly Glu Val Leu Lys Val Gly Asn
865                 870                 875                 880

Lys Ser Cys Glu Thr Ile Tyr Ser Asp Ser Lys Ala Val Leu Cys Lys
                885                 890                 895

Val Pro Asn Asp Leu Leu Lys Leu Asn Asn Glu Leu Asn Ile Glu Trp
            900                 905                 910

Lys Gln Ala Val Ser Ser Thr Val Leu Gly Lys Val Ile Val Gln Pro
        915                 920                 925

Asp Gln Asn Phe Thr Gly Leu Ile Ala Gly Val Ile Ser Ile Ser Thr
    930                 935                 940

Ile Val Leu Leu Leu Leu Gly Leu Phe Leu Trp Leu Lys Arg Lys Lys
945                 950                 955                 960

Gln Ile Lys Asp Leu Gly Ser Glu Leu Val Arg Tyr Asp Ala Arg Val
                965                 970                 975

His Thr Pro His Leu Asp Arg Leu Val Ser Ala Arg Ser Val Ser Pro
            980                 985                 990

Thr Thr Glu Met Val Ser Asn Glu  Ser Val Asp Tyr Arg  Ala Thr Phe
        995                 1000                 1005

Pro Glu  Asp Gln Phe Pro Asn  Ser Ser Gln Asn Gly  Ser Cys Arg
    1010                 1015                 1020

Gln Val Gln Tyr Pro Leu Thr Asp Leu Ser Pro Met Leu Thr Ser
    1025                1030                1035

Gly Asp Ser Asp Ile Ser Ser Pro Leu Leu Gln Asn Thr Val His
    1040                1045                1050

Ile Asp Leu Ser Ala Leu Asn Pro Glu Leu Val Gln Ala Val Gln
    1055                1060                1065

His Val Val Ile Gly Pro Ser Ser Leu Ile Val His Phe Asn Glu
    1070                1075                1080

Val Ile Gly Arg Gly His Phe Gly Cys Val Tyr His Gly Thr Leu
    1085                1090                1095

Leu Asp Asn Asp Asp Lys Lys Ile His Cys Ala Val Lys Ser Leu
    1100                1105                1110

Asn Arg Ile Thr Asp Ile Gly Glu Val Ser Gln Phe Leu Thr Glu
    1115                1120                1125

Gly Ile Ile Met Lys Asp Phe Ser His Pro Asn Val Leu Ser Leu
    1130                1135                1140

Leu Gly Ile Cys Leu Arg Ser Glu Gly Ser Pro Leu Val Val Leu
    1145                1150                1155

Pro Tyr Met Lys His Gly Asp Leu Arg Asn Phe Ile Arg Asn Glu
    1160                1165                1170

Thr His Asn Pro Thr Val Lys Asp Leu Ile Gly Phe Gly Leu Gln
    1175                1180                1185

Val Ala Lys Gly Met Lys Tyr Leu Ala Ser Lys Lys Phe Val His
    1190                1195                1200

Arg Asp Leu Ala Ala Arg Asn Cys Met Leu Asp Glu Lys Phe Thr

1205                 1210                 1215

Val Lys Val Ala Asp Phe Gly Leu Ala Arg Asp Met Tyr Asp Lys
    1220                 1225                 1230

Glu Tyr Tyr Ser Val His Asn Lys Thr Gly Ala Lys Leu Pro Val
    1235                 1240                 1245

Lys Trp Met Ala Leu Glu Ser Leu Gln Thr Gln Lys Phe Thr Thr
    1250                 1255                 1260

Lys Ser Asp Val Trp Ser Phe Gly Val Leu Leu Trp Glu Leu Met
    1265                 1270                 1275

Thr Arg Gly Ala Pro Pro Tyr Pro Asp Val Asn Thr Phe Asp Ile
    1280                 1285                 1290

Thr Val Tyr Leu Leu Gln Gly Arg Arg Leu Leu Gln Pro Glu Tyr
    1295                 1300                 1305

Cys Pro Asp Pro Leu Tyr Glu Val Met Leu Lys Cys Trp His Pro
    1310                 1315                 1320

Arg Ala Glu Leu Arg Pro Ser Phe Ser Glu Leu Val Ser Arg Ile
    1325                 1330                 1335

Ser Ala Ile Phe Ser Thr Phe Ile Gly Glu His Tyr Val His Val
    1340                 1345                 1350

Asn Ala Thr Tyr Val Asn Val Lys Cys Val Ala Pro Tyr Pro Ser
    1355                 1360                 1365

Leu Leu Ser Ser Gln Asp Asn Ile Asp Gly Glu Gly Asp Thr
    1370                 1375                 1380

<210>  27
<211>  4155

<212> DNA
<213> Bos taurus

<220>
<221> CDS
<222> (1)..(4152)

<400> 27
atg aag gcc cct gct gtg ctt gca cct ggc att ctt gtg ctt ctg ttt 48
Met Lys Ala Pro Ala Val Leu Ala Pro Gly Ile Leu Val Leu Leu Phe
1               5                   10                  15

acc ttc gtg cag aag agc aat ggg gag tgt aaa gag gca cta gta aaa 96
Thr Phe Val Gln Lys Ser Asn Gly Glu Cys Lys Glu Ala Leu Val Lys
            20                  25                  30

tcc agg atg aat gtg aac atg caa tat cag ctt cct aac ttc act gca 144
Ser Arg Met Asn Val Asn Met Gln Tyr Gln Leu Pro Asn Phe Thr Ala
        35                  40                  45

gaa aca tcc att cag aat gtt gtt ttg cac aaa cat cac att tac ctc 192
Glu Thr Ser Ile Gln Asn Val Val Leu His Lys His His Ile Tyr Leu
    50                  55                  60

ggt gcc att aac tac att tac gtt tta aat gac aaa gac ctt cag aag 240
Gly Ala Ile Asn Tyr Ile Tyr Val Leu Asn Asp Lys Asp Leu Gln Lys
65                  70                  75                  80

gtt gct gag tac aag act ggg cct gtg ctg gaa cac cca gat tgt ttc 288
Val Ala Glu Tyr Lys Thr Gly Pro Val Leu Glu His Pro Asp Cys Phe
                85                  90                  95

cca tgt cag gac tgc agc cat aaa gcc aat tta tca ggt ggt gtt tgg 336
Pro Cys Gln Asp Cys Ser His Lys Ala Asn Leu Ser Gly Gly Val Trp
            100                 105                 110

aaa gat aac atc aac atg gct ctg ctt gtt gac acg tac tat gat gat 384
Lys Asp Asn Ile Asn Met Ala Leu Leu Val Asp Thr Tyr Tyr Asp Asp
        115                 120                 125

caa ctc att agc tgt ggc agt gtc cac aga ggg acc tgc cag cga cac 432
Gln Leu Ile Ser Cys Gly Ser Val His Arg Gly Thr Cys Gln Arg His
    130                 135                 140

gtc ctt cca ccc aac aat act gca gac ata gag tcg gag gtt cac tgc 480
Val Leu Pro Pro Asn Asn Thr Ala Asp Ile Glu Ser Glu Val His Cys
145                 150                 155                 160

atg tac tct cct cag gca gac gaa gag acc aac cag tgt cct gac tgt 528
Met Tyr Ser Pro Gln Ala Asp Glu Glu Thr Asn Gln Cys Pro Asp Cys
                165                 170                 175

gtg gtg agt gcc ctg gga acc aaa gtc cta ctg tct gaa aag gat cga 576
Val Val Ser Ala Leu Gly Thr Lys Val Leu Leu Ser Glu Lys Asp Arg
            180                 185                 190

ttc atc aac ttc ttc gtg ggc aat acc ata aat tct tct tac ctt cca 624
Phe Ile Asn Phe Phe Val Gly Asn Thr Ile Asn Ser Ser Tyr Leu Pro
        195                 200                 205

gat tat ata ttg cac tcg ata tca gtg aga agg cta aag gaa aca caa 672
Asp Tyr Ile Leu His Ser Ile Ser Val Arg Arg Leu Lys Glu Thr Gln
    210                 215                 220

gat ggt ttt aag ttt ttg aca gac caa tcc tat att gat gtt cta cct 720
Asp Gly Phe Lys Phe Leu Thr Asp Gln Ser Tyr Ile Asp Val Leu Pro
225                 230                 235                 240

gaa ctc cga gat tcc tac ccc att aag tat gtc cac gcc ttt gaa agc 768
Glu Leu Arg Asp Ser Tyr Pro Ile Lys Tyr Val His Ala Phe Glu Ser
                245                 250                 255

aac cat ttt att tac ttt ttg acg gtc cag cgg gaa act cta gat gct 816
Asn His Phe Ile Tyr Phe Leu Thr Val Gln Arg Glu Thr Leu Asp Ala
            260                 265                 270

cag act ttt cac aca aga ata atc agg ttc tgt tcc gca gac tct gga 864
Gln Thr Phe His Thr Arg Ile Ile Arg Phe Cys Ser Ala Asp Ser Gly
        275                 280                 285

ttg cat tcg tac atg gaa atg cct ctg gag tgt att ctg acg gaa aag 912
Leu His Ser Tyr Met Glu Met Pro Leu Glu Cys Ile Leu Thr Glu Lys
    290                 295                 300

aga aga aag aga tcc aca aag cag gaa gtg ttt aat att ctc caa gct 960
Arg Arg Lys Arg Ser Thr Lys Gln Glu Val Phe Asn Ile Leu Gln Ala
305                 310                 315                 320

gca tat gtc agt aag cct ggg gct cag ctc gct agg caa ata ggt gcc 1008
Ala Tyr Val Ser Lys Pro Gly Ala Gln Leu Ala Arg Gln Ile Gly Ala
                325                 330                 335

agc ctg aat gat gac att ctg tac ggg gtg ttt gca caa agc aag cca 1056
Ser Leu Asn Asp Asp Ile Leu Tyr Gly Val Phe Ala Gln Ser Lys Pro
            340                 345                 350

gat tcc tca gaa cca atg aat cgt tct gct gtc tgt gca ttc cct gtc 1104

Asp Ser Ser Glu Pro Met Asn Arg Ser Ala Val Cys Ala Phe Pro Val
        355                 360                 365

aaa tat gtc aat gag ttc ttc aac aaa atc gtc aac aaa aac aat gtg 1152
Lys Tyr Val Asn Glu Phe Phe Asn Lys Ile Val Asn Lys Asn Asn Val
    370                 375                 380

aga tgt ctc cag cac ttt tat ggc ccc aac cat gaa cac tgc ttc aat 1200
Arg Cys Leu Gln His Phe Tyr Gly Pro Asn His Glu His Cys Phe Asn
385                 390                 395                 400

agg aca ctt ttg aga aat tca tca gga tgt gaa gtg cgc aat gat gaa 1248
Arg Thr Leu Leu Arg Asn Ser Ser Gly Cys Glu Val Arg Asn Asp Glu
                405                 410                 415

tat cga acg gag ttt acc aca gct ttg cca cgc gtt gac tta ttc acg 1296
Tyr Arg Thr Glu Phe Thr Thr Ala Leu Pro Arg Val Asp Leu Phe Thr
            420                 425                 430

ggc cag ttc aac caa gtc ctc tta aca tct ata tcc acc ttt atc aaa 1344
Gly Gln Phe Asn Gln Val Leu Leu Thr Ser Ile Ser Thr Phe Ile Lys
        435                 440                 445

gga gat ctc acc atc gct aat ctc ggg aca tca gaa ggt cga ttc atg 1392
Gly Asp Leu Thr Ile Ala Asn Leu Gly Thr Ser Glu Gly Arg Phe Met
    450                 455                 460

cag gtt gtg gtt tct cga tca gga tca tta act cct cat gtg aat ttt 1440
Gln Val Val Val Ser Arg Ser Gly Ser Leu Thr Pro His Val Asn Phe
465                 470                 475                 480

cac ctg gat tcc cac cct gtg tct cca gaa gtg att gtg gag cac ccg 1488
His Leu Asp Ser His Pro Val Ser Pro Glu Val Ile Val Glu His Pro
                485                 490                 495

cta aac cag aat ggc tat aca ctg gtt gtc act ggg aag aag atc acc 1536
Leu Asn Gln Asn Gly Tyr Thr Leu Val Val Thr Gly Lys Lys Ile Thr
            500                 505                 510

aag atc ccg ctg aat ggc ctg ggc tgt gaa cat ttc cag tcc tgc agt 1584
Lys Ile Pro Leu Asn Gly Leu Gly Cys Glu His Phe Gln Ser Cys Ser
        515                 520                 525

cag tgc ctc tca gcc cct tcc ttt gtg caa tgt ggc tgg tgc cat gat 1632
Gln Cys Leu Ser Ala Pro Ser Phe Val Gln Cys Gly Trp Cys His Asp
    530                 535                 540

aaa tgt gtg cga ttg gag gaa tgc tcc agt gga acg tgg act caa gag 1680
Lys Cys Val Arg Leu Glu Glu Cys Ser Ser Gly Thr Trp Thr Gln Glu

545                 550                 555                 560

acc tgt ctg cca aca atc tac aag gtt ttt cca act agt gcc ccc ctt      1728
Thr Cys Leu Pro Thr Ile Tyr Lys Val Phe Pro Thr Ser Ala Pro Leu
                565                 570                 575

gaa gga ggg aca aca ctg act gtt tgt ggc tgg gac ttt gga ttc aag      1776
Glu Gly Gly Thr Thr Leu Thr Val Cys Gly Trp Asp Phe Gly Phe Lys
            580                 585                 590

agg aat aat aaa ttt gat ttg aag aaa acc aga gtt ctc ctt ggc aat      1824
Arg Asn Asn Lys Phe Asp Leu Lys Lys Thr Arg Val Leu Leu Gly Asn
        595                 600                 605

gag agc tgc acc tta acc tta act gag agc aca aca aac atg ttg aaa      1872
Glu Ser Cys Thr Leu Thr Leu Thr Glu Ser Thr Thr Asn Met Leu Lys
    610                 615                 620

tgc aca gtt ggt cct gca atg aat gaa cat ttc aat atg tcc ata gtt      1920
Cys Thr Val Gly Pro Ala Met Asn Glu His Phe Asn Met Ser Ile Val
625                 630                 635                 640

att tca aac agc cgt ggg tca gta gag tat agt gcg ttt tcc tat gtg      1968
Ile Ser Asn Ser Arg Gly Ser Val Glu Tyr Ser Ala Phe Ser Tyr Val
                645                 650                 655

gat cct ata ata aca agt att tct cca aat tat ggt cca aaa act ggt      2016
Asp Pro Ile Ile Thr Ser Ile Ser Pro Asn Tyr Gly Pro Lys Thr Gly
            660                 665                 670

ggc act tta ctt aca tta act gga aag cac cta aac agt gga aat tca      2064
Gly Thr Leu Leu Thr Leu Thr Gly Lys His Leu Asn Ser Gly Asn Ser
        675                 680                 685

aga cac att tca att ggt gga aaa aca tgt act tta aaa agt gtt tca      2112
Arg His Ile Ser Ile Gly Gly Lys Thr Cys Thr Leu Lys Ser Val Ser
    690                 695                 700

cat agt att ctc gaa tgt tat acc cca gcc caa agt gct cca act gag      2160
His Ser Ile Leu Glu Cys Tyr Thr Pro Ala Gln Ser Ala Pro Thr Glu
705                 710                 715                 720

ttt tct gtt aag ttg aaa att gac tta gcc aac cga gag gtg aac agc      2208
Phe Ser Val Lys Leu Lys Ile Asp Leu Ala Asn Arg Glu Val Asn Ser
                725                 730                 735

ttc att tac cgg gaa gac ccc att gtc tat gaa ata cat ccc acc aaa      2256
Phe Ile Tyr Arg Glu Asp Pro Ile Val Tyr Glu Ile His Pro Thr Lys
            740                 745                 750

tct ttt att agt ggt ggg agt aca ata aca ggt gtt gga aaa aac ctg 2304
Ser Phe Ile Ser Gly Gly Ser Thr Ile Thr Gly Val Gly Lys Asn Leu
        755                 760                 765

aat tca gtt agt gtc cta agg atg gta ata aat gtg cat gaa gcc gga 2352
Asn Ser Val Ser Val Leu Arg Met Val Ile Asn Val His Glu Ala Gly
    770                 775                 780

agg aac ttt aca gtg gca tgt caa cat cgc tct aat tca gag ata atc 2400
Arg Asn Phe Thr Val Ala Cys Gln His Arg Ser Asn Ser Glu Ile Ile
785                 790                 795                 800

tgc tgt aca act cct tca ata gaa cag ctg aac cta caa ctc ccc ctg 2448
Cys Cys Thr Thr Pro Ser Ile Glu Gln Leu Asn Leu Gln Leu Pro Leu
                805                 810                 815

aaa acc aaa gcc ttt ttc atg tta gat ggg atc cat tcc aaa tac ttt 2496
Lys Thr Lys Ala Phe Phe Met Leu Asp Gly Ile His Ser Lys Tyr Phe
            820                 825                 830

gat ctc att tat gta cat aat cct gtg ttt aag cct ttt gaa aag cca 2544
Asp Leu Ile Tyr Val His Asn Pro Val Phe Lys Pro Phe Glu Lys Pro
        835                 840                 845

gtg atg atc tca gta ggc aat gaa aat gta ctg gaa att aag gga aat 2592
Val Met Ile Ser Val Gly Asn Glu Asn Val Leu Glu Ile Lys Gly Asn
    850                 855                 860

gat att gac cct gaa gca gtt aaa ggg gag gtg tta aaa gtt gga aat 2640
Asp Ile Asp Pro Glu Ala Val Lys Gly Glu Val Leu Lys Val Gly Asn
865                 870                 875                 880

aag agc tgt gaa aat ata cac tca cac tct gaa gct gtt tta tgt acg 2688
Lys Ser Cys Glu Asn Ile His Ser His Ser Glu Ala Val Leu Cys Thr
                885                 890                 895

gtc ccc agt gac ctg ctg aaa ttg aac agc gaa cta aat ata gag tgg 2736
Val Pro Ser Asp Leu Leu Lys Leu Asn Ser Glu Leu Asn Ile Glu Trp
            900                 905                 910

aag caa gca att tct tca act gtt ctt gga aaa gtg ata gtt caa cca 2784
Lys Gln Ala Ile Ser Ser Thr Val Leu Gly Lys Val Ile Val Gln Pro
        915                 920                 925

gat cag aat ttc aca gga ttg att gtt ggt gtt gtc tca ata tca ata 2832
Asp Gln Asn Phe Thr Gly Leu Ile Val Gly Val Val Ser Ile Ser Ile
    930                 935                 940

ata ctc tta ctg tta ctc ggg ctt ttc ctg tgg ctg aaa aaa agg aag 2880
Ile Leu Leu Leu Leu Leu Gly Leu Phe Leu Trp Leu Lys Lys Arg Lys
945                 950                 955                 960

caa att aaa gat ctg ggc agt gaa tta gtt cgc tat gat gca aga gta 2928
Gln Ile Lys Asp Leu Gly Ser Glu Leu Val Arg Tyr Asp Ala Arg Val
                965                 970                 975

cac act cca cat ttg gat agg ctt gtc agt gcc cga agt gta agc cca 2976
His Thr Pro His Leu Asp Arg Leu Val Ser Ala Arg Ser Val Ser Pro
            980                 985                 990

act aca gaa atg gtt tcc aat gaa tct gta gac tac cga gct act ttt 3024
Thr Thr Glu Met Val Ser Asn Glu Ser Val Asp Tyr Arg Ala Thr Phe
        995                 1000                1005

cca gaa gac cag ttt cct aat gca tct cag aat ggg tca tgc aga 3069
Pro Glu Asp Gln Phe Pro Asn Ala Ser Gln Asn Gly Ser Cys Arg
    1010                1015                1020

caa gtg cag tat cct ctg aca gat cta tcc ccc atc tta act agt 3114
Gln Val Gln Tyr Pro Leu Thr Asp Leu Ser Pro Ile Leu Thr Ser
    1025                1030                1035

ggg gac tct gat ata tcc agt cca tta ctg caa aat acc atc cac 3159
Gly Asp Ser Asp Ile Ser Ser Pro Leu Leu Gln Asn Thr Ile His
    1040                1045                1050

atc gac ctc agt gct cta aat cca gag ctg gtc cag gca gtt cag 3204
Ile Asp Leu Ser Ala Leu Asn Pro Glu Leu Val Gln Ala Val Gln
    1055                1060                1065

cac gta gta att ggg ccg agc agt ctg att gta cat ttc aat gaa 3249
His Val Val Ile Gly Pro Ser Ser Leu Ile Val His Phe Asn Glu
    1070                1075                1080

gtc ata gga aga gga cat ttt ggg tgt gta tat cat gga act ttg 3294
Val Ile Gly Arg Gly His Phe Gly Cys Val Tyr His Gly Thr Leu
    1085                1090                1095

ttg gac aac gat gat aag aaa att cac tgt gct gtg aaa tcc ttg 3339
Leu Asp Asn Asp Asp Lys Lys Ile His Cys Ala Val Lys Ser Leu
    1100                1105                1110

aat aga atc act gac ata gga gaa gtt tcc caa ttt ctg act gag 3384
Asn Arg Ile Thr Asp Ile Gly Glu Val Ser Gln Phe Leu Thr Glu
    1115                1120                1125

gga atc atc atg aaa gat ttt agt cat ccc aat gtt ctc tca ctt 3429

Gly Ile Ile Met Lys Asp Phe Ser His Pro Asn Val Leu Ser Leu
    1130                1135                1140

ttg gga atc tgc ctt cga agt gag ggg tct cca ctg gtg gtc cta 3474
Leu Gly Ile Cys Leu Arg Ser Glu Gly Ser Pro Leu Val Val Leu
    1145                1150                1155

cca tac atg aaa cac ggg gat ctt cga aat ttc att aga aat gag 3519
Pro Tyr Met Lys His Gly Asp Leu Arg Asn Phe Ile Arg Asn Glu
    1160                1165                1170

act cat aac cca act gta aaa gat ctt att ggc ttt ggt ctt caa 3564
Thr His Asn Pro Thr Val Lys Asp Leu Ile Gly Phe Gly Leu Gln
    1175                1180                1185

gta gcc aaa ggc atg gaa tat ctt gca agc aaa aag ttt gtc cac 3609
Val Ala Lys Gly Met Glu Tyr Leu Ala Ser Lys Lys Phe Val His
    1190                1195                1200

aga gac ttg gct gca aga aac tgt atg ctg gat gaa aaa ttc acc 3654
Arg Asp Leu Ala Ala Arg Asn Cys Met Leu Asp Glu Lys Phe Thr
    1205                1210                1215

gtc aag gtt gct gat ttt ggt ctt gcc aga gac gtg tat gat aag 3699
Val Lys Val Ala Asp Phe Gly Leu Ala Arg Asp Val Tyr Asp Lys
    1220                1225                1230

gaa tac tat agc gta cac aac aaa aca ggt gca aaa ctg cca gtg 3744
Glu Tyr Tyr Ser Val His Asn Lys Thr Gly Ala Lys Leu Pro Val
    1235                1240                1245

aaa tgg atg gct tta gaa agc ctg cag act cag aaa ttt acc acc 3789
Lys Trp Met Ala Leu Glu Ser Leu Gln Thr Gln Lys Phe Thr Thr
    1250                1255                1260

aag tca gat gtg tgg tcc ttt ggc gtg ctt ctc tgg gaa ctg atg 3834
Lys Ser Asp Val Trp Ser Phe Gly Val Leu Leu Trp Glu Leu Met
    1265                1270                1275

aca aga gga gca cca cct tat cct gac gtc aac acc ttt gac ata 3879
Thr Arg Gly Ala Pro Pro Tyr Pro Asp Val Asn Thr Phe Asp Ile
    1280                1285                1290

aca gtt tac ttg ttg caa gga aga agg ctc cta caa cct gaa tac 3924
Thr Val Tyr Leu Leu Gln Gly Arg Arg Leu Leu Gln Pro Glu Tyr
    1295                1300                1305

tgc cca gat ccc ttg tat gaa gtg atg ctg aaa tgc tgg cac cct 3969
Cys Pro Asp Pro Leu Tyr Glu Val Met Leu Lys Cys Trp His Pro

1310                1315                1320

aaa gct gaa ctg cgc cca tcc ttt tct gaa ctg gtc tcc agg ata      4014
Lys Ala Glu Leu Arg Pro Ser Phe Ser Glu Leu Val Ser Arg Ile
    1325                1330                1335

tcc gtg ata ttc tct act ttc att ggg gag cac tat gtc cat gtg      4059
Ser Val Ile Phe Ser Thr Phe Ile Gly Glu His Tyr Val His Val
    1340                1345                1350

aat gct act tac gtg aac gtc aaa tgt gtc gct cca tat cct tct      4104
Asn Ala Thr Tyr Val Asn Val Lys Cys Val Ala Pro Tyr Pro Ser
    1355                1360                1365

ctg ttg tca tca caa gat aat gtc agt ggc gag gat gat gat gac      4149
Leu Leu Ser Ser Gln Asp Asn Val Ser Gly Glu Asp Asp Asp Asp
    1370                1375                1380

aca tga                                                          4155
Thr

<210>  28
<211>  1384
<212>  PRT
<213>  Bos taurus

<400>  28

Met Lys Ala Pro Ala Val Leu Ala Pro Gly Ile Leu Val Leu Leu Phe
1               5                   10                  15

Thr Phe Val Gln Lys Ser Asn Gly Glu Cys Lys Glu Ala Leu Val Lys
            20                  25                  30

Ser Arg Met Asn Val Asn Met Gln Tyr Gln Leu Pro Asn Phe Thr Ala
        35                  40                  45

Glu Thr Ser Ile Gln Asn Val Val Leu His Lys His His Ile Tyr Leu
    50                  55                  60

Gly Ala Ile Asn Tyr Ile Tyr Val Leu Asn Asp Lys Asp Leu Gln Lys
65                  70                  75                  80

Val Ala Glu Tyr Lys Thr Gly Pro Val Leu Glu His Pro Asp Cys Phe
                85                  90                  95

Pro Cys Gln Asp Cys Ser His Lys Ala Asn Leu Ser Gly Gly Val Trp
            100                 105                 110

Lys Asp Asn Ile Asn Met Ala Leu Leu Val Asp Thr Tyr Tyr Asp Asp
        115                 120                 125

Gln Leu Ile Ser Cys Gly Ser Val His Arg Gly Thr Cys Gln Arg His
    130                 135                 140

Val Leu Pro Pro Asn Asn Thr Ala Asp Ile Glu Ser Glu Val His Cys
145                 150                 155                 160

Met Tyr Ser Pro Gln Ala Asp Glu Glu Thr Asn Gln Cys Pro Asp Cys
                165                 170                 175

Val Val Ser Ala Leu Gly Thr Lys Val Leu Leu Ser Glu Lys Asp Arg
            180                 185                 190

Phe Ile Asn Phe Phe Val Gly Asn Thr Ile Asn Ser Ser Tyr Leu Pro
        195                 200                 205

Asp Tyr Ile Leu His Ser Ile Ser Val Arg Arg Leu Lys Glu Thr Gln
    210                 215                 220

Asp Gly Phe Lys Phe Leu Thr Asp Gln Ser Tyr Ile Asp Val Leu Pro
225                 230                 235                 240

Glu Leu Arg Asp Ser Tyr Pro Ile Lys Tyr Val His Ala Phe Glu Ser
                245                 250                 255

Asn His Phe Ile Tyr Phe Leu Thr Val Gln Arg Glu Thr Leu Asp Ala
            260                 265                 270

Gln Thr Phe His Thr Arg Ile Ile Arg Phe Cys Ser Ala Asp Ser Gly
        275                 280                 285

Leu His Ser Tyr Met Glu Met Pro Leu Glu Cys Ile Leu Thr Glu Lys
    290                 295                 300

Arg Arg Lys Arg Ser Thr Lys Gln Glu Val Phe Asn Ile Leu Gln Ala
305                 310                 315                 320

Ala Tyr Val Ser Lys Pro Gly Ala Gln Leu Ala Arg Gln Ile Gly Ala
                325                 330                 335

Ser Leu Asn Asp Asp Ile Leu Tyr Gly Val Phe Ala Gln Ser Lys Pro
            340                 345                 350

Asp Ser Ser Glu Pro Met Asn Arg Ser Ala Val Cys Ala Phe Pro Val
        355                 360                 365

Lys Tyr Val Asn Glu Phe Phe Asn Lys Ile Val Asn Lys Asn Asn Val
    370                 375                 380

Arg Cys Leu Gln His Phe Tyr Gly Pro Asn His Glu His Cys Phe Asn
385                 390                 395                 400

Arg Thr Leu Leu Arg Asn Ser Ser Gly Cys Glu Val Arg Asn Asp Glu
                405                 410                 415

Tyr Arg Thr Glu Phe Thr Thr Ala Leu Pro Arg Val Asp Leu Phe Thr
            420                 425                 430

Gly Gln Phe Asn Gln Val Leu Leu Thr Ser Ile Ser Thr Phe Ile Lys
        435                 440                 445

Gly Asp Leu Thr Ile Ala Asn Leu Gly Thr Ser Glu Gly Arg Phe Met
    450                 455                 460

Gln Val Val Val Ser Arg Ser Gly Ser Leu Thr Pro His Val Asn Phe

465                 470                 475                 480

His Leu Asp Ser His Pro Val Ser Pro Glu Val Ile Val Glu His Pro
                485                 490                 495

Leu Asn Gln Asn Gly Tyr Thr Leu Val Val Thr Gly Lys Lys Ile Thr
            500                 505                 510

Lys Ile Pro Leu Asn Gly Leu Gly Cys Glu His Phe Gln Ser Cys Ser
        515                 520                 525

Gln Cys Leu Ser Ala Pro Ser Phe Val Gln Cys Gly Trp Cys His Asp
    530                 535                 540

Lys Cys Val Arg Leu Glu Glu Cys Ser Ser Gly Thr Trp Thr Gln Glu
545                 550                 555                 560

Thr Cys Leu Pro Thr Ile Tyr Lys Val Phe Pro Thr Ser Ala Pro Leu
                565                 570                 575

Glu Gly Gly Thr Thr Leu Thr Val Cys Gly Trp Asp Phe Gly Phe Lys
            580                 585                 590

Arg Asn Asn Lys Phe Asp Leu Lys Lys Thr Arg Val Leu Leu Gly Asn
        595                 600                 605

Glu Ser Cys Thr Leu Thr Leu Thr Glu Ser Thr Thr Asn Met Leu Lys
    610                 615                 620

Cys Thr Val Gly Pro Ala Met Asn Glu His Phe Asn Met Ser Ile Val
625                 630                 635                 640

Ile Ser Asn Ser Arg Gly Ser Val Glu Tyr Ser Ala Phe Ser Tyr Val
                645                 650                 655

Asp Pro Ile Ile Thr Ser Ile Ser Pro Asn Tyr Gly Pro Lys Thr Gly
            660                 665                 670

Gly Thr Leu Leu Thr Leu Thr Gly Lys His Leu Asn Ser Gly Asn Ser
        675                 680                 685

Arg His Ile Ser Ile Gly Gly Lys Thr Cys Thr Leu Lys Ser Val Ser
    690                 695                 700

His Ser Ile Leu Glu Cys Tyr Thr Pro Ala Gln Ser Ala Pro Thr Glu
705                 710                 715                 720

Phe Ser Val Lys Leu Lys Ile Asp Leu Ala Asn Arg Glu Val Asn Ser
                725                 730                 735

Phe Ile Tyr Arg Glu Asp Pro Ile Val Tyr Glu Ile His Pro Thr Lys
            740                 745                 750

Ser Phe Ile Ser Gly Gly Ser Thr Ile Thr Gly Val Gly Lys Asn Leu
        755                 760                 765

Asn Ser Val Ser Val Leu Arg Met Val Ile Asn Val His Glu Ala Gly
    770                 775                 780

Arg Asn Phe Thr Val Ala Cys Gln His Arg Ser Asn Ser Glu Ile Ile
785                 790                 795                 800

Cys Cys Thr Thr Pro Ser Ile Glu Gln Leu Asn Leu Gln Leu Pro Leu
                805                 810                 815

Lys Thr Lys Ala Phe Phe Met Leu Asp Gly Ile His Ser Lys Tyr Phe
            820                 825                 830

Asp Leu Ile Tyr Val His Asn Pro Val Phe Lys Pro Phe Glu Lys Pro
        835                 840                 845

Val Met Ile Ser Val Gly Asn Glu Asn Val Leu Glu Ile Lys Gly Asn
    850                 855                 860

Asp Ile Asp Pro Glu Ala Val Lys Gly Glu Val Leu Lys Val Gly Asn
865                 870                 875                 880

Lys Ser Cys Glu Asn Ile His Ser His Ser Glu Ala Val Leu Cys Thr
                885                 890                 895

Val Pro Ser Asp Leu Leu Lys Leu Asn Ser Glu Leu Asn Ile Glu Trp
            900                 905                 910

Lys Gln Ala Ile Ser Ser Thr Val Leu Gly Lys Val Ile Val Gln Pro
        915                 920                 925

Asp Gln Asn Phe Thr Gly Leu Ile Val Gly Val Val Ser Ile Ser Ile
    930                 935                 940

Ile Leu Leu Leu Leu Leu Gly Leu Phe Leu Trp Leu Lys Lys Arg Lys
945                 950                 955                 960

Gln Ile Lys Asp Leu Gly Ser Glu Leu Val Arg Tyr Asp Ala Arg Val
                965                 970                 975

His Thr Pro His Leu Asp Arg Leu Val Ser Ala Arg Ser Val Ser Pro
            980                 985                 990

Thr Thr Glu Met Val Ser Asn Glu  Ser Val Asp Tyr Arg  Ala Thr Phe
        995                 1000                1005

Pro Glu  Asp Gln Phe Pro Asn  Ala Ser Gln Asn Gly  Ser Cys Arg
    1010                1015                1020

Gln Val  Gln Tyr Pro Leu Thr  Asp Leu Ser Pro Ile  Leu Thr Ser
    1025                1030                1035

Gly Asp  Ser Asp Ile Ser Ser  Pro Leu Leu Gln Asn  Thr Ile His
    1040                1045                1050

Ile Asp Leu Ser Ala Leu Asn Pro Glu Leu Val Gln Ala Val Gln
    1055                1060                1065

His Val Val Ile Gly Pro Ser Ser Leu Ile Val His Phe Asn Glu
    1070                1075                1080

Val Ile Gly Arg Gly His Phe Gly Cys Val Tyr His Gly Thr Leu
    1085                1090                1095

Leu Asp Asn Asp Asp Lys Lys Ile His Cys Ala Val Lys Ser Leu
    1100                1105                1110

Asn Arg Ile Thr Asp Ile Gly Glu Val Ser Gln Phe Leu Thr Glu
    1115                1120                1125

Gly Ile Ile Met Lys Asp Phe Ser His Pro Asn Val Leu Ser Leu
    1130                1135                1140

Leu Gly Ile Cys Leu Arg Ser Glu Gly Ser Pro Leu Val Val Leu
    1145                1150                1155

Pro Tyr Met Lys His Gly Asp Leu Arg Asn Phe Ile Arg Asn Glu
    1160                1165                1170

Thr His Asn Pro Thr Val Lys Asp Leu Ile Gly Phe Gly Leu Gln
    1175                1180                1185

Val Ala Lys Gly Met Glu Tyr Leu Ala Ser Lys Lys Phe Val His
    1190                1195                1200

Arg Asp Leu Ala Ala Arg Asn Cys Met Leu Asp Glu Lys Phe Thr
    1205                1210                1215

Val Lys Val Ala Asp Phe Gly Leu Ala Arg Asp Val Tyr Asp Lys
    1220                1225                1230

Glu Tyr Tyr Ser Val His Asn Lys Thr Gly Ala Lys Leu Pro Val

1235                    1240                    1245

Lys Trp Met Ala Leu Glu Ser Leu Gln Thr Gln Lys Phe Thr Thr
    1250                    1255                    1260

Lys Ser Asp Val Trp Ser Phe Gly Val Leu Leu Trp Glu Leu Met
    1265                    1270                    1275

Thr Arg Gly Ala Pro Pro Tyr Pro Asp Val Asn Thr Phe Asp Ile
    1280                    1285                    1290

Thr Val Tyr Leu Leu Gln Gly Arg Arg Leu Leu Gln Pro Glu Tyr
    1295                    1300                    1305

Cys Pro Asp Pro Leu Tyr Glu Val Met Leu Lys Cys Trp His Pro
    1310                    1315                    1320

Lys Ala Glu Leu Arg Pro Ser Phe Ser Glu Leu Val Ser Arg Ile
    1325                    1330                    1335

Ser Val Ile Phe Ser Thr Phe Ile Gly Glu His Tyr Val His Val
    1340                    1345                    1350

Asn Ala Thr Tyr Val Asn Val Lys Cys Val Ala Pro Tyr Pro Ser
    1355                    1360                    1365

Leu Leu Ser Ser Gln Asp Asn Val Ser Gly Glu Asp Asp Asp Asp
    1370                    1375                    1380

Thr

<210>  29
<211>  38
<212>  DNA
<213>  Artificial

<220>

<223>  synthetic DNA

<400>  29
gaggtcgacg ccaccatgaa ggctcccacc gcgctggc                                38

<210>  30
<211>  24
<212>  DNA
<213>  Artificial

<220>
<223>  synthetic DNA

<400>  30
taaagtacat gttttccctc cgat                                              24

<210>  31
<211>  23
<212>  DNA
<213>  Artificial

<220>
<223>  synthetic DNA

<400>  31
aaatacctca acagcggcaa ttc                                               23

<210>  32
<211>  35
<212>  DNA
<213>  Artificial

<220>
<223>  synthetic DNA

<400>  32
gaggcggccg ctgtgttcgc ttcgccgtca atgtt                                  35